(19) Europäisches Patentamt
European Patent Office
Office européen des brevets

(11) **EP 3 157 549 B1**

(12) **EUROPEAN PATENT SPECIFICATION**

(45) Date of publication and mention
of the grant of the patent:
**29.04.2020 Bulletin 2020/18**

(21) Application number: **15730135.9**

(22) Date of filing: **17.06.2015**

(51) Int Cl.:
*A61K 39/00* (2006.01)     *A61K 47/50* (2017.01)
*C07K 16/00* (2006.01)     *C07K 7/06* (2006.01)
*A61P 35/00* (2006.01)     *A61K 48/00* (2006.01)
*C12N 5/0783* (2010.01)     *A61K 38/00* (2006.01)
*A61K 35/17* (2015.01)

(86) International application number:
**PCT/EP2015/063566**

(87) International publication number:
**WO 2015/193359 (23.12.2015 Gazette 2015/51)**

(54) **NOVEL IMMUNOTHERAPY AGAINST SEVERAL TUMORS OF THE BLOOD, IN PARTICULAR CHRONIC LYMPHOID LEUKEMIA (CLL)**

NEUARTIGE IMMUNTHERAPIE GEGEN VERSCHIEDENE TUMOREN DES BLUTES, INSBESONDERE CHRONISCHER LYMPHOIDER LEUKÄMIE (CLL)

NOUVELLE IMMUNOTHÉRAPIE DIRIGÉE CONTRE PLUSIEURS TUMEURS DU SANG, EN PARTICULIER LA LEUCÉMIE LYMPHOÏDE CHRONIQUE (LLC)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.06.2014 US 201462014849 P
20.06.2014 GB 201411037**

(43) Date of publication of application:
**26.04.2017 Bulletin 2017/17**

(73) Proprietor: **immatics biotechnologies GmbH
72076 Tübingen (DE)**

(72) Inventors:
• **STICKEL, Juliane
72076 Tübingen (DE)**
• **KOWALEWSKI, Daniel
72074 Tübingen (DE)**
• **RAMMENSEE, Hans-Georg
72070 Tübingen (DE)**
• **STEVANOVIC, Stefan
72074 Tübingen (DE)**

(74) Representative: **Michalski Hüttermann & Partner Patentanwälte mbB
Speditionstraße 21
40221 Düsseldorf (DE)**

(56) References cited:
**WO-A2-2005/114221    WO-A2-2005/114221
WO-A2-2005/114221    WO-A2-2007/150077
WO-A2-2007/150077    WO-A2-2007/150077
WO-A2-2008/070047    WO-A2-2008/070047
WO-A2-2008/070047**

• **C BERLIN ET AL: "Mapping the HLA ligandome landscape of acute myeloid leukemia: a targeted approach toward peptide-based immunotherapy", LEUKEMIA, vol. 29, no. 3, 5 August 2014 (2014-08-05), pages 647-659, XP055207425, ISSN: 0887-6924, DOI: 10.1038/leu.2014.233**
• **ETIENNE CARON ET AL: "An open-source computational and data resource to analyze digital maps of immunopeptidomes", ELIFE, vol. 4, 8 July 2015 (2015-07-08), XP055207421, DOI: 10.7554/eLife.07661**

EP 3 157 549 B1

- DANIEL J. KOWALEWSKI ET AL: "HLA ligandome analysis identifies the underlying specificities of spontaneous antileukemia immune responses in chronic lymphocytic leukemia (CLL)", PROCEEDINGS OF THE NATIONAL ACADEMY OF SCIENCES, vol. 112, no. 2, 13 January 2015 (2015-01-13), pages E166-E175, XP055207429, ISSN: 0027-8424, DOI: 10.1073/pnas.1416389112
- KOWALEWSKI DANIEL JOHANNES; SCHUSTER HEIKO; BERLIN CLAUDIA; KANZ LOTHAR; SALIH HELMUT R; RAMMENSEE HANS-GEORG; STEVANOVIC STEFAN;: "Identification of Novel Tumor-Associated Antigens for Chronic Lymphocytic Leukemia (CLL) Based On HLA Ligandome Analysis - New Targets for Peptide Based Immunotherapy", BLOOD, vol. 120, no. 21, suppl., 1 December 2012 (2012-12-01), page 4119, XP055207460, 56TH ANNUAL MEETING OF THE AMERICAN-SOCIETY-OF-HEMATOLOGY; SAN FRANCISCO, CA, USA; DECEMBER 06 -09, 2014 ISSN: 0006-4971
- C BERLIN ET AL: "Mapping the HLA ligandome landscape of acute myeloid leukemia: a targeted approach toward peptide-based immunotherapy", LEUKEMIA, vol. 29, no. 3, 5 August 2014 (2014-08-05) , pages 647-659, XP055207425, ISSN: 0887-6924, DOI: 10.1038/leu.2014.233
- ETIENNE CARON ET AL: "An open-source computational and data resource to analyze digital maps of immunopeptidomes", ELIFE, vol. 4, 8 July 2015 (2015-07-08), XP055207421, DOI: 10.7554/eLife.07661
- DANIEL J. KOWALEWSKI ET AL: "HLA ligandome analysis identifies the underlying specificities of spontaneous antileukemia immune responses in chronic lymphocytic leukemia (CLL)", PROCEEDINGS OF THE NATIONAL ACADEMY OF SCIENCES, vol. 112, no. 2, 13 January 2015 (2015-01-13), pages E166-E175, XP055207429, ISSN: 0027-8424, DOI: 10.1073/pnas.1416389112
- KOWALEWSKI DANIEL JOHANNES; SCHUSTER HEIKO; BERLIN CLAUDIA; KANZ LOTHAR; SALIH HELMUT R; RAMMENSEE HANS-GEORG; STEVANOVIC STEFAN;: "Identification of Novel Tumor-Associated Antigens for Chronic Lymphocytic Leukemia (CLL) Based On HLA Ligandome Analysis - New Targets for Peptide Based Immunotherapy", BLOOD, vol. 120, no. 21, suppl., 1 December 2012 (2012-12-01), page 4119, XP055207460, 56TH ANNUAL MEETING OF THE AMERICAN-SOCIETY-OF-HEMATOLOGY; SAN FRANCISCO, CA, USA; DECEMBER 06 -09, 2014 ISSN: 0006-4971
- C BERLIN ET AL: "Mapping the HLA ligandome landscape of acute myeloid leukemia: a targeted approach toward peptide-based immunotherapy", LEUKEMIA, vol. 29, no. 3, 5 August 2014 (2014-08-05) , pages 647-659, XP055207425, ISSN: 0887-6924, DOI: 10.1038/leu.2014.233
- ETIENNE CARON ET AL: "An open-source computational and data resource to analyze digital maps of immunopeptidomes", ELIFE, vol. 4, 8 July 2015 (2015-07-08), XP055207421, DOI: 10.7554/eLife.07661
- DANIEL J. KOWALEWSKI ET AL: "HLA ligandome analysis identifies the underlying specificities of spontaneous antileukemia immune responses in chronic lymphocytic leukemia (CLL)", PROCEEDINGS OF THE NATIONAL ACADEMY OF SCIENCES, vol. 112, no. 2, 13 January 2015 (2015-01-13), pages E166-E175, XP055207429, ISSN: 0027-8424, DOI: 10.1073/pnas.1416389112
- KOWALEWSKI DANIEL JOHANNES; SCHUSTER HEIKO; BERLIN CLAUDIA; KANZ LOTHAR; SALIH HELMUT R; RAMMENSEE HANS-GEORG; STEVANOVIC STEFAN;: "Identification of Novel Tumor-Associated Antigens for Chronic Lymphocytic Leukemia (CLL) Based On HLA Ligandome Analysis - New Targets for Peptide Based Immunotherapy", BLOOD, vol. 120, no. 21, suppl., 1 December 2012 (2012-12-01), page 4119, XP055207460, US ISSN: 0006-4971

**Description**

The present invention relates to peptides, nucleic acids and cells for use in immunotherapeutic methods. In particular, the present invention relates to the immunotherapy of cancer. The present invention furthermore relates to tumor-associated cytotoxic T cell (CTL) peptide epitopes, alone or in combination with other tumor-associated peptides that serve as active pharmaceutical ingredients of vaccine compositions that stimulate anti-tumor immune responses. The present invention relates to several peptide sequences derived from HLA class I molecules of human tumor cells that can be used in vaccine compositions for eliciting anti-tumor immune responses.

**Background of the invention**

B-cell chronic lymphocytic leukemia (B-CLL), also known as chronic lymphoid leukemia (CLL), is the most common type of leukemia.

Leukemias are cancers of the white blood cells (leukocytes). CLL affects B cell lymphocytes. B cells originate in the bone marrow, develop in the lymph nodes, and normally fight infection by producing antibodies. In CLL, B cells grow out of control and accumulate in the bone marrow and blood, where they crowd out healthy blood cells. CLL is a stage of small lymphocytic lymphoma (SLL), a type of B-cell lymphoma, which presents primarily in the lymph nodes. CLL and SLL are considered the same underlying disease, just with different appearances.

CLL is a disease of adults, but, in rare cases, it can occur in teenagers and occasionally in children (inherited). Most (>75%) people newly diagnosed with CLL are over the age of 50, and the majority are men, with a median age of 70 years at the time of diagnosis. Though less common, CLL sometimes affects people between 30 and 39 years of age. The incidence of CLL increases very quickly with increasing age.

In the United States, during 2012 about 16,060 new cases are expected to be diagnosed, and 4,580 patients are expected to die from CLL.

CLL is very rare in Asian countries, such as Japan and China, and may account for as few as 10 percent of all leukemias in those regions.

In view of the above, there remains a need for new efficacious and safe treatment option for cancers, in particular chronic lymphoid leukemia (CLL) and other cancers of the blood of different phenotypes which improve the well-being of the patients by not using excessive chemotherapeutic agents or other agents that may lead to severe side effects.

The present invention employs peptides that stimulate the immune system of the patient and act as anti-tumor-agents in a non-invasive fashion.

**Summary of the invention**

In a first aspect of the present invention, the present invention relates to a peptide of between 10 and 30 amino acids in length, comprising an amino acid sequence consisting of SEQ ID NO: 167, or a pharmaceutically acceptable salt thereof

The following tables show the peptides according to the present invention, its respective SEQ ID NO:, and the prospective source (underlying) proteins for these peptides. All peptides in Table 1a and 1b bind to HLA A HLA B or HLA C alleles, peptides in Table 2 bind to HLA-DR alleles (MHC class II). The peptides in table 3 are further useful in the diagnosis and/or treatment of CLL, Acute myelogenous leukemia (AML), and other hematological malignancies, which involve an over-expression or over-presentaion of the respective underlying polypeptide.

**Table 1a:** Preferred 49 HLA class I ligandome derived tumor associated antigens (LiTAAs) found represented in ≥20% of CLL patient ligandomes (n=30) and the 225 representing HLA ligands (LiTAPs) annotated with respective HLA restriction. SEQ ID NO: 167 is according to the invention.

| SEQ ID NO: | Underlying source protein/ HLA ligands | Number of positive CLLs (frequency [%]) | HLA |
|---|---|---|---|
| | **APOBEC3D apolipoprotein B mRNA editing enzyme, catalytic polypeptide-like 3D** | **13 (43.3)** | |
| 1 | AEHPNVTLTI | 1 | B*40 |
| 2 | FLAEHPNVTL | 8 | A*02 |
| 3 | ILYGRSYTW | 1 | A*32 |
| 4 | EVAEFLARH | 2 | A*26 |
| 5 | RHSNVNLTI | 1 | C*07 |
| | **CDK14 cyclin-dependent kinase 14** | **12 (40.0)** | |

(continued)

| SEQ ID NO: | Underlying source protein/ HLA ligands | Number of positive CLLs (frequency [%]) | HLA |
|---|---|---|---|
| 6 | HPDNVKLFL | 1 | B*35 |
| 7 | ISDTGELKL | 1 | C*05 |
| 8 | KVNGKLVALK | 1 | A*03 |
| 9 | NRLSAQAAL | 1 | B*39 |
| 10 | TPFTAIREA | 1 | B*55 |
| 11 | FGLARAKSV | 6 | B*08 |
| 12 | KIADFGLAR | 1 | A*03 |
| | **RASGRF1 Ras protein-specific guanine nucleotide-releasing factor 1** | **12 (40.0)** | B*35 |
| 13 | AAANIIRTL | 8 | A*02, B*13, B*51 |
| 14 | GRFKNLREAL | 1 | B*27 |
| 15 | MSPFSKATL | 2 | C*14 |
| 16 | QEDPGDNQITL | 1 | B*40 |
| 17 | SPFSKATL | 2 | B*08, B*07 |
| | **CDCA7L cell division cycle associated 7-like** | **11 (36.7)** | |
| 18 | DALLKRTM | 1 | B*08 |
| 19 | GEDVRSALL | 3 | B*40 |
| 20 | KFAEEFYSF | 2 | A*24 |
| 21 | YGYDNVKEY | 7 | C*03, C*12 |
| | **CELSR1 cadherin, EGF LAG seven-pass G-type receptor 1** | **11 (36.7)** | |
| 22 | LEVEERTKPV | 1 | B*44 |
| 23 | RDSPINANLRY | 1 | B*40 |
| 24 | RPFVIVTA | 1 | B*55 |
| 25 | RPIINTPMV | 1 | B*55 |
| 26 | SPTSSRTSSL | 7 | B*07 |
| 27 | ATSAPLVSR | 1 | A*11 |
| | **AKAP2 A kinase (PRKA) anchor protein 2** | **11 (36.7)** | |
| 28 | AELRSTASLL | 1 | B*40 |
| 29 | APASSHERASM | 2 | B*07 |
| 30 | ASRQAPPHI | 1 | A*30 |
| 31 | AVKKNPGIAA | 2 | A*02 |
| 32 | EEHLESHKKY | 2 | B*44 |
| 33 | GEFTSARAV | 1 | B*49 |
| 34 | GQSTPRLFSI | 1 | B*13 |
| 35 | LVDDPLEY | 1 | A*01 |
| 36 | RPKNLMQTL | 3 | B*07 |
| 37 | RQAPPHIEL | 2 | B*13 |
| 38 | SEAAELRSTA | 1 | B*50 |
| | **CTDP1 CTD phosphatase, subunit 1** | **11 (36.7)** | |
| 39 | AAVRIGSVL | 2 | A*02, B*13 |
| 40 | ERAGVVREL | 1 | C*07 |
| 41 | GAAVRIGSVL | 1 | A*02 |
| 42 | KLYELHVFTF | 1 | A*32 |
| 43 | LYELHVFTF | 2 | A*24, A*23 |
| 44 | YLNKEIEEA | 6 | A*02 |
| | **DNMBP dynamin binding protein** | **10 [33.3]** | |
| 45 | DELPKFHQY | 2 | B*18 |

(continued)

| SEQ ID NO: | Underlying source protein/ HLA ligands | Number of positive CLLs (frequency [%]) | HLA |
|---|---|---|---|
| 46 | DVTGQFPSSF | 1 | A*26 |
| 47 | EHSRVLQQL | 2 | B*39:01 |
| 48 | IKVSKQLL | 1 | B*08 |
| 49 | KPRQSSPQL | 3 | B*07 |
| 50 | KQLLAALEI | 1 | B*13 |
| 51 | RRKDLVLKY | 2 | B*27 |
| 52 | RTRDYASLPPK | 1 | A*03 |
| | **TAGAP T-cell activation RhoGTPase activating protein** | **10 (33.3)** | |
| 53 | APGSVLPRAL | 3 | B*07 |
| 54 | DIKEHPLL | 1 | B*08 |
| 55 | DSAGPQDAR | 1 | A*68 |
| 56 | FQYAKESYI | 1 | B*13 |
| 57 | KVLSWPFLM | 1 | A*32 |
| 58 | LENDQSLSF | 1 | B*44 |
| 59 | SPSRQPQV | 1 | B*07 |
| 60 | SRHQSFTTK | 3 | B*27 |
| 61 | SSHNASKTL | 2 | C*12 |
| | **ABCA6 ATP-binding cassette, sub-family A (ABC1), member 6** | **10 (33.3)** | |
| 62 | EEIDTTMRW | 1 | B*44 |
| 63 | ILDEKPVII | 5 | A*02 |
| 64 | LPQEPRTSL | 2 | B*07 |
| 65 | LTYKLPVA | 1 | B*57 |
| 66 | NEMELAHSSF | 2 | B*18 |
| 67 | REFPEANFEL | 1 | B*40 |
| 68 | THHIPDAKL | 1 | B*38 |
| 69 | TVKENLSLF | 1 | A*26 |
| 70 | VLLKKAVL | 1 | B*08 |
| | **DMXL1 Dmx-like 1** | **10 (33.3)** | |
| 71 | HLKSIPVSL | 2 | B*08, B*07 |
| 72 | KVWYNVENW | 1 | A*32 |
| 73 | LPAYRAQLL | 1 | B*07 |
| 74 | LSEQTSVPL | 1 | A*02 |
| 75 | SLNQWLVSF | 1 | A*32 |
| 76 | SMTSLAQKI | 1 | A*02 |
| 77 | SSSGLHPPK | 2 | A*03, A*11, A*68 |
| | **PARP3 poly (ADP-ribose) polymerase family, member 3** | **10 (33.3)** | |
| 78 | DLDVKKMPL | 4 | B*08 |
| 79 | FYTVIPHNF | 3 | A*24 |
| 80 | HHINTDNPSL | 2 | B*39 |
| 81 | RVGEVGQSK | 2 | A*03 |
| | **TP53I11 tumor protein p53 inducible protein 11** | **8 (26.7)** | |
| 82 | AVFDGAQVTSK | 7 | A*03, A*11 |
| 83 | SQTDL VSRL | 1 | B*15 |
| | **B4GALT1 UDP-Gal:betaGlcNAc beta 1,4-galactosyltransferase, polypeptide 1** | **8 (26.7)** | |

(continued)

| SEQ ID NO: | Underlying source protein/ HLA ligands | Number of positive CLLs (frequency [%]) | HLA |
|---|---|---|---|
| 84 | VPVPHTTAL | 7 | B*07 |
| 85 | YQVLDVQRY | 1 | B*15 |
| | **IRF9 interferon regulatory factor 9** | **8 (26.7)** | |
| 86 | APFQGDQRSL | 2 | B*07 |
| 87 | DVAEPYKVY | 1 | A*26 |
| 88 | IVSGQPGTQK | 3 | A*03 |
| 89 | TPEQQAAIL | 1 | B*35 |
| 90 | VELFRTAYF | 1 | B*37 |
| | **KDM2B lysine (K)-specific demethylase 2B** | **8 (26.7)** | |
| 91 | EHADDDPSL | 1 | B*38 |
| 92 | SEESVKSTTL | 2 | B*40 |
| 93 | SPRPPLGSSL | 4 | B*07 |
| 94 | SPWWRSSL | 1 | B*07 |
| 95 | VYTPVDSLVF | 1 | A*24 |
| | **TBC1D22A TBC1 domain family, member 22A** | **8 (26.7)** | |
| 96 | APLQRSQSL | 6 | B*07, B*08 |
| 97 | DEVHQDTY | 1 | B*18 |
| 98 | LPHSATVTL | 1 | B*07 |
| | **ZNF296 zinc finger protein 296** | **8 (26.7)** | |
| 99 | SEAPEAPLL | 1 | B*40 |
| 100 | SPRASGSGL | 6 | B*07 |
| 101 | WGPAAEAK | 2 | A*03 |
| | **BACH2 BTB and CNC homology 1, basic leucine zipper transcription factor** 2 | 8 (26.7) | |
| 102 | FSITKSVEL | 4 | A*02 |
| 103 | GQTKNDLW | 1 | B*13 |
| 104 | LSQEVCRD | 2 | n.a. |
| 105 | RDIQSPEQI | 1 | B*40 |
| 106 | REDNSSNSL | 1 | B*40 |
| 107 | TEHQEPGL | 2 | B*40 |
| 108 | TKNDLVVSL | 1 | B*39 |
| | **PRR12 proline rich 12** | **8 (26.7)** | |
| 109 | AEEAGGTRL | 1 | B*40 |
| 110 | ENVNKKDY | 1 | A*26 |
| 111 | GLDPNKPPEL | 4 | A*02 |
| 112 | RPAGEPYNRKTL | 2 | B*07 |
| | **ZFAND5 zinc finger, AN1-type domain 5** | **7 (23.3)** | |
| 113 | SASVQRADTSL | 5 | C*03 |
| 114 | YGNPRTNGM | 2 | B*08 |
| | **ATP5G1 ATP synthase, H+ transporting, mitochondrial Fo complex, subunit C1** | **7 (23.3)** | |
| 115 | LIRPVSASF | 3 | B*07 |
| 116 | SPVNSSKQPSY | 3 | B*35 |
| 117 | QLFSYAILGF | 1 | A*32 |
| | **DMD dystrophin** | **7 (23.3)** | |
| 118 | DEHLLIQHY | 2 | B*18 |
| 119 | KQVASSTGF | 1 | B*15 |
| 120 | RDFGPASQHFL | 1 | B*40 |

(continued)

| SEQ ID NO: | Underlying source protein/ HLA ligands | Number of positive CLLs (frequency [%]) | HLA |
|---|---|---|---|
| 121 | RQLGEVASF | 2 | A*32 |
| 122 | TEAETTANVL | 1 | B*40 |
| 123 | GYLPVQTVL | 1 | A*24 |
| **ARID5B AT rich interactive domain 5B (MRF1-like)** | | **7 (23.3)** | |
| 124 | GQKEALLKY | 1 | B*15 |
| 125 | KPSEERKTI | 1 | B*07 |
| 126 | KQTPKVLVV | 1 | B*13 |
| 127 | SVIQHVQSF | 1 | A*26 |
| 128 | TPIERIPYL | 3 | B*51 |
| **ZNF638 zinc finger protein 638** | | **7 (23.3)** | |
| 129 | AEVEKNETV | 1 | B*40 |
| 130 | EVKEEIPLV | 1 | B*08 |
| 131 | KPTSARSGL | 2 | B*07 |
| 132 | KYIETTPLTI | 1 | A*24 |
| 133 | SEIKTSIEV | 1 | B*40 |
| 134 | SVKPTSATK | 4 | A*03 |
| 135 | YPNKGVGQA | 1 | B*55 |
| **DDX46 DEAD (Asp-Glu-Ala-Asp) box polypeptide 46** | | **7 (23.3)** | |
| 136 | ISMKILNSL | 2 | A*02 |
| 137 | KTIAFLLPMF | 1 | A*32 |
| 138 | RDSIINDF | 2 | B*37 |
| 139 | SVKGGGGNEK | 1 | A*03 |
| 140 | GIAKTGSGK | 1 | A*03 |
| **RRM2B ribonucleotide reductase M2 B (TP53 inducible)** | | **7 (23.3)** | |
| 141 | AETTDNVFTL | 1 | B*40 |
| 142 | SEYQRFAVM | 3 | B*37, B*40, B*49 |
| 143 | TFGERVVAF | 1 | A*24 |
| 144 | NENLVERF | 2 | B*18 |
| **BLNK B-cell linker** | | **7 (23.3)** | |
| 145 | KITVPASQK | 1 | A*03 |
| 146 | KITVPASQKL | 7 | A*02 |
| 147 | VPASQKLRQL | 2 | B*07 |
| **HSH2D hematopoietic SH2 domain containing** | | **7 (23.3)** | |
| 148 | HVGYTLSYK | 1 | A*03 |
| 149 | KLPLPLPPRL | 3 | C*14 |
| 150 | KPIEPRREL | 1 | B*07 |
| 151 | SHSHVGYTL | 3 | B*38, B*39 |
| **ERP44 endoplasmic reticulum protein 44** | | **7 (23.3)** | |
| 152 | APSEYRYTL | 1 | B*07 |
| 153 | APSEYRYTLL | 3 | B*07 |
| 154 | EIFQNEVAR | 1 | A*68 |
| 155 | KDVLIPGKL | 1 | B*40 |
| 156 | VPLVREITF | 2 | B*08 |
| **METTL7A methyltransferase like 7A** | | **7 (23.3)** | |
| 157 | DPNPNFEKF | 1 | B*35 |

(continued)

| SEQ ID NO: | Underlying source protein/ HLA ligands | Number of positive CLLs (frequency [%]) | HLA |
|---|---|---|---|
| 158 | IQAPLSWEL | 1 | B*13 |
| 159 | VIYNEQMASK | 3 | A*03 |
| 160 | VLRPGGAFY | 2 | B*15 |
| | **ELP3 elongator acetyltransferase complex subunit 3** | **7 (23.3)** | |
| 161 | EDPDQDILI | 1 | B*18 |
| 162 | HGNLRELAL | 3 | B*08 |
| 163 | KLYPTLVIR | 4 | A*03 |
| 164 | SEETFRFEL | 1 | B*40 |
| | **NLRP2 NLR family, pyrin domain containing 2** | **6 (20.0)** | |
| 165 | ELNKLLEEI | 3 | A*02 |
| 166 | IPFSNPRVL | 2 | B*07 |
| 167 | LLDEGAKLLY | 2 | A*01 |
| 168 | SPADAHRNL | 1 | B*07 |
| | **ZC3H12D zinc finger CCCH-type containing 12D** | **6 (20.0)** | |
| 169 | AELERQAVL | 1 | B*37 |
| 170 | GRVPGPLSL | 1 | B*27 |
| 171 | SDLARLILL | 1 | B*27 |
| 172 | TPIREQHVL | 3 | B*35 |
| | **NELFE negative elongation factor complex member E** | **6 (20.0)** | |
| 173 | APRKGNTL | 1 | B*07 |
| 174 | EEEEALQKKF | 1 | B*44 |
| 175 | KENLVDGF | 2 | B*37 |
| 176 | VYKENLVDGF | 2 | A*23, A*24 |
| | **ATP6V1C1 ATPase, H+ transporting, lysosomal 42kDa, V1 subunit C1** | **6 (20.0)** | |
| 177 | TLLVVVPKL | 6 | A*02 |
| | **HLA-DMA major histocompatibility complex, class II, DM alpha** | **6 (20.0)** | |
| 178 | HEIDRYTAI | 1 | B*40 |
| 179 | VFTLKPLEF | 3 | A*23, A*24 |
| 180 | YWVPRNAL | 2 | B*08 |
| | **TUFM Tu translation elongation factor, mitochondrial** | **6 (20.0)** | |
| 181 | IGVEHVVVY | 5 | C*12 |
| 182 | RDKPHVNV | 1 | B*37 |
| | **EIF6 eukaryotic translation initiation factor 6** | **6 (20.0)** | |
| 183 | ADVLKVEVF | 2 | B*37 |
| 184 | IPVVHASI | 1 | B*51 |
| 185 | RDSLIDSLT | 1 | B*40 |
| 186 | TVADQVLVGSY | 2 | A*26 |
| | **CKAP4 cytoskeleton-associated protein 4** | **6 (20.0)** | |
| 187 | AADTERLAL | 1 | A*02 |
| 188 | DMKAKVASL | 2 | B*08 |
| 189 | HVLEEVQQV | 2 | B*13 |

(continued)

| SEQ ID NO: | Underlying source protein/ HLA ligands | Number of positive CLLs (frequency [%]) | HLA |
|---|---|---|---|
| 190 | KEAADTERL | 1 | B*40 |
| 191 | RISEVLQKL | 1 | A*02 |
| 192 | TEVRELVSL | 2 | B*40 |
| **COBLL1 cordon-bleu WH2 repeat protein-like 1** | | **6 (20.0)** | |
| 193 | AIRSGEAAAK | 2 | A*03 |
| 194 | APNPAPKEL | 4 | B*07 |
| 195 | RQSLLTAI | 1 | B*13 |
| 196 | SPEQTLSPL | 1 | B*35 |
| 197 | TEHQVPSSV | 1 | B*40 |
| 198 | TTYKIVPPK | 1 | A*03 |
| **TMED4 transmembrane emp24 protein transport domain containing 4** | | **6 (20.0)** | |
| 199 | QLLDQVEQI | 4 | A*02 |
| 200 | DETMVIGNY | 1 | B*18 |
| 201 | RQYGSEGRFTF | 1 | B*37 |
| **TNFRSF13C tumor necrosis factor receptor superfamily, member 13C** | | **6 (20.0)** | |
| 202 | SPAPRTAL | 6 | B*07 |
| **UBL7 ubiquitin-like 7** | | **6 (20.0)** | |
| 203 | GPRPITQSEL | 6 | B*07 |
| 204 | KPEPVDKVA | 1 | B*07 |
| 205 | TPSSRPASL | 4 | B*07 |
| **CXorf21 chromosome X open reading frame 21** | | **6 (20.0)** | |
| 206 | DETQVRSLY | 2 | B*18 |
| 207 | KEEETNSVATL | 1 | B*40 |
| 208 | LEQKVVELY | 2 | B*18 |
| 209 | NPISNAVLNEY | 1 | B*35 |
| 210 | SIKEKSSL | 1 | B*08 |
| 211 | TEITEISTPSL | 1 | B*40 |
| **ASUN asunder spermatogenesis regulator** | | **6 (20.0)** | |
| 212 | GRLNSVNNR | 1 | B*27 |
| 213 | SILEDPPSI | 3 | A*02 |
| 214 | TPRTNNIEL | 2 | B*07 |
| **RSL24D1 ribosomal L24 domain containing 1** | | **6 (20.0)** | |
| 215 | DAMKRVEEI | 3 | B*08 |
| 216 | DIKEVKQNI | 3 | B*08 |
| 217 | GPIYPGHGM | 1 | B*07 |
| **Q9UII5, ZNF107 zinc finger protein 107** | | **6 (20.0)** | |
| 218 | GDYGRAFNL | 2 | B*37 |
| 219 | TRHKIVHTK | 2 | B*27 |
| 220 | RIHTGEKPYK | 1 | A*03 |
| 221 | KAFNWFSTL | 1 | A*32 |
| **TRAF3IP3 TRAF3 interacting protein 3** | | **6 (20.0)** | |
| 222 | QSTQRSLAL | 2 | B*08 |
| 223 | RDLQMNQALRF | 1 | B*40 |
| 224 | RELESQLHVL | 2 | B*40 |

(continued)

| SEQ ID NO: | Underlying source protein/ HLA ligands | Number of positive CLLs (frequency [%]) | HLA |
|---|---|---|---|
| 225 | SEAEKLTLV | 1 | B*40 |

**Table 1b:** Additional peptides for CLL - MHC class I

| SEQ ID NO: | Amino acid sequence | HLA |
|---|---|---|
| 226 | AAAKPVATK | A*03, A*11 |
| 227 | ATYHGSFSTK | A*03, A*11 |
| 228 | FMYDRPLRL | A*02 |
| 229 | FRVGNVQEL | A*03, A*11, |
| 230 | GVAPFTIAR | A*68 |
| 231 | KMKPLDGSALY | A*30 |
| 232 | KPAPAKPVA | B*55 |
| 233 | KPVAAKPAA | n.a. |
| 234 | KQFGVAPFTI | B*13 |
| 235 | QEELVKISL | B*40:01 |
| 236 | RQLGTVQQVI | B*13 |
| 237 | RQLINALQI | B*13, A*32 |
| 238 | RVIGGLLAGQTY | B*15:01 |
| 239 | SENAFYLSP | n.a. |
| 240 | SQAPVLDAI | B*13 |
| 241 | STRYPPPAV | A*30 |
| 242 | TEDTLKVYL | B*40:01, B*52 |
| 243 | VAAKPVATK | A*03 |
| 244 | VQRWESL | B*08 |
| 245 | VRNPSVVVK | B*27 |
| 246 | GESEVAIKI | B*49, B*52 |
| 247 | LIYSVGLLLA | A*02 |
| 248 | SAYPHQLSF | A*32 |
| 249 | SVIGVFITK | A*03, A*11, A*68 |
| 250 | AELGNSVQLI | B*49 |
| 251 | ANMTVTRI | n.a |
| 252 | ARISNVEFY | C*07 |
| 253 | AVFIGNQQF | B*15:01 |
| 254 | DIELQAENI | A*02 |
| 255 | DSYTVRVSV | B*51 |
| 256 | DVKIFVNTI | B*51 |
| 257 | EIIPKYGSI | A*25 |
| 258 | EQSKIFIHR | n.a |
| 259 | FVDVGLYQY | A*03 |
| 260 | GHTSTISTL | B*39 |
| 261 | GRIEYVEVF | C*07 |
| 262 | GTSIIPFQK | A*11 |
| 263 | HPFLRGIGY | B*35 |
| 264 | IPVEIHTA | B*55 |
| 265 | KIFVNTIAY | B*15:01 |
| 266 | LPEDKVRIAY | B*35 |
| 267 | LPFSEGLTV | B*51 |
| 268 | LPWANKVTI | B*51 |
| 269 | PWANKVTI | n.a. |

(continued)

| SEQ ID NO: | Amino acid sequence | HLA |
|---|---|---|
| 270 | QAYNRAVTI | B*51 |
| 271 | RSFPQKMAY | B*15:01 |
| 272 | RYPIHWHLL | C*07 |
| 273 | SPQNLRLML | B*07 |
| 274 | SYFSSPTQR | B*27 |
| 275 | VQIKSSLI | B*13 |
| 276 | VYIGHTSTI | C*07 |
| 277 | YHVPGTGESY | C*07 |
| 278 | ATNGDLASR | A*31 |
| 279 | GLHAEVTGVGY | B*15:01 |
| 280 | HVSSTSSSF | A*32 |
| 281 | LQADLQNGL | B*13 |
| 282 | SELPVSEVA | B*45 |
| 283 | SQTKSVFEI | B*13 |
| 284 | THIFTSDGL | B*39 |
| 285 | VIYFPPLQK | A*11 |
| 286 | YPFSSEQKW | B*35 |
| 287 | GQYFGELAL | B*13 |
| 288 | RIIVKNNAK | n.a. |
| 289 | RRIIVKNNAK | B*27 |
| 290 | SFGELALMY | n.a. |
| 291 | AFNAPVINR | B*27 |
| 292 | IMKRNIATY | B*15:01 |
| 293 | KVVDVIGTK | A*11 |
| 294 | LPFLKSLEF | B*07, B*35 |
| 295 | RLKVVDVIGTK | A*03 B*07, B*51, |
| 296 | TPRAATITA | B*55 |
| 297 | KPSEKIQVL | B*07 |
| 298 | VPYPVTTTV | B*35 |
| 299 | ASFPPFVEK | B*15 |
| 300 | AFIHISTAY | A*29 |
| 301 | ATFEKIPFER | A*11 |
| 302 | KLFEKVKEV | A*02 |
| 303 | SQMPKLEAF | B*15:01 |
| 304 | AVLGQHHNY | A*03 |
| 305 | GPPAHKPR | n.a. |
| 306 | RVYDVLVLK | A*03, A*11 |
| 307 | LPRPQGITV | B*07 |
| 308 | VLYVGSKTK | A*03 |
| 309 | KTKEQVTNV | A*11 |
| 310 | MPVDPDNEAY | B*35 |
| 311 | AEKTKQGVA | B*40 |
| 312 | DIADFFTTR | A*68 |
| 313 | HSYLQRQSV | C*12 |
| 314 | KEVTLIEEL | B*40:01 |
| 315 | REDGPGVAL | B*40:01 |
| 316 | REDPLPPGL | B*40:01 |
| 317 | SLFGGSQGLRK | A*03 |
| 318 | AEFQRLKQA | B*50 |
| 319 | EVIDGVPGKW | A*25 |

(continued)

| SEQ ID NO: | Amino acid sequence | HLA |
|---|---|---|
| 320 | IPKAPGKII | B*07, B*08, B*55 |
| 321 | SHNGSAIRY | A*32 |
| 322 | TEVTVVGDKL | B*40:01 |
| 323 | YASVVVKRY | A*28 |
| 324 | ATDLALYIK | A*11 |
| 325 | AYHNWRHAF | C*07 |
| 326 | EPLNIKDAY | B*35 |
| 327 | KIAATIISF | B*15:01 |
| 328 | KIFLHIHGL | B*71 |
| 329 | LEVILKKI | n.a. |
| 330 | SEHPLAQLY | B*44 |
| 331 | VPSAQTLKI | B*51 |
| 332 | AEYRSYVA | B*45 |
| 333 | ALAPGRGTLY | A*24 |
| 334 | GPRGTQAAL | B*07 |
| 335 | IEDPGTLHI | B*49 |
| 336 | IEDPGTLHIW | B*44 |
| 337 | RPIPIAVKY | B*35 |
| 338 | VEKLLTNW | n.a. |
| 339 | FLDPDIGGVAV | A*02 |
| 340 | HTAPPENKTW | A*30 |
| 341 | LLDTPVKTQY | A*01 |
| 342 | NAVKDFTSF | A*03, A*11 |
| 343 | SGLLQIKKL | n.a. |
| 344 | YHDKNIVLL | B*39 |
| 345 | SVDPKNYPK | A*11, A*03 |
| 346 | AVGLVLPAK | A*11 |
| 347 | AVGLVLPAKL | n.a. |
| 348 | ALLEVLSQK | A*03 |
| 349 | HEKQDTLVA | B*45 |
| 350 | KELELQIGM | B*40:01, B*52 |
| 351 | MYSDVWKQL | A*24 |
| 352 | RELQDEKAEL | B*40:01 |
| 353 | RITDVLDQK | A*11 |
| 354 | EVIKITGLK | A*68 |
| 355 | HHVDITKKL | B*39 |
| 356 | LPFNVKVSV | B*51 |
| 357 | TLPRVLEI | B*51 |
| 358 | TVDLPKSPK | A*11 |
| 359 | AEHGLLLTA | B*45 |
| 360 | AQAGALLQV | B*13 |
| 361 | DGGFVLKV | B*51 |
| 362 | IVYPSGKVY | B*15:01 |
| 363 | KLDNQVSKV | A*02 |
| 364 | SENVKLFSA | B*45 |
| 365 | VQKLQNII | |
| 366 | FSTPHGLEV | B*51 |
| 367 | KRFHQKSDM | B*27 |
| 368 | KTFGHAVSL | A*32 |
| 369 | SSNLITHSR | A*31 |

(continued)

| SEQ ID NO: | Amino acid sequence | HLA |
|---|---|---|
| 370 | GVIDGHIYAV | A*02 |
| 371 | IEPAKETTTNV | B*40:01, B*44 |
| 372 | NAPPSEVLL | n.a. |
| 373 | SIEPAKETTTNV | A*02 |
| 374 | AQSQHNQSL | B*13 |
| 375 | AQSRTNPQV | B*13 |
| 376 | KMHDKVFAY | A*03 |
| 377 | TAKAPLSTV | B*51 |
| 378 | IPTRTVAI | B*51 |
| 379 | NHDRKHAV | B*39 |
| 380 | NNHDRKHAV | B*08 |
| 381 | TPGGTRIIY | B*35 |
| 382 | EHWPSPETF | A*68 |
| 383 | EIITNTLSF | A*25 |
| 384 | EVRGALMSAF | A*25 |
| 385 | IPRPILVLL | B*07 |
| 386 | LPNKNRDEL | B*07 |
| 387 | QRIPAGAVL | B*27 |
| 388 | AEGPAGGFMW | B*49 |
| 389 | AYYRDAEAY | C*07 |
| 390 | QVNRPLTMR | A*03 |
| 391 | RHSPVFQVY | A*32 |
| 392 | SLPVPNSAY | B*15:01 |
| 393 | TLGPPGTAHLY | B*15:01 |
| 394 | IEPAKETTTNV | B*40:01, B*44 |
| 395 | NAPPSEVLL | n.a. |
| 396 | SIEPAKETTTNV | A*02 |
| 397 | DLYSGLNQR | A*68 |
| 398 | KAKAKPVTR | A*31 |
| 399 | AVLDKAMKAK | A*03 |
| 400 | LELSTPLKI | B*49 |
| 401 | LPLNLDTKY | B*35 |
| 402 | TVIYRIQAL | A*02 |
| 403 | DAHIYLNHI | B*51 |
| 404 | NHIEPLKIQL | B*39 |
| 405 | AYRPAVHPR | B*27 |
| 406 | LRAPLEHEL | n.a. |
| 407 | RLFMVLLLK | A*03 |
| 408 | RSPDVLKDF | B*15:01 |
| 409 | ETAPGVHKR | A*68 |
| 410 | LYHGYIYTY | A*24 |
| 411 | GQHVATQHF | B*15:01 |
| 412 | LNGQLPNL | n.a. |
| 413 | LPFPDETHERY | B*35 |
| 414 | LPHNTHRW | B*51 |
| 415 | VVFDSPRNR | A*03 |
| 416 | YPLGRILI | B*51 |
| 417 | KEFAEFVTS | B*50 |
| 418 | VMLDVPIRL | A*02 |
| 419 | VPMTPLRTV | B*51 |

(continued)

| SEQ ID NO: | Amino acid sequence | HLA |
|---|---|---|
| 420 | QIDYKTLVL | B*13 |
| 421 | VEDPTIVRI | B*40:01, B*44, B*52 |
| 422 | IPYQDLPHL | B*07 |
| 423 | DTPFLTGHGR | A*68 |
| 424 | EFYRALYI | |
| 425 | RYYPQILTNK | |
| 426 | KAYERHVL | B*08 |
| 427 | LPSPEFHDY | B*35 |
| 428 | SLYAHPIEH | A*03 |
| 429 | LVREPGSQA | B*08 |
| 430 | RLAGPGSEKY | B*15:01 |
| 431 | SPGAGRNSVL | B*07 |
| 432 | SVQSDQGYISR | A*11 |
| 433 | GVRPPAPSL | B*13 |
| 434 | IFSEKPVFV | n.a. |
| 435 | KASNLLLGF | B*58 |
| 436 | KRYIFADAY | n.a. |
| 437 | RNLQLSLPR | A*31 |
| 438 | EASEPVALR | A*68 |
| 439 | RPKVPDQSV | B*07, B*08, B*35 |
| 440 | VLYENALKL | A*02 |
| 441 | EVLDKSQTNY | A*25 |
| 442 | MPSPIPAKY | B*35 |
| 443 | YGIENFTSV | B*51 |
| 444 | ARAAQVFFL | B*27 |
| 445 | EHIVPNAEL | B*39 |
| 446 | EAFEFVKQR | A*68 |
| 447 | NHFEGHYQY | n.a. |
| 448 | DAYPKNPHL | B*51 |
| 449 | DVNIKSTER | A*68 |
| 450 | HINSIKSVF | A*31 |
| 451 | YESEKVGVA | B*50 |
| 452 | ENAPTTVSR | A*68 |
| 453 | RFPHLLAHTY | C*14 |
| 454 | TLDGSLHAV | A*02 |
| 455 | RTVLKNLSLLK | A*03 |
| 456 | FEAKVQAI | B*49 |
| 457 | FFEAKVQAI | C*12 |
| 458 | KELQSTFK | n.a. |
| 459 | NVSSRFEEEI | A*02 |
| 460 | EVWNNLGTTK | A*68 |
| 461 | MIFRSGSLI | n.a. |
| 462 | NHALPLPGF | B*39 |
| 463 | ASVFGTMPLK | A*11 |
| 464 | REFPDRLVGY | B*44 |
| 465 | SVFGTMPLK | A*11 |
| 466 | DEMRFVTQI | n.a. |
| 467 | ETVHFATTQW | A*25 |
| 468 | LPPPATQI | B*51 |
| 469 | LARDLYAF | C*03, C*12 |

(continued)

| SEQ ID NO: | Amino acid sequence | HLA |
|---|---|---|
| 470 | LPGIGLSTSL | B*53 |
| 471 | MEVILPML | n.a. |
| 472 | AILDYILAK | A*03 |
| 473 | KIASQLSKL | A*02 |
| 474 | KVTSTTTVK | A*03, A*11 |
| 475 | YNTLLPYTF | n.a. |
| 476 | FLDPRPLTV | A*02 |
| 477 | SAFADRPAF | C*03 |
| 478 | AAVPVIISR | A*68 |
| 479 | EEIGKVAAA | B*45 |
| 480 | FLKDLVASV | A*02 |
| 481 | VIISRALEL | C*03 |
| 482 | APRTTGTPRTSL | B*07 |
| 483 | ESVGGSPQTK | A*68 |
| 484 | IPKDKAIL | B*08 |
| 485 | LPAYGRTTL | B*07 |
| 486 | HQAAIVSKI | B*13 |
| 487 | QAAIVSKI | B*51 |
| 488 | RQKMPEDGL | B*13 |
| 489 | SVQKSSGVK | A*11 |
| 490 | DSIGSTVSSER | A*68 |
| 491 | LPYNNKDRDAL | B*07 |
| 492 | IYDEIQQEM | C*14 |
| 493 | AQAKGLIQV | B*13 |
| 494 | EVSSEIYQW | A*25 |
| 495 | KWNPVPLSY | A*29 |
| 496 | NRLLAQQSL | B*27 |
| 497 | APRPVAVAV | B*07 |
| 498 | FYRETVQVGR | A*33 |
| 499 | LLAPRPVAV | A*02 |
| 500 | GLAALVILK | A*03 |
| 501 | KIQEVFSSY | B*15:01 |
| 502 | ASLDKFLSH | A*11 |
| 503 | ALYATKTLR | A*03 |
| 504 | MEYVISRI | n.a. |
| 505 | VPVGRQPII | B*51 |
| 506 | KLLIGVIAAV | A*02 |
| 507 | LPSLIKLD | n.a. B*51!!) |
| 508 | PSLIKLDL | n.a. |
| 509 | ARNKELIGK | B*27 |
| 510 | AVKSNAAAY | B*15:01 |
| 511 | EVIIPHSGW | A*25 |
| 512 | SVKEQEAQF | B*15:01 |
| 513 | APRGLEPIAI | B*07 |
| 514 | GRFGGVITI | B*27 |
| 515 | PVAGFFINT | A*68 |
| 516 | TPKTPSRDA | B*08, B*55 |
| 517 | VLFGGKVSGA | A*02 |
| 518 | AEHIESRTL | B*40, B*44 |
| 519 | DQYPYLKSV | C*12 |

(continued)

| SEQ ID NO: | Amino acid sequence | HLA |
|---|---|---|
| 520 | IARNLTQQL | B*07 |
| 521 | IESRTLAIA | B*50 |
| 522 | MTSALPIIQK | A*11 |
| 523 | SLLTSSKGQLQK | A*03 |
| 524 | TSALPIIQK | A*11, A*03 |
| 525 | VRLGSLSTK | B*27 |
| 526 | RINEFSISSF | B*15 |
| 527 | DEKQQHIVY | B*18 |
| 528 | DEVYQVTVY | B*18 |
| 529 | GEISEKAKL | B*40 |
| 530 | YTMKEVLFY | A*03 |
| 531 | SQLTTLSFY | B*15 |
| 532 | LEKQLIEL | B*44 |
| 533 | ELTLGEFLK | A*68, A*33 |
| 534 | LTLGEFLK | A*68 |
| 535 | LTLGEFLKL | A*02 |
| 536 | TLGEFLKL | A*02 |
| 537 | ITARPVLW | B*58 |
| 538 | KLMSPKLYVW | A*32 |
| 539 | KVSAVTLAY | A*03 |
| 540 | VEGSGELFRW | B*44 |
| 541 | RPKSNIVL | B*07 |
| 542 | RPKSNIVLL | B*07 |

**Table 1c:** Additional peptides for CLL - MHC class II

| SEQ ID NO: | Amino acid sequence | MHC |
|---|---|---|
| 543 | GEPLSYTRFSLARQ | class II |
| 544 | GEPLSYTRFSLARQVD | class II |
| 545 | GEPLSYTRFSLARQVDG | class II |
| 546 | GGEPLSYTRFSLARQVD | class II |
| 547 | GGEPLSYTRFSLARQVDG | class II |
| 548 | NPGGYVAYSKAATVTG | class II |
| 549 | NPGGYVAYSKAATVTGK | class II |
| 550 | NPGGYVAYSKAATVTGKL | class II |
| 551 | NSVIIVDKNGRL | class II |
| 552 | NSVIIVDKNGRLV | class II |
| 553 | NSVIIVDKNGRLVY | class II |
| 554 | RVEYHFLSPYVSPK | class II |
| 555 | RVEYHFLSPYVSPKE | class II |
| 556 | RVEYHFLSPYVSPKESPF | class II |
| 557 | SPFRHVFWGSGSHTL | class II |
| 558 | SVIIVDKNGRLV | class II |
| 559 | VEYHFLSPYVSPK | class II |
| 560 | VEYHFLSPYVSPKE | class II |
| 561 | LPSQAFEYILYNKG | class II |
| 562 | LPSQAFEYILYNKGI | class II |
| 563 | LPSQAFEYILYNKGIM | class II |
| 564 | LPSQAFEYILYNKGIMG | class II |
| 565 | MNGYFLIERGKNM | class II |

(continued)

| SEQ ID NO: | Amino acid sequence | MHC |
| --- | --- | --- |
| 566 | NGYFLIERGKNM | class II |
| 567 | PSQAFEYILYNKG | class II |
| 568 | PSQAFEYILYNKGI | class II |
| 569 | PSQAFEYILYNKGIM | class II |
| 570 | EGVQYSYSLFHLM | class II |
| 571 | EGVQYSYSLFHLML | class II |
| 572 | GVQYSYSLFHLM | class II |
| 573 | GVQYSYSLFHLML | class II |
| 574 | SIISIHPKIQEHQPR | class II |
| 575 | SSIRTSTNSQVDK | class II |
| 576 | VLVGYKAVYRIS | class II |
| 577 | YSSIRTSTNSQVDK | class II |
| 578 | GGGYGSGGGSGGYGSRRF | class II |
| 579 | GGSFGGRSSGSP | class II |
| 580 | KGGSFGGRSSGSP | class II |
| 581 | SGQQQSNYGPMKGGSFGGRSSGSPY | class II |
| 582 | SGSPYGGGYGSGGGSGGYGSRRF | class II |
| 583 | SPYGGGYGSGGGSGGYGSRRF | class II |
| 584 | YGGGYGSGGGSGGYGSRRF | class II |
| 585 | GNRINEFSISSF | class II |
| 586 | HGNQITSDKVGRKV | class II |
| 587 | IPPVNTNLENLYLQ | class II |
| 588 | LQVLRLDGNEIKR | class II |
| 589 | LQVLRLDGNEIKRS | class II |
| 590 | LQVLRLDGNEIKRSA | class II |
| 591 | LRELHLDHNQISRVPN | class II |
| 592 | LYVRLSHNSLTNNG | class II |
| 593 | VPSRMKYVYFQNNQ | class II |
| 594 | VPSRMKYVYFQNNQIT | class II |
| 595 | VPSRMKYVYFQNNQITS | class II |
| 596 | WIALHGNQITSD | class II |
| 597 | WIALHGNQITSDK | class II |
| 598 | ADDNVSFRWEALGNT | class II |
| 599 | ADDNVSFRWEALGNTL | class II |
| 600 | DADDNVSFRWEALGNTL | class II |
| 601 | DDNVSFRWEALGNT | class II |
| 602 | DDNVSFRWEALGNTL | class II |
| 603 | DNVSFRWEALGNT | class II |
| 604 | DNVSFRWEALGNTL | class II |
| 605 | DNVSFRWEALGNTLS | class II |
| 606 | DTGSYRAQISTKTSAK | class II |
| 607 | DTGSYRAQISTKTSAKL | class II |
| 608 | DTITIYSTINHSK | class II |
| 609 | EDTGSYRAQISTKTSAK | class II |
| 610 | ENDTITIYSTINHSK | class II |
| 611 | ENDTITIYSTINHSKESKPT | class II |
| 612 | GSYRAQISTKTSAK | class II |
| 613 | NDTITIYSTINH | class II |
| 614 | NDTITIYSTINHS | class II |
| 615 | NDTITIYSTINHSK | class II |

(continued)

| SEQ ID NO: | Amino acid sequence | MHC |
|---|---|---|
| 616 | NVSFRWEALGNTL | class II |
| 617 | SPTNNTVYASVTHSNRET | class II |
| 618 | TGSYRAQISTKTSAK | class II |
| 619 | TPRENDTITIYSTINHSK | class II |
| 620 | TPRENDTITIYSTINHSKESKPT | class II |
| 621 | VSFRWEALGNTL | class II |
| 622 | APIHFTIEKLELNEK | class II |
| 623 | DAQFEVIKGQTIE | class II |
| 624 | DAQFEVIKGQTIEVR | class II |
| 625 | ESYFIPEVRIYDSGT | class II |
| 626 | IPEVRIYDSGTY | class II |
| 627 | KDKAIVAHNRHGNK | class II |
| 628 | KDKAIVAHNRHGNKA | class II |
| 629 | NFVILEFPVEEQDR | class II |
| 630 | SQPRISYDAQFEVIK | class II |
| 631 | SQPRISYDAQFEVIKG | class II |
| 632 | YDAQFEVIKGQTIE | class II |
| 633 | GNPAYRSFSNSLSQ | class II |
| 634 | GPPGEAGYKAFSSLLA | class II |
| 635 | GPPGEAGYKAFSSLLASS | class II |
| 636 | GPPGEAGYKAFSSLLASSA | class II |
| 637 | GPPGEAGYKAFSSLLASSAVSPE | class II |
| 638 | GPPGEAGYKAFSSLLASSAVSPEK | class II |
| 639 | GYKAFSSLLASSAVSP | class II |
| 640 | GYKAFSSLLASSAVSPE | class II |
| 641 | KAFSSLLASSAVSPE | class II |
| 642 | NPAYRSFSNSLSQ | class II |
| 643 | SRDDFQEGREGIVAR | class II |
| 644 | SSSSFHPAPGNAQ | class II |
| 645 | VARLTESLFLDL | class II |
| 646 | VARLTESLFLDLLG | class II |
| 647 | VIAGNPAYRSFSN | class II |
| 648 | VPQPEPETWEQILRRNVLQ | class II |
| 649 | YKAFSSLLASSAVS | class II |
| 650 | YKAFSSLLASSAVSP | class II |
| 651 | YKAFSSLLASSAVSPE | class II |
| 652 | GNQVFSYTANKEIRTDD | class II |
| 653 | IEEIVLVDDASERD | class II |
| 654 | IEEIVLVDDASERDF | class II |
| 655 | LENIYPDSQIPRH | class II |
| 656 | LENIYPDSQIPRHY | class II |
| 657 | NQVFSYTANKEIR | class II |
| 658 | NQVFSYTANKEIRT | class II |
| 659 | NQVFSYTANKEIRTDD | class II |
| 660 | VHSVINRSPRHMIEE | class II |
| 661 | EYVSLYHQPAAM | class II |
| 662 | IKAEYKGRVTLKQYPR | class II |
| 663 | LNVHSEYEPSWEEQP | class II |
| 664 | LPYLFQMPAYASSS | class II |
| 665 | LPYLFQMPAYASSSK | class II |

(continued)

| SEQ ID NO: | Amino acid sequence | MHC |
|---|---|---|
| 666 | NFIKAEYKGRVT | class II |
| 667 | TNFIKAEYKGRVT | class II |
| 668 | TTNFIKAEYKGRVT | class II |
| 669 | VTLNVHSEYEPSWEEQP | class II |
| 670 | YPRKNLFLVEVTQLTESDS | class II |
| 671 | YPRKNLFLVEVTQLTESDSG | class II |
| 672 | ADLSSFKSQELN | class II |
| 673 | ADLSSFKSQELNER | class II |
| 674 | ADLSSFKSQELNERN | class II |
| 675 | ADLSSFKSQELNERNE | class II |
| 676 | ADLSSFKSQELNERNEA | class II |
| 677 | AEQQRLKSQDLELSWNLNG | class II |
| 678 | EQQRLKSQDLELSWN | class II |
| 679 | ISQELEELRAEQQR | class II |
| 680 | ISQELEELRAEQQRLK | class II |
| 681 | KGTKQWVHARYA | class II |
| 682 | QADLSSFKSQELNER | class II |
| 683 | SWNLNGLQADLSSFK | class II |
| 684 | TGSWIGLRNLDLKG | class II |
| 685 | FGNYNNQSSNFGPMKGGNFGGRS | class II |
| 686 | FGPMKGGNFGGRSSGPYGGGGQY | class II |
| 687 | GPMKGGNFGGRSSGP | class II |
| 688 | GPYGGGGQYFAKP | class II |
| 689 | KGGNFGGRSSGP | class II |
| 690 | NDFGNYNNQSSNFGP | class II |
| 691 | SGPYGGGGQYFAKP | class II |
| 692 | DAGSYKAQINQRNFE | class II |
| 693 | DAGSYKAQINQRNFEVT | class II |
| 694 | DGELIRTQPQRLPQ | class II |
| 695 | GELIRTQPQRLPQ | class II |
| 696 | NPSDGELIRTQPQRLP | class II |
| 697 | NPSDGELIRTQPQRLPQ | class II |
| 698 | NPSDGELIRTQPQRLPQL | class II |
| 699 | ASNDMYHSRALQVVR | class II |
| 700 | ASNDMYHSRALQVVRA | class II |
| 701 | EGVRRALDFAVGEYN | class II |
| 702 | EGVRRALDFAVGEYNK | class II |
| 703 | SNDMYHSRALQVVR | class II |
| 704 | VGEYNKASNDMYH | class II |
| 705 | VRARKQIVAGVNY | class II |
| 706 | VRRALDFAVGEYNKASND | class II |
| 707 | VVRARKQIVAGVN | class II |
| 708 | WRARKQIVAGVNY | class II |
| 709 | APLEGARFALVRED | class II |
| 710 | APVELILSDETLPAPE | class II |
| 711 | ELILSDETLPAPE | class II |
| 712 | LAPLEGARFALVRE | class II |
| 713 | LAPLEGARFALVRED | class II |
| 714 | RGEKELLVPRSSTSPD | class II |
| 715 | ASKTFTTQETITNAET | class II |

(continued)

| SEQ ID NO: | Amino acid sequence | MHC |
|---|---|---|
| 716 | DQHFRTTPLEKNAPV | class II |
| 717 | NTPILVDGKDVMPE | class II |
| 718 | NTPILVDGKDVMPEV | class II |
| 719 | NTPILVDGKDVMPEVN | class II |
| 720 | SNTPILVDGKDVMPE | class II |
| 721 | SNTPILVDGKDVMPEVN | class II |
| 722 | TPILVDGKDVMP | class II |
| 723 | TPILVDGKDVMPE | class II |
| 724 | TPILVDGKDVMPEV | class II |
| 725 | TPILVDGKDVMPEVN | class II |
| 726 | GPLKFLHQDIDSGQG | class II |
| 727 | GPLKFLHQDIDSGQGIR | class II |
| 728 | LGDIYFKLFRASG | class II |
| 729 | TGHLFDLSSLSGRAG | class II |
| 730 | VPSPVDCQVTDLAGNE | class II |
| 731 | DGLNSLTYQVLDVQRYPL | class II |
| 732 | HPVLQRQQLDYGIY | class II |
| 733 | LNSLTYQVLDVQR | class II |
| 734 | LNSLTYQVLDVQRYP | class II |
| 735 | LNSLTYQVLDVQRYPL | class II |
| 736 | LPQLVGVSTPLQG | class II |
| 737 | LPQLVGVSTPLQGG | class II |
| 738 | LPQLVGVSTPLQGGS | class II |
| 739 | RLPQLVGVSTPLQGGS | class II |
| 740 | SPHKVAIIIPFRNR | class II |
| 741 | SPHKVAIIIPFRNRQE | class II |
| 742 | SPHKVAIIIPFRNRQEH | class II |
| 743 | AIVQAVSAHRHR | class II |
| 744 | ARNFERNKAIKVI | class II |
| 745 | ARNFERNKAIKVIIA | class II |
| 746 | NFERNKAIKVII | class II |
| 747 | NFERNKAIKVIIA | class II |
| 748 | VAIVQAVSAHRH | class II |
| 749 | VAIVQAVSAHRHR | class II |
| 750 | VAIVQAVSAHRHRA | class II |
| 751 | VAIVQAVSAHRHRAR | class II |
| 752 | EEVITLIRSNQQLE | class II |
| 753 | EEVITLIRSNQQLEN | class II |
| 754 | IPADTFAALKNPNAML | class II |
| 755 | LKQLLSDKQQKRQSG | class II |
| 756 | LKQLLSDKQQKRQSGQ | class II |
| 757 | TPSYVAFTDTER | class II |
| 758 | TPSYVAFTDTERL | class II |
| 759 | EGLYSRTLAGSIT | class II |
| 760 | EGLYSRTLAGSITTPP | class II |
| 761 | EKWYIPDPTGKFN | class II |
| 762 | GAIAAINSIQHNTR | class II |
| 763 | LPILVPSAKKAI | class II |
| 764 | LPILVPSAKKAIY | class II |
| 765 | LPILVPSAKKAIYM | class II |

(continued)

| SEQ ID NO: | Amino acid sequence | MHC |
|---|---|---|
| 766 | LPILVPSAKKAIYMD | class II |
| 767 | LPILVPSAKKAIYMDD | class II |
| 768 | VEEGLYSRTLAGSIT | class II |
| 769 | WEKWYIPDPTGKFN | class II |
| 770 | YKIVNFDPKLLE | class II |
| 771 | YKIVNFDPKLLEG | class II |
| 772 | YKIVNFDPKLLEGKV | class II |
| 773 | LPEFYKTVSPAL | class II |
| 774 | VGQFIQDVKNSRST | class II |
| 775 | VGQFIQDVKNSRSTD | class II |
| 776 | VVGQFIQDVKNSRS | class II |
| 777 | VVGQFIQDVKNSRST | class II |
| 778 | VVGQFIQDVKNSRSTD | class II |
| 779 | VVGQFIQDVKNSRSTDS | class II |
| 780 | DNGHLYREDQTSPAPG | class II |
| 781 | DNGHLYREDQTSPAPGLR | class II |
| 782 | EVQVFAPANALPARSE | class II |
| 783 | GHLYREDQTSPAPG | class II |
| 784 | LPARSEAAAVQPVIG | class II |
| 785 | NGHLYREDQTSPAPG | class II |
| 786 | NGHLYREDQTSPAPGL | class II |
| 787 | NGHLYREDQTSPAPGLR | class II |
| 788 | VFAPANALPARSEAA | class II |
| 789 | VQVFAPANALPARSE | class II |
| 790 | AIVVSDRDGVPVIK | class II |
| 791 | GLHAIVVSDRDGVPV | class II |
| 792 | GLHAIVVSDRDGVPVIK | class II |
| 793 | HAIVVSDRDGVPV | class II |
| 794 | KLPSVEGLHAIVVSDRDG | class II |
| 795 | LHAIVVSDRDGVPV | class II |
| 796 | LHAIVVSDRDGVPVI | class II |
| 797 | LHAIVVSDRDGVPVIK | class II |
| 798 | LPSVEGLHAIVVSDR | class II |
| 799 | VPVIKVANDNAPE | class II |
| 800 | YNTYQVVQFNRLP | class II |
| 801 | YNTYQVVQFNRLPL | class II |
| 802 | YNTYQVVQFNRLPLV | class II |
| 803 | YNTYQVVQFNRLPLVV | class II |
| 804 | YYNTYQVVQFNRLP | class II |
| 805 | YYNTYQVVQFNRLPL | class II |
| 806 | YYNTYQVVQFNRLPLV | class II |
| 807 | DKIYFMAGSSRKE | class II |
| 808 | DVGTDEEEETAKESTAEKDE | class II |
| 809 | EVTFKSILFVPTSAP | class II |
| 810 | KSEKFAFQAEVNR | class II |
| 811 | LPEFDGKRFQNVAK | class II |
| 812 | DGSYRIFSKGASE | class II |
| 813 | GSYRIFSKGASE | class II |
| 814 | SDGSYRIFSKGASE | class II |
| 815 | SVKKMMKDNNLVRH | class II |

(continued)

| SEQ ID NO: | Amino acid sequence | MHC |
|---|---|---|
| 816 | VKKMMKDNNLVRH | class II |
| 817 | NNMRIFGEAAEKN | class II |
| 818 | VDKVLERDQKLSE | class II |
| 819 | VDKVLERDQKLSELD | class II |
| 820 | VDKVLERDQKLSELDD | class II |
| 821 | VDKVLERDQKLSELDDR | class II |
| 822 | VLERDQKLSELDDR | class II |
| 823 | ATRSIQVDGKTIKAQ | class II |
| 824 | ATRSIQVDGKTIKAQI | class II |
| 825 | IGVEFATRSIQVDGK | class II |
| 826 | RSIQVDGKTIKA | class II |
| 827 | RSIQVDGKTIKAQ | class II |
| 828 | RSIQVDGKTIKAQI | class II |
| 829 | TRSIQVDGKTIKAQ | class II |
| 830 | DIMRVNVDKVLERDQK | class II |
| 831 | DIMRVNVDKVLERDQKL | class II |
| 832 | IMRVNVDKVLERDQK | class II |
| 833 | VDKVLERDQKLSE | class II |
| 834 | VDKVLERDQKLSELD | class II |
| 835 | VDKVLERDQKLSELDD | class II |
| 836 | VDKVLERDQKLSELDDR | class II |
| 837 | VLERDQKLSELDDR | class II |
| 838 | ATRSIQVDGKTIKAQ | class II |
| 839 | ATRSIQVDGKTIKAQI | class II |
| 840 | IGVEFATRSIQVDGK | class II |
| 841 | RSIQVDGKTIKA | class II |
| 842 | RSIQVDGKTIKAQ | class II |
| 843 | RSIQVDGKTIKAQI | class II |
| 844 | TRSIQVDGKTIKAQ | class II |
| 845 | GIRVAPVPLYNS | class II |
| 846 | GIRVAPVPLYNSFH | class II |
| 847 | NPNGIRVAPVPLYNSFH | class II |
| 848 | DDPAIDVCKKLLGKYPN | class II |
| 849 | DKQPYSKLPGVSLLKP | class II |
| 850 | DKQPYSKLPGVSLLKPL | class II |
| 851 | HPRYYISANVTGFK | class II |
| 852 | SHPRYYISANVTG | class II |
| 853 | SHPRYYISANVTGFK | class II |
| 854 | TSHPRYYISANVTG | class II |
| 855 | TSHPRYYISANVTGFK | class II |
| 856 | ADIFVDPVLHTA | class II |
| 857 | ADIFVDPVLHTACA | class II |
| 858 | DPGADYRIDRALNEA | class II |
| 859 | IAQDYKVSYSLA | class II |
| 860 | IAQDYKVSYSLAK | class II |
| 861 | ISRDWKLDPVLYRK | class II |
| 862 | LIAQDYKVSYSLA | class II |
| 863 | RQKLIAQDYKVSYS | class II |
| 864 | RQKLIAQDYKVSYSL | class II |
| 865 | RQKLIAQDYKVSYSLA | class II |

(continued)

| SEQ ID NO: | Amino acid sequence | MHC |
|---|---|---|
| 866 | RQKLIAQDYKVSYSLAK | class II |
| 867 | SALDYRLDPQLQLH | class II |
| 868 | SKADIFVDPVLHTA | class II |
| 869 | SPSKNYILSVISGSI | class II |
| 870 | ETTQLTADSHPSYHTDG | class II |
| 871 | SGESLYHVLGLDKNATSDD | class II |
| 872 | TTQLTADSHPSYHT | class II |
| 873 | TTQLTADSHPSYHTD | class II |
| 874 | TTQLTADSHPSYHTDG | class II |
| 875 | SVEEFLSEKLERI | class II |
| 876 | VEEFLSEKLERI | class II |
| 877 | DLSSSILAQSRERVA | class II |
| 878 | EKGVRTLTAAAVSGAQ | class II |
| 879 | EKGVRTLTAAAVSGAQP | class II |
| 880 | EKGVRTLTAAAVSGAQPI | class II |
| 881 | KGVRTLTAAAVSGA | class II |
| 882 | KGVRTLTAAAVSGAQ | class II |
| 883 | VGPFAPGITEKAPEEKK | class II |
| 884 | DPPLIALDKDAPLR | class II |
| 885 | EIITPDVPFTVDKDG | class II |
| 886 | IITPDVPFTVDKDG | class II |
| 887 | PPLIALDKDAPLR | class II |
| 888 | TNVKKSHKATVHIQ | class II |
| 889 | DDNIKTYSDHPE | class II |
| 890 | DDNIKTYSDHPEK | class II |
| 891 | DSAVFFEQGTTRIG | class II |
| 892 | GDKVYVHLKNLASRPY | class II |
| 893 | GDKVYVHLKNLASRPYT | class II |
| 894 | VHLKNLASRPYT | class II |
| 895 | VYVHLKNLASRPY | class II |
| 896 | VYVHLKNLASRPYT | class II |
| 897 | VYVHLKNLASRPYTFH | class II |
| 898 | YVHLKNLASRPY | class II |
| 899 | YVHLKNLASRPYT | class II |
| 900 | YVHLKNLASRPYTFH | class II |
| 901 | SNLIKLAQKVPTAD | class II |
| 902 | YDTRTSALSAKS | class II |
| 903 | ALMTDPKLITWSPV | class II |
| 904 | NDVAWNFEKFLVGPDG | class II |
| 905 | QSVYAFSARPLAG | class II |
| 906 | QSVYAFSARPLAGGEPV | class II |
| 907 | WNFEKFLVGPDG | class II |
| 908 | DVGMFVALTKLGQPD | class II |
| 909 | VGMFVALTKLGQPD | class II |
| 910 | AGVFHVEKNGRY | class II |
| 911 | FAGVFHVEKNGRYS | class II |
| 912 | GPITITIVNRDGTR | class II |
| 913 | NGRYSISRTEAADL | class II |
| 914 | RKSRQGSLAMEELK | class II |
| 915 | RRKSRQGSLAMEELK | class II |

(continued)

| SEQ ID NO: | Amino acid sequence | MHC |
|---|---|---|
| 916 | EEFKKLTSIKIQNDK | class II |
| 917 | INRRMADDNKLFR | class II |
| 918 | TATIVMVTNLKERKE | class II |
| 919 | ELFYKGIRPAINVG | class II |
| 920 | GQKRSTVAQLVKR | class II |
| 921 | SDLDAATQQLLSRGV | class II |
| 922 | FDFSQNTRVPRLPE | class II |
| 923 | GDAPAILFDKEF | class II |
| 924 | VTHEIDRYTAIAY | class II |
| 925 | GQGYLIKDGKLIKNNA | class II |
| 926 | IDTTSKFGHGRFQTM | class II |
| 927 | IDVIGVTKGKGYKGVTSRW | class II |
| 928 | MGPLKKDRIAKEEGA | class II |
| 929 | AAKYQLDPTASISA | class II |
| 930 | IAAKYQLDPTASISA | class II |
| 931 | IAAKYQLDPTASISAK | class II |
| 932 | AGLGRAYALAFAERG | class II |
| 933 | DAFGRIDVVVNNAG | class II |
| 934 | GLGRAYALAFAER | class II |
| 935 | GLGRAYALAFAERG | class II |
| 936 | AKFALNGEEFMNFDL | class II |
| 937 | AKFALNGEEFMNFDLK | class II |
| 938 | ALNGEEFMNFDLK | class II |
| 939 | KFALNGEEFMNFDL | class II |
| 940 | SDGSFHASSSLTVK | class II |
| 941 | EERNLLSVAYKNVVGAR | class II |
| 942 | ERNLLSVAYKNVVGAR | class II |
| 943 | IAELDTLSEESYKD | class II |
| 944 | IAELDTLSEESYKDS | class II |
| 945 | ADSYLDEGFLLDKKIG | class II |
| 946 | DSYLDEGFLLDKK | class II |
| 947 | DSYLDEGFLLDKKIG | class II |
| 948 | VDNIIKAAPRKRVPD | class II |
| 949 | SPPQFRVNGAISN | class II |
| 950 | SPPQFRVNGAISNFE | class II |
| 951 | SPPQFRVNGAISNFEE | class II |
| 952 | SPPQFRVNGAISNFEEF | class II |
| 953 | VGKMFVDVYFQEDKK | class II |
| 954 | VGKMFVDVYFQEDKKE | class II |
| 955 | DPKRTIAQDYGVLKADE | class II |
| 956 | DPKRTIAQDYGVLKADEG | class II |
| 957 | PKRTIAQDYGVLKADEG | class II |
| 958 | GLFIIDDKGILRQ | class II |
| 959 | GLFIIDDKGILRQIT | class II |
| 960 | RGLFIIDDKGILR | class II |
| 961 | RGLFIIDDKGILRQ | class II |
| 962 | RGLFIIDDKGILRQIT | class II |
| 963 | GNTVIHLDQALARMR | class II |
| 964 | NTVIHLDQALARMR | class II |
| 965 | NTVIHLDQALARMRE | class II |

(continued)

| SEQ ID NO: | Amino acid sequence | MHC |
|---|---|---|
| 966 | ENNEIISNIRDSVIN | class II |
| 967 | NNEIISNIRDSVIN | class II |
| 968 | SPTVQVFSASGKPV | class II |
| 969 | SSPTVQVFSASGKPVE | class II |
| 970 | AEPNYHSLPSARTDEQ | class II |
| 971 | SSILAKTASNIIDVS | class II |
| 972 | LEARATAPPAPSAPN | class II |
| 973 | ADDLEGEAFLPL | class II |
| 974 | ADDLEGEAFLPLR | class II |
| 975 | ADDLEGEAFLPLRE | class II |
| 976 | GADDLEGEAFLPLR | class II |
| 977 | AGREINLVDAHLKSE | class II |
| 978 | AGREINLVDAHLKSEQT | class II |
| 979 | GREINLVDAHLKSE | class II |
| 980 | KPGIVYASLNHSVIG | class II |
| 981 | NKPGIVYASLNHSVIG | class II |
| 982 | TTLYVTDVKSASERPS | class II |
| 983 | APSTYAHLSPAKTPPP | class II |
| 984 | APSTYAHLSPAKTPPPP | class II |
| 985 | APSTYAHLSPAKTPPPPA | class II |
| 986 | RDDLYDQDDSRDFPR | class II |
| 987 | TRPYHSLPSEAVFA | class II |
| 988 | TRPYHSLPSEAVFAN | class II |
| 989 | VAVFTFHNHGRT | class II |
| 990 | VAVFTFHNHGRTA | class II |
| 991 | VAVFTFHNHGRTANL | class II |
| 992 | EDDYIKSWEDNQQGDE | class II |
| 993 | ELERIQIQEAAKKKPG | class II |
| 994 | ERIQIQEAAKKKP | class II |
| 995 | ERIQIQEAAKKKPG | class II |
| 996 | ERIQIQEAAKKKPGI | class II |
| 997 | LERIQIQEAAKKKPG | class II |
| 998 | LSSISQYSGKIK | class II |
| 999 | SPAKDSLSFEDF | class II |
| 1000 | SPAKDSLSFEDFLDL | class II |
| 1001 | INSRFPIPSATDPD | class II |
| 1002 | VQHYELLNGQSVFG | class II |
| 1003 | DNQYAVLENQKSSH | class II |
| 1004 | GPPEIYSDTQFPS | class II |
| 1005 | GPPEIYSDTQFPSLQ | class II |
| 1006 | TPQGPPEIYSDTQFPS | class II |
| 1007 | TPQGPPEIYSDTQFPSLQ | class II |
| 1008 | TPQGPPEIYSDTQFPSLQST | class II |
| 1009 | ANLQRAYSLAKEQR | class II |
| 1010 | NLQRAYSLAKEQR | class II |
| 1011 | TPSGITYDRKDIEEH | class II |
| 1012 | VSTLNSEDFVLVSR | class II |
| 1013 | VSTLNSEDFVLVSRQ | class II |
| 1014 | VSTLNSEDFVLVSRQG | class II |
| 1015 | GSSFFGELFNQNPE | class II |

(continued)

| SEQ ID NO: | Amino acid sequence | MHC |
|---|---|---|
| 1016 | SGSSFFGELFNQNPE | class II |

**Table 2:** Peptides suitable for the (combined) treatment of CLL and/or AML

| SEQ ID NO: | Amino acid sequence |
|---|---|
| 710 | APVELILSDETLPAPE |
| 878 | EKGVRTLTAAAVSGAQ |
| 879 | EKGVRTLTAAAVSGAQP |
| 533 | ELTLGEFLK |
| 476 | FLDPRPLTV |
| 892 | GDKVYVHLKNLASRPY |
| 111 | GLDPNKPPEL |
| 178 | HEIDRYTAI |
| 181 | IGVEHVVVY |
| 184 | IPVVHASI |
| 882 | KGVRTLTAAAVSGAQ |
| 363 | KLDNQVSKV |
| 42 | KLYELHVFTF |
| 163 | KLYPTLVIR |
| 137 | KTIAFLLPMF |
| 713 | LAPLEGARFALVRED |
| 532 | LEKQLIEL |
| 734 | LNSLTYQVLDVQRYP |
| 736 | LPQLVGVSTPLQG |
| 737 | LPQLVGVSTPLQGG |
| 738 | LPQLVGVSTPLQGGS |
| 534 | LTLGEFLK |
| 535 | LTLGEFLKL |
| 914 | RKSRQGSLAMEELK |
| 739 | RLPQLVGVSTPLQGGS |
| 477 | SAFADRPAF |
| 164 | SEETFRFEL |
| 364 | SENVKLFSA |
| 531 | SQLTTLSFY |
| 536 | TLGEFLKL |
| 186 | TVADQVLVGSY |
| 179 | VFTLKPLEF |
| 159 | VIYNEQMASK |
| 365 | VQKLQNII |
| 895 | VYVHLKNLASRPY |
| 44 | YLNKEIEEA |
| 180 | YWVPRNAL |

Disclosed is at least one peptide selected from the group consisting of SEQ ID NO: 710, 878, 879, 533, 476, 892, 111, 178, 181, 184, 882, 363, 42, 163, 137, 713, 532, 734, 736, 737, 738, 534, 535, 914, 739, 477, 164, 364, 531, 536, 186, 179, 159, 365, 895, 44, and 180, and the use thereof in the treatment of AML and/or CML as described herein.

The present invention furthermore relates to the peptides according to the present invention for use in the treatment of CLL/AML As shown in the following table 3, many of the peptides as disclosed can also be used in other cancerous and proliferative indications.

**Table 3:** Peptides as disclosed and their specific uses in other proliferative diseases, optionally in other organs. SEQ ID NO: 167 is according to the invention.

| Seq ID | Sequence | Tissue and disease |
|---|---|---|
| 1 | AEHPNVTLTI | colon or rectum, spleen, non-Hodgkin's lymphoma |
| 2 | FLAEHPNVTL | colon or rectum, spleen, non-Hodgkin's lymphoma |
| 3 | ILYGRSYTW | stomach, adenocarcinoma, skin, squamous cell carcinoma |
| 4 | EVAEFLARH | colon or rectum, spleen, non-Hodgkin's lymphoma lymphoma |
| 5 | RHSNVNLTI | colon or rectum, spleen, non-Hodgkin's lymphoma |
| 6 | HPDNVKLFL | pancreas, adenocarcinoma, non-Hodgkin's lymphoma, small lymphocytic type |
| 7 | ISDTGELKL | pancreas, adenocarcinoma, non-Hodgkin's lymphoma, small lymphocytic type |
| 8 | KVNGKLVALK | pancreas, adenocarcinoma, non-Hodgkin's lymphoma, small lymphocytic type |
| 9 | NRLSAQAAL | pancreas, adenocarcinoma, non-Hodgkin's lymphoma, small lymphocytic type |
| 10 | TPFTAIREA | pancreas, adenocarcinoma, non-Hodgkin's lymphoma, small lymphocytic type |
| 11 | FGLARAKSV | kidney, clear cell renal cell carcinoma, |
| 12 | KIADFGLAR | brain, glioblastoma, liver, hepatocellular carcinoma |
| 13 | AAANIIRTL | liver, hepatocellular carcinoma, adrenal gland, adrenal cortical carcinoma |
| 14 | GRFKNLREAL | liver, hepatocellular carcinoma, adrenal gland, adrenal cortical carcinoma |
| 15 | MSPFSKATL | liver, hepatocellular carcinoma, adrenal gland, adrenal cortical carcinoma |
| 16 | QEDPGDNQITL | liver, hepatocellular carcinoma, adrenal gland, adrenal cortical carcinoma |
| 17 | SPFSKATL | liver, hepatocellular carcinoma, adrenal gland, adrenal cortical carcinoma |
| 18 | DALLKRTM | stomach, metastatic, skin, basal cell carcinoma |
| 19 | GEDVRSALL | stomach, metastatic, skin, basal cell carcinoma |
| 20 | KFAEEFYSF | stomach, metastatic, skin, basal cell carcinoma |
| 21 | YGYDNVKEY | stomach, metastatic, skin, basal cell carcinoma |
| 22 | LEVEERTKPV | lung, non-small cell lung carcinoma, breast, carcinoma |
| 23 | RDSPINANLRY | lung, non-small cell lung carcinoma, breast, carcinoma |
| 24 | RPFVIVTA | lung, non-small cell lung carcinoma, breast, carcinoma |
| 25 | RPIINTPMV | lung, non-small cell lung carcinoma, breast, carcinoma |
| 26 | SPTSSRTSSL | lung, non-small cell lung carcinoma, breast, carcinoma |
| 27 | ATSAPLVSR | stomach, metastatic, lung, neuroendocrine carcinoma (non-small cell type) |
| 28 | AELRSTASLL | lipoma |

(continued)

| Seq ID | Sequence | Tissue and disease |
|---|---|---|
| 29 | APASSHERASM | lipoma |
| 30 | ASRQAPPHI | lipoma |
| 31 | AVKKNPGIAA | lipoma |
| 32 | EEHLESHKKY | lipoma |
| 33 | GEFTSARAV | lipoma |
| 34 | GQSTPRLFSI | lipoma |
| 35 | LVDDPLEY | lipoma |
| 36 | RPKNLMQTL | lipoma |
| 37 | RQAPPHIEL | lipoma |
| 38 | SEAAELRSTA | lipoma |
| 39 | AAVRIGSVL | colon, adenoma |
| 40 | ERAGVVREL | colon, adenoma |
| 41 | GAAVRIGSVL | colon, adenoma |
| 42 | KLYELHVFTF | colon, adenoma |
| 43 | LYELHVFTF | colon, adenoma |
| 44 | YLNKEIEEA | colon, adenoma |
| 45 | DELPKFHQY | stomach, adenocarcinoma, white blood cell s, chronic lymphocytic leukemia |
| 46 | DVTGQFPSSF | stomach, adenocarcinoma, white blood cell s, chronic lymphocytic leukemia |
| 47 | EHSRVLQQL | stomach, adenocarcinoma, white blood cell s, chronic lymphocytic leukemia |
| 48 | IKVSKQLL | stomach, adenocarcinoma, white blood cell s, chronic lymphocytic leukemia |
| 49 | KPRQSSPQL | stomach, adenocarcinoma, white blood cell s, chronic lymphocytic leukemia |
| 50 | KQLLAALEI | stomach, adenocarcinoma, white blood cell s, chronic lymphocytic leukemia |
| 51 | RRKDLVLKY | stomach, adenocarcinoma, liver, focal nodular hyperplasia |
| 52 | RTRDYASLPPK | stomach, adenocarcinoma, white blood cell s, chronic lymphocytic leukemia |
| 53 | APGSVLPRAL | stomach, adenocarcinoma, lymph node, Hodgkin's disease |
| 54 | DIKEHPLL | stomach, adenocarcinoma, lymph node, Hodgkin's disease |
| 55 | DSAGPQDAR | stomach, adenocarcinoma, lymph node, Hodgkin's disease |
| 56 | FQYAKESYI | stomach, adenocarcinoma, lymph node, Hodgkin's disease |
| 57 | KVLSWPFLM | stomach, adenocarcinoma, lymph node, Hodgkin's disease |
| 58 | LENDQSLSF | stomach, adenocarcinoma, lymph node, Hodgkin's disease |
| 59 | SPSRQPQV | stomach, adenocarcinoma, lymph node, Hodgkin's disease |
| 60 | SRHQSFTTK | stomach, adenocarcinoma, lymph node, Hodgkin's disease |
| 61 | SSHNASKTL | stomach, adenocarcinoma, lymph node, Hodgkin's disease |
| 62 | EEIDTTMRW | liver, hepatocellular carcinoma, lipoma |

(continued)

| Seq ID | Sequence | Tissue and disease |
|---|---|---|
| 63 | ILDEKPVII | liver, hepatocellular carcinoma, lipoma |
| 64 | LPQEPRTSL | liver, hepatocellular carcinoma, lipoma |
| 65 | LTYKLPVA | liver, hepatocellular carcinoma, lipoma |
| 66 | NEMELAHSSF | liver, hepatocellular carcinoma, lipoma |
| 67 | REFPEANFEL | liver, hepatocellular carcinoma, lipoma |
| 68 | THHIPDAKL | liver, hepatocellular carcinoma, lipoma |
| 69 | TVKENLSLF | liver, hepatocellular carcinoma, lipoma |
| 70 | VLLKKAVL | liver, hepatocellular carcinoma, lipoma |
| 71 | HLKSIPVSL | kidney, clear cell renal cell carcinoma |
| 72 | KVWYNVENW | kidney, clear cell renal cell carcinoma, prostate, adenocarcinoma |
| 73 | LPAYRAQLL | kidney, clear cell renal cell carcinoma, prostate, adenocarcinoma |
| 74 | LSEQTSVPL | kidney, clear cell renal cell carcinoma, prostate, adenocarcinoma |
| 75 | SLNQWLVSF | kidney, clear cell renal cell carcinoma, prostate, adenocarcinoma |
| 76 | SMTSLAQKI | kidney, clear cell renal cell carcinoma, prostate, adenocarcinoma |
| 77 | SSSGLHPPK | kidney, clear cell renal cell carcinoma, prostate, adenocarcinoma |
| 78 | DLDVKKMPL | stomach, metastatic, kidney, carcinoma |
| 79 | FYTVIPHNF | stomach, metastatic, kidney, carcinoma |
| 80 | HHINTDNPSL | stomach, metastatic, kidney, carcinoma |
| 81 | RVGEVGQSK | stomach, metastatic, kidney, carcinoma |
| 82 | AVFDGAQVTSK | lung, non-small cell lung carcinoma, kidney, oncocytoma |
| 83 | SQTDLVSRL | lung, non-small cell lung carcinoma, kidney, oncocytoma |
| 84 | VPVPHTTAL | kidney, clear cell renal cell carcinoma, endometrium, adenocarcinoma, endometrioid type |
| 85 | YQVLDVQRY | kidney, clear cell renal cell carcinoma, endometrium, adenocarcinoma, endometrioid type |
| 86 | APFQGDQRSL | colon or rectum, breast, mucinous carcinoma |
| 87 | DVAEPYKVY | colon or rectum, breast, mucinous carcinoma |
| 88 | IVSGQPGTQK | colon or rectum, breast, mucinous carcinoma |
| 89 | TPEQQAAIL | colon or rectum, breast, mucinous carcinoma |
| 90 | VELFRTAYF | colon or rectum, breast, mucinous carcinoma |
| 91 | EHADDDPSL | brain, cancer, kidney, Wilm's tumor |
| 92 | SEESVKSTTL | brain, cancer, kidney, Wilm's tumor |
| 93 | SPRPPLGSSL | brain, cancer, kidney, Wilm's tumor |
| 94 | SPWWRSSL | brain, cancer, kidney, Wilm's tumor |
| 95 | VYTPVDSLVF | brain, cancer, kidney, Wilm's tumor pancreas, adenocarcinoma, kidney, renal |
| 96 | APLQRSQSL | cell carcinoma |
| 97 | DEVHQDTY | pancreas, adenocarcinoma, kidney, renal cell carcinoma |
| 98 | LPHSATVTL | pancreas, adenocarcinoma, kidney, renal cell carcinoma |

(continued)

| Seq ID | Sequence | Tissue and disease |
|---|---|---|
| 99 | SEAPEAPLL | testis, seminoma |
| 100 | SPRASGSGL | testis, seminoma |
| 101 | VVGPAAEAK | testis, seminoma |
| 102 | FSITKSVEL | non-Hodgkin's lymphoma, small lymphocytic type |
| 103 | GQTKNDLVV | non-Hodgkin's lymphoma, small lymphocytic type |
| 104 | LSQEVCRD | non-Hodgkin's lymphoma, small lymphocytic type |
| 105 | RDIQSPEQI | non-Hodgkin's lymphoma, small lymphocytic type |
| 106 | REDNSSNSL | non-Hodgkin's lymphoma, small lymphocytic type |
| 107 | TEHQEPGL | non-Hodgkin's lymphoma, small lymphocytic type |
| 108 | TKNDLVVSL | non-Hodgkin's lymphoma, small lymphocytic type |
| 109 | AEEAGGTRL | breast, carcinoma |
| 110 | ENVNKKDY | breast, carcinoma |
| 111 | GLDPNKPPEL | breast, carcinoma |
| 112 | RPAGEPYNRKTL | breast, carcinoma |
| 113 | SASVQRADTSL | liver, hepatocellular carcinoma, adrenal gland, adrenal cortical adenoma |
| 114 | YGNPRTNGM | stomach, metastatic, breast, carcinoma |
| 115 | LIRPVSASF | esophagus, adenocarcinoma |
| 116 | SPVNSSKQPSY | esophagus, adenocarcinoma |
| 117 | QLFSYAILGF | liver, hepatocellular carcinoma, colon, non-Hodgkin's lymphoma |
| 118 | DEHLLIQHY | liver, hepatocellular carcinoma, parotid gland, pleomorphic adenoma |
| 119 | KQVASSTGF | liver, hepatocellular carcinoma, parotid gland, pleomorphic adenoma |
| 120 | RDFGPASQHFL | liver, hepatocellular carcinoma, parotid gland, pleomorphic adenoma |
| 121 | RQLGEVASF | liver, hepatocellular carcinoma, parotid gland, pleomorphic adenoma |
| 122 | TEAETTANVL | liver, hepatocellular carcinoma, parotid gland, pleomorphic adenoma |
| 123 | GYLPVQTVL | kidney, clear cell renal cell carcinoma, parotid gland, pleomorphic adenoma |
| 124 | GQKEALLKY | liver, hepatocellular carcinoma, synovial sarcoma |
| 125 | KPSEERKTI | liver, hepatocellular carcinoma, synovial sarcoma |
| 126 | KQTPKVLVV | liver, hepatocellular carcinoma, synovial sarcoma |
| 127 | SVIQHVQSF | liver, hepatocellular carcinoma, synovial sarcoma |
| 128 | TPIERIPYL | liver, hepatocellular carcinoma, synovial sarcoma |
| 129 | AEVEKNETV | kidney, clear cell renal cell carcinoma, spleen, non-Hodgkin's lymphoma |
| 130 | EVKEEIPLV | kidney, clear cell renal cell carcinoma, spleen, non-Hodgkin's lymphoma |
| 131 | KPTSARSGL | kidney, clear cell renal cell carcinoma, spleen, non-Hodgkin's lymphoma |

(continued)

| Seq ID | Sequence | Tissue and disease |
|--------|----------|--------------------|
| 132 | KYIETTPLTI | kidney, clear cell renal cell carcinoma, spleen, non-Hodgkin's lymphoma |
| 133 | SEIKTSIEV | kidney, clear cell renal cell carcinoma, spleen, non-Hodgkin's lymphoma |
| 134 | SVKPTSATK | kidney, clear cell renal cell carcinoma, spleen, non-Hodgkin's lymphoma |
| 135 | YPNKGVGQA | kidney, clear cell renal cell carcinoma, spleen, non-Hodgkin's lymphoma |
| 136 | ISMKILNSL | lung, non-small cell lung carcinoma, thymus, thymoma, benign |
| 137 | KTIAFLLPMF | lung, non-small cell lung carcinoma, thymus, thymoma, benign |
| 138 | RDSIINDF | lung, non-small cell lung carcinoma, thymus, thymoma, benign |
| 139 | SVKGGGGNEK | lung, non-small cell lung carcinoma, thymus, thymoma, benign |
| 140 | GIAKTGSGK | lung, non-small cell lung carcinoma, thymus, thymoma, benign |
| 141 | AETTDNVFTL | kidney, clear cell renal cell carcinoma, thyroid gland, follicular adenoma |
| 142 | SEYQRFAVM | kidney, clear cell renal cell carcinoma, thyroid gland, follicular adenoma |
| 143 | TFGERVVAF | kidney, clear cell renal cell carcinoma, thyroid gland, follicular adenoma |
| 144 | NENLVERF | stomach, adenocarcinoma, colon, adenocarcinoma |
| 145 | KITVPASQK | stomach, adenocarcinoma, colon, non-Hodgkin's lymphoma |
| 146 | KITVPASQKL | stomach, adenocarcinoma, colon, non-Hodgkin's lymphoma |
| 147 | VPASQKLRQL | stomach, adenocarcinoma, colon, non-Hodgkin's lymphoma |
| 148 | HVGYTLSYK | stomach, adenocarcinoma |
| 149 | KLPLPLPPRL | stomach, adenocarcinoma |
| 150 | KPIEPRREL | stomach, adenocarcinoma |
| 151 | SHSHVGYTL | stomach, adenocarcinoma |
| 152 | APSEYRYTL | colon or rectum, stomach, mucinous adenocarcinoma |
| 153 | APSEYRYTLL | colon or rectum, stomach, mucinous adenocarcinoma |
| 154 | EIFQNEVAR | colon or rectum, stomach, mucinous adenocarcinoma |
| 155 | KDVLIPGKL | colon or rectum, stomach, mucinous adenocarcinoma |
| 156 | VPLVREITF | colon or rectum, stomach, mucinous adenocarcinoma |
| 157 | DPNPNFEKF | liver, hepatocellular carcinoma, cancer, liver, focal nodular hyperplasia |
| 158 | IQAPLSWEL | liver, hepatocellular carcinoma, cancer, liver, focal nodular hyperplasia |
| 159 | VIYNEQMASK | liver, hepatocellular carcinoma, cancer, liver, focal nodular hyperplasia |

(continued)

| Seq ID | Sequence | Tissue and disease |
|---|---|---|
| 160 | VLRPGGAFY | liver, hepatocellular carcinoma, cancer, liver, focal nodular hyperplasia |
| 161 | EDPDQDILI | stomach, adenocarcinoma, endometrium, adenocarcinoma, endometrioid |
| 162 | HGNLRELAL | stomach, adenocarcinoma, endometrium, adenocarcinoma, endometrioid |
| 163 | KLYPTLVIR | stomach, adenocarcinoma, endometrium, adenocarcinoma, endometrioid |
| 164 | SEETFRFEL | stomach, adenocarcinoma, endometrium, adenocarcinoma, endometrioid |
| 165 | ELNKLLEEI | stomach, adenocarcinoma, ovary, adenocarcinoma, endometrioid |
| 166 | IPFSNPRVL | stomach, adenocarcinoma, ovary, adenocarcinoma, endometrioid |
| 167 | LLDEGAKLLY | stomach, adenocarcinoma, ovary, adenocarcinoma, endometrioid |
| 168 | SPADAHRNL | stomach, adenocarcinoma, ovary, adenocarcinoma, endometrioid |
| 173 | APRKGNTL | stomach, metastatic, endometrium, Mullerian mixed tumor |
| 174 | EEEEALQKKF | stomach, metastatic, endometrium, Mullerian mixed tumor |
| 175 | KENLVDGF | stomach, metastatic, endometrium, Mullerian mixed tumor |
| 176 | VYKENLVDGF | stomach, metastatic, endometrium, Mullerian mixed tumor |
| 177 | TLLVVVPKL | stomach, adenocarcinoma, bone, giant cell tumor of bone |
| 178 | HEIDRYTAI | kidney, clear cell renal cell carcinoma, non-Hodgkin's lymphoma |
| 179 | VFTLKPLEF | kidney, clear cell renal cell carcinoma, non-Hodgkin's lymphoma |
| 180 | YWVPRNAL | kidney, clear cell renal cell carcinoma, non-Hodgkin's lymphoma |
| 181 | IGVEHVVVY | brain, cancer, kidney, oncocytoma |
| 182 | RDKPHVNV | brain, cancer, omentum, leiomyosarcoma |
| 183 | ADVLKVEVF | stomach, adenocarcinoma, colon, adenocarcinoma |
| 184 | IPVVHASI | stomach, adenocarcinoma, colon, adenocarcinoma |
| 185 | RDSLIDSLT | stomach, adenocarcinoma, colon, adenocarcinoma |
| 186 | TVADQVLVGSY | stomach, adenocarcinoma, colon, adenocarcinoma |
| 187 | AADTERLAL | lung, non-small cell lung carcinoma, chondrosarcoma |
| 188 | DMKAKVASL | lung, non-small cell lung carcinoma, chondrosarcoma |
| 189 | HVLEEVQQV | lung, non-small cell lung carcinoma, chondrosarcoma |
| 190 | KEAADTERL | lung, non-small cell lung carcinoma, chondrosarcoma |

(continued)

| Seq ID | Sequence | Tissue and disease |
|---|---|---|
| 191 | RISEVLQKL | lung, non-small cell lung carcinoma, chondrosarcoma |
| 192 | TEVRELVSL | lung, non-small cell lung carcinoma, chondrosarcoma |
| 193 | AIRSGEAAAK | liver, hepatocellular carcinoma, pleura, malignant mesothelioma |
| 194 | APNPAPKEL | liver, hepatocellular carcinoma, pleura, malignant mesothelioma |
| 195 | RQSLLTAI | liver, hepatocellular carcinoma, liver, hepatocellular carcinoma, cancer, pleura, malignant mesothelioma |
| 196 | SPEQTLSPL | liver, hepatocellular carcinoma, pleura, malignant mesothelioma |
| 197 | TEHQVPSSV | liver, hepatocellular carcinoma, liver, hepatocellular carcinoma, cancer, pleura, malignant mesothelioma |
| 198 | TTYKIVPPK | liver, hepatocellular carcinoma, liver, hepatocellular carcinoma, cancer, pleura, malignant mesothelioma |
| 199 | QLLDQVEQI | stomach, metastatic thyroid gland, papillary carcinoma |
| 200 | DETMVIGNY | stomach, metastatic, rectum, adenocarcinoma |
| 201 | RQYGSEGRFTF | kidney, clear cell renal cell carcinoma, rectum, adenocarcinoma |
| 203 | GPRPITQSEL | stomach, metastatic, lymph node, non-Hodgkin's lymphoma |
| 204 | KPEPVDKVA | stomach, metastatic, lymph node, non-Hodgkin's lymphoma |
| 205 | TPSSRPASL | stomach, metastatic, lymph node, non-Hodgkin's lymphoma |
| 212 | GRLNSVNNR | kidney, clear cell renal cell carcinoma, leiomyosarcoma |
| 213 | SILEDPPSI | kidney, clear cell renal cell carcinoma, leiomyosarcoma |
| 214 | TPRTNNIEL | kidney, clear cell renal cell carcinoma, leiomyosarcoma |
| 215 | DAMKRVEEI | stomach, adenocarcinoma, ovary, thecoma-fibroma |
| 216 | DIKEVKQNI | stomach, adenocarcinoma, ovary, thecoma-fibroma |
| 217 | GPIYPGHGM | stomach, adenocarcinoma, ovary, thecoma-fibroma |
| 218 | GDYGRAFNL | stomach, metastatic, lymph node, non-Hodgkin's lymphoma |
| 219 | TRHKIVHTK | stomach, metastatic, lymph node, non-Hodgkin's lymphoma |
| 220 | RIHTGEKPYK | colon or rectum, thyroid gland, nodular hyperplasia |
| 221 | KAFNWFSTL | stomach, metastatic, lymph node, non-Hodgkin's lymphoma |
| 222 | QSTQRSLAL | liver, hepatocellular carcinoma, uterin cervix, squamous cell carcinoma |

33

(continued)

| Seq ID | Sequence | Tissue and disease |
|---|---|---|
| 223 | RDLQMNQALRF | liver, hepatocellular carcinoma, uterin cervix, squamous cell carcinoma |
| 224 | RELESQLHVL | liver, hepatocellular carcinoma, uterin cervix, squamous cell carcinoma |
| 225 | SEAEKLTLV | liver, hepatocellular carcinoma, uterin cervix, squamous cell carcinoma |
| 226 | AAAKPVATK | pancreas, adenocarcinoma, fibromatosis |
| 227 | ATYHGSFSTK | pancreas, adenocarcinoma, fibromatosis |
| 228 | FMYDRPLRL | pancreas, adenocarcinoma, fibromatosis |
| 229 | FRVGNVQEL | pancreas, adenocarcinoma, fibromatosis |
| 230 | GVAPFTIAR | pancreas, adenocarcinoma, fibromatosis |
| 231 | KMKPLDGSALY | pancreas, adenocarcinoma, fibromatosis |
| 232 | KPAPAKPVA | pancreas, adenocarcinoma, fibromatosis |
| 233 | KPVAAKPAA | pancreas, adenocarcinoma, fibromatosis |
| 234 | KQFGVAPFTI | pancreas, adenocarcinoma, fibromatosis |
| 235 | QEELVKISL | pancreas, adenocarcinoma, fibromatosis |
| 236 | RQLGTVQQVI | pancreas, adenocarcinoma, fibromatosis |
| 237 | RQLINALQI | pancreas, adenocarcinoma, fibromatosis |
| 238 | RVIGGLLAGQTY | pancreas, adenocarcinoma, fibromatosis |
| 239 | SENAFYLSP | pancreas, adenocarcinoma, fibromatosis |
| 240 | SQAPVLDAI | pancreas, adenocarcinoma, fibromatosis |
| 241 | STRYPPPAV | pancreas, adenocarcinoma, fibromatosis |
| 242 | TEDTLKVYL | pancreas, adenocarcinoma, fibromatosis |
| 243 | VAAKPVATK | pancreas, adenocarcinoma, fibromatosis |
| 244 | VQRVVESL | pancreas, adenocarcinoma, fibromatosis |
| 245 | VRNPSVVVK | pancreas, adenocarcinoma, fibromatosis |
| 246 | GESEVAIKI | myometrium, leiomyoma |
| 247 | LIYSVGLLLA | myometrium, leiomyoma |
| 248 | SAYPHQLSF | myometrium, leiomyoma |
| 249 | SVIGVFITK | myometrium, leiomyoma |
| 250 | AELGNSVQLI | liver, hepatocellular carcinoma, thyroid gland, nodular hyperplasia |
| 251 | ANMTVTRI | liver, hepatocellular carcinoma, thyroid gland, nodular hyperplasia |
| 252 | ARISNVEFY | liver, hepatocellular carcinoma, thyroid gland, nodular hyperplasia |
| 253 | AVFIGNQQF | liver, hepatocellular carcinoma, thyroid gland, nodular hyperplasia |
| 254 | DIELQAENI | liver, hepatocellular carcinoma, thyroid gland, nodular hyperplasia |
| 255 | DSYTVRVSV | liver, hepatocellular carcinoma, thyroid gland, nodular hyperplasia |
| 256 | DVKIFVNTI | liver, hepatocellular carcinoma, thyroid gland, nodular hyperplasia |
| 257 | EIIPKYGSI | liver, hepatocellular carcinoma, thyroid gland, nodular hyperplasia |
| 258 | EQSKIFIHR | liver, hepatocellular carcinoma, thyroid gland, nodular hyperplasia |
| 259 | FVDVGLYQY | liver, hepatocellular carcinoma, thyroid gland, nodular hyperplasia |

(continued)

| Seq ID | Sequence | Tissue and disease |
|--------|----------|--------------------|
| 260 | GHTSTISTL | liver, hepatocellular carcinoma, thyroid gland, nodular hyperplasia |
| 261 | GRIEYVEVF | liver, hepatocellular carcinoma, thyroid gland, nodular hyperplasia |
| 262 | GTSIIPFQK | liver, hepatocellular carcinoma, thyroid gland, nodular hyperplasia |
| 263 | HPFLRGIGY | liver, hepatocellular carcinoma, thyroid gland, nodular hyperplasia |
| 264 | IPVEIHTA | liver, hepatocellular carcinoma, thyroid gland, nodular hyperplasia |
| 265 | KIFVNTIAY | liver, hepatocellular carcinoma, thyroid gland, nodular hyperplasia |
| 266 | LPEDKVRIAY | liver, hepatocellular carcinoma, thyroid gland, nodular hyperplasia |
| 267 | LPFSEGLTV | liver, hepatocellular carcinoma, thyroid gland, nodular hyperplasia |
| 268 | LPWANKVTI | liver, hepatocellular carcinoma, thyroid gland, nodular hyperplasia |
| 269 | PWANKVTI | liver, hepatocellular carcinoma, thyroid gland, nodular hyperplasia |
| 270 | QAYNRAVTI | liver, hepatocellular carcinoma, thyroid gland, nodular hyperplasia |
| 271 | RSFPQKMAY | liver, hepatocellular carcinoma, thyroid gland, nodular hyperplasia |
| 272 | RYPIHWHLL | liver, hepatocellular carcinoma, thyroid gland, nodular hyperplasia |
| 273 | SPQNLRLML | liver, hepatocellular carcinoma, thyroid gland, nodular hyperplasia |
| 274 | SYFSSPTQR | liver, hepatocellular carcinoma, thyroid gland, nodular hyperplasia |
| 275 | VQIKSSLI | liver, hepatocellular carcinoma, thyroid gland, nodular hyperplasia |
| 276 | VYIGHTSTI | liver, hepatocellular carcinoma, thyroid gland, nodular hyperplasia |
| 277 | YHVPGTGESY | liver, hepatocellular carcinoma, thyroid gland, nodular hyperplasia |
| 278 | ATNGDLASR | pancreas, adenocarcinoma, prostate, benign nodular hyperplasia |
| 279 | GLHAEVTGVGY | pancreas, adenocarcinoma, prostate, benign nodular hyperplasia |
| 280 | HVSSTSSSF | pancreas, adenocarcinoma, prostate, benign nodular hyperplasia |
| 281 | LQADLQNGL | pancreas, adenocarcinoma, prostate, benign nodular hyperplasia |
| 282 | SELPVSEVA | pancreas, adenocarcinoma, prostate, benign nodular hyperplasia |
| 283 | SQTKSVFEI | pancreas, adenocarcinoma, prostate, benign nodular hyperplasia |
| 284 | THIFTSDGL | pancreas, adenocarcinoma, prostate, benign nodular hyperplasia |
| 285 | VIYFPPLQK | pancreas, adenocarcinoma, prostate, benign nodular hyperplasia |

(continued)

| Seq ID | Sequence | Tissue and disease |
|---|---|---|
| 286 | YPFSSEQKW | pancreas, adenocarcinoma, prostate, benign nodular hyperplasia |
| 287 | GQYFGELAL | stomach, gastrointestinal stromal tumor (GIST) |
| 288 | RIIVKNNAK | stomach, gastrointestinal stromal tumor (GIST) |
| 289 | RRIIVKNNAK | stomach, gastrointestinal stromal tumor (GIST) |
| 290 | SFGELALMY | stomach, gastrointestinal stromal tumor (GIST) |
| 291 | AFNAPVINR | stomach, gastrointestinal stromal tumor (GIST) |
| 292 | IMKRNIATY | stomach, gastrointestinal stromal tumor (GIST) |
| 293 | KVVDVIGTK | stomach, gastrointestinal stromal tumor (GIST) |
| 294 | LPFLKSLEF | stomach, gastrointestinal stromal tumor (GIST) |
| 295 | RLKVVDVIGTK | stomach, gastrointestinal stromal tumor (GIST) |
| 296 | TPRAATITA | stomach, gastrointestinal stromal tumor (GIST) |
| 297 | KPSEKIQVL | lipoma |
| 298 | VPYPVTTTV | lipoma |
| 299 | ASFPPFVEK | lipoma |
| 300 | AFIHISTAY | colon or rectum, colon, adenocarcinoma |
| 301 | ATFEKIPFER | colon or rectum, colon, adenocarcinoma |
| 302 | KLFEKVKEV | colon or rectum, colon, adenocarcinoma |
| 303 | SQMPKLEAF | colon or rectum, colon, adenocarcinoma |
| 304 | AVLGQHHNY | colon or rectum, colon, adenocarcinoma |
| 305 | GPPAHKPR | spleen, chronic myeloid leukemia |
| 306 | RVYDVLVLK | colon or rectum, colon, adenocarcinoma |
| 307 | LPRPQGITV | liver, hepatocellular carcinoma, liver, focal nodular hyperplasia |
| 308 | VLYVGSKTK | brain, glioblastoma, schwannoma |
| 309 | KTKEQVTNV | brain, glioblastoma, schwannoma |
| 310 | MPVDPDNEAY | brain, glioblastoma, schwannoma |
| 311 | AEKTKQGVA | brain, glioblastoma, schwannoma |
| 312 | DIADFFTTR | kidney, clear cell renal cell carcinoma, adrenal gland, adrenal cortical adenoma |
| 313 | HSYLQRQSV | kidney, clear cell renal cell carcinoma, adrenal gland, adrenal cortical adenoma |
| 314 | KEVTLIEEL | kidney, clear cell renal cell carcinoma, adrenal gland, adrenal cortical adenoma |
| 315 | REDGPGVAL | kidney, clear cell renal cell carcinoma, adrenal gland, adrenal cortical adenoma |
| 316 | REDPLPPGL | kidney, clear cell renal cell carcinoma, adrenal gland, adrenal cortical adenoma |
| 317 | SLFGGSQGLRK | kidney, clear cell renal cell carcinoma, adrenal gland, adrenal cortical adenoma |
| 318 | AEFQRLKQA | intramuscular lipoma |
| 319 | EVIDGVPGKW | intramuscular lipoma |
| 320 | IPKAPGKII | intramuscular lipoma |
| 321 | SHNGSAIRY | intramuscular lipoma |
| 322 | TEVTVVGDKL | intramuscular lipoma |
| 323 | YASVVVKRY | intramuscular lipoma |
| 324 | ATDLALYIK | stomach, adenocarcinoma, thyroid gland, papillary carcinoma |
| 325 | AYHNWRHAF | stomach, adenocarcinoma, thyroid gland, papillary carcinoma |

(continued)

| Seq ID | Sequence | Tissue and disease |
|--------|----------|--------------------|
| 326 | EPLNIKDAY | stomach, adenocarcinoma, thyroid gland, papillary carcinoma |
| 327 | KIAATIISF | stomach, adenocarcinoma, thyroid gland, papillary carcinoma |
| 328 | KIFLHIHGL | stomach, adenocarcinoma, thyroid gland, papillary carcinoma |
| 329 | LEVILKKI | stomach, adenocarcinoma, thyroid gland, papillary carcinoma |
| 330 | SEHPLAQLY | stomach, adenocarcinoma, thyroid gland, papillary carcinoma |
| 331 | VPSAQTLKI | stomach, adenocarcinoma, thyroid gland, papillary carcinoma |
| 332 | AEYRSYVA | stomach, metastatic adrenal gland, adrenal cortical carcinoma |
| 333 | ALAPGRGTLY | stomach, metastatic adrenal gland, adrenal cortical carcinoma |
| 334 | GPRGTQAAL | stomach, metastatic adrenal gland, adrenal cortical carcinoma |
| 335 | IEDPGTLHI | stomach, metastatic adrenal gland, adrenal cortical carcinoma |
| 336 | IEDPGTLHIW | stomach, metastatic adrenal gland, adrenal cortical carcinoma |
| 337 | RPIPIAVKY | stomach, metastatic adrenal gland, adrenal cortical carcinoma |
| 338 | VEKLLTNW | stomach, metastatic, pancreas, adenocarcinoma |
| 339 | FLDPDIGGVAV | kidney, clear cell renal cell carcinoma, pancreas, adenocarcinoma |
| 340 | HTAPPENKTW | kidney, clear cell renal cell carcinoma, pancreas, adenocarcinoma |
| 341 | LLDTPVKTQY | kidney, clear cell renal cell carcinoma, pancreas, adenocarcinoma |
| 342 | NAVKDFTSF | kidney, clear cell renal cell carcinoma, pancreas, adenocarcinoma |
| 343 | SGLLQIKKL | kidney, clear cell renal cell carcinoma, pancreas, adenocarcinoma |
| 344 | YHDKNIVLL | kidney, clear cell renal cell carcinoma, pancreas, adenocarcinoma |
| 345 | SVDPKNYPK | pancreas, adenocarcinoma, colon, adenocarcinoma |
| 346 | AVGLVLPAK | liver, hepatocellular carcinoma, cancer, thyroid gland, papillary carcinoma |
| 347 | AVGLVLPAKL | liver, hepatocellular carcinoma, cancer, thyroid gland, papillary carcinoma |
| 348 | ALLEVLSQK | stomach, adenocarcinoma, breast, carcinoma |
| 349 | HEKQDTLVA | kidney, clear cell renal cell carcinoma, spleen, chronic myeloid leukemia |
| 350 | KELELQIGM | kidney, clear cell renal cell carcinoma, spleen, chronic myeloid leukemia |
| 351 | MYSDVWKQL | kidney, clear cell renal cell carcinoma, spleen, chronic myeloid leukemia |
| 352 | RELQDEKAEL | kidney, clear cell renal cell carcinoma, spleen, chronic myeloid leukemia |

(continued)

| Seq ID | Sequence | Tissue and disease |
|--------|----------|--------------------|
| 353 | RITDVLDQK | kidney, clear cell renal cell carcinoma, spleen, chronic myeloid leukemia |
| 354 | EVIKITGLK | stomach, adenocarcinoma |
| 355 | HHVDITKKL | stomach, adenocarcinoma, kidney, carcinoma |
| 356 | LPFNVKVSV | stomach, adenocarcinoma, stomach, gastrointestinal stromal tumor (GIST) |
| 357 | TLPRVLEI | stomach, adenocarcinoma, bone, giant cell tumor of bone |
| 358 | TVDLPKSPK | stomach, adenocarcinoma, thyroid gland, nodular hyperplasia |
| 359 | AEHGLLLTA | stomach, metastatic, uterin cervix, adenocarcinoma |
| 360 | AQAGALLQV | stomach, metastatic, uterin cervix, adenocarcinoma |
| 361 | DGGFVLKV | stomach, metastatic, uterin cervix, adenocarcinoma |
| 362 | IVYPSGKVY | stomach, metastatic, uterin cervix, adenocarcinoma |
| 363 | KLDNQVSKV | colon or rectum, prostate, benign nodular hyperplasia |
| 364 | SENVKLFSA | colon or rectum, prostate, benign nodular hyperplasia |
| 365 | VQKLQNII | colon or rectum, prostate, benign nodular hyperplasia |
| 366 | FSTPHGLEV | pancreas, adenocarcinoma, spleen, chronic myeloid leukemia |
| 367 | KRFHQKSDM | pancreas, adenocarcinoma, spleen, chronic myeloid leukemia |
| 368 | KTFGHAVSL | pancreas, adenocarcinoma, spleen, chronic myeloid leukemia |
| 369 | SSNLITHSR | pancreas, adenocarcinoma, spleen, chronic myeloid leukemia |
| 370 | GVIDGHIYAV | stomach, metastatic, leiomyosarcoma |
| 371 | IEPAKETTTNV | pancreas, adenocarcinoma, lung, adenocarcinoma |
| 372 | NAPPSEVLL | pancreas, adenocarcinoma, lung, adenocarcinoma |
| 373 | SIEPAKETTTNV | pancreas, adenocarcinoma, lung, adenocarcinoma |
| 374 | AQSQHNQSL | spleen, extramedullary hematopoiesis |
| 375 | AQSRTNPQV | spleen, extramedullary hematopoiesis |
| 376 | KMHDKVFAY | spleen, extramedullary hematopoiesis |
| 377 | TAKAPLSTV | spleen, extramedullary hematopoiesis |
| 378 | IPTRTVAI | liver, hepatocellular carcinoma, lipoma |
| 379 | NHDRKHAV | liver, hepatocellular carcinoma, lipoma |
| 380 | NNHDRKHAV | liver, hepatocellular carcinoma, lipoma |
| 381 | TPGGTRIIY | liver, hepatocellular carcinoma, breast, carcinoma |
| 382 | EHWPSPETF | bone, non-ossifying fibroma |
| 383 | EIITNTLSF | bone, non-ossifying fibroma |
| 384 | EVRGALMSAF | bone, non-ossifying fibroma |

(continued)

| Seq ID | Sequence | Tissue and disease |
|---|---|---|
| 385 | IPRPILVLL | bone, non-ossifying fibroma |
| 386 | LPNKNRDEL | bone, non-ossifying fibroma |
| 387 | QRIPAGAVL | bone, non-ossifying fibroma |
| 388 | AEGPAGGFMVV | pancreas, adenocarcinoma, spleen, chronic myeloid leukemia |
| 389 | AYYRDAEAY | pancreas, adenocarcinoma, spleen, chronic myeloid leukemia |
| 390 | QVNRPLTMR | pancreas, adenocarcinoma, spleen, chronic myeloid leukemia |
| 391 | RHSPVFQVY | pancreas, adenocarcinoma, spleen, chronic myeloid leukemia |
| 392 | SLPVPNSAY | pancreas, adenocarcinoma, spleen, chronic myeloid leukemia |
| 393 | TLGPPGTAHLY | pancreas, adenocarcinoma, spleen, chronic myeloid leukemia |
| 394 | IEPAKETTTNV | pancreas, adenocarcinoma, lung, adenocarcinoma |
| 395 | NAPPSEVLL | pancreas, adenocarcinoma, lung, adenocarcinoma |
| 396 | SIEPAKETTTNV | pancreas, adenocarcinoma, lung, adenocarcinoma |
| 397 | DLYSGLNQR | lymph node, Hodgkin's disease |
| 398 | KAKAKPVTR | lymph node, Hodgkin's disease |
| 399 | AVLDKAMKAK | liver, hepatocellular carcinoma, liver, hepatic adenoma |
| 400 | LELSTPLKI | liver, hepatocellular carcinoma, liver, hepatic adenoma |
| 401 | LPLNLDTKY | liver, hepatocellular carcinoma, liver, hepatic adenoma |
| 402 | TVIYRIQAL | liver, hepatocellular carcinoma, liver, hepatic adenoma |
| 403 | DAHIYLNHI | stomach, adenocarcinoma, pancreas, microcystic adenoma |
| 404 | NHIEPLKIQL | stomach, adenocarcinoma, pancreas, microcystic adenoma |
| 405 | AYRPAVHPR | thyroid gland, nodular hyperplasia |
| 406 | LRAPLEHEL | thyroid gland, nodular hyperplasia |
| 407 | RLFMVLLLK | thyroid gland, nodular hyperplasia |
| 408 | RSPDVLKDF | thyroid gland, nodular hyperplasia |
| 409 | ETAPGVHKR | stomach, metastatic, non-Hodgkin's lymphoma |
| 410 | LYHGYIYTY | stomach, metastatic, non-Hodgkin's lymphoma |
| 415 | VVFDSPRNR | liver, hepatocellular carcinoma, pancreas, adenocarcinoma |
| 416 | YPLGRILI | lung, non-small cell lung carcinoma, pancreas, adenocarcinoma |
| 417 | KEFAEFVTS | pancreas, adenocarcinoma, pancreas, adenocarcinoma |
| 418 | VMLDVPIRL | pancreas, adenocarcinoma, pancreas, adenocarcinoma |
| 419 | VPMTPLRTV | liver, hepatocellular carcinoma, cancer, rectum, adenocarcinoma |
| 420 | QIDYKTLVL | stomach, metastatic, leiomyosarcoma |

(continued)

| Seq ID | Sequence | Tissue and disease |
|---|---|---|
| 421 | VEDPTIVRI | stomach, metastatic, leiomyosarcoma |
| 422 | IPYQDLPHL | kidney, clear cell renal cell carcinoma, lipoma |
| 423 | DTPFLTGHGR | stomach, adenocarcinoma, bone, non-ossifying fibroma |
| 424 | EFYRALYI | stomach, adenocarcinoma, bone, non-ossifying fibroma |
| 425 | RYYPQILTNK | stomach, adenocarcinoma, bone, non-ossifying fibroma |
| 426 | KAYERHVL | intestines, malignant carcinoid tumor |
| 427 | LPSPEFHDY | intestines, malignant carcinoid tumor |
| 428 | SLYAHPIEH | intestines, malignant carcinoid tumor |
| 429 | LVREPGSQA | kidney, clear cell renal cell carcinoma, lymph node, Hodgkin's disease |
| 430 | RLAGPGSEKY | kidney, clear cell renal cell carcinoma, lymph node, Hodgkin's disease |
| 431 | SPGAGRNSVL | kidney, clear cell renal cell carcinoma, lymph node, Hodgkin's disease |
| 432 | SVQSDQGYISR | kidney, clear cell renal cell carcinoma, lymph node, Hodgkin's disease |
| 433 | GVRPPAPSL | liver, hepatocellular carcinoma, kidney, carcinoma |
| 434 | IFSEKPVFV | liver, hepatocellular carcinoma, kidney, carcinoma |
| 435 | KASNLLLGF | liver, hepatocellular carcinoma, kidney, carcinoma |
| 436 | KRYIFADAY | liver, hepatocellular carcinoma, kidney, carcinoma |
| 437 | RNLQLSLPR | liver, hepatocellular carcinoma, kidney, carcinoma |
| 438 | EASEPVALR | brain, glioblastoma, liver, hepatic adenoma |
| 439 | RPKVPDQSV | brain, glioblastoma, liver, hepatic adenoma |
| 440 | VLYENALKL | spleen, extramedullary hematopoiesis |
| 441 | EVLDKSQTNY | liver, hepatocellular carcinoma, endometrium, hyperplasia |
| 442 | MPSPIPAKY | liver, hepatocellular carcinoma, endometrium, hyperplasia |
| 443 | YGIENFTSV | liver, hepatocellular carcinoma, endometrium, hyperplasia |
| 444 | ARAAQVFFL | colon or rectum, kidney, renal cell carcinoma |
| 445 | EHIVPNAEL | colon or rectum, kidney, renal cell carcinoma |
| 446 | EAFEFVKQR | stomach, adenocarcinoma, breast, carcinoma |
| 447 | NHFEGHYQY | stomach, adenocarcinoma, breast, carcinoma |
| 448 | DAYPKNPHL | stomach, adenocarcinoma, liver, hepatocellular carcinoma |
| 449 | DVNIKSTER | stomach, adenocarcinoma, liver, hepatocellular carcinoma |
| 450 | HINSIKSVF | stomach, adenocarcinoma, liver, hepatocellular carcinoma |
| 451 | YESEKVGVA | stomach, adenocarcinoma, liver, hepatocellular carcinoma |
| 452 | ENAPTTVSR | stomach, adenocarcinoma, adrenal gland, adrenal cortical adenoma |

(continued)

| Seq ID | Sequence | Tissue and disease |
|---|---|---|
| 453 | RFPHLLAHTY | stomach, adenocarcinoma, adrenal gland, adrenal cortical adenoma |
| 454 | TLDGSLHAV | stomach, adenocarcinoma, adrenal gland, adrenal cortical adenoma |
| 455 | RTVLKNLSLLK | liver, hepatocellular carcinoma, pancreas, microcystic adenoma |
| 456 | FEAKVQAI | stomach, adenocarcinoma, metastatic adenocarcinoma of stomach |
| 457 | FFEAKVQAI | stomach, adenocarcinoma, metastatic adenocarcinoma of stomach |
| 458 | KELQSTFK | stomach, adenocarcinoma, metastatic adenocarcinoma of stomach |
| 459 | NVSSRFEEEI | stomach, adenocarcinoma, metastatic adenocarcinoma of stomach |
| 460 | EVWNNLGTTK | brain, cancer, lymph node, malignant melanoma |
| 461 | MIFRSGSLI | brain, cancer, lymph node, malignant melanoma |
| 462 | NHALPLPGF | brain, cancer, lymph node, malignant melanoma |
| 463 | ASVFGTMPLK | kidney, polycystic kidney disease |
| 464 | REFPDRLVGY | kidney, polycystic kidney disease |
| 465 | SVFGTMPLK | kidney, polycystic kidney disease |
| 466 | DEMRFVTQI | lung, non-small cell lung carcinoma, testis, mixed germ cell tumor |
| 467 | ETVHFATTQW | lung, non-small cell lung carcinoma, testis, mixed germ cell tumor |
| 468 | LPPPATQI | lung, non-small cell lung carcinoma, testis, mixed germ cell tumor |
| 469 | LARDLYAF | liver, hepatocellular carcinoma, neuroblastoma |
| 470 | LPGIGLSTSL | liver, hepatocellular carcinoma, neuroblastoma |
| 471 | MEVILPML | liver, hepatocellular carcinoma, neuroblastoma |
| 472 | AILDYILAK | stomach, metastatic, lung, neuroendocrine carcinoma (non-small cell type) |
| 473 | KIASQLSKL | stomach, metastatic, lung, neuroendocrine carcinoma (non-small cell type) |
| 474 | KVTSTTTVK | stomach, metastatic, lung, neuroendocrine carcinoma (non-small cell type) |
| 475 | YNTLLPYTF | stomach, metastatic, lung, neuroendocrine carcinoma (non-small cell type) |
| 476 | FLDPRPLTV | pancreas, adenocarcinoma, myometrium, leiomyoma |
| 477 | SAFADRPAF | pancreas, adenocarcinoma, myometrium, leiomyoma |
| 478 | AAVPVIISR | lymph node, papillary carcinoma of thyroid |
| 479 | EEIGKVAAA | lymph node, papillary carcinoma of thyroid |
| 480 | FLKDLVASV | lymph node, papillary carcinoma of thyroid |
| 481 | VIISRALEL | lymph node, papillary carcinoma of thyroid |
| 482 | APRTTGTPRTSL | kidney, oncocytoma |
| 483 | ESVGGSPQTK | kidney, oncocytoma |
| 484 | IPKDKAIL | kidney, oncocytoma |
| 485 | LPAYGRTTL | kidney, oncocytoma |
| 486 | HQAAIVSKI | stomach, adenocarcinoma, kidney, angiomyolipoma |

(continued)

| Seq ID | Sequence | Tissue and disease |
| --- | --- | --- |
| 487 | QAAIVSKI | stomach, adenocarcinoma, kidney, angiomyolipoma |
| 488 | RQKMPEDGL | stomach, adenocarcinoma, kidney, angiomyolipoma |
| 489 | SVQKSSGVK | stomach, adenocarcinoma, kidney, angiomyolipoma |
| 490 | DSIGSTVSSER | stomach, adenocarcinoma |
| 491 | LPYNNKDRDAL | stomach, adenocarcinoma |
| 492 | IYDEIQQEM | colon or rectum, colon, adenoma |
| 493 | AQAKGLIQV | thymus, thymoma, benign |
| 494 | EVSSEIYQW | thymus, thymoma, benign |
| 495 | KWNPVPLSY | thymus, thymoma, benign |
| 496 | NRLLAQQSL | thymus, thymoma, benign |
| 497 | APRPVAVAV | stomach, adenocarcinoma |
| 498 | FYRETVQVGR | stomach, adenocarcinoma |
| 499 | LLAPRPVAV | stomach, adenocarcinoma |
| 500 | GLAALVILK | stomach, adenocarcinoma, neurofibroma |
| 501 | KIQEVFSSY | stomach, adenocarcinoma, neurofibroma |
| 502 | ASLDKFLSH | spleen, chronic myeloid leukemia |
| 503 | ALYATKTLR | colon or rectum, pancreas, microcystic adenoma |
| 504 | MEYVISRI | colon or rectum, pancreas, microcystic adenoma |
| 505 | VPVGRQPII | colon or rectum, pancreas, microcystic adenoma |
| 506 | KLLIGVIAAV | stomach, metastatic, colon, adenocarcinoma |
| 507 | LPSLIKLD | stomach, metastatic, colon, adenocarcinoma |
| 508 | PSLIKLDL | stomach, metastatic, colon, adenocarcinoma |
| 509 | ARNKELIGK | stomach, adenocarcinoma |
| 510 | AVKSNAAAY | stomach, adenocarcinoma |
| 511 | EVIIPHSGW | stomach, adenocarcinoma |
| 512 | SVKEQEAQF | stomach, adenocarcinoma |
| 513 | APRGLEPIAI | liver, hepatocellular carcinoma, liver, focal nodular hyperplasia |
| 514 | GRFGGVITI | liver, hepatocellular carcinoma, liver, focal nodular hyperplasia |
| 518 | AEHIESRTL | kidney, clear cell renal cell carcinoma, liver, focal nodular hyperplasia |
| 519 | DQYPYLKSV | kidney, clear cell renal cell carcinoma, liver, focal nodular hyperplasia |
| 520 | IARNLTQQL | kidney, clear cell renal cell carcinoma, liver, focal nodular hyperplasia |
| 521 | IESRTLAIA | kidney, clear cell renal cell carcinoma, liver, focal nodular hyperplasia |
| 522 | MTSALPIIQK | kidney, clear cell renal cell carcinoma, liver, focal nodular hyperplasia |
| 523 | SLLTSSKGQLQK | kidney, clear cell renal cell carcinoma, liver, focal nodular hyperplasia |
| 524 | TSALPIIQK | kidney, clear cell renal cell carcinoma, liver, focal nodular hyperplasia |
| 525 | VRLGSLSTK | kidney, clear cell renal cell carcinoma, liver, focal nodular hyperplasia |
| 526 | RINEFSISSF | chondrosarcoma |
| 527 | DEKQQHIVY | liver, hepatocellular carcinoma, synovial sarcoma |

(continued)

| Seq ID | Sequence | Tissue and disease |
|---|---|---|
| 528 | DEVYQVTVY | liver, hepatocellular carcinoma, synovial sarcoma |
| 529 | GEISEKAKL | liver, hepatocellular carcinoma, synovial sarcoma |
| 530 | YTMKEVLFY | liver, hepatocellular carcinoma, synovial sarcoma |
| 531 | SQLTTLSFY | lung, non-small cell lung carcinoma, omentum, adenocarcinoma |
| 532 | LEKQLIEL | stomach, adenocarcinoma, rectum, adenocarcinoma |
| 533 | ELTLGEFLK | stomach, metastatic, ovary, Mullerian mixed tumor |
| 534 | LTLGEFLK | stomach, metastatic, ovary, Mullerian mixed tumor |
| 535 | LTLGEFLKL | stomach, metastatic, ovary, Mullerian mixed tumor |
| 536 | TLGEFLKL | stomach, metastatic, ovary, Mullerian mixed tumor |
| 537 | ITARPVLW | non-Hodgkin's lymphoma |
| 538 | KLMSPKLYVW | non-Hodgkin's lymphoma |
| 539 | KVSAVTLAY | non-Hodgkin's lymphoma |
| 540 | VEGSGELFRW | non-Hodgkin's lymphoma |
| 541 | RPKSNIVL | non-Hodgkin's lymphoma |
| 542 | RPKSNIVLL | non-Hodgkin's lymphoma |
| 543 | GEPLSYTRFSLARQ | lung, non-small cell lung carcinoma, lung, adenocarcinoma |
| 544 | GEPLSYTRFSLARQVD | lung, non-small cell lung carcinoma, lung, adenocarcinoma |
| 545 | GEPLSYTRFSLARQVDG | lung, non-small cell lung carcinoma, lung, adenocarcinoma |
| 546 | GGEPLSYTRFSLARQVD | lung, non-small cell lung carcinoma, lung, adenocarcinoma |
| 547 | GGEPLSYTRFSLARQVDG | lung, non-small cell lung carcinoma, lung, adenocarcinoma |
| 548 | NPGGYVAYSKAATVTG | lung, non-small cell lung carcinoma, lung, adenocarcinoma |
| 549 | NPGGYVAYSKAATVTGK | lung, non-small cell lung carcinoma, lung, adenocarcinoma |
| 550 | NPGGYVAYSKAATVTGKL | lung, non-small cell lung carcinoma, lung, adenocarcinoma |
| 551 | NSVIIVDKNGRL | lung, non-small cell lung carcinoma, lung, adenocarcinoma |
| 552 | NSVIIVDKNGRLV | lung, non-small cell lung carcinoma, lung, adenocarcinoma |
| 553 | NSVIIVDKNGRLVY | lung, non-small cell lung carcinoma, lung, adenocarcinoma |
| 554 | RVEYHFLSPYVSPK | lung, non-small cell lung carcinoma, lung, adenocarcinoma |
| 555 | RVEYHFLSPYVSPKE | lung, non-small cell lung carcinoma, lung, adenocarcinoma |
| 556 | RVEYHFLSPYVSPKESPF | lung, non-small cell lung carcinoma, lung, adenocarcinoma |
| 557 | SPFRHVFWGSGSHTL | lung, non-small cell lung carcinoma, lung, adenocarcinoma |

(continued)

| Seq ID | Sequence | Tissue and disease |
|---|---|---|
| 558 | SVIIVDKNGRLV | lung, non-small cell lung carcinoma, lung, adenocarcinoma |
| 559 | VEYHFLSPYVSPK | lung, non-small cell lung carcinoma, lung, adenocarcinoma |
| 560 | VEYHFLSPYVSPKE | lung, non-small cell lung carcinoma, lung, adenocarcinoma |
| 561 | LPSQAFEYILYNKG | lung, non-small cell lung carcinoma, lung, adenocarcinoma |
| 562 | LPSQAFEYILYNKGI | lung, non-small cell lung carcinoma, lung, adenocarcinoma |
| 563 | LPSQAFEYILYNKGIM | lung, non-small cell lung carcinoma, lung, adenocarcinoma |
| 564 | LPSQAFEYILYNKGIMG | lung, non-small cell lung carcinoma, lung, adenocarcinoma |
| 565 | MNGYFLIERGKNM | lung, non-small cell lung carcinoma, lung, adenocarcinoma |
| 566 | NGYFLIERGKNm | lung, non-small cell lung carcinoma, lung, adenocarcinoma |
| 567 | PSQAFEYILYNKG | lung, non-small cell lung carcinoma, lung, adenocarcinoma |
| 568 | PSQAFEYILYNKGI | lung, non-small cell lung carcinoma, lung, adenocarcinoma |
| 569 | PSQAFEYILYNKGIM | lung, non-small cell lung carcinoma, lung, adenocarcinoma |
| 570 | EGVQYSYSLFHLM | stomach, metastatic, stomach, gastrointestinal stromal tumor (GIST) |
| 571 | EGVQYSYSLFHLML | stomach, metastatic, stomach, gastrointestinal stromal tumor (GIST) |
| 572 | GVQYSYSLFHLM | stomach, metastatic, stomach, gastrointestinal stromal tumor (GIST) |
| 573 | GVQYSYSLFHLML | stomach, metastatic, stomach, gastrointestinal stromal tumor (GIST) |
| 574 | SIISIHPKIQEHQPR | stomach, metastatic, stomach, gastrointestinal stromal tumor (GIST) |
| 575 | SSIRTSTNSQVDK | stomach, metastatic, stomach, gastrointestinal stromal tumor (GIST) |
| 576 | VLVGYKAVYRIS | stomach, metastatic, stomach, gastrointestinal stromal tumor (GIST) |
| 577 | KSSIRTSTNSQVDK | stomach, metastatic, stomach, gastrointestinal stromal tumor (GIST) |
| 578 | GGGYGSGGGSGGYGSRRF | colon or rectum, thymus, thymoma, malignant |
| 579 | GGSFGGRSSGSP | colon or rectum, thymus, thymoma, malignant |
| 580 | KGGSFGGRSSGSP | colon or rectum, thymus, thymoma, malignant |
| 581 | SGQQQSNYGPMKGGSFGGRSSGSPY | colon or rectum, thymus, thymoma, malignant |
| 582 | SGSPYGGGYGSGGGSGGYGSRRF | colon or rectum, thymus, thymoma, malignant |
| 583 | SPYGGGYGSGGGSGGYGSRRF | colon or rectum, thymus, thymoma, malignant |

(continued)

| Seq ID | Sequence | Tissue and disease |
|---|---|---|
| 584 | YGGGYGSGGGSGGYGSR RF | colon or rectum, thymus, thymoma, malignant |
| 585 | GNRINEFSISSF | chondrosarcoma |
| 586 | HGNQITSDKVGRKV | chondrosarcoma |
| 587 | IPPVNTNLENLYLQ | chondrosarcoma |
| 588 | LQVLRLDGNEIKR | chondrosarcoma |
| 589 | LQVLRLDGNEIKRS | chondrosarcoma |
| 590 | LQVLRLDGNEIKRSA | chondrosarcoma |
| 591 | LRELHLDHNQISRVPN | chondrosarcoma |
| 592 | LYVRLSHNSLTNNG | chondrosarcoma |
| 593 | VPSRMKYVYFQNNQ | chondrosarcoma |
| 594 | VPSRMKYVYFQNNQIT | chondrosarcoma |
| 595 | VPSRMKYVYFQNNQITS | chondrosarcoma |
| 596 | WIALHGNQITSD | chondrosarcoma |
| 597 | WIALHGNQITSDK | chondrosarcoma |
| 598 | ADDNVSFRWEALGNT | chondrosarcoma |
| 599 | ADDNVSFRWEALGNTL | colon or rectum |
| 600 | DADDNVSFRWEALGNTL | colon or rectum |
| 601 | DDNVSFRWEALGNT | colon or rectum |
| 602 | DDNVSFRWEALGNTL | colon or rectum |
| 603 | DNVSFRWEALGNT | colon or rectum |
| 604 | DNVSFRWEALGNTL | colon or rectum |
| 605 | DNVSFRWEALGNTLS | colon or rectum |
| 606 | DTGSYRAQISTKTSAK | colon or rectum |
| 607 | DTGSYRAQISTKTSAKL | colon or rectum |
| 608 | DTITIYSTINHSK | colon or rectum |
| 609 | EDTGSYRAQISTKTSAK | colon or rectum |
| 610 | ENDTITIYSTINHSK | colon or rectum |
| 611 | ENDTITIYSTINHSKESKPT | colon or rectum |
| 612 | GSYRAQISTKTSAK | colon or rectum |
| 613 | NDTITIYSTINH | colon or rectum |
| 614 | NDTITIYSTINHS | colon or rectum |
| 615 | NDTITIYSTINHSK | colon or rectum |
| 616 | NVSFRWEALGNTL | colon or rectum |
| 617 | SPTNNTVYASVTHSNRET | colon or rectum |
| 618 | TGSYRAQISTKTSAK | colon or rectum |
| 619 | | colon or rectum |
| 620 | TPRENDTITIYSTINHSKESK PT | colon or rectum |
| 621 | VSFRWEALGNTL | colon or rectum |
| 622 | APIHFTIEKLELNEK | lipoma |
| 623 | DAQFEVIKGQTIE | lipoma |
| 624 | DAQFEVIKGQTIEVR | lipoma |
| 625 | ESYFIPEVRIYDSGT | lipoma |
| 626 | IPEVRIYDSGTY | lipoma |
| 627 | KDKAIVAHNRHGNK | lipoma |
| 628 | KDKAIVAHNRHGNKA | lipoma |
| 629 | NFVILEFPVEEQDR | lipoma |
| 630 | SQPRISYDAQFEVIK | lipoma |

(continued)

| Seq ID | Sequence | Tissue and disease |
|---|---|---|
| 631 | SQPRISYDAQFEVIKG | lipoma |
| 632 | YDAQFEVIKGQTIE | lipoma |
| 633 | GNPAYRSFSNSLSQ | colon or rectum, kidney, angiomyolipoma |
| 634 | GPPGEAGYKAFSSLLA | colon or rectum, kidney, angiomyolipoma |
| 635 | GPPGEAGYKAFSSLLASS | colon or rectum, kidney, angiomyolipoma |
| 636 | GPPGEAGYKAFSSLLASSA | colon or rectum, kidney, angiomyolipoma |
| 637 | GPPGEAGYKAFSSLLASSA VSPE | colon or rectum, kidney, angiomyolipoma |
| 638 | GPPGEAGYKAFSSLLASSA VSPEK | colon or rectum, kidney, angiomyolipoma |
| 639 | GYKAFSSLLASSAVSP | colon or rectum, kidney, angiomyolipoma |
| 640 | GYKAFSSLLASSAVSPE | colon or rectum, kidney, angiomyolipoma |
| 641 | KAFSSLLASSAVSPE | colon or rectum, kidney, angiomyolipoma |
| 642 | NPAYRSFSNSLSQ | colon or rectum, kidney, angiomyolipoma |
| 643 | SRDDFQEGREGIVAR | colon or rectum, kidney, angiomyolipoma |
| 644 | SSSSFHPAPGNAQ | colon or rectum, kidney, angiomyolipoma |
| 645 | VARLTESLFLDL | colon or rectum, kidney, angiomyolipoma |
| 646 | VARLTESLFLDLLG | colon or rectum, kidney, angiomyolipoma |
| 647 | VIAGNPAYRSFSN | colon or rectum, kidney, angiomyolipoma |
| 648 | VPQPEPETWEQILRRNVLQ | colon or rectum, kidney, angiomyolipoma |
| 649 | YKAFSSLLASSAVS | colon or rectum, kidney, angiomyolipoma |
| 650 | YKAFSSLLASSAVSP | colon or rectum, kidney, angiomyolipoma |
| 651 | YKAFSSLLASSAVSPE | colon or rectum, kidney, angiomyolipoma, |
| 652 | GNQVFSYTANKEIRTDD | colon or rectum, urinary bladder, transitional cell carcinoma |
| 653 | IEEIVLVDDASERD | colon or rectum, urinary bladder, transitional cell carcinoma |
| 654 | IEEIVLVDDASERDF | colon or rectum, urinary bladder, transitional cell carcinoma |
| 655 | LENIYPDSQIPRH | colon or rectum, urinary bladder, transitional cell carcinoma |
| 656 | LENIYPDSQIPRHY | colon or rectum, urinary bladder, transitional cell carcinoma |
| 657 | NQVFSYTANKEIR | colon or rectum, urinary bladder, transitional cell carcinoma |
| 658 | NQVFSYTANKEIRT | colon or rectum, urinary bladder, transitional cell carcinoma |
| 659 | NQVFSYTANKEIRTDD | colon or rectum, urinary bladder, transitional cell carcinoma |
| 660 | VHSVINRSPRHMIEE | colon or rectum, urinary bladder, transitional cell carcinoma |
| 661 | EYVSLYHQPAAM | non-Hodgkin's lymphoma |
| 662 | IKAEYKGRVTLKQYPR | non-Hodgkin's lymphoma |
| 663 | LNVHSEYEPSWEEQP | non-Hodgkin's lymphoma |
| 664 | LPYLFQmPAYASSS | non-Hodgkin's lymphoma |
| 665 | LPYLFQmPAYASSSK | non-Hodgkin's lymphoma |
| 666 | NFIKAEYKGRVT | non-Hodgkin's lymphoma |
| 667 | TNFIKAEYKGRVT | non-Hodgkin's lymphoma |
| 668 | TTNFIKAEYKGRVT | non-Hodgkin's lymphoma |
| 669 | VTLNVHSEYEPSWEEQP | non-Hodgkin's lymphoma |

(continued)

| Seq ID | Sequence | Tissue and disease |
|---|---|---|
| 670 | | non-Hodgkin's lymphoma |
| 671 | YPRKNLFLVEVTQLTESDSG | non-Hodgkin's lymphoma |
| 672 | ADLSSFKSQELN | lymph node, papillary carcinoma of thyroid |
| 673 | ADLSSFKSQELNER | lymph node, papillary carcinoma of thyroid |
| 674 | ADLSSFKSQELNERN | lymph node, papillary carcinoma of thyroid |
| 675 | ADLSSFKSQELNERNE | lymph node, papillary carcinoma of thyroid |
| 676 | | lymph node, papillary carcinoma of thyroid |
| 677 | AEQQRLKSQDLELSWNLNG | lymph node, papillary carcinoma of thyroid, metastatic |
| 678 | EQQRLKSQDLELSWN | lymph node, papillary carcinoma of thyroid |
| 679 | ISQELEELRAEQQR | lymph node, papillary carcinoma of thyroid |
| 680 | ISQELEELRAEQQRLK | lymph node, papillary carcinoma of thyroid |
| 681 | KGTKQWVHARYA | lymph node, papillary carcinoma of thyroid |
| 682 | QADLSSFKSQELNER | lymph node, papillary carcinoma of thyroid, metastatic |
| 683 | SWNLNGLQADLSSFK | lymph node, papillary carcinoma of thyroid |
| 684 | TGSWIGLRNLDLKG | lymph node, papillary carcinoma of thyroid |
| 685 | FGNYNNQSSNFGPMKGGNFGGRS | pancreas, adenocarcinoma, thymus, thymoma, malignant |
| 686 | FGPMKGGNFGGRSSGPYGGGGQY | pancreas, adenocarcinoma, thymus, thymoma, malignant |
| 687 | GPMKGGNFGGRSSGP | pancreas, adenocarcinoma, thymus, thymoma, malignant |
| 688 | GPYGGGGQYFAKP | pancreas, adenocarcinoma, thymus, thymoma, malignant |
| 689 | KGGNFGGRSSGP | pancreas, adenocarcinoma, thymus, thymoma, malignant |
| 690 | NDFGNYNNQSSNFGP | pancreas, adenocarcinoma, thymus, thymoma, malignant |
| 691 | SGPYGGGGQYFAKP | pancreas, adenocarcinoma, thymus, thymoma, malignant |
| 692 | DAGSYKAQINQRNFE | lung, non-small cell lung carcinoma, lymph node, non-Hodgkin's lymphoma |
| 693 | DAGSYKAQINQRNFEVT | lung, non-small cell lung carcinoma, lymph node, non-Hodgkin's lymphoma |
| 694 | DGELIRTQPQRLPQ | pancreas, adenocarcinoma, intramuscular lipoma |
| 695 | GELIRTQPQRLPQ | pancreas, adenocarcinoma, intramuscular lipoma |
| 696 | NPSDGELIRTQPQRLP | pancreas, adenocarcinoma, intramuscular lipoma |
| 697 | NPSDGELIRTQPQRLPQ | pancreas, adenocarcinoma, intramuscular lipoma |
| 698 | NPSDGELIRTQPQRLPQL | pancreas, adenocarcinoma, intramuscular lipoma |
| 699 | ASNDMYHSRALQVVR | colon or rectum, bone, giant cell tumor of bone |
| 700 | ASNDMYHSRALQVVRA | colon or rectum, bone, giant cell tumor of bone |
| 701 | EGVRRALDFAVGEYN | colon or rectum, bone, giant cell tumor of bone |
| 702 | EGVRRALDFAVGEYNK | colon or rectum, bone, giant cell tumor of bone |

(continued)

| Seq ID | Sequence | Tissue and disease |
|---|---|---|
| 703 | SNDMYHSRALQVVR | colon or rectum, bone, giant cell tumor of bone |
| 704 | VGEYNKASNDMYH | colon or rectum, bone, giant cell tumor of bone |
| 705 | VRARKQIVAGVNY | colon or rectum, bone, giant cell tumor of bone |
| 706 | VRRALDFAVGEYNKASND | colon or rectum, bone, giant cell tumor of bone |
| 707 | VVRARKQIVAGVN | colon or rectum, bone, giant cell tumor of bone |
| 708 | WRARKQIVAGVNY | colon or rectum, bone, giant cell tumor of bone |
| 709 | APLEGARFALVRED | liver, hepatocellular carcinoma |
| 710 | APVELILSDETLPAPE | liver, hepatocellular carcinoma |
| 711 | ELILSDETLPAPE | liver, hepatocellular carcinoma |
| 712 | LAPLEGARFALVRE | liver, hepatocellular carcinoma |
| 713 | LAPLEGARFALVRED | liver, hepatocellular carcinoma |
| 714 | RGEKELLVPRSSTSPD | liver, hepatocellular carcinoma |
| 715 | ASKTFTTQETITNAET | kidney, clear cell renal cell carcinoma, kidney, angiomyolipoma |
| 716 | DQHFRTTPLEKNAPV | kidney, clear cell renal cell carcinoma, kidney, angiomyolipoma |
| 717 | NTPILVDGKDVMPE | kidney, clear cell renal cell carcinoma, kidney, angiomyolipoma |
| 718 | NTPILVDGKDVMPEV | kidney, clear cell renal cell carcinoma, kidney, angiomyolipoma |
| 719 | NTPILVDGKDVMPEVN | kidney, clear cell renal cell carcinoma, kidney, angiomyolipoma |
| 720 | SNTPILVDGKDVMPE | kidney, clear cell renal cell carcinoma, kidney, angiomyolipoma |
| 721 | SNTPILVDGKDVMPEVN | kidney, clear cell renal cell carcinoma, kidney, angiomyolipoma |
| 722 | TPILVDGKDVMP | kidney, clear cell renal cell carcinoma, kidney, angiomyolipoma |
| 723 | TPILVDGKDVMPE | kidney, clear cell renal cell carcinoma, kidney, angiomyolipoma |
| 724 | TPILVDGKDVMPEV | kidney, clear cell renal cell carcinoma, kidney, angiomyolipoma |
| 725 | TPILVDGKDVMPEVN | kidney, clear cell renal cell carcinoma, kidney, angiomyolipoma |
| 726 | GPLKFLHQDIDSGQG | kidney, renal cell carcinoma |
| 727 | GPLKFLHQDIDSGQGIR | kidney, renal cell carcinoma |
| 728 | LGDIYFKLFRASG | kidney, renal cell carcinoma |
| 729 | TGHLFDLSSLSGRAG | kidney, renal cell carcinoma |
| 730 | VPSPVDCQVTDLAGNE | kidney, renal cell carcinoma |
| 731 | DGLNSLTYQVLDVQRYPL | kidney, clear cell renal cell carcinoma, endometrium, adenocarcinoma, endometrioid type |
| 732 | HPVLQRQQLDYGIY | kidney, clear cell renal cell carcinoma, endometrium, adenocarcinoma, endometrioid type |
| 733 | LNSLTYQVLDVQR | kidney, clear cell renal cell carcinoma, endometrium, adenocarcinoma, endometrioid type |
| 734 | LNSLTYQVLDVQRYP | kidney, clear cell renal cell carcinoma, endometrium, adenocarcinoma, endometrioid type |

(continued)

| Seq ID | Sequence | Tissue and disease |
|---|---|---|
| 735 | LNSLTYQVLDVQRYPL | kidney, clear cell renal cell carcinoma, endometrium, adenocarcinoma, endometrioid type |
| 736 | LPQLVGVSTPLQG | kidney, clear cell renal cell carcinoma, endometrium, adenocarcinoma, endometrioid type |
| 737 | LPQLVGVSTPLQGG | kidney, clear cell renal cell carcinoma, endometrium, adenocarcinoma, endometrioid type |
| 738 | LPQLVGVSTPLQGGS | kidney, clear cell renal cell carcinoma, endometrium, adenocarcinoma, endometrioid type |
| 739 | RLPQLVGVSTPLQGGS | kidney, clear cell renal cell carcinoma, endometrium, adenocarcinoma, endometrioid type |
| 740 | SPHKVAIIIPFRNR | kidney, clear cell renal cell carcinoma, endometrium, adenocarcinoma, endometrioid type |
| 741 | SPHKVAIIIPFRNRQE | kidney, clear cell renal cell carcinoma, endometrium, adenocarcinoma, endometrioid type |
| 742 | SPHKVAIIIPFRNRQEH | kidney, clear cell renal cell carcinoma, endometrium, adenocarcinoma, endometrioid type |
| 743 | AIVQAVSAHRHR | non-Hodgkin's lymphoma, peripheral T cell type |
| 744 | ARNFERNKAIKVI | non-Hodgkin's lymphoma, peripheral T cell type |
| 745 | ARNFERNKAIKVIIA | non-Hodgkin's lymphoma, peripheral T cell type |
| 746 | NFERNKAIKVII | non-Hodgkin's lymphoma, peripheral T cell type |
| 747 | NFERNKAIKVIIA | non-Hodgkin's lymphoma, peripheral T cell type |
| 748 | VAIVQAVSAHRH | non-Hodgkin's lymphoma, peripheral T cell type |
| 749 | VAIVQAVSAHRHR | non-Hodgkin's lymphoma, peripheral T cell type |
| 750 | VAIVQAVSAHRHRA | non-Hodgkin's lymphoma, peripheral T cell type |
| 751 | VAIVQAVSAHRHRAR | non-Hodgkin's lymphoma, peripheral T cell type |
| 752 | EEVITLIRSNQQLE | lung, non-small cell lung carcinoma, pancreas, adenocarcinoma |
| 753 | EEVITLIRSNQQLEN | lung, non-small cell lung carcinoma, pancreas, adenocarcinoma |
| 754 | IPADTFAALKNPNAML | lung, non-small cell lung carcinoma, pancreas, adenocarcinoma |
| 755 | LKQLLSDKQQKRQSG | lung, non-small cell lung carcinoma, pancreas, adenocarcinoma |
| 756 | LKQLLSDKQQKRQSGQ | lung, non-small cell lung carcinoma, pancreas, adenocarcinoma |
| 757 | TPSYVAFTDTER | pancreas, adenocarcinoma, rectum, adenocarcinoma |
| 758 | TPSYVAFTDTERL | pancreas, adenocarcinoma, rectum, adenocarcinoma |
| 759 | EGLYSRTLAGSIT | liver, hepatocellular carcinoma, cancer, thyroid gland, nodular hyperplasia |
| 760 | EGLYSRTLAGSITTPP | liver, hepatocellular carcinoma, cancer, thyroid gland, nodular hyperplasia |

(continued)

| Seq ID | Sequence | Tissue and disease |
|--------|----------|--------------------|
| 761 | EKWYIPDPTGKFN | liver, hepatocellular carcinoma, cancer, thyroid gland, nodular hyperplasia |
| 762 | GAIAAINSIQHNTR | liver, hepatocellular carcinoma, cancer, thyroid gland, nodular hyperplasia |
| 763 | LPILVPSAKKAI | liver, hepatocellular carcinoma, cancer, thyroid gland, nodular hyperplasia |
| 764 | LPILVPSAKKAIY | liver, hepatocellular carcinoma, cancer, thyroid gland, nodular hyperplasia |
| 765 | LPILVPSAKKAIYM | liver, hepatocellular carcinoma, cancer, thyroid gland, nodular hyperplasia |
| 766 | LPILVPSAKKAIYMD | liver, hepatocellular carcinoma, cancer, thyroid gland, nodular hyperplasia |
| 767 | LPILVPSAKKAIYMDD | liver, hepatocellular carcinoma, cancer, thyroid gland, nodular hyperplasia |
| 768 | VEEGLYSRTLAGSIT | liver, hepatocellular carcinoma, cancer, thyroid gland, nodular hyperplasia |
| 769 | WEKWYIPDPTGKFN | liver, hepatocellular carcinoma, cancer, thyroid gland, nodular hyperplasia |
| 770 | YKIVNFDPKLLE | liver, hepatocellular carcinoma, cancer, thyroid gland, nodular hyperplasia |
| 771 | YKIVNFDPKLLEG | liver, hepatocellular carcinoma, cancer, thyroid gland, nodular hyperplasia |
| 772 | YKIVNFDPKLLEGKV | liver, hepatocellular carcinoma, cancer, thyroid gland, nodular hyperplasia |
| 773 | LPEFYKTVSPAL | colon or rectum, endometrium, adenocarcinoma, endometrioid type |
| 774 | VGQFIQDVKNSRST | colon or rectum, endometrium, adenocarcinoma, endometrioid type |
| 775 | VGQFIQDVKNSRSTD | colon or rectum, endometrium, adenocarcinoma, endometrioid type |
| 776 | VVGQFIQDVKNSRS | colon or rectum, endometrium, adenocarcinoma, endometrioid type |
| 777 | VVGQFIQDVKNSRST | colon or rectum, endometrium, adenocarcinoma, endometrioid type |
| 778 | VVGQFIQDVKNSRSTD | colon or rectum, endometrium, adenocarcinoma, endometrioid type |
| 779 | VVGQFIQDVKNSRSTDS | colon or rectum, endometrium, adenocarcinoma, endometrioid type |
| 780 | DNGHLYREDQTSPAPG | pancreas, adenocarcinoma, kidney, angiomyolipoma |
| 781 | DNGHLYREDQTSPAPGLR | pancreas, adenocarcinoma, kidney, angiomyolipoma |
| 782 | EVQVFAPANALPARSE | pancreas, adenocarcinoma, kidney, angiomyolipoma |
| 783 | GHLYREDQTSPAPG | pancreas, adenocarcinoma, kidney, angiomyolipoma |
| 784 | LPARSEAAAVQPVIG | pancreas, adenocarcinoma, kidney, angiomyolipoma |
| 785 | NGHLYREDQTSPAPG | pancreas, adenocarcinoma, kidney, angiomyolipoma |
| 786 | NGHLYREDQTSPAPGL | pancreas, adenocarcinoma, kidney, angiomyolipoma |

(continued)

| Seq ID | Sequence | Tissue and disease |
|---|---|---|
| 787 | NGHLYREDQTSPAPGLR | pancreas, adenocarcinoma, kidney, angiomyolipoma |
| 788 | VFAPANALPARSEAA | pancreas, adenocarcinoma, kidney, angiomyolipoma |
| 789 | VQVFAPANALPARSE | pancreas, adenocarcinoma, kidney, angiomyolipoma |
| 790 | AIVVSDRDGVPVIK | stomach, adenocarcinoma, parathyroid gland, adenoma |
| 791 | GLHAIVVSDRDGVPV | stomach, adenocarcinoma, parathyroid gland, adenoma |
| 792 | GLHAIVVSDRDGVPVIK | stomach, adenocarcinoma, parathyroid gland, adenoma |
| 793 | HAIVVSDRDGVPV | stomach, adenocarcinoma, parathyroid gland, adenoma |
| 794 | KLPSVEGLHAIVVSDRDG | stomach, adenocarcinoma, parathyroid gland, adenoma |
| 795 | LHAIVVSDRDGVPV | stomach, adenocarcinoma, parathyroid gland, adenoma |
| 796 | LHAIVVSDRDGVPVI | stomach, adenocarcinoma, parathyroid gland, adenoma |
| 797 | LHAIVVSDRDGVPVIK | stomach, adenocarcinoma, parathyroid gland, adenoma |
| 798 | LPSVEGLHAIVVSDR | stomach, adenocarcinoma, parathyroid gland, adenoma |
| 799 | VPVIKVANDNAPE | stomach, adenocarcinoma, parathyroid gland, adenoma |
| 800 | YNTYQVVQFNRLP | stomach, adenocarcinoma, parathyroid gland, adenoma |
| 801 | YNTYQVVQFNRLPL | stomach, adenocarcinoma, parathyroid gland, adenoma |
| 802 | YNTYQVVQFNRLPLV | stomach, adenocarcinoma, parathyroid gland, adenoma |
| 803 | YNTYQVVQFNRLPLVV | stomach, adenocarcinoma, parathyroid gland, adenoma |
| 804 | YYNTYQVVQFNRLP | stomach, adenocarcinoma, parathyroid gland, adenoma |
| 805 | YYNTYQVVQFNRLPL | stomach, adenocarcinoma, parathyroid gland, adenoma |
| 806 | YYNTYQVVQFNRLPLV | stomach, adenocarcinoma, parathyroid gland, adenoma |
| 807 | DKIYFmAGSSRKE | liver, hepatocellular carcinoma, thyroid gland, nodular hyperplasia |
| 808 | DVGTDEEEETAKESTAEKDE | liver, hepatocellular carcinoma, thyroid gland, nodular hyperplasia |
| 809 | EVTFKSILFVPTSAP | liver, hepatocellular carcinoma, thyroid gland, nodular hyperplasia |
| 810 | KSEKFAFQAEVNR | liver, hepatocellular carcinoma, thyroid gland, nodular hyperplasia |
| 811 | LPEFDGKRFQNVAK | liver, hepatocellular carcinoma, thyroid gland, nodular hyperplasia |
| 812 | DGSYRIFSKGASE | colon or rectum, liposarcoma |
| 813 | GSYRIFSKGASE | colon or rectum, liposarcoma |

(continued)

| Seq ID | Sequence | Tissue and disease |
|---|---|---|
| 814 | SDGSYRIFSKGASE | colon or rectum, liposarcoma |
| 815 | SVKKMMKDNNLVRH | colon or rectum, liver, hepatocellular carcinoma |
| 816 | VKKMMKDNNLVRH | colon or rectum, liver, hepatocellular carcinoma |
| 817 | NNmRIFGEAAEKN | stomach, adenocarcinoma, thyroid gland, papillary carcinoma |
| 818 | VDKVLERDQKLSE | lung, non-small cell lung carcinoma, lymph node, papillary carcinoma of thyroid |
| 819 | VDKVLERDQKLSELD | lung, non-small cell lung carcinoma, lymph node, papillary carcinoma of thyroid |
| 820 | VDKVLERDQKLSELDD | lung, non-small cell lung carcinoma, lymph node, papillary carcinoma of thyroid |
| 821 | VDKVLERDQKLSELDDR | stomach, adenocarcinoma, lymph node, papillary carcinoma of thyroid |
| 822 | VLERDQKLSELDDR | lung, non-small cell lung carcinoma, lymph node, papillary carcinoma of thyroid |
| 823 | ATRSIQVDGKTIKAQ | stomach, adenocarcinoma, kidney, angiomyolipoma |
| 824 | ATRSIQVDGKTIKAQI | stomach, adenocarcinoma, kidney, angiomyolipoma |
| 825 | IGVEFATRSIQVDGK | stomach, adenocarcinoma, kidney, angiomyolipoma |
| 826 | RSIQVDGKTIKA | stomach, adenocarcinoma, kidney, angiomyolipoma |
| 827 | RSIQVDGKTIKAQ | stomach, adenocarcinoma, kidney, angiomyolipoma |
| 828 | RSIQVDGKTIKAQI | stomach, adenocarcinoma, kidney, angiomyolipoma |
| 829 | TRSIQVDGKTIKAQ | stomach, adenocarcinoma, kidney, angiomyolipoma |
| 830 | DIMRVNVDKVLERDQK | stomach, adenocarcinoma, medullary carcinoma of thyroid origin |
| 831 | DIMRVNVDKVLERDQKL | stomach, adenocarcinoma, medullary carcinoma of thyroid origin |
| 832 | IMRVNVDKVLERDQK | lung, non-small cell lung carcinoma, lymph node, Hodgkin's disease |
| 833 | VDKVLERDQKLSE | lung, non-small cell lung carcinoma, lymph node, papillary carcinoma of thyroid |
| 834 | VDKVLERDQKLSELD | lung, non-small cell lung carcinoma, lymph node, papillary carcinoma of thyroid |
| 835 | VDKVLERDQKLSELDD | lung, non-small cell lung carcinoma, lymph node, papillary carcinoma of thyroid |
| 836 | VDKVLERDQKLSELDDR | stomach, adenocarcinoma, lymph node, papillary carcinoma of thyroid |
| 837 | VLERDQKLSELDDR | lung, non-small cell lung carcinoma, lymph node, papillary carcinoma of thyroid |
| 838 | ATRSIQVDGKTIKAQ | stomach, adenocarcinoma |
| 839 | ATRSIQVDGKTIKAQI | stomach, adenocarcinoma, kidney, angiomyolipoma |
| 840 | IGVEFATRSIQVDGK | stomach, adenocarcinoma, kidney, angiomyolipoma |
| 841 | RSIQVDGKTIKA | stomach, adenocarcinoma, kidney, angiomyolipoma |

(continued)

| Seq ID | Sequence | Tissue and disease |
|--------|----------|---------------------|
| 842 | RSIQVDGKTIKAQ | stomach, adenocarcinoma, kidney, angiomyolipoma |
| 843 | RSIQVDGKTIKAQI | stomach, adenocarcinoma, kidney, angiomyolipoma |
| 844 | TRSIQVDGKTIKAQ | stomach, adenocarcinoma, kidney, angiomyolipoma |
| 845 | GIRVAPVPLYNS | lung, non-small cell lung carcinoma, liver, hepatocellular carcinoma |
| 846 | GIRVAPVPLYNSFH | lung, non-small cell lung carcinoma, liver, hepatocellular carcinoma |
| 847 | NPNGIRVAPVPLYNSFH | lung, non-small cell lung carcinoma, liver, hepatocellular carcinoma |
| 848 | DDPAIDVCKKLLGKYPN | kidney, clear cell renal cell carcinoma, pancreas, adenocarcinoma |
| 849 | DKQPYSKLPGVSLLKP | kidney, clear cell renal cell carcinoma, pancreas, adenocarcinoma |
| 850 | DKQPYSKLPGVSLLKPL | kidney, clear cell renal cell carcinoma, pancreas, adenocarcinoma |
| 851 | HPRYYISANVTGFK | kidney, clear cell renal cell carcinoma, pancreas, adenocarcinoma |
| 852 | SHPRYYISANVTG | kidney, clear cell renal cell carcinoma, pancreas, adenocarcinoma |
| 853 | SHPRYYISANVTGFK | kidney, clear cell renal cell carcinoma, pancreas, adenocarcinoma |
| 854 | TSHPRYYISANVTG | kidney, clear cell renal cell carcinoma, pancreas, adenocarcinoma |
| 855 | TSHPRYYISANVTGFK | kidney, clear cell renal cell carcinoma, pancreas, adenocarcinoma |
| 856 | ADIFVDPVLHTA | kidney, renal cell carcinoma |
| 857 | ADIFVDPVLHTACA | kidney, renal cell carcinoma |
| 858 | DPGADYRIDRALNEA | kidney, renal cell carcinoma |
| 859 | IAQDYKVSYSLA | kidney, renal cell carcinoma |
| 860 | IAQDYKVSYSLAK | kidney, renal cell carcinoma |
| 861 | ISRDWKLDPVLYRK | kidney, renal cell carcinoma |
| 862 | LIAQDYKVSYSLA | kidney, renal cell carcinoma |
| 863 | RQKLIAQDYKVSYS | kidney, renal cell carcinoma |
| 864 | RQKLIAQDYKVSYSL | kidney, renal cell carcinoma |
| 865 | RQKLIAQDYKVSYSLA | kidney, renal cell carcinoma |
| 866 | RQKLIAQDYKVSYSLAK | kidney, renal cell carcinoma |
| 867 | SALDYRLDPQLQLH | kidney, renal cell carcinoma |
| 868 | SKADIFVDPVLHTA | kidney, renal cell carcinoma |
| 869 | SPSKNYILSVISGSI | kidney, renal cell carcinoma |
| 870 | ETTQLTADSHPSYHTDG | stomach, metastatic, skin, squamous cell carcinoma |
| 871 | SGESLYHVLGLDKNATSDD | stomach, metastatic, skin, squamous cell carcinoma |
| 872 | TTQLTADSHPSYHT | stomach, metastatic, skin, squamous cell carcinoma |
| 873 | TTQLTADSHPSYHTD | stomach, metastatic, skin, squamous cell carcinoma |

(continued)

| Seq ID | Sequence | Tissue and disease |
|---|---|---|
| 874 | TTQLTADSHPSYHTDG | stomach, metastatic, skin, squamous cell carcinoma |
| 875 | SVEEFLSEKLERI | pancreas, adenocarcinoma, liver, hepatic adenoma |
| 876 | VEEFLSEKLERI | pancreas, adenocarcinoma, liver, hepatic adenoma |
| 877 | DLSSSILAQSRERVA | pancreas, adenocarcinoma, bone, giant cell tumor of bone |
| 878 | EKGVRTLTAAAVSGAQ | pancreas, adenocarcinoma, bone, giant cell tumor of bone |
| 879 | EKGVRTLTAAAVSGAQP | pancreas, adenocarcinoma, bone, giant cell tumor of bone |
| 880 | EKGVRTLTAAAVSGAQPI | pancreas, adenocarcinoma, bone, giant cell tumor of bone |
| 881 | KGVRTLTAAAVSGA | pancreas, adenocarcinoma, bone, giant cell tumor of bone |
| 882 | KGVRTLTAAAVSGAQ | pancreas, adenocarcinoma, bone, giant cell tumor of bone |
| 883 | VGPFAPGITEKAPEEKK | pancreas, adenocarcinoma, bone, giant cell tumor of bone |
| 884 | DPPLIALDKDAPLR | brain, glioblastoma, parotid gland, pleomorphic adenoma |
| 885 | EIITPDVPFTVDKDG | brain, glioblastoma, parotid gland, pleomorphic adenoma |
| 886 | IITPDVPFTVDKDG | brain, glioblastoma, parotid gland, pleomorphic adenoma |
| 887 | PPLIALDKDAPLR | brain, glioblastoma, parotid gland, pleomorphic adenoma |
| 888 | TNVKKSHKATVHIQ | brain, glioblastoma, parotid gland, pleomorphic adenoma |
| 889 | DDNIKTYSDHPE | kidney, clear cell renal cell carcinoma, liver, hepatocellular carcinoma |
| 890 | DDNIKTYSDHPEK | kidney, clear cell renal cell carcinoma, liver, hepatocellular carcinoma |
| 891 | DSAVFFEQGTTRIG | kidney, clear cell renal cell carcinoma, liver, hepatocellular carcinoma |
| 892 | GDKVYVHLKNLASRPY | kidney, clear cell renal cell carcinoma, liver, hepatocellular carcinoma |
| 893 | GDKVYVHLKNLASRPYT | kidney, clear cell renal cell carcinoma, liver, hepatocellular carcinoma |
| 894 | VHLKNLASRPYT | kidney, clear cell renal cell carcinoma, liver, hepatocellular carcinoma |
| 895 | VYVHLKNLASRPY | kidney, clear cell renal cell carcinoma, liver, hepatocellular carcinoma |
| 896 | VYVHLKNLASRPYT | kidney, clear cell renal cell carcinoma, liver, hepatocellular carcinoma |
| 897 | VYVHLKNLASRPYTFH | kidney, clear cell renal cell carcinoma, liver, hepatocellular carcinoma |
| 898 | YVHLKNLASRPY | kidney, clear cell renal cell carcinoma, liver, hepatocellular carcinoma |
| 899 | YVHLKNLASRPYT | kidney, clear cell renal cell carcinoma, liver, hepatocellular carcinoma |

(continued)

| Seq ID | Sequence | Tissue and disease |
|---|---|---|
| 900 | YVHLKNLASRPYTFH | kidney, clear cell renal cell carcinoma, liver, hepatocellular carcinoma |
| 901 | SNLIKLAQKVPTAD | liver, hepatocellular carcinom |
| 902 | YDTRTSALSAKS | liver, hepatocellular carcinoma |
| 903 | ALMTDPKLITWSPV | bone, non-ossifying fibroma |
| 904 | NDVAWNFEKFLVGPDG | bone, non-ossifying fibroma |
| 905 | QSVYAFSARPLAG | bone, non-ossifying fibroma |
| 906 | QSVYAFSARPLAGGEPV | bone, non-ossifying fibroma |
| 907 | WNFEKFLVGPDG | colon or rectum, bone, non-ossifying fibroma |
| 908 | DVGMFVALTKLGQPD | stomach, adenocarcinoma, uterin cervix, squamous cell carcinoma |
| 909 | VGMFVALTKLGQPD | stomach, adenocarcinoma, uterin cervix, squamous cell carcinoma |
| 910 | AGVFHVEKNGRY | stomach, adenocarcinoma, colon, adenocarcinoma |
| 911 | FAGVFHVEKNGRYS | stomach, adenocarcinoma, colon, adenocarcinoma |
| 912 | GPITITIVNRDGTR | stomach, adenocarcinoma, colon, adenocarcinoma |
| 913 | NGRYSISRTEAADL | stomach, adenocarcinoma, colon, adenocarcinoma |
| 914 | RKSRQGSLAMEELK | rectum, adenocarcinoma |
| 915 | RRKSRQGSLAMEELK | rectum, adenocarcinoma |
| 916 | EEFKKLTSIKIQNDK | brain, glioblastoma, small Intestine, gastrointestinal stromal tumor (GIST) |
| 917 | INRRMADDNKLFR | brain, glioblastoma, small Intestine, gastrointestinal stromal tumor (GIST) |
| 918 | TATIVMVTNLKERKE | brain, glioblastoma, small Intestine, gastrointestinal stromal tumor (GIST) |
| 919 | ELFYKGIRPAINVG | liver, hepatocellular carcinoma, kidney, oncocytoma |
| 920 | GQKRSTVAQLVKR | liver, hepatocellular carcinoma, kidney, oncocytoma |
| 921 | SDLDAATQQLLSRGV | liver, hepatocellular carcinoma, kidney, oncocytoma |
| 922 | FDFSQNTRVPRLPE | kidney, clear cell renal cell carcinoma, non-Hodgkin's lymphoma |
| 923 | GDAPAILFDKEF | kidney, clear cell renal cell carcinoma, non-Hodgkin's lymphoma |
| 924 | VTHEIDRYTAIAY | kidney, clear cell renal cell carcinoma, non-Hodgkin's lymphoma |
| 929 | AAKYQLDPTASISA | kidney, oncocytoma |
| 930 | IAAKYQLDPTASISA | kidney, oncocytoma |
| 931 | IAAKYQLDPTASISAK | kidney, oncocytoma |
| 932 | AGLGRAYALAFAERG | liver, hepatocellular carcinoma, hepatic adenoma |
| 933 | DAFGRIDVVVNNAG | liver, hepatocellular carcinoma, hepatic adenoma |
| 934 | GLGRAYALAFAER | liver, hepatocellular carcinoma, hepatic adenoma |
| 935 | GLGRAYALAFAERG | liver, hepatocellular carcinoma, hepatic adenoma |
| 936 | AKFALNGEEFMNFDL | liver, hepatocellular carcinoma, liposarcoma |
| 937 | AKFALNGEEFMNFDLK | liver, hepatocellular carcinoma, liposarcoma |
| 938 | ALNGEEFMNFDLK | liver, hepatocellular carcinoma, liposarcoma |

(continued)

| Seq ID | Sequence | Tissue and disease |
|--------|----------|--------------------|
| 939 | KFALNGEEFMNFDL | liver, hepatocellular carcinoma, liposarcoma |
| 940 | SDGSFHASSSLTVK | liver, hepatocellular carcinoma, liposarcoma |
| 941 | EERNLLSVAYKNVVGAR | colon or rectum, esophagus, adenocarcinoma |
| 942 | ERNLLSVAYKNVVGAR | colon or rectum, esophagus, adenocarcinoma |
| 943 | IAELDTLSEESYKD | colon or rectum, Vulva, squamous cell carcinoma |
| 944 | IAELDTLSEESYKDS | colon or rectum, Vulva, squamous cell carcinoma |
| 945 | ADSYLDEGFLLDKKIG | lung, non-small cell lung carcinoma, ovary, Mullerian mixed tumor |
| 946 | DSYLDEGFLLDKK | lung, non-small cell lung carcinoma, ovary, Mullerian mixed tumor |
| 947 | DSYLDEGFLLDKKIG | lung, non-small cell lung carcinoma, ovary, Mullerian mixed tumor |
| 948 | VDNIIKAAPRKRVPD | lung, non-small cell lung carcinoma, ovary, Mullerian mixed tumor |
| 949 | SPPQFRVNGAISN | colon or rectum, ovary, granulosa cell tumor |
| 950 | SPPQFRVNGAISNFE | colon or rectum, ovary, granulosa cell tumor |
| 951 | SPPQFRVNGAISNFEE | colon or rectum, ovary, granulosa cell tumor |
| 952 | SPPQFRVNGAISNFEEF | colon or rectum, ovary, granulosa cell tumor |
| 953 | VGKMFVDVYFQEDKK | colon or rectum, ovary, granulosa cell tumor |
| 954 | VGKMFVDVYFQEDKKE | colon or rectum, ovary, granulosa cell tumor |
| 955 | DPKRTIAQDYGVLKADE | lung, non-small cell lung carcinoma, thyroid gland, nodular hyperplasia |
| 956 | DPKRTIAQDYGVLKADEG | lung, non-small cell lung carcinoma, thyroid gland, nodular hyperplasia |
| 957 | PKRTIAQDYGVLKADEG | lung, non-small cell lung carcinoma, thyroid gland, nodular hyperplasia |
| 958 | GLFIIDDKGILRQ | lung, non-small cell lung carcinoma, thyroid gland, nodular hyperplasia |
| 959 | GLFIIDDKGILRQIT | lung, non-small cell lung carcinoma, thyroid gland, nodular hyperplasia |
| 960 | RGLFIIDDKGILR | lung, non-small cell lung carcinoma, thyroid gland, nodular hyperplasia |
| 961 | RGLFIIDDKGILRQ | lung, non-small cell lung carcinoma, thyroid gland, nodular hyperplasia |
| 962 | RGLFIIDDKGILRQIT | lung, non-small cell lung carcinoma, thyroid gland, nodular hyperplasia |
| 963 | GNTVIHLDQALARMR | brain, glioblastoma, lung, small cell carcinoma |
| 964 | NTVIHLDQALARMR | brain, glioblastoma, lung, small cell carcinoma |
| 965 | NTVIHLDQALARMRE | brain, glioblastoma, lung, small cell carcinoma |
| 966 | ENNEIISNIRDSVIN | stomach, adenocarcinoma, kidney, oncocytoma |
| 967 | NNEIISNIRDSVIN | stomach, adenocarcinoma, kidney, oncocytoma |
| 968 | SPTVQVFSASGKPV | stomach, adenocarcinoma, kidney, oncocytoma |
| 969 | SSPTVQVFSASGKPVE | stomach, adenocarcinoma, kidney, oncocytoma |
| 970 | AEPNYHSLPSARTDEQ | thyroid gland, follicular adenoma |
| 971 | SSILAKTASNIIDVS | thyroid gland, follicular adenoma |
| 973 | ADDLEGEAFLPL | stomach, adenocarcinoma, spleen, chronic myeloid leukemia |
| 974 | ADDLEGEAFLPLR | stomach, adenocarcinoma, spleen, chronic myeloid leukemia |
| 975 | ADDLEGEAFLPLRE | stomach, adenocarcinoma, spleen, chronic myeloid leukemia |

(continued)

| Seq ID | Sequence | Tissue and disease |
|--------|----------|--------------------|
| 976 | GADDLEGEAFLPLR | stomach, adenocarcinoma, spleen, chronic myeloid leukemia |
| 977 | AGREINLVDAHLKSE | lymph node, Hodgkin's disease |
| 978 | AGREINLVDAHLKSEQT | lymph node, Hodgkin's disease |
| 979 | GREINLVDAHLKSE | lymph node, Hodgkin's disease |
| 980 | KPGIVYASLNHSVIG | lymph node, Hodgkin's disease |
| 981 | NKPGIVYASLNHSVIG | lymph node, Hodgkin's disease |
| 982 | TTLYVTDVKSASERPS | lymph node, Hodgkin's disease |
| 983 | APSTYAHLSPAKTPPP | stomach, adenocarcinoma, pancreas, adenocarcinoma |
| 984 | APSTYAHLSPAKTPPPP | stomach, adenocarcinoma, pancreas, adenocarcinoma |
| 985 | APSTYAHLSPAKTPPPPA | stomach, adenocarcinoma, pancreas, adenocarcinoma |
| 986 | RDDLYDQDDSRDFPR | stomach, adenocarcinoma, pancreas, adenocarcinoma |
| 987 | TRPYHSLPSEAVFA | adrenal gland, adrenal cortical adenoma |
| 988 | TRPYHSLPSEAVFAN | adrenal gland, adrenal cortical adenoma |
| 989 | VAVFTFHNHGRT | adrenal gland, adrenal cortical adenoma |
| 990 | VAVFTFHNHGRTA | adrenal gland, adrenal cortical adenoma |
| 991 | VAVFTFHNHGRTANL | adrenal gland, adrenal cortical adenoma |
| 992 | EDDYIKSWEDNQQGDE | brain, glioblastoma, pleura, malignant mesothelioma |
| 993 | ELERIQIQEAAKKKPG | brain, glioblastoma, pleura, malignant mesothelioma |
| 994 | ERIQIQEAAKKKP | brain, glioblastoma, pleura, malignant mesothelioma |
| 995 | ERIQIQEAAKKKPG | brain, glioblastoma, pleura, malignant mesothelioma |
| 996 | ERIQIQEAAKKKPGI | brain, glioblastoma, pleura, malignant mesothelioma |
| 997 | LERIQIQEAAKKKPG | brain, glioblastoma, pleura, malignant mesothelioma |
| 998 | LSSISQYSGKIK | brain, glioblastoma, pleura, malignant mesothelioma |
| 999 | SPAKDSLSFEDF | rectum, adenocarcinoma |
| 1000 | SPAKDSLSFEDFLDL | rectum, adenocarcinoma |
| 1001 | INSRFPIPSATDPD | brain, glioblastoma, brain, oligodendroglioma |
| 1002 | VQHYELLNGQSVFG | brain, glioblastoma, brain, oligodendroglioma |
| 1003 | DNQYAVLENQKSSH | colon or rectum, pleura, malignant mesothelioma |
| 1004 | GPPEIYSDTQFPS | colon or rectum, pleura, malignant mesothelioma |
| 1005 | GPPEIYSDTQFPSLQ | colon or rectum, pleura, malignant mesothelioma |
| 1006 | TPQGPPEIYSDTQFPS | colon or rectum, pleura, malignant mesothelioma |
| 1007 | TPQGPPEIYSDTQFPSLQ | colon or rectum, pleura, malignant mesothelioma |
| 1008 | TPQGPPEIYSDTQFPSLQST | colon or rectum, pleura, malignant mesothelioma |
| 1009 | ANLQRAYSLAKEQR | kidney, clear cell renal cell carcinoma, adrenal gland, adrenal cortical carcinoma |
| 1010 | NLQRAYSLAKEQR | kidney, clear cell renal cell carcinoma, adrenal gland, adrenal cortical carcinoma |

(continued)

| Seq ID | Sequence | Tissue and disease |
|---|---|---|
| 1011 | TPSGITYDRKDIEEH | kidney, clear cell renal cell carcinoma, adrenal gland, adrenal cortical carcinoma |
| 1012 | VSTLNSEDFVLVSR | brain, glioblastoma, kidney, angiomyolipoma |
| 1013 | VSTLNSEDFVLVSRQ | brain, glioblastoma, kidney, angiomyolipoma |
| 1014 | VSTLNSEDFVLVSRQG | brain, glioblastoma, kidney, angiomyolipoma |
| 1015 | GSSFFGELFNQNPE | brain, glioblastoma, thyroid gland, papillary carcinoma |
| 1016 | SGSSFFGELFNQNPE | brain, glioblastoma, thyroid gland, papillary carcinoma |

Thus, disclosed is the use of the peptides as disclosed for the - preferably combined - treatment of a proliferative disease selected from the group of adrenal cortical adenoma; non-ossifying fibroma; brain cancer and a proliferative disease selected from kidney oncocytoma, kidney Wilm's tumor, lymph node malignant melanoma, and omentum leiomyosarcoma; glioblastoma and a proliferative disease selected from oligodendroglioma, kidney angiomyolipoma, liver hepatic adenoma, liver hepatocellular carcinoma, lung small cell carcinoma, parotid gland pleomorphic adenoma, pleura malignant mesothelioma, schwannoma, small intestine gastrointestinal stromal tumor (GIST), and thyroid gland papillary carcinoma; breast carcinoma; chondrosarcoma; colonal or rectal cancer and a proliferative disease selected from bone giant cell tumor of bone, bone, non-ossifying fibroma, breast mucinous carcinoma, colon adenocarcinoma, colon adenoma, endometrium adenocarcinoma endometrioid type, esophagus adenocarcinoma, kidney angiomyolipoma, kidney renal cell carcinoma, liposarcoma, liver hepatocellular carcinoma, ovary granulosa cell tumor, pancreas microcystic adenoma, pleura malignant mesothelioma, prostate benign nodular hyperplasia, spleen non-Hodgkin's lymphoma, stomach mucinous adenocarcinoma, thymus thymoma, malignant, thyroid gland nodular hyperplasia, urinary bladder, transitional cell carcinoma, and vulva squamous cell carcinoma; colon adenoma; esophagus adenocarcinoma; intestines malignant carcinoid tumor; intramuscular lipoma; kidney clear cell renal cell carcinoma and a proliferative disease selected from adrenal gland, adrenal cortical carcinoma, endometrium adenocarcinoma endometrioid type, endometrium adenocarcinoma endometrioid type, kidney angiomyolipoma leiomyosarcoma, lipoma liver hepatocellular carcinoma, lymph node Hodgkin's disease, non-Hodgkin's lymphoma, pancreas adenocarcinoma, parotid gland pleomorphic adenoma, prostate adenocarcinoma, rectum adenocarcinoma, spleen chronic myeloid leukemia, spleen non-Hodgkin's lymphoma, and thyroid gland follicular adenoma; kidney oncocytoma; kidney polycystic kidney disease; kidney renal cell carcinoma; lipoma; liver hepatocellular carcinoma and a proliferative disease selected from, adrenal gland adrenal cortical adenoma, breast carcinoma, liver focal nodular hyperplasia, cancer rectum adenocarcinoma, cancer thyroid gland, nodular hyperplasia, cancer thyroid gland, papillary carcinoma, colon non-Hodgkin's lymphoma, endometrium hyperplasia, hepatic adenoma, kidney carcinoma, kidney oncocytoma, lipoma, liposarcoma, liver focal nodular hyperplasia, liver hepatic adenoma, pleura malignant mesothelioma, neuroblastoma, pancreas adenocarcinoma, pancreas microcystic adenoma, parotid gland pleomorphic adenoma, pleura malignant mesothelioma, synovial sarcoma, thyroid gland nodular hyperplasia, and uterine cervix squamous cell carcinoma; lung, non-small cell lung carcinoma, and a proliferative disease selected from breast carcinoma, chondrosarcoma, kidney oncocytoma, liver hepatocellular carcinoma, lung adenocarcinoma, lymph node Hodgkin's disease, lymph node non-Hodgkin's lymphoma, lymph node papillary carcinoma of thyroid, omentum adenocarcinoma, ovary Mullerian mixed tumor, pancreas adenocarcinoma, testis mixed germ cell tumor, thymus thymoma benign, and thyroid gland, nodular hyperplasia; lymph node Hodgkin's disease; lymph node papillary carcinoma of thyroid; lymph node papillary carcinoma of thyroid metastatic; myometrium leiomyoma; non-Hodgkin's lymphoma; non-Hodgkin's lymphoma, peripheral T cell type or small lymphocytic type; pancreas adenocarcinoma and a proliferative disease selected from bone giant cell tumor of bone, colon adenocarcinoma, fibromatosis, intramuscular lipoma, kidney angiomyolipoma, kidney renal cell carcinoma, liver hepatic adenoma, lung adenocarcinoma, myometrium leiomyoma, non-Hodgkin's lymphoma small lymphocytic type, pancreas adenocarcinoma, prostate benign nodular hyperplasia, rectum adenocarcinoma, spleen chronic myeloid leukemia, and thymus, thymoma, malignant; rectum adenocarcinoma; spleen chronic myeloid leukemia; spleen extramedullary hematopoiesis; stomach, adenocarcinoma and a proliferative disease selected from, adrenal gland adrenal cortical adenoma, bone giant cell tumor of bone, bone non-ossifying fibroma, breast carcinoma, colon adenocarcinoma, colon non-Hodgkin's lymphoma, endometrium adenocarcinoma endometrioid, kidney angiomyolipoma, kidney carcinoma, kidney oncocytoma, liver, focal nodular hyperplasia, liver hepatocellular carcinoma, lymph node Hodgkin's disease, lymph node papillary carcinoma of thyroid, medullary carcinoma of thyroid origin, metastatic adenocarcinoma of stomach, neurofibroma, ovary thecoma-fibroma, pancreas adenocarcinoma, pancreas microcystic adenoma, parathyroid gland adenoma, rectum adenocarcinoma, skin squamous cell carcinoma, spleen chronic myeloid leukemia, stomach gastrointestinal stromal tumor (GIST), thyroid gland nodular hyperplasia, thyroid gland papillary carcinoma, uterin cervix squamous cell carcinoma, and white blood cells chronic lymphocytic leukemia; stomach gastrointestinal stromal tumor (GIST); stomach cancer metastatic and a proliferative disease selected from adrenal gland adrenal cortical carcinoma, thyroid gland papillary carcinoma, skin, squamous cell carcinoma, breast carcinoma, colon adenocarcinoma , endometrium Mullerian mixed tumor, kidney carcinoma, leiomyosarcoma, lung neuroendocrine carcinoma (non-small cell type), lymph node non-Hodgkin's lymphoma, non-Hodgkin's lymphoma, ovary Mullerian mixed tumor, pancreas adenocarcinoma, rectum adenocarcinoma, skin basal cell carcinoma, stomach gastrointestinal stromal tumor (GIST), and uterine cervix adenocarcinoma; testis seminoma; thymus benign thymoma; thyroid gland follicular adenoma; and thyroid gland nodular hyperplasia.

Disclosed is the use of the peptides as disclosed for the - preferably combined - preferred immunotherapy of diseases according to the following table 4.

**Table 4:** Preferred peptides and diseases to be treated. SEQ ID NO: 167 is according to the invention.

| Seq ID | Sequence | Tissue and disease |
|---|---|---|
| 22 | LEVEERTKPV | lung, non-small cell lung carcinoma, breast, carcinoma |
| 23 | RDSPINANLRY | lung, non-small cell lung carcinoma, breast, carcinoma |
| 24 | RPFVIVTA | lung, non-small cell lung carcinoma, breast, carcinoma |
| 25 | RPIINTPMV | lung, non-small cell lung carcinoma, breast, carcinoma |
| 26 | SPTSSRTSSL | lung, non-small cell lung carcinoma, breast, carcinoma |
| 27 | ATSAPLVSR | stomach, metastatic, lung, neuroendocrine carcinoma |
| 114 | YGNPRTNGM | stomach, metastatic, breast, carcinoma |
| 102 | FSITKSVEL | non-Hodgkin's lymphoma, small lymphocytic type |
| 103 | GQTKNDLVV | non-Hodgkin's lymphoma, small lymphocytic type |
| 104 | LSQEVCRD | non-Hodgkin's lymphoma, small lymphocytic type |
| 105 | RDIQSPEQI | non-Hodgkin's lymphoma, small lymphocytic type |
| 106 | REDNSSNSL | non-Hodgkin's lymphoma, small lymphocytic type |
| 107 | TEHQEPGL | non-Hodgkin's lymphoma, small lymphocytic type |
| 108 | TKNDLVVSL | non-Hodgkin's lymphoma, small lymphocytic type |
| 977 | AGREINLVDAHLKSE | lymph node, Hodgkin's disease |
| 979 | GREINLVDAHLKSE | lymph node, Hodgkin's disease |
| 980 | KPGIVYASLNHSVIG | lymph node, Hodgkin's disease |
| 220 | RIHTGEKPYK | colon or rectum, thyroid gland, nodular hyperplasia |
| 53 | APGSVLPRAL | lymph node, Hodgkin's disease |
| 54 | DIKEHPLL | lymph node, Hodgkin's disease |
| 55 | DSAGPQDAR | lymph node, Hodgkin's disease |
| 56 | FQYAKESYI | lymph node, Hodgkin's disease |
| 57 | KVLSWPFLM | lymph node, Hodgkin's disease |
| 58 | LENDQSLSF | lymph node, Hodgkin's disease |
| 59 | SPSRQPQV | lymph node, Hodgkin's disease |
| 60 | SRHQSFTTK | lymph node, Hodgkin's disease |
| 61 | SSHNASKTL | lymph node, Hodgkin's disease |
| 1003 | DNQYAVLENQKSSH | colon or rectum, pleura, malignant mesothelioma, |
| 1004 | GPPEIYSDTQFPS | colon or rectum, pleura, malignant mesothelioma, |
| 1005 | GPPEIYSDTQFPSLQ | colon or rectum, pleura, malignant mesothelioma, |
| 1006 | TPQGPPEIYSDTQFPS | colon or rectum, pleura, malignant mesothelioma, |
| 1007 | TPQGPPEIYSDTQFPSLQ | colon or rectum, pleura, malignant mesothelioma, |
| 1008 | TPQGPPEIYSDTQFPSLQST | colon or rectum, pleura, malignant mesothelioma, |
| 91 | EHADDDPSL | kidney, Wilm's tumor |

(continued)

| Seq ID | Sequence | Tissue and disease |
|--------|----------|---------------------|
| 92 | SEESVKSTTL | kidney, Wilm's tumor |
| 93 | SPRPPLGSSL | kidney, Wilm's tumor |
| 94 | SPWWRSSL | kidney, Wilm's tumor |
| 95 | VYTPVDSLVF | kidney, Wilm's tumor |
| 18 | DALLKRTM | stomach, metastatic, skin, basal cell carcinoma |
| 19 | GEDVRSALL | stomach, metastatic, skin, basal cell carcinoma |
| 20 | KFAEEFYSF | stomach, metastatic, skin, basal cell carcinoma |
| 21 | YGYDNVKEY | stomach, metastatic, skin, basal cell carcinoma |
| 661 | EYVSLYHQPAAM | non-Hodgkin's lymphoma, peripheral T cell type |
| 664 | LPYLFQMPAYASSS | non-Hodgkin's lymphoma, peripheral T cell type |
| 665 | LPYLFQMPAYASSSK | non-Hodgkin's lymphoma, peripheral T cell type |
| 666 | NFIKAEYKGRVT | non-Hodgkin's lymphoma, peripheral T cell type |
| 667 | TNFIKAEYKGRVT | non-Hodgkin's lymphoma, peripheral T cell type |
| 668 | TTNFIKAEYKGRVT | non-Hodgkin's lymphoma, peripheral T cell type |
| 780 | DNGHLYREDQTSPAPG | kidney, angiomyolipoma |
| 781 | DNGHLYREDQTSPAPGLR | kidney, angiomyolipoma |
| 782 | EVQVFAPANALPARSE | kidney, angiomyolipoma |
| 783 | GHLYREDQTSPAPG | kidney, angiomyolipoma |
| 784 | LPARSEAAAVQPVIG | kidney, angiomyolipoma |
| 785 | NGHLYREDQTSPAPG | kidney, angiomyolipoma |
| 786 | NGHLYREDQTSPAPGL | kidney, angiomyolipoma |
| 787 | NGHLYREDQTSPAPGLR | kidney, angiomyolipoma |
| 788 | VFAPANALPARSEAA | kidney, angiomyolipoma |
| 789 | VQVFAPANALPARSE | kidney, angiomyolipoma |
| 178 | HEIDRYTAI | non-Hodgkin's lymphoma, follicular type, |
| 179 | VFTLKPLEF | non-Hodgkin's lymphoma, follicular type, |
| 180 | YWVPRNAL | non-Hodgkin's lymphoma, follicular type, |
| 694 | DGELIRTQPQRLPQ | pancreas, adenocarcinoma, intramuscular lipoma |
| 695 | GELIRTQPQRLPQ | pancreas, adenocarcinoma, intramuscular lipoma |
| 696 | NPSDGELIRTQPQRLP | pancreas, adenocarcinoma, intramuscular lipoma |
| 697 | NPSDGELIRTQPQRLPQ | pancreas, adenocarcinoma, intramuscular lipoma |
| 698 | NPSDGELIRTQPQRLPQL | pancreas, adenocarcinoma, intramuscular lipoma |
| 922 | FDFSQNTRVPRLPE | non-Hodgkin's lymphoma, follicular type |
| 923 | GDAPAILFDKEF | non-Hodgkin's lymphoma, follicular type |
| 924 | VTHEIDRYTAIAY | non-Hodgkin's lymphoma, follicular type |
| 692 | DAGSYKAQINQRNFE | lymph node, non-Hodgkin's lymphoma |
| 693 | DAGSYKAQINQRNFEVT | lymph node, non-Hodgkin's lymphoma |
| 1 | AEHPNVTLTI | spleen, non-Hodgkin's lymphoma |
| 2 | FLAEHPNVTL | spleen, non-Hodgkin's lymphoma |
| 4 | EVAEFLARH | spleen, non-Hodgkin's lymphoma |
| 5 | RHSNVNLTI | spleen, non-Hodgkin's lymphoma |
| 222 | QSTQRSLAL | uterine cervix, squamous cell carcinoma |
| 223 | RDLQMNQALRF | uterine cervix, squamous cell carcinoma |
| 224 | RELESQLHVL | uterine cervix, squamous cell carcinoma |
| 225 | SEAEKLTLV | uterine cervix, squamous cell carcinoma |
| 6 | HPDNVKLFL | pancreas, adenocarcinoma, non-Hodgkin's lymphoma, small lymphocytic type |
| 7 | ISDTGELKL | pancreas, adenocarcinoma, non-Hodgkin's lymphoma, small lymphocytic type |

(continued)

| Seq ID | Sequence | Tissue and disease |
|---|---|---|
| 8 | KVNGKLVALK | pancreas, adenocarcinoma, non-Hodgkin's lymphoma, small lymphocytic type |
| 9 | NRLSAQAAL | pancreas, pancreas, adenocarcinoma, non-Hodgkin's lymphoma, small lymphocytic type |
| 10 | TPFTAIREA | pancreas, pancreas, adenocarcinoma, non-Hodgkin's lymphoma, small lymphocytic type |
| 11 | FGLARAKSV | kidney, clear cell renal cell carcinoma, kidney, renal cell carcinoma, clear cell type |
| 12 | KIADFGLAR | brain, glioblastoma, liver, hepatocellular carcinoma |
| 812 | DGSYRIFSKGASE | colon or rectum, liposarcoma |
| 813 | GSYRIFSKGASE | colon or rectum, liposarcoma |
| 814 | SDGSYRIFSKGASE | colon or rectum, liposarcoma |
| 815 | SVKKMMKDNNLVRH | colon or rectum, liver, hepatocellular carcinoma |
| 816 | VKKMMKDNNLVRH | colon or rectum, liver, hepatocellular carcinoma |
| 145 | KITVPASQK | colon, non-Hodgkin's lymphoma |
| 146 | KITVPASQKL | colon, non-Hodgkin's lymphoma |
| 147 | VPASQKLRQL | colon, non-Hodgkin's lymphoma |
| 537 | ITARPVLW | non-Hodgkin's lymphoma, diffuse large B-cell type |
| 538 | KLMSPKLYVW | non-Hodgkin's lymphoma, diffuse large B-cell type |
| 539 | KVSAVTLAY | non-Hodgkin's lymphoma, diffuse large B-cell type |
| 540 | VEGSGELFRW | non-Hodgkin's lymphoma, diffuse large B-cell type |
| 672 | ADLSSFKSQELN | lymph node, papillary carcinoma of thyroid, metastatic |
| 673 | ADLSSFKSQELNER | lymph node, papillary carcinoma of thyroid, metastatic |
| 674 | ADLSSFKSQELNERN | lymph node, papillary carcinoma of thyroid, metastatic |
| 679 | ISQELEELRAEQQR | lymph node, papillary carcinoma of thyroid, metastatic |
| 680 | ISQELEELRAEQQRLK | lymph node, papillary carcinoma of thyroid, metastatic |
| 681 | KGTKQWVHARYA | lymph node, papillary carcinoma of thyroid, metastatic |
| 682 | QADLSSFKSQELNER | lymph node, papillary carcinoma of thyroid, metastatic |
| 684 | TGSWIGLRNLDLKG | lymph node, papillary carcinoma of thyroid, metastatic |
| 743 | AIVQAVSAHRHR | non-Hodgkin's lymphoma, peripheral T cell type |
| 744 | ARNFERNKAIKVI | non-Hodgkin's lymphoma, peripheral T cell type |
| 745 | ARNFERNKAIKVIIA | non-Hodgkin's lymphoma, peripheral T cell type |
| 746 | NFERNKAIKVII | non-Hodgkin's lymphoma, peripheral T cell type |
| 747 | NFERNKAIKVIIA | non-Hodgkin's lymphoma, peripheral T cell type |
| 748 | VAIVQAVSAHRH | non-Hodgkin's lymphoma, peripheral T cell type |
| 749 | VAIVQAVSAHRHR | non-Hodgkin's lymphoma, peripheral T cell type |
| 750 | VAIVQAVSAHRHRA | non-Hodgkin's lymphoma, peripheral T cell type |
| 818 | VDKVLERDQKLSE | lung, non-small cell lung carcinoma, lymph node, papillary carcinoma of thyroid, metastatic |
| 819 | VDKVLERDQKLSELD | lung, non-small cell lung carcinoma, lymph node, papillary carcinoma of thyroid, metastatic |

(continued)

| Seq ID | Sequence | Tissue and disease |
|---|---|---|
| 820 | VDKVLERDQKLSELDD | lung, non-small cell lung carcinoma, lymph node, papillary carcinoma of thyroid, metastatic |
| 821 | VDKVLERDQKLSELDDR | stomach, diffuse subtype adenocarcinoma, lymph node, papillary carcinoma of thyroid, metastatic |
| 822 | VLERDQKLSELDDR | lung, non-small cell lung carcinoma, lymph node, papillary carcinoma of thyroid, metastatic |
| 833 | VDKVLERDQKLSE | lung, non-small cell lung carcinoma, lymph node, papillary carcinoma of thyroid, metastatic |
| 834 | VDKVLERDQKLSELD | lung, non-small cell lung carcinoma, lymph node, papillary carcinoma of thyroid, etastatic |
| 835 | VDKVLERDQKLSELDD | lung, non-small cell lung carcinoma, lymph node, papillary carcinoma of thyroid, metastatic |
| 836 | VDKVLERDQKLSELDDR | stomach, diffuse subtype adenocarcinoma, lymph node, papillary carcinoma of thyroid, metastatic |
| 837 | VLERDQKLSELDDR | lung, non-small cell lung carcinoma, lymph node, papillary carcinoma of thyroid, metastatic |
| 848 | DDPAIDVCKKLLGKYPN | kidney, clear cell renal cell carcinoma, pancreas, adenocarcinoma |
| 849 | DKQPYSKLPGVSLLKP | kidney, clear cell renal cell carcinoma, pancreas, adenocarcinoma |
| 850 | DKQPYSKLPGVSLLKPL | kidney, clear cell renal cell carcinoma, pancreas, adenocarcinoma |
| 851 | HPRYYISANVTGFK | kidney, clear cell renal cell carcinoma, pancreas, adenocarcinoma |
| 852 | SHPRYYISANVTG | kidney, clear cell renal cell carcinoma, pancreas, adenocarcinoma |
| 853 | SHPRYYISANVTGFK | kidney, clear cell renal cell carcinoma, pancreas, adenocarcinoma |
| 854 | TSHPRYYISANVTG | kidney, clear cell renal cell carcinoma, pancreas, adenocarcinoma |
| 855 | TSHPRYYISANVTGFK | kidney, clear cell renal cell carcinoma, pancreas, adenocarcinoma |
| 908 | DVGMFVALTKLGQPD | stomach, differentiated subtype adenocarcinoma, uterine cervix, squamous cell carcinoma, |
| 909 | VGMFVALTKLGQPD | stomach, differentiated subtype adenocarcinoma, uterine cervix, squamous cell carcinoma |
| 1015 | GSSFFGELFNQNPE | brain, glioblastoma, thyroid gland, papillary carcinoma |
| 1016 | SGSSFFGELFNQNPE | brain, glioblastoma, thyroid gland, papillary carcinoma |
| 466 | DEMRFVTQI | testis, mixed germ cell tumor |
| 467 | ETVHFATTQW | testis, mixed germ cell tumor |
| 468 | LPPPATQI | testis, mixed germ cell tumor |
| 633 | GNPAYRSFSNSLSQ | kidney, angiomyolipoma |
| 634 | GPPGEAGYKAFSSLLA | kidney, angiomyolipoma |
| 635 | GPPGEAGYKAFSSLLASS | kidney, angiomyolipoma |
| 636 | GPPGEAGYKAFSSLLASSA | kidney, angiomyolipoma |
| 637 | GPPGEAGYKAFSSLLASSA VSPE | kidney, angiomyolipoma |

(continued)

| Seq ID | Sequence | Tissue and disease |
|---|---|---|
| 638 | GPPGEAGYKAFSSLLASSA VSPEK | kidney, angiomyolipoma |
| 639 | GYKAFSSLLASSAVSP | kidney, angiomyolipoma |
| 640 | GYKAFSSLLASSAVSPE | kidney, angiomyolipoma |
| 641 | KAFSSLLASSAVSPE | kidney, angiomyolipoma |
| 642 | NPAYRSFSNSLSQ | kidney, angiomyolipoma |
| 643 | SRDDFQEGREGIVAR | kidney, angiomyolipoma |
| 644 | SSSSFHPAPGNAQ | kidney, angiomyolipoma |
| 645 | VARLTESLFLDL | kidney, angiomyolipoma |
| 646 | VARLTESLFLDLLG | kidney, angiomyolipoma |
| 647 | VIAGNPAYRSFSN | kidney, angiomyolipoma |
| 648 | VPQPEPETWEQILRRNVLQ | kidney, angiomyolipoma |
| 649 | YKAFSSLLASSAVS | kidney, angiomyolipoma |
| 650 | YKAFSSLLASSAVSP | kidney, angiomyolipoma |
| 651 | YKAFSSLLASSAVSPE | kidney, angiomyolipoma |
| 992 | EDDYIKSWEDNQQGDE | pleura, malignant mesothelioma |
| 993 | ELERIQIQEAAKKKPG | pleura, malignant mesothelioma |
| 994 | ERIQIQEAAKKKP | pleura, malignant mesothelioma |
| 995 | ERIQIQEAAKKKPG | pleura, malignant mesothelioma |
| 996 | ERIQIQEAAKKKPGI | pleura, malignant mesothelioma |
| 997 | LERIQIQEAAKKKPG | pleura, malignant mesothelioma |
| 998 | LSSISQYSGKIK | pleura, malignant mesothelioma |
| 941 | EERNLLSVAYKNVVGAR | colon or rectum, esophagus, adenocarcinoma, |
| 942 | ERNLLSVAYKNVVGAR | colon or rectum, esophagus, adenocarcinoma, |
| 943 | IAELDTLSEESYKD | colon or rectum, vulva, squamous cell carcinoma, |
| 944 | IAELDTLSEESYKDS | colon or rectum, vulva, squamous cell carcinoma, |
| 218 | GDYGRAFNL | stomach, metastatic, lymph node, non-Hodgkin's lymphoma, small lymphocytic type |
| 219 | TRHKIVHTK | stomach, metastatic, lymph node, non-Hodgkin's lymphoma, small lymphocytic type |
| 221 | KAFNWFSTL | stomach, metastatic, lymph node, non-Hodgkin's lymphoma, small lymphocytic type |
| 541 | RPKSNIVL | non-Hodgkin's lymphoma, diffuse large B-cell type |
| 542 | RPKSNIVLL | non-Hodgkin's lymphoma, diffuse large B-cell type |
| 1001 | INSRFPIPSATDPD | brain, glioblastoma, brain, oligodendroglioma, |
| 1002 | VQHYELLNGQSVFG | brain, glioblastoma, brain, oligodendroglioma, |
| 910 | AGVFHVEKNGRY | stomach, diffuse subtype adenocarcinoma, colon, adenocarcinoma |
| 911 | FAGVFHVEKNGRYS | stomach, diffuse subtype adenocarcinoma, colon, adenocarcinoma |
| 912 | GPITITIVNRDGTR | stomach, diffuse subtype adenocarcinoma, colon, adenocarcinoma |
| 913 | NGRYSISRTEAADL | stomach, diffuse subtype adenocarcinoma, colon, adenocarcinoma |
| 45 | DELPKFHQY | stomach, adenocarcinoma, white blood cells, chronic lymphocytic leukemia |
| 46 | DVTGQFPSSF | white blood cells, chronic lymphocytic leukemia |

(continued)

| Seq ID | Sequence | Tissue and disease |
|---|---|---|
| 47 | EHSRVLQQL | white blood cells, chronic lymphocytic leukemia |
| 48 | IKVSKQLL | white blood cells, chronic lymphocytic leukemia |
| 49 | KPRQSSPQL | white blood cells, chronic lymphocytic leukemia |
| 50 | KQLLAALEI | white blood cells, chronic lymphocytic leukemia |
| 51 | RRKDLVLKY | liver, focalnodular hyperplasia |
| 52 | RTRDYASLPPK | white blood cells, chronic lymphocytic leukemia |
| 124 | GQKEALLKY | liver, hepatocellular carcinoma, synovial sarcoma |
| 125 | KPSEERKTI | liver, hepatocellular carcinoma, synovial sarcoma |
| 126 | KQTPKVLVV | liver, hepatocellular carcinoma, synovial sarcoma |
| 127 | SVIQHVQSF | liver, hepatocellular carcinoma, synovial sarcoma |
| 128 | TPIERIPYL | liver, hepatocellular carcinoma, synovial sarcoma |
| 773 | LPEFYKTVSPAL | colon or rectum, endometrium, adenocarcinoma, endometrioid type |
| 774 | VGQFIQDVKNSRST | colon or rectum, endometrium, adenocarcinoma, endometrioid type |
| 775 | VGQFIQDVKNSRSTD | colon or rectum, endometrium, adenocarcinoma, endometrioid type |
| 776 | WGQFIQDVKNSRS | colon or rectum, endometrium, adenocarcinoma, endometrioid type |
| 777 | WGQFIQDVKNSRST | colon or rectum, endometrium, adenocarcinoma, endometrioid type |
| 778 | WGQFIQDVKNSRSTD | colon or rectum, endometrium, adenocarcinoma, endometrioid type |
| 779 | WGQFIQDVKNSRSTDS | colon or rectum, endometrium, adenocarcinoma, endometrioid type |
| 685 | FGNYNNQSSNFGPMKGGNFGGRS | pancreas, adenocarcinoma, thymus, thymoma, malignant |
| 686 | FGPMKGGNFGGRSSGPYGGGGQY | pancreas, adenocarcinoma, thymus, thymoma, malignant |
| 687 | GPMKGGNFGGRSSGP | pancreas, adenocarcinoma, thymus, thymoma, malignant |
| 688 | GPYGGGGQYFAKP | pancreas, adenocarcinoma, thymus, thymoma, malignant |
| 689 | KGGNFGGRSSGP | pancreas, adenocarcinoma, thymus, thymoma, malignant |
| 690 | NDFGNYNNQSSNFGP | pancreas, adenocarcinoma, thymus, thymoma, malignant |
| 691 | SGPYGGGGQYFAKP | pancreas, adenocarcinoma, thymus, thymoma, malignant |
| 13 | AAANIIRTL | liver, hepatocellular carcinoma, adrenal gland, adrenal cortical carcinoma |
| 14 | GRFKNLREAL | liver, hepatocellular carcinoma, adrenal gland, adrenal cortical carcinoma, |
| 15 | MSPFSKATL | liver, hepatocellular carcinoma, adrenal gland, adrenal cortical carcinoma, |
| 16 | QEDPGDNQITL | liver, hepatocellular carcinoma, adrenal gland, adrenal cortical carcinoma, |
| 17 | SPFSKATL | liver, hepatocellular carcinoma, adrenal gland, adrenal cortical carcinoma, |

(continued)

| Seq ID | Sequence | Tissue and disease |
|---|---|---|
| 129 | AEVEKNETV | kidney, clear cell renal cell carcinoma, spleen, non-Hodgkin's lymphoma, follicular type |
| 130 | EVKEEIPLV | kidney, clear cell renal cell carcinoma, spleen, non-Hodgkin's lymphoma, follicular type |
| 131 | KPTSARSGL | kidney, clear cell renal cell carcinoma, spleen, non-Hodgkin's lymphoma, follicular type |
| 132 | KYIETTPLTI | kidney, clear cell renal cell carcinoma, spleen, non-Hodgkin's lymphoma, follicular type |
| 133 | SEIKTSIEV | kidney, clear cell renal cell carcinoma, spleen, non-Hodgkin's lymphoma, follicular type |
| 134 | SVKPTSATK | kidney, clear cell renal cell carcinoma, spleen, non-Hodgkin's lymphoma, follicular type |
| 135 | YPNKGVGQA | kidney, clear cell renal cell carcinoma, spleen, non-Hodgkin's lymphoma, follicular type |
| 966 | ENNEIISNIRDSVIN | stomach, adenocarcinoma, kidney, oncocytoma |
| 967 | NNEIISNIRDSVIN | stomach, adenocarcinoma, kidney, oncocytoma |
| 968 | SPTVQVFSASGKPV | stomach, adenocarcinoma, kidney, oncocytoma |
| 969 | SSPTVQVFSASGKPVE | stomach, adenocarcinoma, kidney, oncocytoma |
| 830 | DIMRVNVDKVLERDQK | stomach, diffuse subtype adenocarcinoma, Medullary carcinoma of thyroid Origin |
| 831 | DIMRVNVDKVLERDQKL | stomach, diffuse subtype adenocarcinoma, medullary carcinoma |
| 832 | IMRVNVDKVLERDQK | lung, non-small cell lung carcinoma, lymph node, Hodgkin's disease |
| 752 | EEVITLIRSNQQLE | pancreas, adenocarcinoma |
| 753 | EEVITLIRSNQQLEN | pancreas, adenocarcinoma |
| 754 | IPADTFAALKNPNAML | pancreas, adenocarcinoma |
| 755 | LKQLLSDKQQKRQSG | pancreas, adenocarcinoma |
| 756 | LKQLLSDKQQKRQSGQ | pancreas, adenocarcinoma |
| 118 | DEHLLIQHY | parotid gland, pleomorphic adenoma |
| 119 | KQVASSTGF | parotid gland, pleomorphic adenoma |
| 120 | RDFGPASQHFL | parotid gland, pleomorphic adenoma |
| 121 | RQLGEVASF | parotid gland, pleomorphic adenoma |
| 122 | TEAETTANVL | parotid gland, pleomorphic adenoma |
| 123 | GYLPVQTVL | kidney, clear cell renal cell carcinoma, parotid gland, pleomorphic adenoma |
| 987 | TRPYHSLPSEAVFA | adrenal gland, adrenal cortical adenoma |
| 988 | TRPYHSLPSEAVFAN | adrenal gland, adrenal cortical adenoma |
| 989 | VAVFTFHNHGRT | adrenal gland, adrenal cortical adenoma |
| 990 | VAVFTFHNHGRTA | adrenal gland, adrenal cortical adenoma |
| 991 | VAVFTFHNHGRTANL | adrenal gland, adrenal cortical adenoma |
| 339 | FLDPDIGGVAV | kidney, clear cell renal cell carcinoma, pancreas, adenocarcinoma |
| 340 | HTAPPENKTW | kidney, clear cell renal cell carcinoma, pancreas, adenocarcinoma |
| 341 | LLDTPVKTQY | kidney, clear cell renal cell carcinoma, pancreas, adenocarcinoma |
| 342 | NAVKDFTSF | kidney, clear cell renal cell carcinoma, pancreas, adenocarcinoma |

(continued)

| Seq ID | Sequence | Tissue and disease |
|---|---|---|
| 343 | SGLLQIKKL | kidney, clear cell renal cell carcinoma, pancreas, adenocarcinoma |
| 344 | YHDKNIVLL | kidney, clear cell renal cell carcinoma, pancreas, adenocarcinoma |
| 71 | HLKSIPVSL | kidney, clear cell renal cell carcinoma, prostate, adenocarcinoma |
| 72 | KVWYNVENW | kidney, clear cell renal cell carcinoma, prostate, adenocarcinoma |
| 73 | LPAYRAQLL | kidney, clear cell renal cell carcinoma, prostate, adenocarcinoma |
| 74 | LSEQTSVPL | kidney, clear cell renal cell carcinoma, prostate, adenocarcinoma |
| 75 | SLNQWLVSF | kidney, clear cell renal cell carcinoma, prostate, adenocarcinoma |
| 76 | SMTSLAQKI | kidney, clear cell renal cell carcinoma, prostate, adenocarcinoma |
| 77 | SSSGLHPPK | kidney, clear cell renal cell carcinoma, prostate, adenocarcinoma |
| 578 | GGGYGSGGGSGGYGSRRF | colon or rectum, thymus, thymoma, malignant, |
| 579 | GGSFGGRSSGSP | colon or rectum, thymus, thymoma, malignant |
| 580 | KGGSFGGRSSGSP | colon or rectum, thymus, thymoma, malignant |
| 581 | SGQQQSNYGPMKGGSFGGRSSGSPY | colon or rectum, thymus, thymoma, malignant |
| 582 | SGSPYGGGYGSGGGSGGYGSRRF | colon or rectum, thymus, thymoma, malignant |
| 583 | SPYGGGYGSGGGSGGYGSRRF | colon or rectum, thymus, thymoma, malignant |
| 584 | YGGGYGSGGGSGGYGSRRF | colon or rectum, thymus, thymoma, malignant |
| 84 | VPVPHTTAL | kidney, clear cell renal cell carcinoma, endometrium, adenocarcinoma, endometrioid type |
| 85 | YQVLDVQRY | kidney, clear cell renal cell carcinoma, endometrium, adenocarcinoma, endometrioid type |
| 731 | DGLNSLTYQVLDVQRYPL | kidney, clear cell renal cell carcinoma, endometrium, adenocarcinoma, endometrioid type |
| 732 | HPVLQRQQLDYGIY | kidney, clear cell renal cell carcinoma, endometrium, adenocarcinoma, endometrioid type |
| 733 | LNSLTYQVLDVQR | kidney, clear cell renal cell carcinoma, endometrium, adenocarcinoma, endometrioid type |
| 734 | LNSLTYQVLDVQRYP | kidney, clear cell renal cell carcinoma, endometrium, adenocarcinoma, endometrioid type |
| 735 | LNSLTYQVLDVQRYPL | kidney, clear cell renal cell carcinoma, endometrium, adenocarcinoma, endometrioid type |
| 736 | LPQLVGVSTPLQG | kidney, clear cell renal cell carcinoma, endometrium, adenocarcinoma, endometrioid type |
| 737 | LPQLVGVSTPLQGG | kidney, clear cell renal cell carcinoma, endometrium, adenocarcinoma, endometrioid type |
| 738 | LPQLVGVSTPLQGGS | kidney, clear cell renal cell carcinoma, endometrium, adenocarcinoma, endometrioid type |

(continued)

| Seq ID | Sequence | Tissue and disease |
|---|---|---|
| 739 | RLPQLVGVSTPLQGGS | kidney, clear cell renal cell carcinoma, endometrium, adenocarcinoma, endometrioid type |
| 740 | SPHKVAIIIPFRNR | kidney, clear cell renal cell carcinoma, endometrium, adenocarcinoma, endometrioid type |
| 741 | SPHKVAIIIPFRNRQE | kidney, clear cell renal cell carcinoma, endometrium, adenocarcinoma, endometrioid type |
| 742 | SPHKVAIIIPFRNRQEH | kidney, clear cell renal cell carcinoma, endometrium, adenocarcinoma, endometrioid type |
| 527 | DEKQQHIVY | liver, hepatocellular carcinoma, synovial sarcoma |
| 528 | DEVYQVTVY | liver, hepatocellular carcinoma, synovial sarcoma |
| 529 | GEISEKAKL | liver, hepatocellular carcinoma, synovial sarcoma |
| 530 | YTMKEVLFY | liver, hepatocellular carcinoma, synovial sarcoma |
| 203 | GPRPITQSEL | lymph node, non-Hodgkin's lymphoma, marginal zone B-cell type |
| 204 | KPEPVDKVA | lymph node, non-Hodgkin's lymphoma |
| 205 | TPSSRPASL | lymph node, non-Hodgkin's lymphoma |
| 949 | SPPQFRVNGAISN | ovary, granulosa cell tumor |
| 950 | SPPQFRVNGAISNFE | ovary, granulosa cell tumor |
| 951 | SPPQFRVNGAISNFEE | ovary, granulosa cell tumor |
| 952 | SPPQFRVNGAISNFEEF | ovary, granulosa cell tumor |
| 953 | VGKMFVDVYFQEDKK | ovary, granulosa cell tumor |
| 954 | VGKmFVDVYFQEDKKE | ovary, granulosa cell tumor |
| 916 | EEFKKLTSIKIQNDK | brain, glioblastoma, small intestine, gastrointestinal stromal tumor (GIST) |
| 917 | INRRMADDNKLFR | brain, glioblastoma, small intestine, gastrointestinal stromal tumor (GIST) |
| 918 | TATIVMVTNLKERKE | brain, glioblastoma, small intestine, gastrointestinal stromal tumor (GIST) |
| 526 | RINEFSISSF | chondrosarcoma |
| 585 | GNRINEFSISSF | chondrosarcoma |
| 586 | HGNQITSDKVGRKV | chondrosarcoma |
| 587 | IPPVNTNLENLYLQ | chondrosarcoma |
| 588 | LQVLRLDGNEIKR | chondrosarcoma |
| 589 | LQVLRLDGNEIKRS | chondrosarcoma |
| 590 | LQVLRLDGNEIKRSA | chondrosarcoma |
| 592 | LYVRLSHNSLTNNG | chondrosarcoma |
| 596 | WIALHGNQITSD | chondrosarcoma |
| 597 | WIALHGNQITSDK | chondrosarcoma |
| 165 | ELNKLLEEI | ovary, adenocarcinoma |
| 166 | IPFSNPRVL | ovary, adenocarcinoma |
| 167 | LLDEGAKLLY | ovary, adenocarcinoma |
| 168 | SPADAHRNL | ovary, adenocarcinoma |
| 96 | APLQRSQSL | kidney, renal cell carcinoma, clear cell type |
| 97 | DEVHQDTY | kidney, renal cell carcinoma, clear cell type |
| 98 | LPHSATVTL | kidney, renal cell carcinoma, clear cell type |
| 152 | APSEYRYTL | stomach, mucinous adenocarcinoma |
| 153 | APSEYRYTLL | stomach, mucinous adenocarcinoma |
| 154 | EIFQNEVAR | stomach, mucinous adenocarcinoma |
| 155 | KDVLIPGKL | stomach, mucinous adenocarcinoma |

(continued)

| Seq ID | Sequence | Tissue and disease |
|---|---|---|
| 156 | VPLVREITF | stomach, mucinous adenocarcinoma |
| 62 | EEIDTTMRW | liver, hepatocellular carcinoma, lipoma |
| 63 | ILDEKPVII | liver, hepatocellular carcinoma, lipoma |
| 64 | LPQEPRTSL | liver, hepatocellular carcinoma, lipoma |
| 65 | LTYKLPVA | liver, hepatocellular carcinoma, lipoma |
| 66 | NEMELAHSSF | liver, hepatocellular carcinoma, lipoma |
| 67 | REFPEANFEL | liver, hepatocellular carcinoma, lipoma |
| 68 | THHIPDAKL | liver, hepatocellular carcinoma, lipoma |
| 69 | TVKENLSLF | liver, hepatocellular carcinoma, lipoma |
| 70 | VLLKKAVL | liver, hepatocellular carcinoma, lipoma |
| 136 | ISMKILNSL | lung, non-small cell lung carcinoma, thymus, thymoma |
| 137 | KTIAFLLPMF | lung, non-small cell lung carcinoma, thymus, thymoma |
| 138 | RDSIINDF | lung, non-small cell lung carcinoma, thymus, thymoma |
| 139 | SVKGGGGNEK | lung, non-small cell lung carcinoma, thymus, thymoma |
| 140 | GIAKTGSGK | lung, non-small cell lung carcinoma, thymus, thymoma |
| 503 | ALYATKTLR | pancreas, microcystic adenoma |
| 504 | MEYVISRI | pancreas, microcystic adenoma |
| 505 | VPVGRQPII | pancreas, microcystic adenoma |
| 278 | ATNGDLASR | pancreas, adenocarcinoma, prostate, benign nodular hyperplasia |
| 279 | GLHAEVTGVGY | pancreas, adenocarcinoma, prostate, benign nodular hyperplasia |
| 280 | HVSSTSSSF | pancreas, adenocarcinoma, prostate, benign nodular hyperplasia |
| 281 | LQADLQNGL | pancreas, adenocarcinoma, prostate, benign nodular hyperplasia |
| 282 | SELPVSEVA | pancreas, adenocarcinoma, prostate, benign nodular hyperplasia |
| 283 | SQTKSVFEI | pancreas, adenocarcinoma, prostate, benign nodular hyperplasia |
| 284 | THIFTSDGL | pancreas, adenocarcinoma, prostate, benign nodular hyperplasia |
| 285 | VIYFPPLQK | pancreas, adenocarcinoma, prostate, benign nodular hyperplasia |
| 286 | YPFSSEQKW | pancreas, adenocarcinoma, prostate, benign nodular hyperplasia |
| 78 | DLDVKKMPL | kidney, carcinoma |
| 79 | FYTVIPHNF | kidney, carcinoma |
| 80 | HHINTDNPSL | kidney, carcinoma |
| 81 | RVGEVGQSK | kidney, carcinoma |
| 28 | AELRSTASLL | lipoma |
| 29 | APASSHERASM | lipoma |
| 30 | ASRQAPPHI | lipoma |
| 31 | AVKKNPGIAA | lipoma |
| 32 | EEHLESHKKY | lipoma |

(continued)

| Seq ID | Sequence | Tissue and disease |
|---|---|---|
| 33 | GEFTSARAV | lipoma |
| 34 | GQSTPRLFSI | lipoma |
| 35 | LVDDPLEY | lipoma |
| 36 | RPKNLMQTL | lipoma |
| 37 | RQAPPHIEL | lipoma |
| 38 | SEAAELRSTA | lipoma |
| 490 | DSIGSTVSSER | stomach, adenocarcinoma, signet ring cell type, |
| 491 | LPYNNKDRDAL | stomach, adenocarcinoma, signet ring cell type, |
| 215 | DAMKRVEEI | ovary, thecoma-fibroma |
| 216 | DIKEVKQNI | ovary, thecoma-fibroma |
| 217 | GPIYPGHGM | ovary, thecoma-fibroma |
| 963 | GNTVIHLDQALARMR | lung, small cell carcinoma |
| 964 | NTVIHLDQALARMR | lung, small cell carcinoma |
| 965 | NTVIHLDQALARMRE | lung, small cell carcinoma |
| 187 | AADTERLAL | chondrosarcoma |
| 188 | DMKAKVASL | chondrosarcoma |
| 189 | HVLEEVQQV | chondrosarcoma |
| 190 | KEAADTERL | chondrosarcoma |
| 191 | RISEVLQKL | chondrosarcoma |
| 192 | TEVRELVSL | chondrosarcoma |
| 875 | SVEEFLSEKLERI | liver, hepatic adenoma |
| 876 | VEEFLSEKLERI | liver, hepatic adenoma |
| 973 | ADDLEGEAFLPL | spleen, chronic myeloid leukemia |
| 974 | ADDLEGEAFLPLR | spleen, chronic myeloid leukemia |
| 975 | ADDLEGEAFLPLRE | spleen, chronic myeloid leukemia |
| 976 | GADDLEGEAFLPLR | spleen, chronic myeloid leukemia |
| 141 | AETTDNVFTL | kidney, clear cell renal cell carcinoma, thyroid gland, follicular adenoma |
| 142 | SEYQRFAVM | kidney, clear cell renal cell carcinoma, thyroid gland, follicular adenoma |
| 143 | TFGERWAF | kidney, clear cell renal cell carcinoma, thyroid gland, follicular adenoma |
| 144 | NENLVERF | stomach, colon, adenocarcinoma, mucinous type |
| 117 | QLFSYAILGF | liver, hepatocellular carcinoma, colon, non-Hodgkin's lymphoma |
| 845 | GIRVAPVPLYNS | lung, non-small cell lung carcinoma, liver, hepatocellular carcinoma |
| 846 | GIRVAPVPLYNSFH | lung, non-small cell lung carcinoma, liver, hepatocellular carcinoma |
| 847 | NPNGIRVAPVPLYNSFH | lung, non-small cell lung carcinoma, liver, hepatocellular carcinoma |
| 478 | AAVPVIISR | lymph node, papillary carcinoma of thyroid, metastatic |
| 479 | EEIGKVAAA | lymph node, papillary carcinoma of thyroid, metastatic |
| 480 | FLKDLVASV | lymph node, papillary carcinoma of thyroid, metastatic |
| 481 | VIISRALEL | lymph node, papillary carcinoma of thyroid, metastatic |
| 420 | QIDYKTLVL | stomach, metastatic, leiomyosarcoma |

(continued)

| Seq ID | Sequence | Tissue and disease |
|---|---|---|
| 421 | VEDPTIVRI | stomach, metastatic, leiomyosarcoma |
| 543 | GEPLSYTRFSLARQ | lung, non-small cell lung carcinoma, lung, adenocarcinoma |
| 544 | GEPLSYTRFSLARQVD | lung, non-small cell lung carcinoma, lung, adenocarcinoma |
| 545 | GEPLSYTRFSLARQVDG | lung, non-small cell lung carcinoma, lung, adenocarcinoma |
| 546 | GGEPLSYTRFSLARQVD | lung, non-small cell lung carcinoma, lung, adenocarcinoma |
| 547 | GGEPLSYTRFSLARQVDG | lung, non-small cell lung carcinoma, lung, adenocarcinoma |
| 548 | NPGGYVAYSKAATVTG | lung, non-small cell lung carcinoma, lung, adenocarcinoma |
| 549 | NPGGYVAYSKAATVTGK | lung, non-small cell lung carcinoma, lung, adenocarcinoma |
| 550 | NPGGYVAYSKAATVTGKL | lung, non-small cell lung carcinoma, lung, adenocarcinoma |
| 551 | NSVIIVDKNGRL | lung, non-small cell lung carcinoma, lung, adenocarcinoma |
| 552 | NSVIIVDKNGRLV | lung, non-small cell lung carcinoma, lung, adenocarcinoma |
| 553 | NSVIIVDKNGRLVY | lung, non-small cell lung carcinoma, lung, adenocarcinoma |
| 554 | RVEYHFLSPYVSPK | lung, non-small cell lung carcinoma, lung, adenocarcinoma |
| 555 | RVEYHFLSPYVSPKE | lung, non-small cell lung carcinoma, lung, adenocarcinoma |
| 556 | RVEYHFLSPYVSPKESPF | lung, non-small cell lung carcinoma, lung, adenocarcinoma |
| 557 | SPFRHVFWGSGSHTL | lung, non-small cell lung carcinoma, lung, adenocarcinoma |
| 558 | SVIIVDKNGRLV | lung, non-small cell lung carcinoma, lung, adenocarcinoma |
| 559 | VEYHFLSPYVSPK | lung, non-small cell lung carcinoma, lung, adenocarcinoma |
| 560 | VEYHFLSPYVSPKE | lung, non-small cell lung carcinoma, lung, adenocarcinoma |
| 388 | AEGPAGGFMW | spleen, chronic myeloid leukemia |
| 389 | AYYRDAEAY | spleen, chronic myeloid leukemia |
| 390 | QVNRPLTMR | spleen, chronic myeloid leukemia |
| 391 | RHSPVFQVY | spleen, chronic myeloid leukemia |
| 392 | SLPVPNSAY | spleen, chronic myeloid leukemia |
| 393 | TLGPPGTAHLY | spleen, chronic myeloid leukemia |
| 308 | VLYVGSKTK | schwannoma |
| 309 | KTKEQVTNV | schwannoma |
| 310 | MPVDPDNEAY | schwannoma |
| 311 | AEKTKQGVA | schwannoma |
| 446 | EAFEFVKQR | stomach, adenocarcinoma, breast, carcinoma |
| 447 | NHFEGHYQY | stomach, adenocarcinoma, breast, carcinoma |

Disclosed is the use of the peptides as disclosed for the - preferably combined - more preferred immunotherapy of diseases according to the following table 5.

**Table 5:** More preferred peptides and diseases to be treated. SEQ ID NO: 167 is according to the invention.

| Seq ID | Sequence | Tissue and disease |
|---|---|---|
| 22 | LEVEERTKPV | breast, carcinoma |
| 23 | RDSPINANLRY | breast, carcinoma |
| 24 | RPFVIVTA | breast, carcinoma |
| 25 | RPIINTPMV | breast, carcinoma |
| 26 | SPTSSRTSSL | breast, carcinoma |
| 27 | ATSAPLVSR | lung, neuroendocrine carcinoma |
| 114 | YGNPRTNGM | breast, carcinoma |
| 102 | FSITKSVEL | non-Hodgkin's lymphoma, small lymphocytic type |
| 103 | GQTKNDLVV | non-Hodgkin's lymphoma, small lymphocytic type |
| 104 | LSQEVCRD | non-Hodgkin's lymphoma, small lymphocytic type |
| 105 | RDIQSPEQI | non-Hodgkin's lymphoma, small lymphocytic type |
| 106 | REDNSSNSL | non-Hodgkin's lymphoma, small lymphocytic type |
| 107 | TEHQEPGL | non-Hodgkin's lymphoma, small lymphocytic type |
| 108 | TKNDLVVSL | non-Hodgkin's lymphoma, small lymphocytic type |
| 977 | AGREINLVDAHLKSE | lymph node, Hodgkin's disease |
| 978 | AGREINLVDAHLKSEQT | lymph node, Hodgkin's disease |
| 979 | GREINLVDAHLKSE | lymph node, Hodgkin's disease |
| 980 | KPGIVYASLNHSVIG | lymph node, Hodgkin's disease |
| 981 | NKPGIVYASLNHSVIG | lymph node, Hodgkin's disease |
| 982 | TTLYVTDVKSASERPS | lymph node, Hodgkin's disease |
| 220 | RIHTGEKPYK | thyroid gland, nodular hyperplasia |
| 53 | APGSVLPRAL | lymph node, Hodgkin's disease |
| 54 | DIKEHPLL | lymph node, Hodgkin's disease |
| 55 | DSAGPQDAR | lymph node, Hodgkin's disease |
| 56 | FQYAKESYI | lymph node, Hodgkin's disease |
| 57 | KVLSWPFLM | lymph node, Hodgkin's disease |
| 58 | LENDQSLSF | lymph node, Hodgkin's disease |
| 59 | SPSRQPQV | lymph node, Hodgkin's disease |
| 60 | SRHQSFTTK | lymph node, Hodgkin's disease |
| 61 | SSHNASKTL | lymph node, Hodgkin's disease |
| 1003 | DNQYAVLENQKSSH | pleura, malignant mesothelioma |
| 1004 | GPPEIYSDTQFPS | pleura, malignant mesothelioma |
| 1005 | GPPEIYSDTQFPSLQ | pleura, malignant mesothelioma |
| 1006 | TPQGPPEIYSDTQFPS | pleura, malignant mesothelioma |
| 1007 | TPQGPPEIYSDTQFPSLQ | pleura, malignant mesothelioma |
| 1008 | TPQGPPEIYSDTQFPSLQST | pleura, malignant mesothelioma |
| 91 | EHADDDPSL | kidney, Wilm's tumor |
| 92 | SEESVKSTTL | kidney, Wilm's tumor |
| 93 | SPRPPLGSSL | kidney, Wilm's tumor |
| 94 | SPWWRSSL | kidney, Wilm's tumor |

(continued)

| Seq ID | Sequence | Tissue and disease |
|---|---|---|
| 95 | VYTPVDSLVF | kidney, Wilm's tumor |
| 18 | DALLKRTM | skin, basal cell carcinoma |
| 19 | GEDVRSALL | skin, basal cell carcinoma |
| 20 | KFAEEFYSF | skin, basal cell carcinoma |
| 21 | YGYDNVKEY | skin, basal cell carcinoma |
| 661 | EYVSLYHQPAAM | non-Hodgkin's lymphoma, peripheral T cell type, |
| 662 | IKAEYKGRVTLKQYPR | non-Hodgkin's lymphoma, peripheral T cell type |
| 663 | LNVHSEYEPSWEEQP | non-Hodgkin's lymphoma, peripheral T cell type |
| 664 | LPYLFQmPAYASSS | non-Hodgkin's lymphoma, peripheral T cell type |
| 665 | LPYLFQmPAYASSSK | non-Hodgkin's lymphoma, peripheral T cell type |
| 666 | NFIKAEYKGRVT | non-Hodgkin's lymphoma, peripheral T cell type |
| 667 | TNFIKAEYKGRVT | non-Hodgkin's lymphoma, peripheral T cell type |
| 668 | TTNFIKAEYKGRVT | non-Hodgkin's lymphoma, peripheral T cell type |
| 669 | VTLNVHSEYEPSWEEQP | non-Hodgkin's lymphoma, peripheral T cell type |
| 670 | YPRKNLFLVEVTQLTESDS | non-Hodgkin's lymphoma, peripheral T cell type |
| 671 | YPRKNLFLVEVTQLTESDSG | non-Hodgkin's lymphoma, peripheral T cell type |
| 780 | DNGHLYREDQTSPAPG | kidney, angiomyolipoma |
| 781 | DNGHLYREDQTSPAPGLR | kidney, angiomyolipoma |
| 782 | EVQVFAPANALPARSE | kidney, angiomyolipoma |
| 783 | GHLYREDQTSPAPG | kidney, angiomyolipoma |
| 784 | LPARSEAAAVQPVIG | kidney, angiomyolipoma |
| 785 | NGHLYREDQTSPAPG | kidney, angiomyolipoma |
| 786 | NGHLYREDQTSPAPGL | kidney, angiomyolipoma |
| 787 | NGHLYREDQTSPAPGLR | kidney, angiomyolipoma |
| 788 | VFAPANALPARSEAA | kidney, angiomyolipoma |
| 789 | VQVFAPANALPARSE | kidney, angiomyolipoma |
| 178 | HEIDRYTAI | non-Hodgkin's lymphoma |
| 179 | VFTLKPLEF | non-Hodgkin's lymphoma |
| 180 | YWVPRNAL | non-Hodgkin's lymphoma |
| 694 | DGELIRTQPQRLPQ | intramuscular lipoma |
| 695 | GELIRTQPQRLPQ | intramuscular lipoma |
| 696 | NPSDGELIRTQPQRLP | intramuscular lipoma |
| 697 | NPSDGELIRTQPQRLPQ | intramuscular lipoma |
| 698 | NPSDGELIRTQPQRLPQL | intramuscular lipoma |
| 922 | FDFSQNTRVPRLPE | non-Hodgkin's lymphoma |
| 923 | GDAPAILFDKEF | non-Hodgkin's lymphoma |
| 924 | VTHEIDRYTAIAY | non-Hodgkin's lymphoma |
| 692 | DAGSYKAQINQRNFE | lymph node, non-Hodgkin's lymphoma |
| 693 | DAGSYKAQINQRNFEVT | lymph node, non-Hodgkin's lymphoma |

(continued)

| Seq ID | Sequence | Tissue and disease |
|---|---|---|
| 1 | AEHPNVTLTI | spleen, non-Hodgkin's lymphoma |
| 2 | FLAEHPNVTL | spleen, non-Hodgkin's lymphoma |
| 4 | EVAEFLARH | spleen, non-Hodgkin's lymphoma |
| 5 | RHSNVNLTI | spleen, non-Hodgkin's lymphoma |
| 222 | QSTQRSLAL | uterine cervix, squamous cell carcinoma |
| 223 | RDLQMNQALRF | uterine cervix, squamous cell carcinoma |
| 224 | RELESQLHVL | uterine cervix, squamous cell carcinoma |
| 225 | SEAEKLTLV | uterine cervix, squamous cell carcinoma |
| 6 | HPDNVKLFL | non-Hodgkin's lymphoma, small lymphocytic type |
| 7 | ISDTGELKL | non-Hodgkin's lymphoma, small lymphocytic type |
| 8 | KVNGKLVALK | non-Hodgkin's lymphoma, small lymphocytic type |
| 9 | NRLSAQAAL | non-Hodgkin's lymphoma, small lymphocytic type |
| 10 | TPFTAIREA | non-Hodgkin's lymphoma, small lymphocytic type |
| 11 | FGLARAKSV | kidney, renal cell carcinoma, clear cell type |
| 12 | KIADFGLAR | liver, hepatocellular carcinoma |
| 812 | DGSYRIFSKGASE | liposarcoma |
| 813 | GSYRIFSKGASE | liposarcoma |
| 814 | SDGSYRIFSKGASE | liposarcoma |
| 815 | SVKKMMKDNNLVRH | liver, hepatocellular carcinoma |
| 816 | VKKMMKDNNLVRH | liver, hepatocellular carcinoma |
| 145 | KITVPASQK | colon, non-Hodgkin's lymphoma |
| 146 | KITVPASQKL | colon, non-Hodgkin's lymphoma |
| 147 | VPASQKLRQL | colon, non-Hodgkin's lymphoma |
| 537 | ITARPVLW | non-Hodgkin's lymphoma, diffuse large B-cell type |
| 538 | KLMSPKLYVW | non-Hodgkin's lymphoma, diffuse large B-cell type |
| 539 | KVSAVTLAY | non-Hodgkin's lymphoma, diffuse large B-cell type |
| 540 | VEGSGELFRW | non-Hodgkin's lymphoma, diffuse large B-cell type |
| 672 | ADLSSFKSQELN | lymph node, papillary carcinoma of thyroid, metastatic |
| 673 | ADLSSFKSQELNER | lymph node, papillary carcinoma of thyroid, metastatic |
| 674 | ADLSSFKSQELNERN | lymph node, papillary carcinoma of thyroid, metastatic |
| 675 | ADLSSFKSQELNERNE | lymph node, papillary carcinoma of thyroid, metastatic |
| 676 | ADLSSFKSQELNERNEA | lymph node, papillary carcinoma of thyroid, metastatic |
| 677 | AEQQRLKSQDLELSWNLNG | lymph node, papillary carcinoma of thyroid, metastatic |
| 678 | EQQRLKSQDLELSWN | lymph node, papillary carcinoma of thyroid, metastatic |

73

(continued)

| Seq ID | Sequence | Tissue and disease |
|---|---|---|
| 679 | ISQELEELRAEQQR | lymph node, papillary carcinoma of thyroid, metastatic |
| 680 | ISQELEELRAEQQRLK | lymph node, papillary carcinoma of thyroid, metastatic |
| 681 | KGTKQWVHARYA | lymph node, papillary carcinoma of thyroid, metastatic |
| 682 | QADLSSFKSQELNER | lymph node, papillary carcinoma of thyroid, metastatic |
| 683 | SWNLNGLQADLSSFK | lymph node, papillary carcinoma of thyroid, metastatic |
| 684 | TGSWIGLRNLDLKG | lymph node, papillary carcinoma of thyroid, metastatic |
| 743 | AIVQAVSAHRHR | non-Hodgkin's lymphoma, peripheral T cell type |
| 744 | ARNFERNKAIKVI | non-Hodgkin's lymphoma, peripheral T cell type |
| 745 | ARNFERNKAIKVIIA | non-Hodgkin's lymphoma, peripheral T cell type |
| 746 | NFERNKAIKVII | non-Hodgkin's lymphoma, peripheral T cell type |
| 747 | NFERNKAIKVIIA | non-Hodgkin's lymphoma, peripheral T cell type |
| 748 | VAIVQAVSAHRH | non-Hodgkin's lymphoma, peripheral T cell type |
| 749 | VAIVQAVSAHRHR | non-Hodgkin's lymphoma, peripheral T cell type |
| 750 | VAIVQAVSAHRHRA | non-Hodgkin's lymphoma, peripheral T cell type |
| 751 | VAIVQAVSAHRHRAR | non-Hodgkin's lymphoma, peripheral T cell type |
| 818 | VDKVLERDQKLSE | lymph node, papillary carcinoma of thyroid, metastatic |
| 819 | VDKVLERDQKLSELD | lymph node, papillary carcinoma of thyroid, metastatic |
| 820 | VDKVLERDQKLSELDD | lymph node, papillary carcinoma of thyroid, metastatic |
| 821 | VDKVLERDQKLSELDDR | lymph node, papillary carcinoma of thyroid, metastatic |
| 822 | VLERDQKLSELDDR | lymph node, papillary carcinoma of thyroid, metastatic |
| 833 | VDKVLERDQKLSE | lymph node, papillary carcinoma of thyroid, metastatic |
| 834 | VDKVLERDQKLSELD | lymph node, papillary carcinoma of thyroid, metastatic |
| 835 | VDKVLERDQKLSELDD | lymph node, papillary carcinoma of thyroid, metastatic |
| 836 | VDKVLERDQKLSELDDR | lymph node, papillary carcinoma of thyroid, metastatic |
| 837 | VLERDQKLSELDDR | lymph node, papillary carcinoma of thyroid, metastatic |
| 848 | DDPAIDVCKKLLGKYPN | pancreas, adenocarcinoma |
| 849 | DKQPYSKLPGVSLLKP | pancreas, adenocarcinoma |

(continued)

| Seq ID | Sequence | Tissue and disease |
|---|---|---|
| 850 | DKQPYSKLPGVSLLKPL | pancreas, adenocarcinoma |
| 851 | HPRYYISANVTGFK | pancreas, adenocarcinoma |
| 852 | SHPRYYISANVTG | pancreas, adenocarcinoma |
| 853 | SHPRYYISANVTGFK | pancreas, adenocarcinoma |
| 854 | TSHPRYYISANVTG | pancreas, adenocarcinoma |
| 855 | TSHPRYYISANVTGFK | pancreas, adenocarcinoma |
| 908 | DVGMFVALTKLGQPD | uterine cervix, squamous cell carcinoma |
| 909 | VGmFVALTKLGQPD | uterine cervix, squamous cell carcinoma |
| 1015 | GSSFFGELFNQNPE | thyroid gland, papillary carcinoma |
| 1016 | SGSSFFGELFNQNPE | thyroid gland, papillary carcinoma |
| 466 | DEMRFVTQI | testis, mixed germ cell tumor |
| 467 | ETVHFATTQW | testis, mixed germ cell tumor |
| 468 | LPPPATQI | testis, mixed germ cell tumor |
| 633 | GNPAYRSFSNSLSQ | kidney, angiomyolipoma |
| 634 | GPPGEAGYKAFSSLLA | kidney, angiomyolipoma |
| 635 | GPPGEAGYKAFSSLLASS | kidney, angiomyolipoma |
| 636 | GPPGEAGYKAFSSLLASSA | kidney, angiomyolipoma |
| 637 | GPPGEAGYKAFSSLLASSAVSPE | kidney, angiomyolipoma |
| 639 | GYKAFSSLLASSAVSP | kidney, angiomyolipoma |
| 640 | GYKAFSSLLASSAVSPE | kidney, angiomyolipoma |
| 641 | KAFSSLLASSAVSPE | kidney, angiomyolipoma |
| 642 | NPAYRSFSNSLSQ | kidney, angiomyolipoma |
| 643 | SRDDFQEGREGIVAR | kidney, angiomyolipoma |
| 644 | SSSSFHPAPGNAQ | kidney, angiomyolipoma |
| 645 | VARLTESLFLDL | kidney, angiomyolipoma |
| 646 | VARLTESLFLDLLG | kidney, angiomyolipoma |
| 647 | VIAGNPAYRSFSN | kidney, angiomyolipoma |
| 648 | VPQPEPETWEQILRRNVLQ | kidney, angiomyolipoma |
| 649 | YKAFSSLLASSAVS | kidney, angiomyolipoma |
| 650 | YKAFSSLLASSAVSP | kidney, angiomyolipoma |
| 651 | YKAFSSLLASSAVSPE | kidney, angiomyolipoma |
| 992 | EDDYIKSWEDNQQGDE | pleura, malignant mesothelioma |
| 993 | ELERIQIQEAAKKKPG | pleura, malignant mesothelioma |
| 994 | ERIQIQEAAKKKP | pleura, malignant mesothelioma |
| 995 | ERIQIQEAAKKKPG | pleura, malignant mesothelioma |
| 996 | ERIQIQEAAKKKPGI | pleura, malignant mesothelioma |
| 997 | LERIQIQEAAKKKPG | pleura, malignant mesothelioma |
| 998 | LSSISQYSGKIK | pleura, malignant mesothelioma, |
| 941 | EERNLLSVAYKNVVGAR | esophagus, adenocarcinoma |
| 942 | ERNLLSVAYKNVVGAR | esophagus, adenocarcinoma |
| 943 | IAELDTLSEESYKD | vulva, squamous cell carcinoma |
| 944 | IAELDTLSEESYKDS | vulva, squamous cell carcinoma |
| 218 | GDYGRAFNL | stomach, metastatic, lymph node, non-Hodgkin's lymphoma, small lymphocytic type |
| 219 | TRHKIVHTK | stomach, metastatic, lymph node, non-Hodgkin's lymphoma, small lymphocytic type |

(continued)

| Seq ID | Sequence | Tissue and disease |
|---|---|---|
| 221 | KAFNWFSTL | stomach, metastatic, lymph node, non-Hodgkin's lymphoma, small lymphocytic type |
| 541 | RPKSNIVL | non-Hodgkin's lymphoma, diffuse large B-cell type |
| 542 | RPKSNIVLL | non-Hodgkin's lymphoma, diffuse large B-cell type |
| 1001 | INSRFPIPSATDPD | brain, glioblastoma, brain, oligodendroglioma, |
| 1002 | VQHYELLNGQSVFG | brain, glioblastoma, brain, oligodendroglioma, |
| 910 | AGVFHVEKNGRY | colon, adenocarcinoma, mucinous type |
| 911 | FAGVFHVEKNGRYS | colon, adenocarcinoma, mucinous type |
| 912 | GPITITIVNRDGTR | colon, adenocarcinoma, mucinous type |
| 913 | NGRYSISRTEAADL | colon, adenocarcinoma, mucinous type |
| 45 | DELPKFHQY | white blood cells, chronic lymphocytic leukemia |
| 46 | DVTGQFPSSF | white blood cells, chronic lymphocytic leukemia |
| 47 | EHSRVLQQL | white blood cells, chronic lymphocytic leukemia |
| 48 | IKVSKQLL | white blood cells, chronic lymphocytic leukemia |
| 49 | KPRQSSPQL | white blood cells, chronic lymphocytic leukemia |
| 50 | KQLLAALEI | white blood cells, chronic lymphocytic leukemia |
| 51 | RRKDLVLKY | liver, focalnodular hyperplasia |
| 52 | RTRDYASLPPK | white blood cells, chronic lymphocytic leukemia |
| 124 | GQKEALLKY | synovial sarcoma |
| 125 | KPSEERKTI | synovial sarcoma |
| 126 | KQTPKVLVV | synovial sarcoma |
| 127 | SVIQHVQSF | synovial sarcoma |
| 128 | TPIERIPYL | synovial sarcoma |
| 773 | LPEFYKTVSPAL | endometrium, adenocarcinoma, endometrioid type |
| 774 | VGQFIQDVKNSRST | endometrium, adenocarcinoma, endometrioid type |
| 775 | VGQFIQDVKNSRSTD | endometrium, adenocarcinoma, endometrioid type |
| 776 | WGQFIQDVKNSRS | endometrium, adenocarcinoma, endometrioid type |
| 777 | WGQFIQDVKNSRST | endometrium, adenocarcinoma, endometrioid type |
| 778 | WGQFIQDVKNSRSTD | endometrium, adenocarcinoma, endometrioid type |
| 779 | WGQFIQDVKNSRSTDS | endometrium, adenocarcinoma, endometrioid type |
| 687 | GPMKGGNFGGRSSGP | thymus, thymoma, malignant |
| 688 | GPYGGGGQYFAKP | thymus, thymoma, malignant |
| 689 | KGGNFGGRSSGP | thymus, thymoma, malignant |
| 690 | NDFGNYNNQSSNFGP | thymus, thymoma, malignant |
| 691 | SGPYGGGGQYFAKP | thymus, thymoma, malignant |

(continued)

| Seq ID | Sequence | Tissue and disease |
|---|---|---|
| 13 | AAANIIRTL | adrenal gland, adrenal cortical carcinoma |
| 14 | GRFKNLREAL | adrenal gland, adrenal cortical carcinoma |
| 15 | MSPFSKATL | adrenal gland, adrenal cortical carcinoma |
| 16 | QEDPGDNQITL | adrenal gland, adrenal cortical carcinoma |
| 17 | SPFSKATL | adrenal gland, adrenal cortical carcinoma |
| 129 | AEVEKNETV | spleen, non-Hodgkin's lymphoma |
| 130 | EVKEEIPLV | spleen, non-Hodgkin's lymphoma |
| 131 | KPTSARSGL | spleen, non-Hodgkin's lymphoma |
| 132 | KYIETTPLTI | spleen, non-Hodgkin's lymphoma |
| 133 | SEIKTSIEV | spleen, non-Hodgkin's lymphoma |
| 134 | SVKPTSATK | spleen, non-Hodgkin's lymphoma |
| 135 | YPNKGVGQA | spleen, non-Hodgkin's lymphoma |
| 966 | ENNEIISNIRDSVIN | kidney, oncocytoma |
| 967 | NNEIISNIRDSVIN | kidney, oncocytoma |
| 968 | SPTVQVFSASGKPV | kidney, oncocytoma |
| 969 | SSPTVQVFSASGKPVE | kidney, oncocytoma |
| 830 | DIMRVNVDKVLERDQK | Medullary carcinoma of thyroid origin |
| 831 | DIMRVNVDKVLERDQKL | Medullary carcinoma of thyroid origin |
| 832 | IMRVNVDKVLERDQK | lymph node, Hodgkin's disease |
| 752 | EEVITLIRSNQQLE | pancreas, adenocarcinoma, |
| 753 | EEVITLIRSNQQLEN | pancreas, adenocarcinoma, |
| 754 | IPADTFAALKNPNAML | pancreas, adenocarcinoma |
| 755 | LKQLLSDKQQKRQSG | pancreas, adenocarcinoma |
| 756 | LKQLLSDKQQKRQSGQ | pancreas, adenocarcinoma |
| 339 | FLDPDIGGVAV | pancreas, adenocarcinoma |
| 340 | HTAPPENKTW | pancreas, adenocarcinoma |
| 341 | LLDTPVKTQY | pancreas, adenocarcinoma |
| 342 | NAVKDFTSF | pancreas, adenocarcinoma |
| 343 | SGLLQIKKL | pancreas, adenocarcinoma |
| 344 | YHDKNIVLL | pancreas, adenocarcinoma |
| 71 | HLKSIPVSL | prostate, adenocarcinoma |
| 72 | KVWYNVENW | prostate, adenocarcinoma |
| 73 | LPAYRAQLL | prostate, adenocarcinoma |
| 74 | LSEQTSVPL | prostate, adenocarcinoma |
| 75 | SLNQWLVSF | prostate, adenocarcinoma |
| 76 | SMTSLAQKI | prostate, adenocarcinoma |
| 77 | SSSGLHPPK | prostate, adenocarcinoma |
| 578 | GGGYGSGGGSGGYGSRRF | thymus, thymoma, malignant |
| 579 | GGSFGGRSSGSP | thymus, thymoma, malignant |
| 580 | KGGSFGGRSSGSP | thymus, thymoma, malignant |
| 583 | SPYGGGYGSGGGSGGYGSRRF | thymus, thymoma, malignant |
| 584 | YGGGYGSGGGSGGYGSRRF | thymus, thymoma, malignant |
| 84 | VPVPHTTAL | endometrium, adenocarcinoma |
| 85 | YQVLDVQRY | endometrium, adenocarcinoma |
| 731 | DGLNSLTYQVLDVQRYPL | endometrium, adenocarcinoma |
| 732 | HPVLQRQQLDYGIY | endometrium, adenocarcinoma |

(continued)

| Seq ID | Sequence | Tissue and disease |
|---|---|---|
| 733 | LNSLTYQVLDVQR | endometrium, adenocarcinoma |
| 734 | LNSLTYQVLDVQRYP | endometrium, adenocarcinoma |
| 735 | LNSLTYQVLDVQRYPL | endometrium, adenocarcinoma |
| 736 | LPQLVGVSTPLQG | endometrium, adenocarcinoma |
| 737 | LPQLVGVSTPLQGG | endometrium, adenocarcinoma |
| 738 | LPQLVGVSTPLQGGS | endometrium, adenocarcinoma |
| 739 | RLPQLVGVSTPLQGGS | endometrium, adenocarcinoma |
| 740 | SPHKVAIIIPFRNR | endometrium, adenocarcinoma |
| 741 | SPHKVAIIIPFRNRQE | endometrium, adenocarcinoma |
| 742 | SPHKVAIIIPFRNRQEH | endometrium, adenocarcinoma |
| 527 | DEKQQHIVY | synovial sarcoma |
| 528 | DEVYQVTVY | synovial sarcoma |
| 529 | GEISEKAKL | synovial sarcoma |
| 530 | YTMKEVLFY | synovial sarcoma |
| 203 | GPRPITQSEL | lymph node, non-Hodgkin's lymphoma, marginal Zone B-cell type |
| 204 | KPEPVDKVA | lymph node, non-Hodgkin's lymphoma, marginal Zone B-cell type |
| 205 | TPSSRPASL | lymph node, non-Hodgkin's lymphoma, marginal Zone B-cell type |
| 949 | SPPQFRVNGAISN | ovary, granulosa cell tumor |
| 950 | SPPQFRVNGAISNFE | ovary, granulosa cell tumor |
| 951 | SPPQFRVNGAISNFEE | ovary, granulosa cell tumor |
| 952 | SPPQFRVNGAISNFEEF | ovary, granulosa cell tumor |
| 953 | VGKMFVDVYFQEDKK | ovary, granulosa cell tumor |
| 954 | VGKmFVDVYFQEDKKE | ovary, granulosa cell tumor |
| 916 | EEFKKLTSIKIQNDK | small intestine, gastrointestinal stromal tumor (GIST) |
| 917 | INRRMADDNKLFR | small intestine, gastrointestinal stromal tumor (GIST) |
| 918 | TATIVMVTNLKERKE | small intestine, gastrointestinal stromal tumor (GIST) |
| 526 | RINEFSISSF | connective tissues, chondrosarcoma |
| 585 | GNRINEFSISSF | connective tissues, chondrosarcoma |
| 586 | HGNQITSDKVGRKV | connective tissues, chondrosarcoma |
| 587 | IPPVNTNLENLYLQ | connective tissues, chondrosarcoma |
| 588 | LQVLRLDGNEIKR | connective tissues, chondrosarcoma |
| 589 | LQVLRLDGNEIKRS | connective tissues, chondrosarcoma |
| 590 | LQVLRLDGNEIKRSA | connective tissues, chondrosarcoma |
| 591 | LRELHLDHNQISRVPN | connective tissues, chondrosarcoma |
| 592 | LYVRLSHNSLTNNG | connective tissues, chondrosarcoma, |
| 593 | VPSRMKYVYFQNNQ | connective tissues, chondrosarcoma |
| 594 | VPSRMKYVYFQNNQIT | connective tissues, chondrosarcoma |
| 595 | VPSRMKYVYFQNNQITS | connective tissues, chondrosarcoma |
| 596 | WIALHGNQITSD | connective tissues, chondrosarcoma |
| 597 | WIALHGNQITSDK | connective tissues, chondrosarcoma |
| 165 | ELNKLLEEI | ovary, adenocarcinoma, endometrioid type |
| 166 | IPFSNPRVL | ovary, adenocarcinoma, endometrioid type |
| 167 | LLDEGAKLLY | ovary, adenocarcinoma, endometrioid type |
| 168 | SPADAHRNL | ovary, adenocarcinoma, endometrioid type |

(continued)

| Seq ID | Sequence | Tissue and disease |
|---|---|---|
| 96 | APLQRSQSL | kidney, renal cell carcinoma, clear cell type |
| 97 | DEVHQDTY | kidney, renal cell carcinoma, clear cell type |
| 98 | LPHSATVTL | kidney, renal cell carcinoma, clear cell type |
| 152 | APSEYRYTL | stomach, mucinous adenocarcinoma |
| 153 | APSEYRYTLL | stomach, mucinous adenocarcinoma |
| 154 | EIFQNEVAR | stomach, mucinous adenocarcinoma |
| 155 | KDVLIPGKL | stomach, mucinous adenocarcinoma |
| 156 | VPLVREITF | stomach, mucinous adenocarcinoma |
| 136 | ISMKILNSL | thymus, thymoma, benign |
| 137 | KTIAFLLPMF | thymus, thymoma, benign |
| 138 | RDSIINDF | thymus, thymoma, benign |
| 139 | SVKGGGGNEK | thymus, thymoma, benign |
| 140 | GIAKTGSGK | thymus, thymoma, benign |
| 503 | ALYATKTLR | pancreas, microcystic adenoma |
| 504 | MEYVISRI | pancreas, microcystic adenoma |
| 505 | VPVGRQPII | pancreas, microcystic adenoma |
| 278 | ATNGDLASR | prostate, benign nodular hyperplasia |
| 279 | GLHAEVTGVGY | prostate, benign nodular hyperplasia |
| 280 | HVSSTSSSF | prostate, benign nodular hyperplasia |
| 281 | LQADLQNGL | prostate, benign nodular hyperplasia |
| 282 | SELPVSEVA | prostate, benign nodular hyperplasia |
| 283 | SQTKSVFEI | prostate, benign nodular hyperplasia |
| 284 | THIFTSDGL | prostate, benign nodular hyperplasia |
| 285 | VIYFPPLQK | prostate, benign nodular hyperplasia |
| 286 | YPFSSEQKW | prostate, benign nodular hyperplasia |
| 963 | GNTVIHLDQALARMR | lung, small cell carcinoma |
| 964 | NTVIHLDQALARMR | lung, small cell carcinoma |
| 965 | NTVIHLDQALARMRE | lung, small cell carcinoma |
| 187 | AADTERLAL | connective tissues, chondrosarcoma |
| 188 | DMKAKVASL | connective tissues, chondrosarcoma |
| 189 | HVLEEVQQV | connective tissues, chondrosarcoma |
| 190 | KEAADTERL | connective tissues, chondrosarcoma |
| 191 | RISEVLQKL | connective tissues, chondrosarcoma |
| 192 | TEVRELVSL | |
| 973 | ADDLEGEAFLPL | spleen, chronic myeloid leukemia |
| 974 | ADDLEGEAFLPLR | spleen, chronic myeloid leukemia |
| 975 | ADDLEGEAFLPLRE | spleen, chronic myeloid leukemia |
| 976 | GADDLEGEAFLPLR | spleen, chronic myeloid leukemia |
| 141 | AETTDNVFTL | thyroid gland, follicular adenoma |
| 142 | SEYQRFAVM | thyroid gland, follicular adenoma |
| 143 | TFGERWAF | thyroid gland, follicular adenoma |
| 144 | NENLVERF | stomach, colon, adenocarcinoma, mucinous type |
| 117 | QLFSYAILGF | colon, non-Hodgkin's lymphoma |
| 845 | GIRVAPVPLYNS | lung, non-small cell lung carcinoma, liver, hepatocellular carcinoma |
| 846 | GIRVAPVPLYNSFH | lung, non-small cell lung carcinoma, liver, hepatocellular carcinoma |
| 847 | NPNGIRVAPVPLYNSFH | lung, non-small cell lung carcinoma, liver, hepatocellular carcinoma |

(continued)

| Seq ID | Sequence | Tissue and disease |
|---|---|---|
| 478 | AAVPVIISR | lymph node, papillary carcinoma of thyroid, metastatic |
| 479 | EEIGKVAAA | lymph node, papillary carcinoma of thyroid, metastatic |
| 480 | FLKDLVASV | lymph node, papillary carcinoma of thyroid, metastatic |
| 481 | VIISRALEL | lymph node, papillary carcinoma of thyroid, metastatic |
| 420 | QIDYKTLVL | leiomyosarcoma |
| 421 | VEDPTIVRI | leiomyosarcoma |
| 543 | GEPLSYTRFSLARQ | lung, non-small cell lung carcinoma, lung, adenocarcinoma |
| 544 | GEPLSYTRFSLARQVD | lung, non-small cell lung carcinoma, lung, adenocarcinoma |
| 545 | GEPLSYTRFSLARQVDG | lung, non-small cell lung carcinoma, lung, adenocarcinoma |
| 546 | GGEPLSYTRFSLARQVD | lung, non-small cell lung carcinoma, lung, adenocarcinoma |
| 547 | GGEPLSYTRFSLARQVDG | lung, non-small cell lung carcinoma, lung, adenocarcinoma |
| 548 | NPGGYVAYSKAATVTG | lung, non-small cell lung carcinoma, lung, adenocarcinoma |
| 549 | NPGGYVAYSKAATVTGK | lung, non-small cell lung carcinoma, lung, adenocarcinoma |
| 550 | NPGGYVAYSKAATVTGKL | lung, non-small cell lung carcinoma, lung, adenocarcinoma |
| 551 | NSVIIVDKNGRL | lung, non-small cell lung carcinoma, lung, adenocarcinoma |
| 552 | NSVIIVDKNGRLV | lung, non-small cell lung carcinoma, lung, adenocarcinoma |
| 553 | NSVIIVDKNGRLVY | lung, non-small cell lung carcinoma, lung, adenocarcinoma |
| 554 | RVEYHFLSPYVSPK | lung, non-small cell lung carcinoma, lung, adenocarcinoma |
| 555 | RVEYHFLSPYVSPKE | lung, non-small cell lung carcinoma, lung, adenocarcinoma |
| 556 | RVEYHFLSPYVSPKESPF | lung, non-small cell lung carcinoma, lung, adenocarcinoma |
| 557 | SPFRHVFWGSGSHTL | lung, non-small cell lung carcinoma, lung, adenocarcinoma |
| 558 | SVIIVDKNGRLV | lung, non-small cell lung carcinoma, lung, adenocarcinoma |
| 559 | VEYHFLSPYVSPK | lung, non-small cell lung carcinoma, lung, adenocarcinoma |
| 560 | VEYHFLSPYVSPKE | lung, non-small cell lung carcinoma, lung, adenocarcinoma |
| 388 | AEGPAGGFmW | spleen, chronic myeloid leukemia |
| 389 | AYYRDAEAY | spleen, chronic myeloid leukemia |
| 390 | QVNRPLTMR | spleen, chronic myeloid leukemia |
| 391 | RHSPVFQVY | spleen, chronic myeloid leukemia |
| 392 | SLPVPNSAY | spleen, chronic myeloid leukemia |
| 393 | TLGPPGTAHLY | spleen, chronic myeloid leukemia |

(continued)

| Seq ID | Sequence | Tissue and disease |
|---|---|---|
| 308 | VLYVGSKTK | schwannoma |
| 309 | KTKEQVTNV | schwannoma |
| 310 | MPVDPDNEAY | schwannoma |
| 311 | AEKTKQGVA | schwannoma |
| 446 | EAFEFVKQR | stomach, diffuse subtype adenocarcinoma, breast, carcinoma |
| 447 | NHFEGHYQY | stomach, diffuse subtype adenocarcinoma, breast, carcinoma |

Finally a most preferred aspect as disclosed relates to the use of the peptides as disclosed for the - preferably combined - most preferred immunotherapy of diseases according to the following table 6.

Table 6: Most preferred peptides as disclosed and diseases to be treated. SEQ ID No. 167 is according to the invention.

| Seq ID | Sequence | Tissue and disease |
|---|---|---|
| 22 | LEVEERTKPV | breast, carcinoma |
| 23 | RDSPINANLRY | breast, carcinoma |
| 24 | RPFVIVTA | breast, carcinoma |
| 25 | RPIINTPMV | breast, carcinoma |
| 26 | SPTSSRTSSL | breast, carcinoma |
| 27 | ATSAPLVSR | lung, neuroendocrine carcinoma (non-small cell type) |
| 114 | YGNPRTNGM | breast, carcinoma |
| 102 | FSITKSVEL | non-Hodgkin's lymphoma, small lymphocytic type |
| 103 | GQTKNDLVV | non-Hodgkin's lymphoma, small lymphocytic type |
| 104 | LSQEVCRD | non-Hodgkin's lymphoma, small lymphocytic type |
| 105 | RDIQSPEQI | non-Hodgkin's lymphoma, small lymphocytic t type |
| 106 | REDNSSNSL | non-Hodgkin's lymphoma, small lymphocytic type |
| 107 | TEHQEPGL | non-Hodgkin's lymphoma, small lymphocytic type |
| 108 | TKNDLVVSL | non-Hodgkin's lymphoma, small lymphocytic type |
| 977 | AGREINLVDAHLKSE | lymph node, Hodgkin's disease |
| 978 | AGREINLVDAHLKSEQT | lymph node, Hodgkin's disease |
| 979 | GREINLVDAHLKSE | lymph node, Hodgkin's disease |
| 980 | KPGIVYASLNHSVIG | lymph node, Hodgkin's disease |
| 981 | NKPGIVYASLNHSVIG | lymph node, Hodgkin's disease |
| 982 | TTLYVTDVKSASERPS | lymph node, Hodgkin's disease colon or rectum, thyroid gland, nodular hyperplasia |
| 220 | RIHTGEKPYK | |
| 53 | APGSVLPRAL | lymph node, Hodgkin's disease |
| 54 | DIKEHPLL | lymph node, Hodgkin's disease |
| 55 | DSAGPQDAR | lymph node, Hodgkin's disease |
| 56 | FQYAKESYI | lymph node, Hodgkin's disease |
| 57 | KVLSWPFLM | lymph node, Hodgkin's disease |
| 58 | LENDQSLSF | lymph node, Hodgkin's disease |
| 59 | SPSRQPQV | lymph node, Hodgkin's disease |

(continued)

| Seq ID | Sequence | Tissue and disease |
|---|---|---|
| 60 | SRHQSFTTK | lymph node, Hodgkin's disease |
| 61 | SSHNASKTL | lymph node, Hodgkin's disease |
| 100 3 | DNQYAVLENQKSSH | pleura, malignant mesothelioma |
| 100 4 | GPPEIYSDTQFPS | pleura, malignant mesothelioma |
| 100 5 | GPPEIYSDTQFPSLQ | pleura, malignant mesothelioma |
| 100 6 | TPQGPPEIYSDTQFPS | pleura, malignant mesothelioma |
| 100 7 | TPQGPPEIYSDTQFPSLQ | pleura, malignant mesothelioma |
| 100 8 | TPQGPPEIYSDTQFPSLQS T | pleura, malignant mesothelioma |
| 661 | EYVSLYHQPAAM | non-Hodgkin's lymphoma, peripheral T cell type |
| 662 | IKAEYKGRVTLKQYPR | non-Hodgkin's lymphoma, peripheral T cell type |
| 663 | LNVHSEYEPSWEEQP | non-Hodgkin's lymphoma, peripheral T cell type |
| 664 | LPYLFQMPAYASSS | non-Hodgkin's lymphoma, peripheral T cell type |
| 665 | LPYLFQMPAYASSSK | non-Hodgkin's lymphoma, peripheral T cell type |
| 666 | NFIKAEYKGRVT | non-Hodgkin's lymphoma, peripheral T cell type |
| 667 | TNFIKAEYKGRVT | non-Hodgkin's lymphoma, peripheral T cell type |
| 668 | TTNFIKAEYKGRVT | non-Hodgkin's lymphoma, peripheral T cell type |
| 669 | VTLNVHSEYEPSWEEQP | non-Hodgkin's lymphoma, peripheral T cell type |
| 670 | YPRKNLFLVEVTQLTESDS | non-Hodgkin's lymphoma, peripheral T cell type |
| 671 | YPRKNLFLVEVTQLTESDS G | non-Hodgkin's lymphoma, peripheral T cell type |
| 780 | DNGHLYREDQTSPAPG | kidney, angiomyolipoma |
| 781 | DNGHLYREDQTSPAPGLR | kidney, angiomyolipoma |
| 782 | EVQVFAPANALPARSE | kidney, angiomyolipoma |
| 783 | GHLYREDQTSPAPG | kidney, angiomyolipoma |
| 784 | LPARSEAAAVQPVIG | kidney, angiomyolipoma |
| 785 | NGHLYREDQTSPAPG | kidney, angiomyolipoma |
| 786 | NGHLYREDQTSPAPGL | kidney, angiomyolipoma |
| 787 | NGHLYREDQTSPAPGLR | kidney, angiomyolipoma |
| 788 | VFAPANALPARSEAA | kidney, angiomyolipoma |
| 789 | VQVFAPANALPARSE | kidney, angiomyolipoma |
| 222 | QSTQRSLAL | uterine cervix, squamous cell carcinoma |
| 223 | RDLQMNQALRF | uterine cervix, squamous cell carcinoma |
| 224 | RELESQLHVL | uterine cervix, squamous cell carcinoma |
| 225 | SEAEKLTLV | uterine cervix, squamous cell carcinoma |
| 12 | KIADFGLAR | liver, hepatocellular carcinoma |
| 812 | DGSYRIFSKGASE | colon or rectum |
| 813 | GSYRIFSKGASE | colon or rectum |
| 814 | SDGSYRIFSKGASE | colon or rectum |
| 815 | SVKKMMKDNNLVRH | colon or rectum, liver, hepatocellular carcinoma |

(continued)

| Seq ID | Sequence | Tissue and disease |
|--------|----------|--------------------|
| 816 | VKKMMKDNNLVRH | colon or rectum, liver, hepatocellular carcinoma |
| 743 | AIVQAVSAHRHR | non-Hodgkin's lymphoma, peripheral T cell type |
| 744 | ARNFERNKAIKVI | non-Hodgkin's lymphoma, peripheral T cell type |
| 745 | ARNFERNKAIKVIIA | non-Hodgkin's lymphoma, peripheral T cell type |
| 746 | NFERNKAIKVII | non-Hodgkin's lymphoma, peripheral T cell type |
| 747 | NFERNKAIKVIIA | non-Hodgkin's lymphoma, peripheral T cell type |
| 748 | VAIVQAVSAHRH | non-Hodgkin's lymphoma, peripheral T cell type |
| 749 | VAIVQAVSAHRHR | non-Hodgkin's lymphoma, peripheral T cell type |
| 750 | VAIVQAVSAHRHRA | non-Hodgkin's lymphoma, peripheral T cell type |
| 751 | VAIVQAVSAHRHRAR | non-Hodgkin's lymphoma, peripheral T cell type |
| 818 | VDKVLERDQKLSE | lymph node, papillary carcinoma of thyroid, metastatic |
| 819 | VDKVLERDQKLSELD | lymph node, papillary carcinoma of thyroid, metastatic |
| 820 | VDKVLERDQKLSELDD | lymph node, papillary carcinoma of thyroid, metastatic |
| 821 | VDKVLERDQKLSELDDR | lymph node, papillary carcinoma of thyroid, metastatic |
| 822 | VLERDQKLSELDDR | lymph node, papillary carcinoma of thyroid, metastatic |
| 833 | VDKVLERDQKLSE | lymph node, papillary carcinoma of thyroid, metastatic |
| 834 | VDKVLERDQKLSELD | lymph node, papillary carcinoma of thyroid, metastatic |
| 835 | VDKVLERDQKLSELDD | lymph node, papillary carcinoma of thyroid, metastatic |
| 836 | VDKVLERDQKLSELDDR | lymph node, papillary carcinoma of thyroid, metastatic |
| 837 | VLERDQKLSELDDR | lymph node, papillary carcinoma of thyroid, metastatic |
| 908 | DVGMFVALTKLGQPD | uterine cervix, squamous cell carcinoma |
| 909 | VGmFVALTKLGQPD | uterine cervix, squamous cell carcinoma |
| 218 | GDYGRAFNL | lymph node, non-Hodgkin's lymphoma, small lymphocytic type |
| 219 | TRHKIVHTK | lymph node, non-Hodgkin's lymphoma, small lymphocytic type |
| 221 | KAFNWFSTL | lymph node, non-Hodgkin's lymphoma, small lymphocytic type |
| 541 | RPKSNIVL | non-Hodgkin's lymphoma, diffuse large B-cell type |
| 542 | RPKSNIVLL | non-Hodgkin's lymphoma, diffuse large B-cell type |

(continued)

| Seq ID | Sequence | Tissue and disease |
|--------|----------|--------------------|
| 752 | EEVITLIRSNQQLE | pancreas, adenocarcinoma |
| 753 | EEVITLIRSNQQLEN | pancreas, adenocarcinoma |
| 754 | IPADTFAALKNPNAML | pancreas, adenocarcinoma |
| 755 | LKQLLSDKQQKRQSG | pancreas, adenocarcinoma |
| 756 | LKQLLSDKQQKRQSGQ | pancreas, adenocarcinoma |
| 71 | HLKSIPVSL | prostate, adenocarcinoma |
| 72 | KVWYNVENW | prostate, adenocarcinoma |
| 73 | LPAYRAQLL | prostate, adenocarcinoma |
| 74 | LSEQTSVPL | prostate, adenocarcinoma |
| 75 | SLNQWLVSF | prostate, adenocarcinoma |
| 76 | SMTSLAQKI | prostate, adenocarcinoma |
| 77 | SSSGLHPPK | prostate, adenocarcinoma |
| 527 | DEKQQHIVY | synovial sarcoma |
| 528 | DEVYQVTVY | synovial sarcoma |
| 529 | GEISEKAKL | synovial sarcoma |
| 530 | YTMKEVLFY | synovial sarcoma |
| 165 | ELNKLLEEI | ovary, adenocarcinoma, endometrioid type |
| 166 | IPFSNPRVL | ovary, adenocarcinoma, endometrioid type |
| 167 | LLDEGAKLLY | ovary, adenocarcinoma, endometrioid type |
| 168 | SPADAHRNL | ovary, adenocarcinoma, endometrioid type |
| 96 | APLQRSQSL | kidney, renal cell carcinoma, clear cell type |
| 97 | DEVHQDTY | kidney, renal cell carcinoma, clear cell type |
| 98 | LPHSATVTL | kidney, renal cell carcinoma, clear cell type |
| 278 | ATNGDLASR | prostate, benign nodular hyperplasia |
| 279 | GLHAEVTGVGY | prostate, benign nodular hyperplasia |
| 280 | HVSSTSSSF | prostate, benign nodular hyperplasia |
| 281 | LQADLQNGL | prostate, benign nodular hyperplasia |
| 282 | SELPVSEVA | prostate, benign nodular hyperplasia |
| 283 | SQTKSVFEI | prostate, benign nodular hyperplasia |
| 284 | THIFTSDGL | prostate, benign nodular hyperplasia |
| 285 | VIYFPPLQK | prostate, benign nodular hyperplasia |
| 286 | YPFSSEQKW | prostate, benign nodular hyperplasia |
| 973 | ADDLEGEAFLPL | spleen, chronic myeloid leukemia |
| 974 | ADDLEGEAFLPLR | spleen, chronic myeloid leukemia |
| 975 | ADDLEGEAFLPLRE | spleen, chronic myeloid leukemia |
| 976 | GADDLEGEAFLPLR | spleen, chronic myeloid leukemia |
| 141 | AETTDNVFTL | thyroid gland, follicular adenoma |
| 142 | SEYQRFAVM | thyroid gland, follicular adenoma |
| 143 | TFGERWAF | thyroid gland, follicular adenoma |
| 144 | NENLVERF | colon, adenocarcinoma, mucinous type |
| 845 | GIRVAPVPLYNS | liver, hepatocellular carcinoma |
| 846 | GIRVAPVPLYNSFH | liver, hepatocellular carcinoma |
| 847 | NPNGIRVAPVPLYNSFH | liver, hepatocellular carcinoma |
| 478 | AAVPVIISR | lymph node, papillary carcinoma of thyroid, metastatic |
| 479 | EEIGKVAAA | lymph node, papillary carcinoma of thyroid, metastatic |
| 480 | FLKDLVASV | lymph node, papillary carcinoma of thyroid, metastatic |

(continued)

| Seq ID | Sequence | Tissue and disease |
|---|---|---|
| 481 | VIISRALEL | lymph node, papillary carcinoma of thyroid, metastatic |
| 420 | QIDYKTLVL | leiomyosarcoma |
| 421 | VEDPTIVRI | leiomyosarcoma |
| 543 | GEPLSYTRFSLARQ | lung, non-small cell lung carcinoma, lung, adenocarcinoma |
| 544 | GEPLSYTRFSLARQVD | lung, non-small cell lung carcinoma, lung, adenocarcinoma |
| 545 | GEPLSYTRFSLARQVDG | lung, non-small cell lung carcinoma, lung, adenocarcinoma |
| 546 | GGEPLSYTRFSLARQVD | lung, non-small cell lung carcinoma, lung, adenocarcinoma |
| 547 | GGEPLSYTRFSLARQVDG | lung, non-small cell lung carcinoma, lung, adenocarcinoma |
| 548 | NPGGYVAYSKAATVTG | lung, non-small cell lung carcinoma, lung, adenocarcinoma |
| 549 | NPGGYVAYSKAATVTGK | lung, non-small cell lung carcinoma, lung, adenocarcinoma |
| 550 | NPGGYVAYSKAATVTGKL | lung, non-small cell lung carcinoma, lung, adenocarcinoma |
| 551 | NSVIIVDKNGRL | lung, non-small cell lung carcinoma, lung, adenocarcinoma |
| 552 | NSVIIVDKNGRLV | lung, non-small cell lung carcinoma, lung, adenocarcinoma |
| 553 | NSVIIVDKNGRLVY | lung, non-small cell lung carcinoma, lung, adenocarcinoma |
| 554 | RVEYHFLSPYVSPK | lung, non-small cell lung carcinoma, lung, adenocarcinoma |
| 555 | RVEYHFLSPYVSPKE | lung, non-small cell lung carcinoma, lung, adenocarcinoma |
| 556 | RVEYHFLSPYVSPKESPF | lung, non-small cell lung carcinoma, lung, adenocarcinoma |
| 557 | SPFRHVFWGSGSHTL | lung, non-small cell lung carcinoma, lung, adenocarcinoma |
| 558 | SVIIVDKNGRLV | lung, non-small cell lung carcinoma, lung, adenocarcinoma |
| 559 | VEYHFLSPYVSPK | lung, non-small cell lung carcinoma, lung, adenocarcinoma |
| 560 | VEYHFLSPYVSPKE | lung, non-small cell lung carcinoma, lung, adenocarcinoma |

The present invention furthermore relates to the peptides according to the present invention that have the ability to bind to a molecule of the human major histocompatibility complex (MHC) class-I.

The present invention further relates to the peptides according to the present invention, wherein said peptide includes non-peptide bonds.

The present invention further relates to a fusion protein comprising (a) an amino acid sequence consisting of SEQ ID NO: 167; and (b) N-terminal amino acids 1-80 of HLA-DR antigen-associated invariant chain (Ii).

The present invention further relates to a nucleic acid, encoding the peptides according to the present invention.

The present invention further relates to the nucleic acid according to the present invention that is DNA, cDNA, PNA, RNA or combinations thereof.

The present invention further relates to an expression vector expressing and/or presenting a nucleic acid according to the present invention.

The present invention further relates An antibody that specifically binds to a human major histocompatibility complex (MHC) class I being complexed with the peptide according according to SEQ ID NO: 167, and methods of making them.

The present invention further relates to a T-cell receptor that binds to a human major histocompatibility complex (MHC) class I being complexed with the peptide according according to SEQ ID NO: 167, and methods of making them.

The present invention further relates to a host cell comprising a nucleic acid according to the present invention or an expression vector as described before.

The present invention further relates to the host cell according to the present invention that is an antigen presenting cell.

The present invention further relates to the host cell according to the present invention wherein the antigen presenting cell is a dendritic cell.

The present invention further relates to a method of producing a peptide according to the present invention, the method comprising culturing the host cell according to the present invention, and isolating the peptide from the host cell or its culture medium.

The present invention further relates to an *in vitro* method for producing activated cytotoxic T lymphocytes (CTL), the method comprising contacting in vitro CTL with antigen loaded human class I MHC molecules expressed on the surface of a suitable antigen-presenting cell for a period of time sufficient to activate said CTL in an antigen specific manner, wherein said antigen is a peptide according to the present invention.

The present invention further relates to the method according to the present invention, wherein the antigen is loaded onto class I MHC molecules expressed on the surface of a suitable antigen-presenting cell by contacting a sufficient amount of the antigen with an antigen-presenting cell.

The present invention further relates to the method according to the present invention, wherein the antigen-presenting cell comprises an expression vector expressing said peptide containing SEQ ID NO: 167.

The present invention further relates to activated cytotoxic T lymphocytes (CTL), produced by the method according to the present invention, which selectively recognize a cell which aberrantly expresses a polypeptide comprising an amino acid sequence according to the present invention.

The present invention further discloses a method of killing target cells in a patient which target cells aberrantly express a polypeptide comprising any amino acid sequence according to the present invention, the method comprising administering to the patient an effective number of cytotoxic T lymphocytes (CTL) as according to the present invention.

The present invention further discloses the use of any peptide described, a nucleic acid according to the present invention, an expression vector according to the present invention, a cell according to the present invention, or an activated cytotoxic T lymphocyte according to the present invention as a medicament or in the manufacture of a medicament.

The present invention further relates to a use according to the present invention, wherein said medicament is a vaccine.

The present invention further relates to a use according to the present invention, wherein the medicament is active against cancer.

The present invention further relates to a use according to the present invention, wherein said cancer cells are cells of haematological malignancies, such as, CLL or AML cells.

The present invention further discloses particular marker proteins and biomarkers based on the peptides according to the present invention that can be used in the diagnosis and/or prognosis of haematological malignancies, in particular chronic lymphoid leukemia (CLL) cells.

Further, the present invention discloses the use of these novel targets for cancer treatment.

Further, the present invention discloses a method for producing a personalized anticancer vaccine for an individual patient using a database ("warehouse") of prescreened tumour associated peptides.

Stimulation of an immune response is dependent upon the presence of antigens recognised as foreign by the host immune system. The discovery of the existence of tumor associated antigens has raised the possibility of using a host's immune system to intervene in tumor growth. Various mechanisms of harnessing both the humoral and cellular arms of the immune system are currently being explored for cancer immunotherapy.

Specific elements of the cellular immune response are capable of specifically recognising and destroying tumor cells. The isolation of cytotoxic T-cells (CTL) from tumor-infiltrating cell populations or from peripheral blood suggests that such cells play an important role in natural immune defences against cancer. CD8-positive T-cells in particular, which recognise Class I molecules of the major histocompatibility complex (MHC)-bearing peptides of usually 8 to 10 amino acid residues derived from proteins or defect ribosomal products (DRIPS) located in the cytosol, play an important role in this response. The MHC-molecules of the human are also designated as human leukocyte-antigens (HLA).

There are two classes of MHC-molecules: MHC class I molecules that can be found on most cells having a nucleus. MHC molecules are composed of an alpha heavy chain and beta-2-microglobulin (MHC class I receptors) or an alpha and a beta chain (MHC class II receptors), respectively. Their three-dimensional conformation results in a binding groove, which is used for non-covalent interaction with peptides. MHC class I present peptides that result from proteolytic cleavage of predominantly endogenous proteins, DRIPs and larger peptides. MHC class II molecules can be found predominantly on professional antigen presenting cells (APCs), and primarily present peptides of exogenous or transmembrane proteins that are taken up by APCs during the course of endocytosis, and are subsequently processed. Complexes of peptide and MHC class I molecules are recognized by CD8-positive cytotoxic T-lymphocytes bearing the appropriate TCR (T-cell receptor), whereas complexes of peptide and MHC class II molecules are recognized by CD4-positive-helper-T cells bearing the appropriate TCR. It is well known that the TCR, the peptide and the MHC are thereby present in a stoichiometric amount of 1:1:1.

CD4-positive helper T cells play an important role in inducing and sustaining effective responses by CD8-positive cytotoxic T cells. The identification of CD4-positive T-cell epitopes derived from tumor associated antigens (TAA) is of great importance for the development of pharmaceutical products for triggering anti-tumor immune responses (Gnjatic S, et al. Survey of naturally occurring CD4+ T cell responses against NY-ESO-1 in cancer patients: correlation with antibody responses. Proc Natl Acad Sci U S A. 2003 Jul 22;100(15):8862-7). At the tumor site, T helper cells, support a CTL friendly cytokine milieu Mortara L, et al. CIITA-induced MHC class II expression in mammary adenocarcinoma leads to a Th1 polarization of the tumor microenvironment, tumor rejection, and specific antitumor memory. Clin Cancer Res. 2006 Jun 1;12(11 Pt 1):3435-43) and attract effector cells, e.g. CTLs, NK cells, macrophages, granulocytes (Hwang ML, et al. Cognate memory CD4+ T cells generated with dendritic cell priming influence the expansion, trafficking, and differentiation of secondary CD8+ T cells and enhance tumor control. J Immunol. 2007 Nov 1;179(9):5829-38).

In the absence of inflammation, expression of MHC class II molecules is mainly restricted to cells of the immune system, especially professional antigen-presenting cells (APC), e.g., monocytes, monocyte-derived cells, macrophages, dendritic cells. In cancer patients, cells of the tumor have surprisingly been found to express MHC class II molecules (Dengjel J, et al. Unexpected abundance of HLA class II presented peptides in primary renal cell carcinomas. Clin Cancer Res. 2006 Jul 15;12(14 Pt 1):4163-70).

It was shown in mammalian animal models, e.g., mice, that even in the absence of CTL effector cells (i.e., CD8-positive T lymphocytes), CD4-positive T cells are sufficient for inhibiting manifestation of tumors via inhibition of angiogenesis by secretion of interferon-gamma (IFNγ).

Additionally, it was shown that CD4-positive T cells recognizing peptides from tumor-associated antigens presented by HLA class II molecules can counteract tumor progression via the induction of antibody (Ab) responses.

In contrast to tumor-associated peptides binding to HLA class I molecules, only a small number of class II ligands of tumor associated antigens (TAA) have been described to date.

Since the constitutive expression of HLA class II molecules is usually limited to cells of the immune system, the possibility of isolating class II peptides directly from primary tumors was not considered possible. However, Dengjel et al. were successful in identifying a number of MHC Class II epitopes directly from tumors (WO 2007/028574, EP 1 760 088 B1; (Dengjel et al., 2006).

The antigens that are recognized by the tumor specific cytotoxic T lymphocytes, that is, their epitopes, can be molecules derived from all protein classes, such as enzymes, receptors, transcription factors, etc. which are expressed and, as compared to unaltered cells of the same origin, up-regulated in cells of the respective tumor.

Since both types of response, CD8 and CD4 dependent, contribute jointly and synergistically to the anti-tumor effect, the identification and characterization of tumor-associated antigens recognized by either CD8+ CTLs (ligand: MHC class I molecule + peptide epitope) or by CD4-positive T-helper cells (ligand: MHC class II molecule + peptide epitope) is important in the development of tumor vaccines.

The length variants are generally N- and/or C-terminally extended (between 1 and 5, preferably 1 to 10 amino acids) or N- and/or C-terminally shortened (between 1 and 5 amino acids) peptides, which still can bind to MHC, and elicit a cellular immune response as described herein.

For a peptide to trigger (elicit) a cellular immune response, it must bind to an MHC-molecule. This process is dependent on the allele of the MHC-molecule and specific polymorphisms of the amino acid sequence of the peptide. MHC-class-I-binding peptides are usually 8-12 amino acid residues in length and usually contain two conserved residues ("anchors") in their sequence that interact with the corresponding binding groove of the MHC-molecule. In this way each MHC allele has a "binding motif" determining which peptides can bind specifically to the binding groove.

In the MHC class I dependent immune reaction, peptides not only have to be able to bind to certain MHC class I molecules being expressed by tumor cells, they also have to be recognized by T cells bearing specific T cell receptors (TCR).

The antigens that are recognized by the tumor specific cytotoxic T lymphocytes, that is, their epitopes, can be molecules derived from all protein classes, such as enzymes, receptors, transcription factors, etc. which are expressed and, as compared to unaltered cells of the same origin, up-regulated in cells of the respective tumor.

The current classification of tumor associated antigens comprises the following major groups:

> a) Cancer-testis antigens: The first TAAs ever identified that can be recognized by T cells belong to this class, which was originally called cancer-testis (CT) antigens because of the expression of its members in histologically different human tumors and, among normal tissues, only in spermatocytes/spermatogonia of testis and, occasionally, in placenta. Since the cells of testis do not express class I and II HLA molecules, these antigens cannot be recognized by T cells in normal tissues and can therefore be considered as immunologically tumor-specific. Well-known examples for CT antigens are the MAGE family members or NY-ESO-1.

b) Differentiation antigens: These TAAs are shared between tumors and the normal tissue from which the tumor arose; most are found in melanomas and normal melanocytes. Many of these melanocyte lineage-related proteins are involved in the biosynthesis of melanin and are therefore not tumor specific but nevertheless are widely used for cancer immunotherapy. Examples include, but are not limited to, tyrosinase and Melan-A/MART-1 for melanoma or PSA for prostate cancer.

c) Overexpressed TAAs: Genes encoding widely expressed TAAs have been detected in histologically different types of tumors as well as in many normal tissues, generally with lower expression levels. It is possible that many of the epitopes processed and potentially presented by normal tissues are below the threshold level for T-cell recognition, while their overexpression in tumor cells can trigger an anticancer response by breaking previously established tolerance. Prominent examples for this class of TAAs are Her-2/neu, Survivin, Telomerase or WT1.

d) Tumor specific antigens: These unique TAAs arise from mutations of normal genes (such as β-catenin, CDK4, etc.). Some of these molecular changes are associated with neoplastic transformation and/or progression. Tumor specific antigens are generally able to induce strong immune responses without bearing the risk for autoimmune reactions against normal tissues. On the other hand, these TAAs are in most cases only relevant to the exact tumor on which they were identified and are usually not shared between many individual tumors.

e) TAAs arising from abnormal post-translational modifications: Such TAAs may arise from proteins which are neither specific nor overexpressed in tumors but nevertheless become tumor associated by posttranslational processes primarily active in tumors. Examples for this class arise from altered glycosylation patterns leading to novel epitopes in tumors as for MUC1 or events like protein splicing during degradation which may or may not be tumor specific.

f) Oncoviral proteins: These TAAs are viral proteins that may play a critical role in the oncogenic process and, because they are foreign (not of human origin), they can evoke a T-cell response. Examples of such proteins are the human papilloma type 16 virus proteins, E6 and E7, which are expressed in cervical carcinoma.

For proteins to be recognized by cytotoxic T-lymphocytes as tumor-specific or - associated antigens, and to be used in a therapy, particular prerequisites must be fulfilled. The antigen should be expressed mainly by tumor cells and not or in comparably small amounts by normal healthy tissues or in another preferred embodiment the peptide should be over-presented by tumor cells as compared to normal healthy tissues. It is furthermore desirable, that the respective antigen is not only present in a type of tumor, but also in high concentrations (i.e. copy numbers of the respective peptide per cell). Tumor-specific and tumor-associated antigens are often derived from proteins directly involved in transformation of a normal cell to a tumor cell due to a function e.g. in cell cycle control or suppression of apoptosis. Additionally, downstream targets of the proteins directly causative for a transformation may be upregulated und thus may be indirectly tumor-associated. Such indirect tumor-associated antigens may also be targets of a vaccination approach (Singh-Jasuja et al., 2004). In both cases it is essential that epitopes are present in the amino acid sequence of the antigen, since such a peptide ("immunogenic peptide") that is derived from a tumor associated antigen should lead to an *in vitro* or *in vivo* T-cell-response.

Basically, any peptide able to bind a MHC molecule may function as a T-cell epitope. A prerequisite for the induction of an *in vitro* or *in vivo* T-cell-response is the presence of a T cell with a corresponding TCR and the absence of immunological tolerance for this particular epitope.

Therefore, TAAs are a starting point for the development of a tumor vaccine. The methods for identifying and characterizing the TAAs are based on the use of CTL that can be isolated from patients or healthy subjects, or they are based on the generation of differential transcription profiles or differential peptide expression patterns between tumors and normal tissues.

However, the identification of genes over-expressed in tumor tissues or human tumor cell lines, or selectively expressed in such tissues or cell lines, does not provide precise information as to the use of the antigens being transcribed from these genes in an immune therapy. This is because only an individual subpopulation of epitopes of these antigens are suitable for such an application since a T cell with a corresponding TCR has to be present and immunological tolerance for this particular epitope needs to be absent or minimal. In a very preferred embodiment of the invention it is therefore important to select only those over- or selectively presented peptides against which a functional and/or a proliferating T cell can be found. Such a functional T cell is defined as a T cell, which upon stimulation with a specific antigen can be clonally expanded and is able to execute effector functions ("effector T cell").

In case of TCRs and antibodies according to the invention the immunogenicity of the underlying peptides is secondary. For TCRs and antibodies according to the invention the presentation is the determining factor.

T-helper cells play an important role in orchestrating the effector function of CTLs in anti-tumor immunity. T-helper cell epitopes that trigger a T-helper cell response of the $T_{H1}$ type support effector functions of CD8-positive killer T cells, which include cytotoxic functions directed against tumor cells displaying tumor-associated peptide/MHC complexes on their cell surfaces. In this way tumor-associated T-helper cell peptide epitopes, alone or in combination with other tumor-associated peptides, can serve as active pharmaceutical ingredients of vaccine compositions that stimulate anti-tumor immune responses.

The inventors identified a novel category of ligandome-derived tumor-associated antigens (LiTAAs), which were frequently and exclusively detected in CLL patients. Specific immune recognition of the corresponding HLA ligands (LiTAPs) was observed exclusively in CLL patients, remarkably showing a direct correlation with the frequency of HLA restricted presentation. Furthermore, retrospective survival analysis of 33 CLL patients indicated a potential association of LiTAP-specific immune responses with improved overall survival in CLL patients.

Uses against further cancers are disclosed in the following description of the proteins of the peptides according to the invention.

**Detailed description of the invention**

As used herein and except as noted otherwise all terms are defined as given below.

The term "peptide" is used herein to designate a series of amino acid residues, connected one to the other typically by peptide bonds between the alpha-amino and carbonyl groups of the adjacent amino acids. The peptides are preferably 9 amino acids in length, but can be as short as 8 amino acids in length, and as long as 10, 11, 12, 13 or 14 and in case of MHC class II peptides they can be as long as 15, 16, 17, 18, 19 or 20 amino acids in length.

Furthermore, the term "peptide" shall include salts of a series of amino acid residues, connected one to the other typically by peptide bonds between the alpha-amino and carbonyl groups of the adjacent amino acids. Preferably, the salts are pharmaceutical acceptable salts of the peptides, such as, for example, the chloride or acetate (trifluoroacetate) salts.

The term "peptide" shall include "oligopeptide". The term "oligopeptide" is used herein to designate a series of amino acid residues, connected one to the other typically by peptide bonds between the alpha-amino and carbonyl groups of the adjacent amino acids. The length of the oligopeptide is not critical to the invention, as long as the correct epitope or epitopes are maintained therein. The oligopeptides are typically less than about 30 amino acid residues in length, and greater than about 15 amino acids in length.

The term "the peptides of the present invention" shall include the peptides consisting of or comprising a peptide as defined above according to SEQ ID NO: 167.

The term "polypeptide" designates a series of amino acid residues, connected one to the other typically by peptide bonds between the alpha-amino and carbonyl groups of the adjacent amino acids. The length of the polypeptide is not critical to the invention as long as the correct epitopes are maintained. In contrast to the terms peptide or oligopeptide, the term polypeptide is meant to refer to molecules containing more than about 30 amino acid residues.

A peptide, oligopeptide, protein or polynucleotide coding for such a molecule is "immunogenic" (and thus is an "immunogen" within the present invention), if it is capable of inducing an immune response. In the case of the present invention, immunogenicity is more specifically defined as the ability to induce a T-cell response. Thus, an "immunogen" would be a molecule that is capable of inducing an immune response, and in the case of the present invention, a molecule capable of inducing a T-cell response. In another aspect, the immunogen can be the peptide, the complex of the peptide with MHC, oligopeptide, and/or protein that is used to raise specific antibodies or TCRs against it.

A class I T cell "epitope" requires a short peptide that is bound to a class I MHC receptor, forming a ternary complex (MHC class I alpha chain, beta-2-microglobulin, and peptide) that can be recognized by a T cell bearing a matching T-cell receptor binding to the MHC/peptide complex with appropriate affinity. Peptides binding to MHC class I molecules are typically 8-14 amino acids in length, and most typically 9 amino acids in length.

In humans there are three different genetic loci that encode MHC class I molecules (the MHC-molecules of the human are also designated human leukocyte antigens (HLA)): HLA-A, HLA-B, and HLA-C. HLA-A*01, HLA-A*02, and HLA-B*07 are examples of different MHC class I alleles that can be expressed from these loci.

**Table 7:** Expression frequencies F of HLA*A02 and the most frequent HLA-DR serotypes. Frequencies are deduced from haplotype frequencies $G_f$ within the American population adapted from Mori et al. (Mori M, et al. HLA gene and haplotype frequencies in the North American population: the National Marrow Donor Program Donor Registry. Transplantation. 1997 Oct 15;64(7):1017-27) employing the Hardy-Weinberg formula $F=1-(1-G_f)^2$. Combinations of A*02 with certain HLA-DR alleles might be enriched or less frequent than expected from their single frequencies due to linkage disequilibrium. For details refer to Chanock et al. (S.J. Chanock, et al (2004) HLA-A, -B, -Cw, -DQA1 and DRB1 in an African American population from Bethesda, USA Human Immunology, 65: 1223-1235).

| | Expression frequencies of HLA*02 and HLA-DR serotypes within North American subpopulations | | | |
|---|---|---|---|---|
| HLA Allele | Caucasian American | African American | Asian American | Latin American |
| A*02 | 49.1% | 34.1% | 43.2% | 48.3% |
| DR1 | 19.4% | 13.2% | 6.8% | 15.3% |
| DR2 | 28.2% | 29.8% | 33.8% | 21.2% |
| DR3 | 20.6% | 24.8% | 9.2% | 15.2% |
| DR4 | 30.7% | 11.1% | 28.6% | 36.8% |
| DR5 | 23.3% | 31.1% | 30.0% | 20.0% |
| DR6 | 26.7% | 33.7% | 25.1% | 31.1% |
| DR7 | 24.8% | 19.2% | 13.4% | 20.2% |
| DR8 | 5.7% | 12.1% | 12.7% | 18.6% |
| DR9 | 2.1% | 5.8% | 18.6% | 2.1% |

Therefore, for therapeutic and diagnostic purposes a peptide that binds with appropriate affinity to several different HLA class II receptors is highly desirable. A peptide binding to several different HLA class II molecules is called a promiscuous binder.

As used herein, reference to a DNA sequence includes both single stranded and double stranded DNA. Thus, the specific sequence, unless the context indicates otherwise, refers to the single strand DNA of such sequence, the duplex of such sequence with its complement (double stranded DNA) and the complement of such sequence. The term "coding region" refers to that portion of a gene which either naturally or normally codes for the expression product of that gene in its natural genomic environment, i.e., the region coding *in vivo* for the native expression product of the gene.

The coding region can be from a non-mutated ("normal"), mutated or altered gene, or can even be from a DNA sequence, or gene, wholly synthesized in the laboratory using methods well known to those of skill in the art of DNA synthesis.

The term "nucleotide sequence" refers to a heteropolymer of deoxyribonucleotides.

The nucleotide sequence coding for a particular peptide, oligopeptide, or polypeptide may be naturally occurring or they may be synthetically constructed. Generally, DNA segments encoding the peptides, polypeptides, and proteins of this invention are assembled from cDNA fragments and short oligonucleotide linkers, or from a series of oligonucleotides, to provide a synthetic gene that is capable of being expressed in a recombinant transcriptional unit comprising regulatory elements derived from a microbial or viral operon.

As used herein the term "a nucleotide coding (or encoding) for a peptide" refers to a nucleotide sequence coding for the peptide including artificial (man-made) start and stop codons compatible for the biological system the sequence is going to be expressed by.

The term "expression product" means the polypeptide or protein that is the natural translation product of the gene and any nucleic acid sequence coding equivalents resulting from genetic code degeneracy and thus coding for the same amino acid(s).

The term "fragment", when referring to a coding sequence, means a portion of DNA comprising less than the complete coding region, whose expression product retains essentially the same biological function or activity as the expression product of the complete coding region.

The term "DNA segment" refers to a DNA polymer, in the form of a separate fragment or as a component of a larger DNA construct, which has been derived from DNA isolated at least once in substantially pure form, i.e., free of contaminating endogenous materials and in a quantity or concentration enabling identification, manipulation, and recovery of the segment and its component nucleotide sequences by standard biochemical methods, for example, by using a cloning vector. Such segments are provided in the form of an open reading frame uninterrupted by internal non-translated sequences, or introns, which are typically present in eukaryotic genes. Sequences of non-translated DNA may be present downstream from the open reading frame, where the same do not interfere with manipulation or expression of the coding regions.

The term "primer" means a short nucleic acid sequence that can be paired with one strand of DNA and provides a free 3'-OH end at which a DNA polymerase starts synthesis of a deoxyribonucleotide chain.

The term "promoter" means a region of DNA involved in binding of RNA polymerase to initiate transcription.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The polynucleotides, and recombinant or immunogenic polypeptides, disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art. For example, individual clones isolated from a cDNA library have been conventionally purified to electrophoretic homogeneity. Purification of starting material or natural material to at least one order of magnitude, preferably two or three orders, and more preferably four or five orders of magnitude is expressly contemplated. Furthermore, a claimed polypeptide which has a purity of preferably 99.999%, or at least 99.99% or 99.9%; and even desirably 99% by weight or greater is expressly contemplated.

The nucleic acids and polypeptide expression products disclosed according to the present invention, as well as expression vectors containing such nucleic acids and/or such polypeptides, may be in "enriched form". As used herein, the term "enriched" means that the concentration of the material is at least about 2, 5, 10, 100, or 1000 times its natural concentration (for example), advantageously 0.01 %, by weight, preferably at least about 0.1% by weight. Enriched preparations of about 0.5%, 1%, 5%, 10%, and 20% by weight are also contemplated. The sequences, constructs, vectors, clones, and other materials comprising the present invention can advantageously be in enriched or isolated form.

The term "active fragment" means a fragment that generates an immune response (i.e., has immunogenic activity) when administered, alone or optionally with a suitable adjuvant, to an animal, such as a mammal, for example, a rabbit or a mouse, and also including a human, such immune response taking the form of stimulating a T-cell response within the recipient animal, such as a human. Alternatively, the "active fragment" may also be used to induce a T-cell response *in vitro.*

As used herein, the terms "portion", "segment" and "fragment," when used in relation to polypeptides, refer to a continuous sequence of residues, such as amino acid residues, which sequence forms a subset of a larger sequence. For example, if a polypeptide were subjected to treatment with any of the common endopeptidases, such as trypsin or chymotrypsin, the oligopeptides resulting from such treatment would represent portions, segments or fragments of the starting polypeptide. When used in relation to polynucleotides, these terms refer to the products produced by treatment of said polynucleotides with any of the endonucleases.

In accordance with the present invention, the term "percent identity" or "percent identical", when referring to a sequence, means that a sequence is compared to a claimed or described sequence after alignment of the sequence to be compared (the "Compared Sequence") with the described or claimed sequence (the "Reference Sequence"). The Percent Identity is then determined according to the following formula:

$$\text{Percent Identity} = 100 \, [1 - (C/R)]$$

wherein C is the number of differences between the Reference Sequence and the Compared Sequence over the length of alignment between the Reference Sequence and the Compared Sequence, wherein

(i) each base or amino acid in the Reference Sequence that does not have a corresponding aligned base or amino acid in the Compared Sequence and

(ii) each gap in the Reference Sequence and

(iii) each aligned base or amino acid in the Reference Sequence that is different from an aligned base or amino acid in the Compared Sequence, constitutes a difference and

(iiii) the alignment has to start at position 1 of the aligned sequences;

and R is the number of bases or amino acids in the Reference Sequence over the length of the alignment with the Compared Sequence with any gap created in the Reference Sequence also being counted as a base or amino acid.

If an alignment exists between the Compared Sequence and the Reference Sequence for which the Percent Identity as calculated above is about equal to or greater than a specified minimum Percent Identity then the Compared Sequence has the specified minimum Percent Identity to the Reference Sequence even though alignments may exist in which the herein above calculated Percent Identity is less than the specified Percent Identity.

The peptides of the invention can be elongated by up to four amino acids, that is 1, 2, 3 or 4 amino acids can be added to either end in any combination between 4:0 and 0:4.

Combinations of the elongations according to the invention can be depicted from table 8:

| C-terminus | N-terminus |
|---|---|
| 4 | 0 |
| 3 | 0 or 1 |
| 2 | 0 or 1 or 2 |
| 1 | 0 or 1 or 2 or 3 |
| 0 | 0 or 1 or 2 or 3 or 4 |

| N-terminus | C-terminus |
|---|---|
| 4 | 0 |
| 3 | 0 or 1 |
| 2 | 0 or 1 or 2 |
| 1 | 0 or 1 or 2 or 3 |
| 0 | 0 or 1 or 2 or 3 or 4 |

The amino acids for the elongation can be the peptides of the original sequence of the protein or any other amino acid. The elongation can be used to enhance the stability or solubility of the peptides.

The term "T-cell response" means the specific proliferation and activation of effector functions induced by a peptide *in vitro* or *in vivo.* For MHC class I restricted CTLs, effector functions may be lysis of peptide-pulsed, peptide-precursor pulsed or naturally peptide-presenting target cells, secretion of cytokines, preferably Interferon-gamma, TNF-alpha, or IL-2 induced by peptide, secretion of effector molecules, preferably granzymes or perforins induced by peptide, or degranulation.

Preferably, when the CTLs specific for a peptide according to the present invention are tested against the substituted peptides, the peptide concentration at which the substituted peptides achieve half the maximal increase in lysis relative to background is no more than about 1 mM, preferably no more than about 1 $\mu$M, more preferably no more than about 1 nM, and still more preferably no more than about 100 pM, and most preferably no more than about 10 pM. It is also preferred that the substituted peptide be recognized by CTLs from more than one individual, at least two, and more preferably three individuals.

Stimulation of an immune response is dependent upon the presence of antigens recognized as foreign by the host immune system. The discovery of the existence of tumor associated antigens has now raised the possibility of using a host's immune system to intervene in tumor growth. Various mechanisms of harnessing both the humoral and cellular arms of the immune system are currently explored for cancer immunotherapy.

Specific elements of the cellular immune response are capable of specifically recognizing and destroying tumor cells. The isolation of cytotoxic T-cells (CTL) from tumor-infiltrating cell populations or from peripheral blood suggests that such cells play an important role in natural immune defences against cancer. CD8-positive T-cells in particular, which recognize class I molecules of the major histocompatibility complex (MHC)-bearing peptides of usually 8 to 12 residues derived from proteins or defect ribosomal products (DRIPS) located in the cytosols, play an important role in this response. The MHC-molecules of the human are also designated as human leukocyte-antigens (HLA).

MHC class I molecules can be found on most cells having a nucleus which present peptides that result from proteolytic cleavage of mainly endogenous, cytosolic or nuclear proteins, DRIPS, and larger peptides. However, peptides derived from endosomal compartments or exogenous sources are also frequently found on MHC class I molecules. This non-classical way of class I presentation is referred to as cross-presentation in literature.

Since both types of response, CD8 and CD4 dependent, contribute jointly and synergistically to the anti-tumor effect, the identification and characterization of tumor-associated antigens recognized by either CD8-positive CTLs (MHC class I molecule) or by CD4-positive CTLs (MHC class II molecule) is important in the development of tumor vaccines. It is therefore an object of the present invention, to provide compositions of peptides that contain peptides binding to MHC complexes of either class.

Considering the severe side-effects and expense associated with treating cancer better prognosis and diagnostic methods are desperately needed. Therefore, there is a need to identify other factors representing biomarkers for cancer in general and CLL in particular. Furthermore, there is a need to identify factors that can be used in the treatment of cancer in general and CLL in particular.

The present invention provides peptides that are useful in treating cancers / tumors, preferably CLL that over- or exclusively present the peptides of the invention. These peptides were shown by mass spectrometry to be naturally presented by HLA molecules on primary human CLL samples.

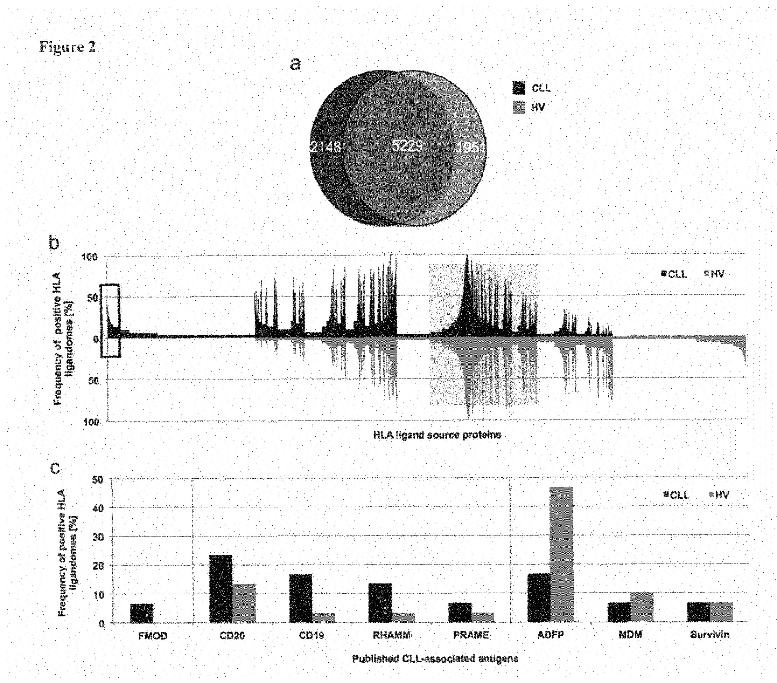

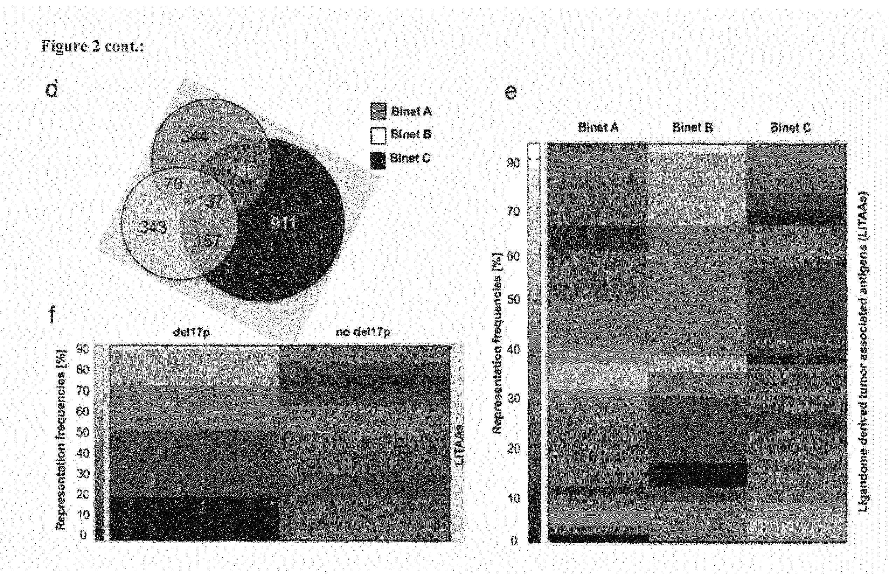

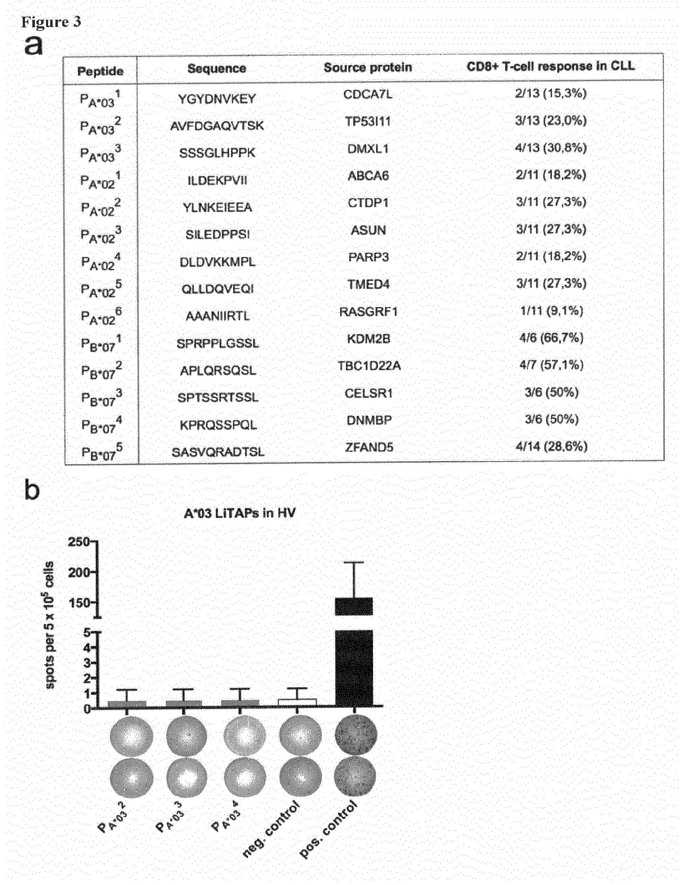

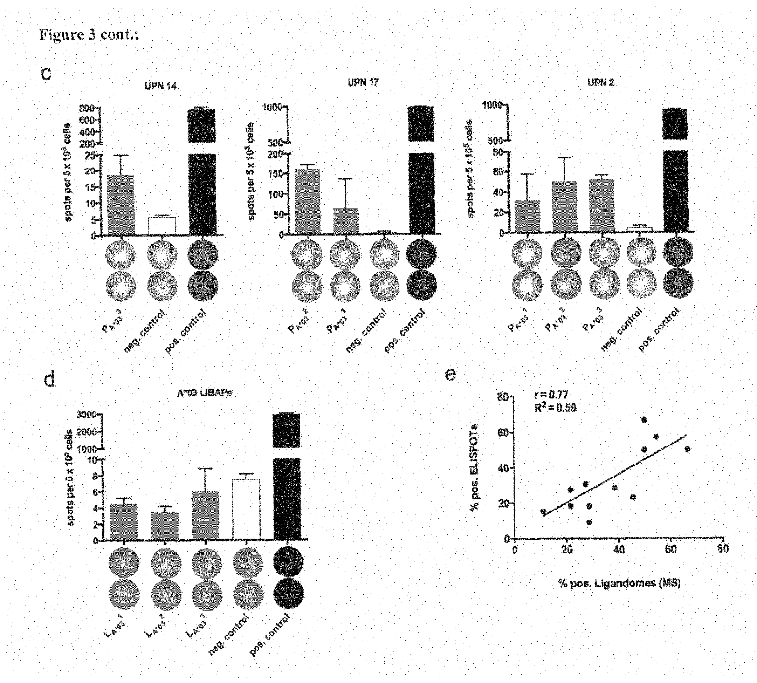

The source gene/protein (also designated "full-length protein" or "underlying protein") from which the peptides are derived were shown to be highly overexpressed in diseased (e.g. cancerous) compared with normal tissues. "Normal tissues" in relation to this invention shall particularly mean a blood sample from a healthy donor and sub-populations of blood cells, especially white blood cells, (see example 2, and figure 2) demonstrating a high degree of tumor association of the source genes. Moreover, the peptides themselves are strongly over-presented on tumor tissue - "tumor tissue" in relation to this invention shall mean a blood sample from a patient suffering from CLL and sub-populations of blood cells, especially white blood cells, but not on normal tissues (see example 3 and Figure 3).

HLA-bound peptides can be recognized by the immune system, specifically T lymphocytes/T cells. T cells can destroy the cells presenting the recognized HLA/peptide complex, e.g. cells presenting the peptides of the present invention that are derived from their underlying proteins.

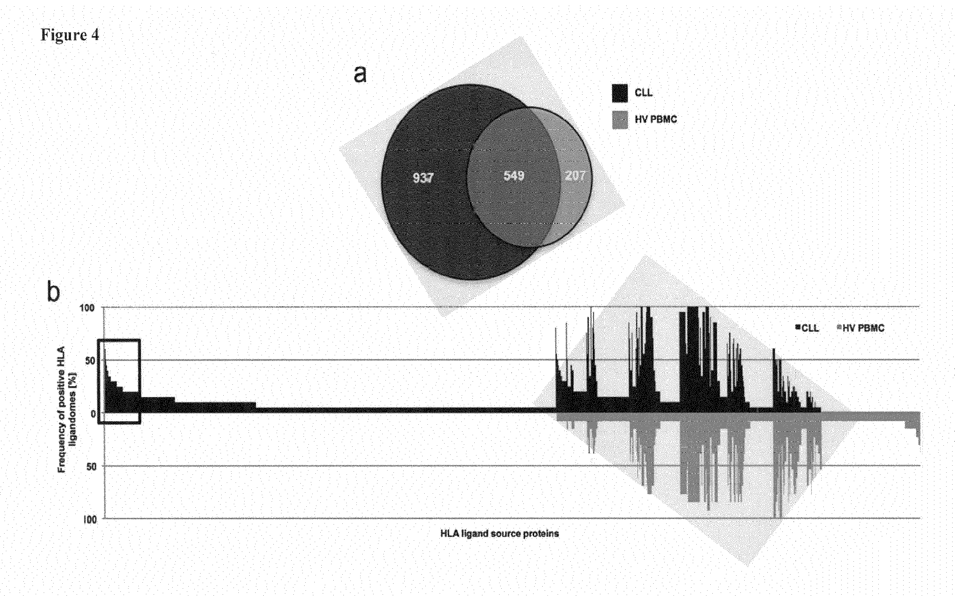

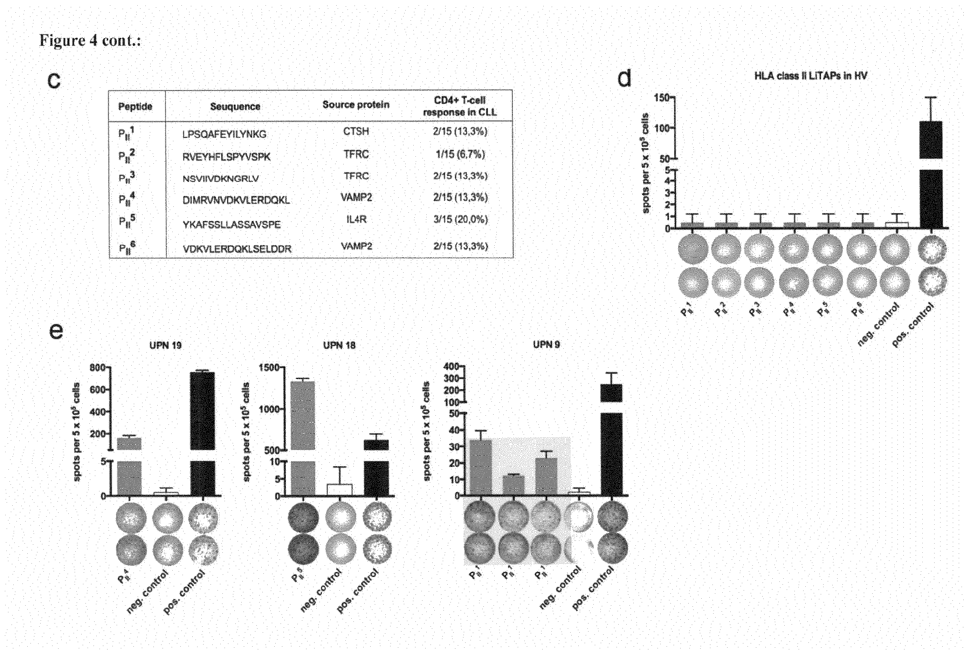

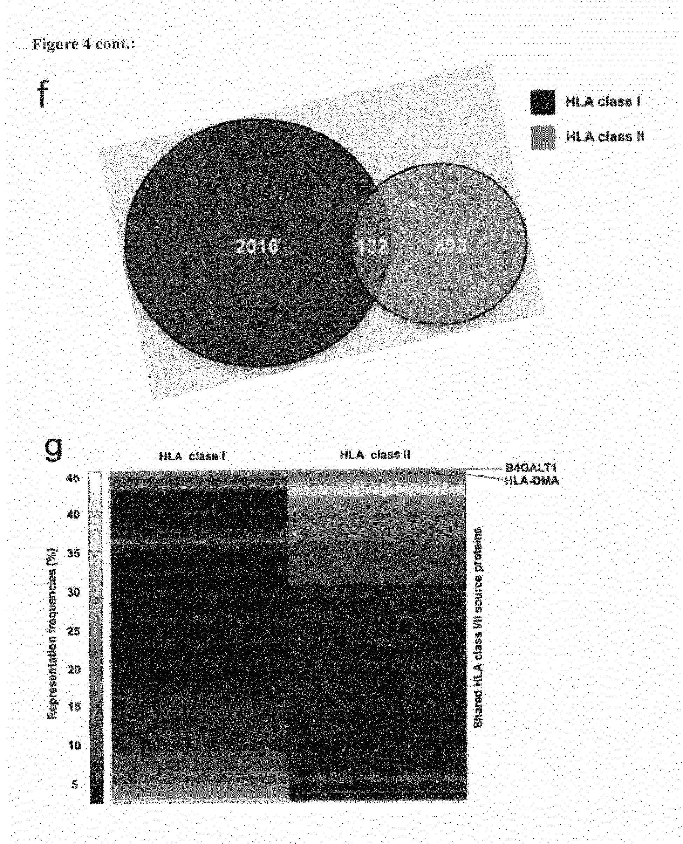

The peptides as disclosed have been shown to be capable of stimulating T cell responses and/or are over-presented and thus can be used for the production of antibodies and/or TCRs, in particular sTCRs, according to the present invention (see example 4 and figure 4). Furthermore, the peptides when complexed with the respective MHC can be used for the production of antibodies and / or TCRs, in particular sTCRs, according to the present invention, as well. Respective methods are well known to the person of skill, and can be found in the respective literature as well. Thus, the peptides of the present invention are useful for generating an immune response in a patient by which tumor cells can be destroyed. An immune response in a patient can be induced by direct administration of the described peptides or suitable precursor substances (e.g. elongated peptides, proteins, or nucleic acids encoding these peptides) to the patient, ideally in combination with an agent enhancing the immunogenicity (i.e. an adjuvant). The immune response originating from such a therapeutic vaccination can be expected to be highly specific against tumor cells because the target peptides of the present invention are not presented on normal tissues in comparable copy numbers, preventing the risk of undesired autoimmune reactions against normal cells in the patient.

A "pharmaceutical composition" is a composition suitable for administration to a human being in a medical setting. Preferably, said pharmaceutical composition is sterile and produced according to the GMP guidelines.

The pharmaceutical compositions comprise the peptides either in the free form or in the form of a pharmaceutically acceptable salt (see also above). As used herein, "a pharmaceutically acceptable salt" refers to a derivative of the disclosed peptides wherein the peptide is modified by making acid or base salts of the agent. For example, acid salts are prepared from the free base (typically wherein the neutral form of the drug has a neutral -NH$_2$ group) involving reaction with a suitable acid. Suitable acids for preparing acid salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methane sulfonic acid, ethane sulfonic acid, p-toluenesulfonic acid, salicylic acid, and the like, as well as inorganic acids, e.g., hydrochloric acid, hydrobromic acid, sulfuric acid, nitric acid phosphoric acid and the like. Conversely, preparation of basic salts of acid moieties which may be present on a peptide are prepared using a pharmaceutically acceptable base such as sodium hydroxide, potassium hydroxide, ammonium hydroxide, calcium hydroxide, trimethylamine or the like.

In an especially preferred embodiment, the pharmaceutical compositions comprise the peptides as salts of acetic acid (acetates), trifluoro acetates or hydrochloric acid (chlorides).

The peptides of the present invention can be used to generate and develop specific antibodies against MHC/peptide complexes. These can be used for therapy, targeting toxins or radioactive substances to the diseased tissue. Another use of these antibodies can be targeting radionuclides to the diseased tissue for imaging purposes such as PET. This use can help to detect small metastases or to determine the size and precise localization of diseased tissues.

Therefore, it is a further aspect of the invention to provide a method for producing a recombinant antibody specifically binding to a human major histocompatibility complex (MHC) class I being complexed with a HLA-restricted antigen, the method comprising: immunizing a genetically engineered non-human mammal comprising cells expressing said human major histocompatibility complex (MHC) class I with a soluble form of a MHC class I molecule being complexed with said HLA-restricted antigen; isolating mRNA molecules from antibody producing cells of said non-human mammal; producing a phage display library displaying protein molecules encoded by said mRNA molecules; and isolating at least one phage from said phage display library, said at least one phage displaying said antibody specifically binding to said human major histocompatibility complex (MHC) class I being complexed with said HLA-restricted antigen.

It is a further aspect of the invention to provide an antibody that specifically binds to a human major histocompatibility complex (MHC) class I being complexed with a HLA-restricted antigen, wherein the antibody preferably is a polyclonal antibody, monoclonal antibody, bi-specific antibody and/or a chimeric antibody.

Yet another aspect of the present invention then relates to a method of producing said antibody specifically binding to a human major histocompatibility complex (MHC) class I being complexed with a HLA-restricted antigen, the method comprising: immunizing a genetically engineered non-human mammal comprising cells expressing said human major histocompatibility complex (MHC) class I with a soluble form of a MHC class I molecule being complexed with said HLA-restricted antigen; isolating mRNA molecules from antibody producing cells of said non-human mammal; producing a phage display library displaying protein molecules encoded by said mRNA molecules; and isolating at least one phage from said phage display library, said at least one phage displaying said antibody specifically bindable to said human major histocompatibility complex (MHC) class I being complexed with said HLA-restricted antigen. Respective methods for producing such antibodies and single chain class I major histocompatibility complexes, as well as other tools for the production of these antibodies are disclosed in WO 03/068201, WO 2004/084798, WO 01/72768, WO 03/070752, and Cohen CJ, et al. Recombinant antibodies with MHC-restricted, peptide-specific, T-cell receptor-like specificity: new tools to study antigen presentation and TCR-peptide-MHC interactions. J Mol Recognit. 2003 Sep-Oct;16(5):324-32.; Denkberg G, et al. Selective targeting of melanoma and APCs using a recombinant antibody with TCR-like specificity directed toward a melanoma differentiation antigen. J Immunol. 2003 Sep 1;171(5):2197-207; and Cohen CJ, et al. Direct phenotypic analysis of human MHC class I antigen presentation: visualization, quantitation, and in situ detection of human viral epitopes using peptide-specific, MHC-restricted human recombinant antibodies. J Immunol. 2003 Apr 15; 170(8):4349-61.

Preferably, the antibody is binding with a binding affinity of below 20 nanomolar, preferably of below 10 nanomolar, to the complex, which is regarded as "specific" in the context of the present invention.

It is a further aspect of the invention to provide a method for producing a soluble T-cell receptor recognizing a specific peptide-MHC complex. Such soluble T-cell receptors can be generated from specific T-cell clones, and their affinity can be increased by mutagenesis targeting the complementarity-determining regions. For the purpose of T-cell receptor selection, phage display can be used (US 2010/0113300, Liddy N, et al. Monoclonal TCR-redirected tumor cell killing. Nat Med 2012 Jun;18(6):980-987). For the purpose of stabilization of T-cell receptors during phage display and in case of practical use as drug, alpha and beta chain can be linked e.g. by non-native disulfide bonds, other covalent bonds (single-chain T-cell receptor), or by dimerization domains (see Boulter JM, et al. Stable, soluble T-cell receptor molecules for crystallization and therapeutics. Protein Eng 2003 Sep;16(9):707-711.; Card KF, et al. A soluble single-chain T-cell receptor IL-2 fusion protein retains MHC-restricted peptide specificity and IL-2 bioactivity. Cancer Immunol Immunother 2004 Apr;53(4):345-357; and Willcox BE, et al. Production of soluble alphabeta T-cell receptor heterodimers suitable for biophysical analysis of ligand binding. Protein Sci 1999 Nov; 8 (11):2418-2423). The T-cell receptor can be linked to toxins, drugs, cytokines (see US 2013/0115191), domains recruiting effector cells such as an anti-CD3 domain, etc., in order to execute particular functions on target cells. Moreover, it could be expressed in T cells used for adoptive transfer.

Further information can be found in WO 2004/033685A1 and WO 2004/074322A1. A combination of sTCRs is described in WO 2012/056407A1. Further methods for the production are disclosed in WO 2013/057586A1.

In addition, they can be used to verify a pathologist's diagnosis of a cancer based on a biopsied sample.

In order to select over-presented peptides, a presentation profile is calculated showing the median sample presentation as well as replicate variation. The profile juxtaposes samples of the tumor entity of interest to a baseline of normal tissue samples. Each of these profiles can then be consolidated into an over-presentation score by calculating the p-value of a Linear Mixed-Effects Model (J. Pinheiro, et al. The nlme Package: Linear and Nonlinear Mixed Effects Models. 2007) adjusting for multiple testing by False Discovery Rate (Y. Benjamini and Y. Hochberg. Controlling the False Discovery Rate: A Practical and Powerful Approach to Multiple Testing. Journal of the Royal Statistical Society. Series B (Methodological), Vol.57 (No.1):289-300, 1995).

For the identification and relative quantitation of HLA ligands by mass spectrometry, HLA molecules from shock-frozen tissue samples were purified and HLA-associated peptides were isolated. The isolated peptides were separated and sequences were identified by online nano-electrospray-ionization (nanoESI) liquid chromatography-mass spectrometry (LC-MS) experiments. The resulting peptide sequences were verified by comparison of the fragmentation pattern of natural TUMAPs recorded from CLL samples with the fragmentation patterns of corresponding synthetic reference peptides of identical sequences. Since the peptides were directly identified as ligands of HLA molecules of primary tumors, these results provide direct evidence for the natural processing and presentation of the identified peptides on primary cancer tissue obtained from CLL patients.

The discovery pipeline XPRESIDENT® v2.1 (see, for example, US 2013-0096016,) allows the identification and selection of relevant over-presented peptide vaccine candidates based on direct relative quantitation of HLA-restricted peptide levels on cancer tissues in comparison to several different non-cancerous tissues and organs. This was achieved by the development of label-free differential quantitation using the acquired LC-MS data processed by a proprietary data analysis pipeline, combining algorithms for sequence identification, spectral clustering, ion counting, retention time alignment, charge state deconvolution and normalization.

Presentation levels including error estimates for each peptide and sample were established. Peptides exclusively presented on tumor tissue and peptides over-presented in tumor versus non-cancerous tissues and organs have been identified.

HLA-peptide complexes from CLL tissue samples were purified and HLA-associated peptides were isolated and analysed by LC-MS (see examples). All TUMAPs contained in the present application were identified with this approach on primary CLL samples confirming their presentation on primary CLL.

All TUMAPs contained in the application at hand were identified with this approach on primary CLL samples confirming their presentation on primary CLL.

TUMAPs identified on multiple CLL tumor and normal tissues were quantified using ion-counting of label-free LC-MS data. The method assumes that LC-MS signal areas of a peptide correlate with its abundance in the sample. All quantitative signals of a peptide in various LC-MS experiments were normalized based on central tendency, averaged per sample and merged into a bar plot, called presentation profile. The presentation profile consolidates different analysis methods like protein database search, spectral clustering, charge state deconvolution (decharging) and retention time alignment and normalization.

The present invention therefore relates to a peptide of between 10 and 30 amino acids in length, comprising an amino acid sequence consisting of SEQ ID NO: 167, or a pharmaceutically acceptable salt thereof.

The present invention further relates to the peptides according to the invention that have the ability to bind to a molecule of the human major histocompatibility complex (MHC) class-I.

The present invention further relates to the peptides according to the invention wherein the peptide consists of an amino acid sequence according to SEQ ID NO: 167.

The present invention further relates to the peptides according to the invention, wherein the peptide includes non-peptide bonds.

The present invention further relates to the peptides according to the invention, wherein the peptide is a fusion protein, comprising (a) an amino acid sequence consisting of SEQ ID NO: 167; and (b) N-terminal amino acids 1-80 of HLA-DR antigen-associated invariant chain (Ii).

The present invention further relates to a nucleic acid, encoding the peptides according to the invention.

The present invention further relates to the nucleic acid according to the invention that is DNA, cDNA, PNA, RNA or combinations thereof.

The present invention further relates to an expression vector expressing a nucleic acid according to the invention.

The present invention further relates to a host cell comprising a nucleic acid according to the invention or an expression vector according to the invention.

The present invention further relates to the host cell according to the invention that is an antigen presenting cell.

The present invention further relates to the host cell according to the invention wherein the antigen presenting cell is a dendritic cell.

The present invention further relates to a method for producing a peptide according to the invention, the method comprising culturing the host cell described, and isolating the peptide from the host cell or its culture medium.

The present invention further relates to an *in vitro* method for producing activated cytotoxic T lymphocytes (CTL), the method comprising contacting *in vitro* CTL with antigen loaded human class I MHC molecules expressed on the surface of a suitable antigen-presenting cell for a period of time sufficient to activate said CTL in an antigen specific manner, wherein said antigen is any peptide according to the invention.

The present invention further relates to the method as described, wherein said antigen is loaded onto class I MHC molecules expressed on the surface of a suitable antigen-presenting cell by contacting a sufficient amount of the antigen with an antigen-presenting cell.

The present invention further relates to the method according to the invention, wherein the antigen-presenting cell comprises an expression vector expressing said peptide containing SEQ ID NO: 167.

The present invention further relates to activated cytotoxic T lymphocytes (CTL), produced by the method according to the invention, which selectively recognise a cell which aberrantly expresses a polypeptide comprising an amino acid sequence described.

The present invention further discloses a method of killing target cells in a patient which target cells aberrantly express a polypeptide comprising any amino acid sequence according to the invention, the method comprising administering to the patient an effective number of cytotoxic T lymphocytes (CTL) according to the invention.

The present invention further relates to any peptide according to the invention, a nucleic acid according to the invention, an expression vector according to the invention, a cell according to the invention, or an activated cytotoxic T lymphocyte according to the invention for use as a medicament against cancer.

The present invention further relates to a use according to the invention, wherein the medicament is a vaccine.

The present invention further relates to a use according to the invention, wherein said cancer cells are CLL cells or other non solid tumor cells.

The term "antibody" or "antibodies" is used herein in a broad sense and includes both polyclonal and monoclonal antibodies. In addition to intact or "full" immunoglobulin molecules, also included in the term "antibodies" are fragments or polymers of those immunoglobulin molecules and humanized versions of immunoglobulin molecules, so long as they exhibit any of the desired properties (e.g., specific binding of an CLL marker polypeptide, delivery of a toxin to an CLL (leukimea) cells expressing a CLL marker gene at an increased level, and/or inhibiting the activity of a CLL marker polypeptide) according to the invention.

Whenever possible, the antibodies of the invention may be purchased from commercial sources. The antibodies of the invention may also be generated using well-known methods. The skilled artisan will understand that either full length CLL marker polypeptides or fragments thereof may be used to generate the antibodies of the invention. A polypeptide to be used for generating an antibody of the invention may be partially or fully purified from a natural source, or may be produced using recombinant DNA techniques.

For example, a cDNA encoding a peptide according to the present invention, such as a peptide according to SEQ ID NO: 167 polypeptide, or a variant or fragment thereof, can be expressed in prokaryotic cells (e.g., bacteria) or eukaryotic cells (e.g., yeast, insect, or mammalian cells), after which the recombinant protein can be purified and used to generate a monoclonal or polyclonal antibody preparation that specifically bind the CLL marker polypeptide used to generate the antibody according to the invention.

One of skill in the art will realize that the generation of two or more different sets of monoclonal or polyclonal antibodies maximizes the likelihood of obtaining an antibody with the specificity and affinity required for its intended use (e.g., ELISA, immunohistochemistry, *in vivo* imaging, immunotoxin therapy). The antibodies are tested for their desired activity by known methods, in accordance with the purpose for which the antibodies are to be used (e.g., ELISA, immunohistochemistry, immunotherapy, etc.; for further guidance on the generation and testing of antibodies, see, e.g., Harlow and Lane, Antibodies: A Laboratory Manual, Cold Spring Harbor Laboratory Press, Cold Spring Harbor, N.Y., 1988, new 2nd edition 2013). For example, the antibodies may be tested in ELISA assays or Western blots. After their initial *in vitro* characterization, antibodies intended for therapeutic or *in vivo* diagnostic use are tested according to known clinical testing methods.

The term "monoclonal antibody" as used herein refers to an antibody obtained from a substantially homogeneous population of antibodies, i.e.; the individual antibodies comprising the population are identical except for possible naturally occurring mutations that may be present in minor amounts. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired antagonistic activity (U.S. Pat. No. 4,816,567).

Monoclonal antibodies of the invention may be prepared using hybridoma methods. In a hybridoma method, a mouse or other appropriate host animal is typically immunized with an immunizing agent to elicit lymphocytes that produce or are capable of producing antibodies that will specifically bind to the immunizing agent. Alternatively, the lymphocytes may be immunized *in vitro.*

The monoclonal antibodies may also be made by recombinant DNA methods, such as those described in U.S. Pat. No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies).

*In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 and U.S. Pat. No. 4,342,566. Papain digestion of antibodies typically produces two identical antigen binding fragments, called Fab fragments, each with a single antigen binding site, and a residual Fc fragment. Pepsin treatment yields a a F(ab')$_2$ fragment and a pFc' fragment.

The antibody fragments, whether attached to other sequences or not, can also include insertions, deletions, substitutions, or other selected modifications of particular regions or specific amino acids residues, provided the activity of the fragment is not significantly altered or impaired compared to the non-modified antibody or antibody fragment. These modifications can provide for some additional property, such as to remove/add amino acids capable of disulfide bonding, to increase its bio-longevity, to alter its secretory characteristics, etc. In any case, the antibody fragment must possess a bioactive property, such as binding activity, regulation of binding at the binding domain, etc. Functional or active regions of the antibody may be identified by mutagenesis of a specific region of the protein, followed by expression and testing of the expressed polypeptide. Such methods are readily apparent to a skilled practitioner in the art and can include site-specific mutagenesis of the nucleic acid encoding the antibody fragment.

The antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab' or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework (FR) residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin.

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed by substituting rodent CDRs or CDR sequences for the corresponding sequences of a human antibody. Accordingly, such "humanized" antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Transgenic animals (e.g., mice) that are capable, upon immunization, of producing a full repertoire of human antibodies in the absence of endogenous immunoglobulin production can be employed. For example, it has been described that the homozygous deletion of the antibody heavy chain joining region gene in chimeric and germ-line mutant mice results in complete inhibition of endogenous antibody production. Transfer of the human germ-line immunoglobulin gene array in such germ-line mutant mice will result in the production of human antibodies upon antigen challenge. Human antibodies can also be produced in phage display libraries.

Antibodies of the invention are preferably administered to a subject in a pharmaceutically acceptable carrier. Typically, an appropriate amount of a pharmaceutically-acceptable salt is used in the formulation to render the formulation isotonic. Examples of the pharmaceutically-acceptable carrier include saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of antibody being administered.

The antibodies can be administered to the subject, patient, or cell by injection (e.g., intravenous, intraperitoneal, subcutaneous, intramuscular), or by other methods such as infusion that ensure its delivery to the bloodstream in an effective form. The antibodies may also be administered by intratumoral or peritumoral routes, to exert local as well as systemic therapeutic effects. Local or intravenous injection is preferred.

Effective dosages and schedules for administering the antibodies may be determined empirically, and making such determinations is within the skill in the art. Those skilled in the art will understand that the dosage of antibodies that must be administered will vary depending on, for example, the subject that will receive the antibody, the route of administration, the particular type of antibody used and other drugs being administered. A typical daily dosage of the antibody used alone might range from about 1 ($\mu$g/kg to up to 100 mg/kg of body weight or more per day, depending on the factors mentioned above. Following administration of an antibody for treating CLL, the efficacy of the therapeutic antibody can be assessed in various ways well known to the skilled practitioner.

The antibodies may also be used for *in vivo* diagnostic assays. Generally, the antibody is labeled with a radionucleotide (such as $^{111}$In, $^{99}$Tc, $^{14}$C, $^{131}$I, $^{3}$H, $^{32}$P or $^{35}$S) so that the tumor can be localized using immunoscintiography.

Antibodies for diagnostic use may be labeled with probes suitable for detection by various imaging methods. Methods for detection of probes include, but are not limited to, fluorescence, light, confocal and electron microscopy; magnetic resonance imaging and spectroscopy; fluoroscopy, computed tomography and positron emission tomography. Suitable probes include, but are not limited to, fluorescein, rhodamine, eosin and other fluorophores, radioisotopes, gold, gadolinium and other lanthanides, paramagnetic iron, fluorine-18 and other positron-emitting radionuclides. Additionally, probes may be bi- or multi-functional and be detectable by more than one of the methods listed. These antibodies may be directly or indirectly labeled with said probes. Attachment of probes to the antibodies includes covalent attachment of the probe, incorporation of the probe into the antibody, and the covalent attachment of a chelating compound for binding of probe, amongst others well recognized in the art. For immunohistochemistry, the disease tissue sample may be fresh or frozen or may be embedded in paraffin and fixed with a preservative such as formalin. The fixed or embedded section contains the sample are contacted with a labeled primary antibody and secondary antibody, wherein the antibody is used to detect the expression of the proteins *in situ*.

The present invention thus provides a peptide of between 10 and 30 amino acids in length, comprising an amino acid sequence consisting of SEQ ID NO: 167, or a pharmaceutically acceptable salt thereof.

The peptides of the invention have the ability to bind to a molecule of the human major histocompatibility complex (MHC) class-I.

In the present invention, the term "homologous" refers to the degree of identity (see Percent Identity above) between sequences of two amino acid sequences, i.e. peptide or polypeptide sequences. The aforementioned "homology" is determined by comparing two sequences aligned under optimal conditions over the sequences to be compared. Such a sequence homology can be calculated by creating an alignment using, for example, the ClustalW algorithm. Commonly available sequence analysis software, more specifically, Vector NTI, GENETYX or other analysis tools are provided by public databases.

A person skilled in the art will be able to assess, whether T cells induced by a variant of a specific peptide will be able to cross-react with the peptide itself (Fong L, et al. Altered peptide ligand vaccination with Flt3 ligand expanded dendritic cells for tumor immunotherapy. Proc Natl Acad Sci USA. 2001 Jul 17;98(15):8809-14; Zaremba S, et al. Identification of an enhancer agonist cytotoxic T lymphocyte peptide from human carcinoembryonic antigen. Cancer Res. 1997 Oct 15;57(20):4570-7; Colombetti S, et al. Impact of orthologous melan-A peptide immunizations on the anti-self melan-A/HLA-A2 T cell cross-reactivity. J Immunol. 2006 Jun 1;176(11):6560-7; Appay V, et al. Decreased specific CD8+ T cell cross-reactivity of antigen recognition following vaccination with Melan-A peptide. Eur J Immunol. 2006 Jul;36(7):1805-14).

CTL can subsequently cross-react with cells and kill cells that express a polypeptide that contains the natural amino acid sequence of the cognate peptide as defined in the aspects of the invention. As can be derived from the scientific literature (Godkin A, et al. Use of eluted peptide sequence data to identify the binding characteristics of peptides to the insulin-dependent diabetes susceptibility allele HLA-DQ8 (DQ 3.2). Int Immunol. 1997 Jun;9(6):905-11) and databases (Rammensee H. et al. SYFPEITHI: database for MHC ligands and peptide motifs. Immunogenetics. 1999 Nov;50(3-4):213-9), certain positions of HLA binding peptides are typically anchor residues forming a core sequence fitting to the binding motif of the HLA receptor, which is defined by polar, electrophysical, hydrophobic and spatial properties of the polypeptide chains constituting the binding groove.

Longer peptides may also be suitable. It is also possible, that MHC class I epitopes, although usually between 8 and 11 amino acids long, are generated by peptide processing from longer peptides or proteins that include the actual epitope. It is preferred that the residues that flank the actual epitope are residues that do not substantially affect proteolytic cleavage necessary to expose the actual epitope during processing.

Accordingly, the present invention also provides peptides of MHC class I epitopes wherein the peptide has an overall length of between 10 and 30 amino acids.

Of course, the peptide according to the present invention will have the ability to bind to a molecule of the human major histocompatibility complex (MHC) class I. Binding of a peptide to a MHC complex may be tested by methods known in the art.

In a particularly preferred embodiment of the invention the peptide consists of an amino acid sequence according to SEQ ID NO: 167.

In one embodiment of the present invention, the peptide is a fusion protein which comprises, for example, the 80 N-terminal amino acids of the HLA-DR antigen-associated invariant chain (p33, in the following "Ii") as derived from the NCBI, GenBank Accession number X00497. In other fusions, the peptides of the present invention can be fused to an antibody as described herein, or a functional part thereof, in particular into a sequence of an antibody, so as to be specifically targeted by said antibody, or, for example, to or into an antibody that is specific for dendritic cells.

In a reverse peptide bond amino acid residues are not joined by peptide (-CO-NH-) linkages but the peptide bond is reversed. Such retro-inverso peptidomimetics may be made using methods known in the art, for example such as those described in Meziere et al (1997) J. Immunol. 159, 3230-3237. This approach involves making pseudopeptides containing changes involving the backbone, and not the orientation of side chains. Meziere et al (1997) show that for MHC binding and T helper cell responses, these pseudopeptides are useful. Retro-inverse peptides, which contain NH-CO bonds instead of CO-NH peptide bonds, are much more resistant to proteolysis.

A non-peptide bond is, for example, $-CH_2-NH$, $-CH_2S-$, $-CH_2CH_2-$, $-CH=CH-$, $-COCH_2-$, $-CH(OH)CH_2-$, and $-CH_2SO-$. United States Patent 4,897,445 provides a method for the solid phase synthesis of non-peptide bonds ($-CH_2-NH$) in polypeptide chains which involves polypeptides synthesized by standard procedures and the non-peptide bond synthesized by reacting an amino aldehyde and an amino acid in the presence of $NaCNBH_3$.

Peptides comprising the sequences described above may be synthesized with additional chemical groups present at their amino and/or carboxy termini, to enhance the stability, bioavailability, and/or affinity of the peptides. For example, hydrophobic groups such as carbobenzoxyl, dansyl, or t-butyloxycarbonyl groups may be added to the peptides' amino termini. Likewise, an acetyl group or a 9-fluorenylmethoxycarbonyl group may be placed at the peptides' amino termini. Additionally, the hydrophobic group, t-butyloxycarbonyl, or an amido group may be added to the peptides' carboxy termini.

A peptide, wherein the peptide includes non-peptide bonds is a preferred embodiment of the invention. Generally, peptides (at least those containing peptide linkages between amino acid residues) may be synthesized by the Fmoc-polyamide mode of solid-phase peptide synthesis as disclosed by Lukas et al. (Solid-phase peptide synthesis under continuous-flow conditions. Proc Natl Acad Sci U S A. May 1981; 78(5): 2791-2795) and references therein. Temporary N-amino group protection is afforded by the 9-fluorenylmethyloxycarbonyl (Fmoc) group. Repetitive cleavage of this highly base-labile protecting group is done using 20% piperidine in N, N-dimethylformamide. Side-chain functionalities may be protected as their butyl ethers (in the case of serine threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylbenzenesulphonyl derivative (in the case of arginine). Where glutamine or asparagine are C-terminal residues, use is made of the 4,4'-dimethoxybenzhydryl group for protection of the side chain amido functionalities. The solid-phase support is based on a polydimethyl-acrylamide polymer constituted from the three monomers dimethylacrylamide (backbone-monomer), bisacryloylethylene diamine (cross linker) and acryloylsarcosine methyl ester (functionalizing agent). The peptide-to-resin cleavable linked agent used is the acid-labile 4-hydroxymethyl-phenoxyacetic acid derivative. All amino acid derivatives are added as their preformed symmetrical anhydride derivatives with the exception of asparagine and glutamine, which are added using a reversed N, N-dicyclohexylcarbodiimide/lhydroxybenzotriazole mediated coupling procedure. All coupling and deprotection reactions are monitored using ninhydrin, trinitrobenzene sulphonic acid or isotin test procedures. Upon completion of synthesis, peptides are cleaved from the resin support with concomitant removal of side-chain protecting groups by treatment with 95% trifluoroacetic acid containing a 50 % scavenger mix. Scavengers commonly used include ethandithiol, phenol, anisole and water, the exact choice depending on the constituent amino acids of the peptide being synthesized. Also a combination of solid phase and solution phase methodologies for the synthesis of peptides is possible (see, for example, Bruckdorfer et al., 2004, and the references as cited therein).

Trifluoroacetic acid is removed by evaporation *in vacuo,* with subsequent trituration with diethyl ether affording the crude peptide. Any scavengers present are removed by a simple extraction procedure which on lyophilisation of the aqueous phase affords the crude peptide free of scavengers. Reagents for peptide synthesis are generally available from e.g. Calbiochem-Novabiochem (UK) Ltd, Nottingham NG7 2QJ, UK.

Purification may be performed by any one, or a combination of, techniques such as re-crystallization, size exclusion chromatography, ion-exchange chromatography, hydrophobic interaction chromatography and (usually) reverse-phase high performance liquid chromatography using e.g. acetonitril/water gradient separation.

Analysis of peptides may be carried out using thin layer chromatography, electrophoresis, in particular capillary electrophoresis, solid phase extraction (CSPE), reverse-phase high performance liquid chromatography, amino-acid analysis after acid hydrolysis and by fast atom bombardment (FAB) mass spectrometric analysis, as well as MALDI and ESI-Q-TOF mass spectrometric analysis.

A further aspect of the invention provides a nucleic acid (for example a polynucleotide) encoding a peptide of the invention. The polynucleotide may be, for example, DNA, cDNA, PNA, RNA or combinations thereof, either single- and/or double-stranded, or native or stabilized forms of polynucleotides, such as, for example, polynucleotides with a phosphorothioate backbone and it may or may not contain introns so long as it codes for the peptide. Of course, only peptides that contain naturally occurring amino acid residues joined by naturally occurring peptide bonds are encodable by a polynucleotide. A still further aspect of the invention provides an expression vector capable of expressing a polypeptide according to the invention.

A variety of methods have been developed to link polynucleotides, especially DNA, to vectors for example via complementary cohesive termini. For instance, complementary homopolymer tracts can be added to the DNA segment to be inserted to the vector DNA. The vector and DNA segment are then joined by hydrogen bonding between the complementary homopolymeric tails to form recombinant DNA molecules.

Synthetic linkers containing one or more restriction sites provide an alternative method of joining the DNA segment to vectors. Synthetic linkers containing a variety of restriction endonuclease sites are commercially available from a number of sources including International Biotechnologies Inc. New Haven, CN, USA.

A desirable method of modifying the DNA encoding the polypeptide of the invention employs the polymerase chain reaction as disclosed by Saiki RK, et al. (Diagnosis of sickle cell anemia and beta-thalassemia with enzymatically amplified DNA and nonradioactive allele-specific oligonucleotide probes. N Engl J Med. 1988 Sep 1;319(9):537-41). This method may be used for introducing the DNA into a suitable vector, for example by engineering in suitable restriction sites, or it may be used to modify the DNA in other useful ways as is known in the art. If viral vectors are used, pox- or adenovirus vectors are preferred.

The DNA (or in the case of retroviral vectors, RNA) may then be expressed in a suitable host to produce a polypeptide comprising the peptide of the invention. Thus, the DNA encoding the peptide or variant of the invention may be used in accordance with known techniques, appropriately modified in view of the teachings contained herein, to construct an expression vector, which is then used to transform an appropriate host cell for the expression and production of the polypeptide of the invention. Such techniques include those disclosed in US Patent Nos. 4,440,859, 4,530,901, 4,582,800, 4,677,063, 4,678,751, 4,704,362, 4,710,463, 4,757,006, 4,766,075, and 4,810,648.

The DNA (or in the case of retroviral vectors, RNA) encoding the polypeptide constituting the compound of the invention may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognized by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance.

Alternatively, the gene for such selectable trait can be on another vector, which is used to co-transform the desired host cell.

Host cells that have been transformed by the recombinant DNA of the invention are then cultured for a sufficient time and under appropriate conditions known to those skilled in the art in view of the teachings disclosed herein to permit the expression of the polypeptide, which can then be recovered.

Many expression systems are known, including bacteria (for example *E. coli* and *Bacillus subtilis*), yeasts (for example *Saccharomyces cerevisiae*), filamentous fungi (for example *Aspergillus spec.*), plant cells, animal cells and insect cells. Preferably, the system can be mammalian cells such as CHO cells available from the ATCC Cell Biology Collection.

A typical mammalian cell vector plasmid for constitutive expression comprises the CMV or SV40 promoter with a suitable poly A tail and a resistance marker, such as neomycin. One example is pSVL available from Pharmacia, Piscataway, NJ, USA. An example of an inducible mammalian expression vector is pMSG, also available from Pharmacia. Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (Yips) and incorporate the yeast selectable markers HIS3, TRP1, LEU2 and URA3. Plasmids pRS413-416 are Yeast Centromere plasmids (Ycps). CMV promoter-based vectors (for example from Sigma-Aldrich) provide transient or stable expression, cytoplasmic expression or secretion, and N-terminal or C-terminal tagging in various combinations of FLAG, 3xFLAG, c-*myc* or MAT. These fusion proteins allow for detection, purification and analysis of recombinant protein. Dual-tagged fusions provide flexibility in detection.

The strong human cytomegalovirus (CMV) promoter regulatory region drives constitutive protein expression levels as high as 1 mg/L in COS cells. For less potent cell lines, protein levels are typically ~0.1 mg/L. The presence of the SV40 replication origin will result in high levels of DNA replication in SV40 replication permissive COS cells. CMV vectors, for example, can contain the pMB1 (derivative of pBR322) origin for replication in bacterial cells, the b-lactamase gene for ampicillin resistance selection in bacteria, hGH polyA, and the f1 origin. Vectors containing the preprotrypsin leader (PPT) sequence can direct the secretion of FLAG fusion proteins into the culture medium for purification using ANTI-FLAG antibodies, resins, and plates. Other vectors and expression systems are well known in the art for use with a variety of host cells.

In another embodiment two or more peptides of the invention are encoded and thus expressed in a successive order (similar to "beads on a string" constructs). In doing so, the peptides may be linked or fused together by stretches of linker amino acids, such as for example LLLLLL, or may be linked without any additional peptide(s) between them.

The present invention also relates to a host cell transformed with a polynucleotide vector construct of the present invention. The host cell can be either prokaryotic or eukaryotic. Bacterial cells may be preferred prokaryotic host cells in some circumstances and typically are a strain of E. coli such as, for example, the E. coli strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include yeast, insect and mammalian cells, preferably vertebrate cells such as those from a mouse, rat, monkey or human fibroblastic and colon cell lines. Yeast host cells include YPH499, YPH500 and YPH501, which are generally available from Stratagene Cloning Systems, La Jolla, CA 92037, USA. Preferred mammalian host cells include Chinese hamster ovary (CHO) cells available from the ATCC as CCL61, NIH Swiss mouse embryo cells NIH/3T3 available from the ATCC as CRL 1658, monkey kidney-derived COS-1 cells available from the ATCC as CRL 1650 and 293 cells which are human embryonic kidney cells. Preferred insect cells are Sf9 cells which can be transfected with baculovirus expression vectors. An overview regarding the choice of suitable host cells for expression can be found in, for example, the textbook of Paulina Balbás and Argelia Lorence "Methods in Molecular Biology Recombinant Gene Expression, Reviews and Protocols," Part One, Second Edition, ISBN 978-1-58829-262-9, and other literature known to the person of skill.

Transformation of appropriate cell hosts with a DNA construct of the present invention is accomplished by well-known methods that typically depend on the type of vector used. With regard to transformation of prokaryotic host cells, see, for example, Cohen et al (1972) Proc. Natl. Acad. Sci. USA 69, 2110, and Sambrook et al (1989) Molecular Cloning, A Laboratory Manual, Cold Spring Harbor Laboratory, Cold Spring Harbor, NY. Transformation of yeast cells is described in Sherman et al (1986) Methods In Yeast Genetics, A Laboratory Manual, Cold Spring Harbor, NY. The method of Beggs (1978) Nature 275,104-109 is also useful. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA. Electroporation is also useful for transforming and/or transfecting cells and is well known in the art for transforming yeast cell, bacterial cells, insect cells and vertebrate cells.

Successfully transformed cells, i.e. cells that contain a DNA construct of the present invention, can be identified by well-known techniques such as PCR. Alternatively, the presence of the protein in the supernatant can be detected using antibodies.

It will be appreciated that certain host cells of the invention are useful in the preparation of the peptides of the invention, for example bacterial, yeast and insect cells. However, other host cells may be useful in certain therapeutic methods. For example, antigen-presenting cells, such as dendritic cells, may usefully be used to express the peptides of the invention such that they may be loaded into appropriate MHC molecules. Thus, the current invention provides a host cell comprising a nucleic acid or an expression vector according to the invention.

In a preferred embodiment the host cell is an antigen presenting cell, in particular a dendritic cell or antigen presenting cell. APCs loaded with a recombinant fusion protein containing prostatic acid phosphatase (PAP) were approved by the U.S. Food and Drug Administration (FDA) on April 29, 2010, to treat asymptomatic or minimally symptomatic metastatic HRPC (Sipuleucel-T) (Small EJ, et al. Placebo-controlled phase III trial of immunologic therapy with sipuleucel-T (APC8015) in patients with metastatic, asymptomatic hormone refractory prostate cancer. J Clin Oncol. 2006 Jul 1;24(19):3089-94. Rini et al. Combination immunotherapy with prostatic acid phosphatase pulsed antigen-presenting cells (provenge) plus bevacizumab in patients with serologic progression of prostate cancer after definitive local therapy. Cancer. 2006 Jul 1:107(1):67-74).

A further aspect of the invention provides a method of producing a peptide, the method comprising culturing a host cell and isolating the peptide from the host cell or its culture medium.

In another embodiment the peptide, the nucleic acid or the expression vector of the invention are used in medicine. For example, the peptide may be prepared for intravenous (i.v.) injection, sub-cutaneous (s.c.) injection, intradermal (i.d.) injection, intraperitoneal (i.p.) injection, intramuscular (i.m.) injection. Preferred methods of peptide injection include s.c., i.d., i.p., i.m., and i.v. Preferred methods of DNA injection include i.d., i.m., s.c., i.p. and i.v. Doses of e.g. between 50 $\mu$g and 1.5 mg, preferably 125 $\mu$g to 500 $\mu$g, of peptide or DNA may be given and will depend on the respective peptide or DNA. Dosages of this range were successfully used in previous trials (Walter et al Nature Medicine 18, 1254-1261 (2012)).

Another aspect of the present invention includes an *in vitro* method for producing activated T cells, the method comprising contacting *in vitro* T cells with antigen loaded human MHC molecules expressed on the surface of a suitable antigen-presenting cell for a period of time sufficient to activate the T cell in an antigen specific manner, wherein the antigen is a peptide according to the invention. Preferably a sufficient amount of the antigen is used with an antigen-presenting cell.

Preferably the mammalian cell lacks or has a reduced level or function of the TAP peptide transporter. Suitable cells that lack the TAP peptide transporter include T2, RMA-S and Drosophila cells. TAP is the transporter associated with antigen processing.

The human peptide loading deficient cell line T2 is available from the American Type Culture Collection, 12301 Parklawn Drive, Rockville, Maryland 20852, USA under Catalogue No CRL 1992; the Drosophila cell line Schneider line 2 is available from the ATCC under Catalogue No CRL 19863; the mouse RMA-S cell line is described in Karre et al 1985 (Ljunggren, H.-G., and K. Karre. 1985. J. Exp. Med. 162:1745).

Preferably, the host cell before transfection expresses substantially no MHC class I molecules. It is also preferred that the stimulator cell expresses a molecule important for providing a co-stimulatory signal for T-cells such as any of B7.1, B7.2, ICAM-1 and LFA 3. The nucleic acid sequences of numerous MHC class I molecules and of the co-stimulator molecules are publicly available from the GenBank and EMBL databases.

In case of a MHC class I epitope being used as an antigen, the T cells are CD8-positive CTLs.

If an antigen-presenting cell is transfected to express such an epitope, preferably the cell comprises an expression vector expressing a peptide containing SEQ ID NO: 167.

A number of other methods may be used for generating CTL *in vitro*. For example, autologous tumor-infiltrating lymphocytes can be used in the generation of CTL. Plebanski et al (1995) (Induction of peptide-specific primary cytotoxic T lymphocyte responses from human peripheral blood.Eur J Immunol. 1995 Jun;25(6):1783-7) make use of autologous peripheral blood lymphocytes (PLBs) in the preparation of CTL. Furthermore, the production of autologous CTL by pulsing dendritic cells with peptide or polypeptide, or via infection with recombinant virus is possible. Also, B cells can be used in the production of autologous CTL. In addition, macrophages pulsed with peptide or polypeptide, or infected with recombinant virus, may be used in the preparation of autologous CTL. S. Walter et al. 2003 (Cutting edge: prede-

termed avidity of human CD8 T cells expanded on calibrated MHC/anti-CD28-coated microspheres.J Immunol. 2003 Nov 15;171(10):4974-8) describe the *in vitro* priming of T cells by using artificial antigen presenting cells (aAPCs), which is also a suitable way for generating T cells against the peptide of choice. In the present invention, aAPCs were generated by the coupling of preformed MHC:peptide complexes to the surface of polystyrene particles (microbeads) by biotin:streptavidin biochemistry. This system permits the exact control of the MHC density on aAPCs, which allows to selectively elicit high- or low-avidity antigen-specific T cell responses with high efficiency from blood samples. Apart from MHC:peptide complexes, aAPCs should carry other proteins with co-stimulatory activity like anti-CD28 antibodies coupled to their surface. Furthermore such aAPC-based systems often require the addition of appropriate soluble factors, e. g. cytokines, like interleukin-12.

Allogeneic cells may also be used in the preparation of T cells and a method is described in detail in WO 97/26328. For example, in addition to Drosophila cells and T2 cells, other cells may be used to present antigens such as CHO cells, baculovirus-infected insect cells, bacteria, yeast, vaccinia-infected target cells. In addition plant viruses may be used (see, for example, Porta et al. (1994) Development of cowpea mosaic virus as a high-yielding system for the presentation of foreign peptides.Virology. 1994 Aug 1;202(2):949-55) which describes the development of cowpea mosaic virus as a high-yielding system for the presentation of foreign peptides.

The activated T cells that are directed against the peptides of the invention are useful in therapy. Thus, a further aspect of the invention provides activated T cells obtainable by the foregoing methods of the invention.

Activated T cells, which are produced by the above method, will selectively recognize a cell that aberrantly expresses a polypeptide that comprises an amino acid sequence of SEQ ID NO: 167.

Preferably, the T cell recognizes the cell by interacting through its TCR with the HLA/peptide-complex (for example, binding). The T cells are useful in a method of killing target cells in a patient whose target cells aberrantly express a polypeptide comprising an amino acid sequence of the invention wherein the patient is administered an effective number of the activated T cells. The T cells that are administered to the patient may be derived from the patient and activated as described above (i.e. they are autologous T cells). Alternatively, the T cells are not from the patient but are from another individual. Of course, it is preferred if the individual is a healthy individual. By "healthy individual" the inventors mean that the individual is generally in good health, preferably has a competent immune system and, more preferably, is not suffering from any disease that can be readily tested for, and detected.

*In vivo,* the target cells for the CD8-positive T cells according to the present invention can be cells of the tumor (which sometimes express MHC class II) and/or stromal cells surrounding the tumor (tumor cells) (which sometimes also express MHC class II; (Dengjel et al., 2006)).

The T cells of the present invention may be used as active ingredients of a therapeutic composition. Thus, the invention also provides a method of killing target cells in a patient whose target cells aberrantly express a polypeptide comprising an amino acid sequence of the invention, the method comprising administering to the patient an effective number of T cells as defined above.

By "aberrantly expressed" the inventors also mean that the polypeptide is over-expressed compared to normal levels of expression or that the gene is silent in the tissue from which the tumor is derived but in the tumor it is expressed. By "over-expressed" the inventors mean that the polypeptide is present at a level at least 1.2-fold of that present in normal tissue; preferably at least 2-fold, and more preferably at least 5-fold or 10-fold the level present in normal tissue.

T cells may be obtained by methods known in the art, e.g. those described above.

Protocols for this so-called adoptive transfer of T cells are well known in the art. Reviews can be found in: Gattinoni L, et al. Adoptive immunotherapy for cancer: building on success. Nat Rev Immunol. 2006 May;6(5):383-93. Review, and Morgan RA, et al. Cancer regression in patients after transfer of genetically engineered lymphocytes. Science. 2006 Oct 6;314(5796):126-9.

Any molecule of the invention, i.e. the peptide, nucleic acid, antibody, expression vector, cell, activated CTL, T-cell receptor or the nucleic acid encoding it is useful for the treatment of disorders, characterized by cells escaping an immune response.

Therefore any molecule of the present invention may be used as medicament or in the manufacture of a medicament. The molecule may be used by itself or combined with other molecule(s) of the invention or (a) known molecule(s).

Preferably, the medicament of the present invention is a vaccine. It may be administered directly into the patient, into the affected organ or systemically i.d., i.m., s.c., i.p. and i.v., or applied ex vivo to cells derived from the patient or a human cell line which are subsequently administered to the patient, or used *in vitro* to select a subpopulation of immune cells derived from the patient, which are then re-administered to the patient. If the nucleic acid is administered to cells *in vitro,* it may be useful for the cells to be transfected so as to co-express immune-stimulating cytokines, such as interleukin-2. The peptide may be substantially pure, or combined with an immune-stimulating adjuvant (see below) or used in combination with immune-stimulatory cytokines, or be administered with a suitable delivery system, for example liposomes. The peptide may also be conjugated to a suitable carrier such as keyhole limpet haemocyanin (KLH) or mannan (see WO 95/18145 and Longenecker, 1993). The peptide may also be tagged, may be a fusion protein, or may be a hybrid molecule. The peptides whose sequence is given in the present invention are expected to stimulate CD4 or CD8 T cells. However, stimulation of CD8 CTLs is more efficient in the presence of help provided by CD4 T-helper cells. Thus, for MHC Class I epitopes that stimulate CD8 CTL the fusion partner or sections of a hybrid molecule suitably provide epitopes which stimulate CD4-positive T cells. CD4- and CD8-stimulating epitopes are well known in the art and include those identified in the present invention.

In one aspect, the vaccine comprises at least one peptide having the amino acid sequence set forth SEQ ID No. 167, and at least one additional peptide, preferably two to 50, more preferably two to 25, even more preferably two to 20 and most preferably two, three, four, five, six, seven, eight, nine, ten, eleven, twelve, thirteen, fourteen, fifteen, sixteen, seventeen or eighteen peptides. The peptide(s) may be derived from one or more specific TAAs and may bind to MHC class I molecules.

In another aspect, the vaccine comprises at least one peptide having the amino acid sequence set forth in SEQ ID NO: 167, and at least one additional peptide, preferably two to 50, more preferably two to 25, even more preferably two to 20 and most preferably two, three, four, five, six, seven, eight, nine, ten, eleven, twelve, thirteen, fourteen, fifteen, sixteen, seventeen or eighteen peptides. The peptide(s) may be derived from one or more specific TAAs and may bind to MHC class I molecules.

The polynucleotide may be substantially pure, or contained in a suitable vector or delivery system. The nucleic acid may be DNA, cDNA, PNA, RNA or a combination thereof. Methods for designing and introducing such a nucleic acid are well known in the art. An overview is provided by e.g. (Pascolo et al., Human peripheral blood mononuclear cells transfected with messenger RNA stimulate antigen-specific cytotoxic T-lymphocytes in vitro.Cell Mol Life Sci. 2005 Aug;62(15):1755-62). Polynucleotide vaccines are easy to prepare, but the mode of action of these vectors in inducing an immune response is not fully understood. Suitable vectors and delivery systems include viral DNA and/or RNA, such as systems based on adenovirus, vaccinia virus, retroviruses, herpes virus, adeno-associated virus or hybrids containing elements of more than one virus. Non-viral delivery systems include cationic lipids and cationic polymers and are well known in the art of DNA delivery. Physical delivery, such as via a "gene-gun" may also be used. The peptide or peptides encoded by the nucleic acid may be a fusion protein, for example with an epitope that stimulates T cells for the respective opposite CDR as noted above.

The medicament of the invention may also include one or more adjuvants. Adjuvants are substances that non-specifically enhance or potentiate the immune response (e.g., immune responses mediated by CTLs and helper-T ($T_H$) cells to an antigen, and would thus be considered useful in the medicament of the present invention. Suitable adjuvants include, but are not limited to, 1018 ISS, aluminium salts, AMPLIVAX®, AS15, BCG, CP-870,893, CpG7909, CyaA, dSLIM, flagellin or TLR5 ligands derived from flagellin, FLT3 ligand, GM-CSF, IC30, IC31, Imiquimod (ALDARA®), resiquimod, ImuFact IMP321, Interleukins as IL-2, IL-13, IL-21, Interferon-alpha or -beta, or pegylated derivatives thereof, IS Patch, ISS, ISCOMATRIX, ISCOMs, JuvImmune®, LipoVac, MALP2, MF59, monophosphoryl lipid A, Montanide IMS 1312, Montanide ISA 206, Montanide ISA 50V, Montanide ISA-51, water-in-oil and oil-in-water emulsions, OK-432, OM-174, OM-197-MP-EC, ONTAK, OspA, PepTel® vector system, poly(lactid co-glycolid) [PLG]-based and dextran microparticles, talactoferrin SRL172, Virosomes and other Virus-like particles, YF-17D, VEGF trap, R848, beta-glucan, Pam3Cys, Aquila's QS21 stimulon, which is derived from saponin, mycobacterial extracts and synthetic bacterial cell wall mimics, and other proprietary adjuvants such as Ribi's Detox, Quil, or Superfos. Adjuvants such as Freund's or GM-CSF are preferred. Several immunological adjuvants (e.g., MF59) specific for dendritic cells and their preparation have been described previously (Allison and Krummel, 1995 The Yin and Yang of T cell costimulation.Science. 1995 Nov 10;270(5238):932-3. Review). Also cytokines may be used. Several cytokines have been directly linked to influencing dendritic cell migration to lymphoid tissues (e.g., TNF-), accelerating the maturation of dendritic cells into efficient antigen-presenting cells for T-lymphocytes (e.g., GM-CSF, IL-1 and IL-4) (U.S. Pat. No. 5,849,589) and acting as immunoadjuvants (e.g., IL-12, IL-15, IL-23, IL-7, IFN-alpha. IFN-beta) (Gabrilovich, 1996 Production of vascular endothelial growth factor by human tumors inhibits the functional maturation of dendritic cells Nat Med. 1996 Oct;2(10):1096-103).

CpG immunostimulatory oligonucleotides have also been reported to enhance the effects of adjuvants in a vaccine setting. Without being bound by theory, CpG oligonucleotides act by activating the innate (non-adaptive) immune system via Toll-like receptors (TLR), mainly TLR9. CpG triggered TLR9 activation enhances antigen-specific humoral and cellular responses to a wide variety of antigens, including peptide or protein antigens, live or killed viruses, dendritic cell vaccines, autologous cellular vaccines and polysaccharide conjugates in both prophylactic and therapeutic vaccines. More importantly it enhances dendritic cell maturation and differentiation, resulting in enhanced activation of $T_{H1}$ cells and strong cytotoxic T-lymphocyte (CTL) generation, even in the absence of CD4 T cell help. The $T_{H1}$ bias induced by TLR9 stimulation is maintained even in the presence of vaccine adjuvants such as alum or incomplete Freund's adjuvant (IFA) that normally promote a $T_{H2}$ bias. CpG oligonucleotides show even greater adjuvant activity when formulated or co-administered with other adjuvants or in formulations such as microparticles, nanoparticles, lipid emulsions or similar formulations, which are especially necessary for inducing a strong response when the antigen is relatively weak. They also accelerate the immune response and enable the antigen doses to be reduced by approximately two orders of magnitude, with comparable antibody responses to the full-dose vaccine without CpG in some experiments (Krieg, 2006).

US Pat. No. 6,406,705 B1 describes the combined use of CpG oligonucleotides, non-nucleic acid adjuvants and an antigen to induce an antigen-specific immune response. A CpG TLR9 antagonist is dSLIM (double Stem Loop Immunomodulator) by Mologen (Berlin, Germany) which is a preferred component of the pharmaceutical composition of the present invention. Other TLR binding molecules such as RNA binding TLR 7, TLR 8 and/or TLR 9 may also be used.

Other examples for useful adjuvants include, but are not limited to chemically modified CpGs (e.g. CpR, Idera), dsRNA analogues such as Poly(I:C) and derivates thereof (e.g. AmpliGen®, Hiltonol®, poly-(ICLC), poly(IC-R), poly(I:C12U), non-CpG bacterial DNA or RNA as well as immunoactive small molecules and antibodies such as cyclophosphamide, sunitinib, Bevacizumab®, celebrex, NCX-4016, sildenafil, tadalafil, vardenafil, sorafenib, temozolomide, temsirolimus, XL-999, CP-547632, pazopanib, VEGF Trap, ZD2171, AZD2171, anti-CTLA4, other antibodies targeting key structures of the immune system (e.g. anti-CD40, anti-TGFbeta, anti-TNFalpha receptor) and SC58175, which may act therapeutically and/or as an adjuvant. The amounts and concentrations of adjuvants and additives useful in the context of the present invention can readily be determined by the skilled artisan without undue experimentation.

Preferred adjuvants are imiquimod, resiquimod, GM-CSF, cyclophosphamide, sunitinib, bevacizumab, interferon-alpha, CpG oligonucleotides and derivates, poly-(I:C) and derivates, RNA, sildenafil, and particulate formulations with PLG or virosomes.

In a preferred embodiment, the pharmaceutical composition according to the invention the adjuvant is selected from the group consisting of colony-stimulating factors, such as Granulocyte Macrophage Colony Stimulating Factor (GM-CSF, sargramostim), cyclophosphamide, imiquimod, resiquimod, and interferon-alpha.

In a preferred embodiment, the pharmaceutical composition according to the invention the adjuvant is selected from the group consisting of colony-stimulating factors, such as Granulocyte Macrophage Colony Stimulating Factor (GM-CSF, sargramostim), cyclophosphamide, immiquimod and resiquimod.

In a preferred embodiment of the pharmaceutical composition according to the invention, the adjuvant is cyclophosphamide, imiquimod or resiquimod.

Even more preferred adjuvants are Montanide IMS 1312, Montanide ISA 206, Montanide ISA 50V, Montanide ISA-51, poly-ICLC (Hiltonol®) and anti-CD40 mAB or combinations thereof.

This composition is used for parenteral administration, such as subcutaneous, intradermal, intramuscular or oral administration. For this, the peptides and optionally other molecules are dissolved or suspended in a pharmaceutically acceptable, preferably aqueous carrier. In addition, the composition can contain excipients, such as buffers, binding agents, blasting agents, diluents, flavours, lubricants, etc. The peptides can also be administered together with immune stimulating substances, such as cytokines. An extensive listing of excipients that can be used in such a composition, can be, for example, taken from A. Kibbe, Handbook of Pharmaceutical Excipients, 3rd Ed., 2000, American Pharmaceutical Association and pharmaceutical press. The composition can be used for a prevention, prophylaxis and/or therapy of adenomateous or cancerous diseases. Exemplary formulations can be found in, for example, EP2112253.

Nevertheless depending on the number and the physico-chemical characteristics of the peptides of the invention further research is needed to provide formulations for specific combinations of peptides, especially combinations with more than 20 peptides that are stable for more than 12 to 18 months.

The present invention provides a medicament that useful in treating cancer, in particular AML, Chronic lymphoid leukemia (CLL) and other hematological malignancies.

The present invention further discloses a kit comprising:

> (a) a container containing a pharmaceutical composition as described above, in solution or in lyophilized form;
> (b) optionally a second container containing a diluent or reconstituting solution for the lyophilized formulation; and
> (c) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

Kits as disclosed preferably comprise a lyophilized formulation of the present invention in a suitable container and instructions for its reconstitution and/or use. Suitable containers include, for example, bottles, vials (e.g. dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

The container holding the formulation may be a multi-use vial, which allows for repeat administrations (e.g., from 2-6 administrations) of the reconstituted formulation. The kit may further comprise a second container comprising a suitable diluent (e.g., sodium bicarbonate solution).

Upon mixing of the diluent and the lyophilized formulation, the final peptide concentration in the reconstituted formulation is preferably at least 0.15 mg/mL/peptide (=75 μg) and preferably not more than 3 mg/mL/peptide (=1500 μg). The kit may further include other materials desirable from a commercial and user standpoint, including other buffers, diluents, filters, needles, syringes, and package inserts with instructions for use.

Kits as disclosed may have a single container that contains the formulation of the pharmaceutical compositions according to the present invention with or without other components (e.g., other compounds or pharmaceutical compositions of these other compounds) or may have distinct container for each component.

Preferably, kits as disclosed include a formulation of the invention packaged for use in combination with the co-administration of a second compound (such as adjuvants (e.g. GM-CSF), a chemotherapeutic agent, a natural product, a hormone or antagonist, an anti-angiogenesis agent or inhibitor, a apoptosis-inducing agent or a chelator) or a pharmaceutical composition thereof. The components of the kit may be pre-complexed or each component may be in a separate distinct container prior to administration to a patient. The components of the kit may be provided in one or more liquid solutions, preferably, an aqueous solution, more preferably, a sterile aqueous solution. The components of the kit may also be provided as solids, which may be converted into liquids by addition of suitable solvents, which are preferably provided in another distinct container.

The container of a therapeutic kit may be a vial, test tube, flask, bottle, syringe, or any other means of enclosing a solid or liquid. Usually, when there is more than one component, the kit will contain a second vial or other container, which allows for separate dosing. The kit may also contain another container for a pharmaceutically acceptable liquid. Preferably, a therapeutic kit will contain an apparatus (e.g., one or more needles, syringes, eye droppers, pipette, etc.), which enables administration of the agents of the invention that are components of the present kit.

The present formulation is one that is suitable for administration of the peptides by any acceptable route such as oral (enteral), nasal, ophthal, subcutaneous, intradermal, intramuscular, intravenous or transdermal. Preferably the administration is s.c., and most preferably i.d. Administration may be by infusion pump.

Since the peptides of the invention were isolated from CLL, the medicament of the invention is preferably used to treat CLL.

The present invention further discloses a method for producing a personalized pharmaceutical for an individual patient comprising manufacturing a pharmaceutical composition comprising at least one peptide selected from a warehouse of pre-screened TUMAPs, wherein the at least one peptide used in the pharmaceutical composition is selected for suitability in the individual patient. Preferably, the pharmaceutical composition is a vaccine. The method could also be adapted to produce T cell clones for down-stream applications such as TCR isolations.

A "personalized pharmaceutical" shall mean specifically tailored therapies for one individual patient that will only be used for therapy in such individual patient, including actively personalized cancer vaccines and adoptive cellular therapies using autologous patient tissue.

As used herein, the term "warehouse" shall refer to a group of peptides that have been pre-screened for immunogenicity and over-presentation in a particular tumour type. The term "warehouse" is not intended to imply that the particular peptides included in the vaccine have been pre-manufactured and stored in a physical facility, although that possibility is contemplated. It is expressly contemplated that the peptides may be manufactured *de novo* for each individualized vaccine produced, or may be pre-manufactured and stored.

The warehouse (e.g. in the form of a database) is composed of tumour-associated peptides which were highly overexpressed in the tumour tissue of several HLA-A, HLA-B and HLA-C positive CLL patients analyzed (see tables above). It contains MHC class I and MHC class II peptides. In addition to the tumor associated peptides collected from several GBM tissues, the warehouse may contain an HLA-A*02 and an HLA-A*24 marker peptide. These peptides allow comparison of the magnitude of T-cell immunity induced by TUMAPS in a quantitative manner and hence allow important conclusion to be drawn on the capacity of the vaccine to elicit anti-tumor responses. Secondly, it functions as an important positive control peptide derived from a "non-self' antigen in the case that any vaccine-induced T-cell responses to TUMAPs derived from "self antigens in a patient are not observed. And thirdly, it may allow conclusions to be drawn, regarding the status of immunocompetence of the patient population.

HLA class I and II TUMAPs for the warehouse are identified by using an integrated functional genomics approach combining gene expression analysis, mass spectrometry, and T-cell immunology. The approach assures that only TUMAPs truly present on a high percentage of tumours but not or only minimally expressed on normal tissue, are chosen for further analysis. For peptide selection, CLL samples from patients and blood from healthy donors were analyzed in a stepwise approach:

1. HLA ligands from the malignant material were identified by mass spectrometry
2. Genome-wide messenger ribonucleic acid (mRNA) expression analysis by microarrays was used to identify genes over-expressed in the malignant tissue (CLL) compared with a range of normal organs and tissues
3. Identified HLA ligands were compared to gene expression data. Peptides encoded by selectively expressed or over-expressed genes as detected in step 2 were considered suitable TUMAP candidates for a multi-peptide vaccine.
4. Literature research was performed in order to identify additional evidence supporting the relevance of the identified peptides as TUMAPs

5. The relevance of over-expression at the mRNA level was confirmed by redetection of selected TUMAPs from step 3 on tumor tissue and lack of (or infrequent) detection on healthy tissues

6. To assess whether an induction of in vivo T-cell responses by the selected peptides may be feasible, in vitro immunogenicity assays were performed using human T cells from healthy donors as well as from CLL patients.

In an aspect, the peptides are pre-screened for immunogenicity before being included in the warehouse. By way of example, and not limitation, the immunogenicity of the peptides included in the warehouse is determined by a method comprising in vitro T-cell priming through repeated stimulations of CD8+ T cells from healthy donors with artificial antigen presenting cells loaded with peptide/MHC complexes and anti-CD28 antibody.

This method is preferred for rare cancers and patients with a rare expression profile. In contrast to multi-peptide cocktails with a fixed composition as currently developed the warehouse allows a significantly higher matching of the actual expression of antigens in the tumour with the vaccine. Selected single or combinations of several "off-the-shelf" peptides will be used for each patient in a multitarget approach. In theory an approach based on selection of e.g. 5 different antigenic peptides from a library of 50 would already lead to approximately 17 million possible drug product (DP) compositions.

In an aspect, the peptides are selected for inclusion in the vaccine based on their suitability for the individual patient based on the method as disclosed, and as follows.

The HLA phenotype, transcriptomic and peptidomic data will be gathered from the patient's tumour material and blood samples to identify the most suitable peptides for each patient containing warehouse and patient-unique (ie. mutated) TUMAPs. Those peptides will be chosen, which are selectively or over-expressed in the patients tumor and, where possible, showed strong in vitro immunogenicity if tested with the patients individual PBMCs.

Preferably, the peptides included in the vaccine are identified by a method comprising: (a) identifying tumour-associated peptides (TUMAPs) presented by a tumor sample from the individual patient; (b) comparing the peptides identified in (a) with a warehouse (database) of peptides as described above; and (c) selecting at least one peptide from the warehouse (database) that correlates with a tumour-associated peptide identified in the patient. For example, the TUMAPs presented by the tumor sample are identified by: (a1) comparing expression data from the tumor sample to expression data from a sample of normal tissue corresponding to the tissue type of the tumor sample to identify proteins that are over-expressed or aberrantly expressed in the tumor sample; and (a2) correlating the expression data with sequences of MHC ligands bound to MHC class I and/or class II molecules in the tumor sample to identify MHC ligands derived from proteins over-expressed or aberrantly expressed by the tumor. Preferably, the sequences of MHC ligands are identified by eluting bound peptides from MHC molecules isolated from the tumor sample, and sequencing the eluted ligands. Preferably, the tumor sample and the normal tissue are obtained from the same patient.

In addition to, or as an alternative to, selecting peptides using a warehousing (database) model, TUMAPs may be identified in the patient *de novo* and then included in the vaccine. As one example, candidate TUMAPs may be identified in the patient by (a1) comparing expression data from the tumor sample to expression data from a sample of normal tissue corresponding to the tissue type of the tumor sample to identify proteins that are over-expressed or aberrantly expressed in the tumor sample; and (a2) correlating the expression data with sequences of MHC ligands bound to MHC class I and/or class II molecules in the tumor sample to identify MHC ligands derived from proteins over-expressed or aberrantly expressed by the tumor. As another example, proteins may be identified containing mutations that are unique to the tumor sample relative to normal corresponding tissue from the individual patient, and TUMAPs can be identified that specifically target the mutation. For example, the genome of the tumour and of corresponding normal tissue can be sequenced by whole genome sequencing: For discovery of non-synonymous mutations in the protein-coding regions of genes, genomic DNA and RNA are extracted from tumour tissues and normal non-mutated genomic germline DNA is extracted from peripheral blood mononuclear cells (PBMCs). The applied NGS approach is confined to the re-sequencing of protein coding regions (exome re-sequencing). For this purpose, exonic DNA from human samples is captured using vendor-supplied target enrichment kits, followed by sequencing with e.g. a HiSeq2000 (Illumina). Additionally, tumour mRNA is sequenced for direct quantification of gene expression and validation that mutated genes are expressed in the patients' tumours. The resultant millions of sequence reads are processed through software algorithms. The output list contains mutations and gene expression. Tumour-specific somatic mutations are determined by comparison with the PBMC-derived germline variations and prioritized.. The *de novo* identified peptides may then be tested for immunogenicity as described above for the warehouse, and candidate TUMAPs possessing suitable immunogenicity are selected for inclusion in the vaccine.

In one exemplary embodiment, the peptides included in the vaccine are identified by: (a) identifying tumour-associated peptides (TUMAPs) presented by a tumor sample from the individual patient by the methdos described above; (b) comparing the peptides identified in a) with a warehouse of peptides that have been prescreened for immunogenicity and overpresentation in tumors as compared to corresponding normal tissue; (c) selecting at least one peptide from the warehouse that correlates with a tumour-associated peptide identified in the patient; and (d) optionally, selecting at least one peptide identified *de novo* in (a) confirming its immunogenicity.

In one exemplary embodiment, the peptides included in the vaccine are identified by: (a) identifying tumour-associated peptides (TUMAPs) presented by a tumor sample from the individual patient; and (b) selecting at least one peptide identified *de novo* in (a) and confirming its immunogenicity.

Once the peptides are selected, the vaccine is manufactured.

The vaccine preferably is a liquid formulation consisting of the individual peptides dissolved in 33% DMSO. Each peptide to be included into a product is dissolved in DMSO. The concentration of the single peptide solutions has to be chosen depending on the number of peptides to be included into the product. The single peptide-DMSO solutions are mixed in equal parts to achieve a solution containing all peptides to be included in the product with a concentration of ~2.5 mg/ml per peptide. The mixed solution is then diluted 1:3 with water for injection to achieve a concentration of 0.826 mg/ml per peptide in 33% DMSO. The diluted solution is filtered through a 0.22 μm sterile filter. The final bulk solution is obtained.

Final bulk solution is filled into vials and stored at -20°C until use. One vial contains 700 μL solution containing 0.578 mg of each peptide. Of this, 500 μL (approx. 400 μg per peptide) will be applied for intradermal injection.

The present invention will now be described in the following examples that describe preferred embodiments thereof, nevertheless, without being limited thereto.

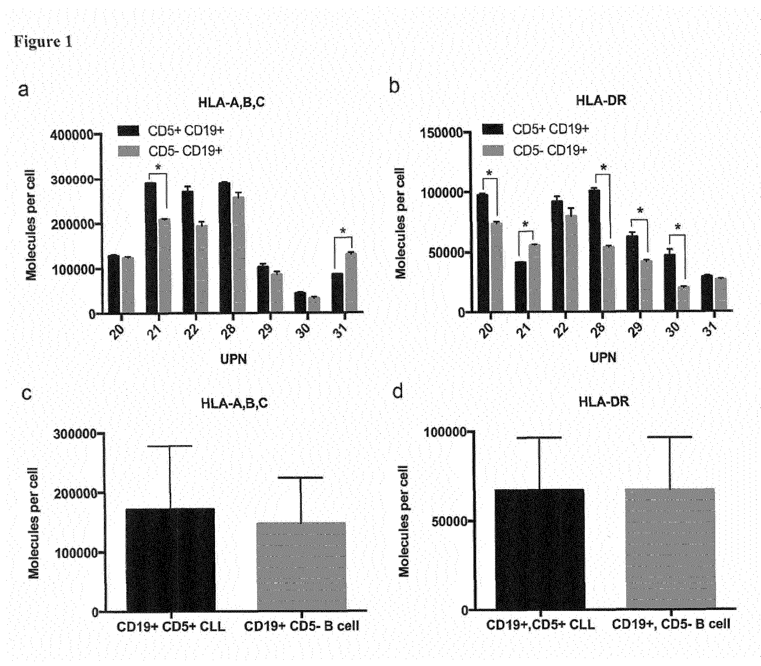

**Figure 1** shows the HLA surface expression of primary CLL samples. (a) HLA class I and (b) HLA class II expression of CD5$^+$CD19$^+$ CLL cells compared to autologous CD5$^-$CD19$^+$ B cells in 7 primary CLL samples. Data are expressed as mean $\pm$ s.d. of triplicate experiments. (c) Mean HLA class I and (d) HLA class II expression CD5$^+$CD19$^+$ CLL cells compared to autologous CD5$^-$CD19$^+$ B cells (n=7). * P<0.01 Abbreviations: UPN, uniform patient number

**Figure 2** shows the identification of a novel category of tumor-associated antigens by HLA ligandome profiling. (a) Overlap of HLA class I ligand source proteins of primary CLL samples (n=30) and HV PBMC (n=30). (b) Comparative profiling of HLA class I ligand source proteins based on the frequency of HLA restricted representation in CLL and HV PBMC ligandomes. Frequencies [%] of CLL patients/HVs positive for HLA restricted presentation of the respective source protein (x-axis) are indicated on the y-axis. The box on the left-hand side highlights the subset of source proteins showing CLL-exclusive representation in >20% of patients (LiTAAs: ligandome-derived tumor-associated antigens). (c) Representation of published CLL-associated antigens in HLA class I ligandomes. Bars indicate relative representation [%] of respective antigens by HLA class I ligands in CLL and HV PBMC. Dashed lines separate the antigens into three groups according to their degree of CLL-association. (d) Source protein overlaps of CLL samples from different stages of disease (Binet A (n=9), Binet B (n=7), Binet C (n=14)). (e) Heatmap analysis of the representation frequencies [%] of LiTAAs across different disease stages (Binet A-C, as in (d)) (f) Heatmap analysis of LiTAA representation [%] on primary CLL samples with del17p (n=5) and without del17p (n=25). Abbreviations: CLL, chronic lymphocytic leukemia; HV, healthy volunteer

**Figure 3** shows that LiTAAs are specifically recognized by CLL patient immune responses. (a) HLA class I LiTAAs and corresponding LiTAPs (3 HLA-A*03, 5 HLA-A*02, 5 HLA-B*07) functionally evaluated in IFNy ELISPOT assays. Absolute numbers and frequencies of peptide-specific immune recognition by CLL patient PBMC are summarized in the right hand column. (b) Example of A*03 LiTAPs evaluated in ELISPOT using HV PBMC as a control. An EBV epitope mix containing 4 frequently recognized peptides (....) was used as positive control, HIV GAG$_{18-26}$ A*03 peptide served as negative control. (c) Example of ELISPOT assays using HLA-A*03 LiTAPs (n=3) on PBMC of 3 different CLL patients. Results are shown for immunoreactive LiTAPs. EBV epitope mix served as positive control, HIV GAG$_{18-26}$ A*03 peptide as negative control. (d) Example of HLA-A*03 benign tissue-derived LiBAPs (n=3) tested on CLL patient PBMC as internal control for the target selection strategy. EBV epitope mix served as positive control, HIV GAG$_{18-26}$ A*03 peptide as negative control. (e) Scatterplot of the allele-adjusted frequencies of LiTAP presentation in CLL ligandomes (as detected by MS) and the corresponding allele-adjusted frequencies of immune recognition by CLL patient PBMC in IFNy ELISPOT. Data points are shown for the 14/15 LiTAPs showing immune recognition. Abbreviations: LiTAP, ligandome-derived tumor-associated peptide; HV, healthy volunteer; neg., negative; pos., positive; UPN, uniform patient number; LiBAP, ligandome-derived benign tissue-associated peptide; MS, mass spectrometry .

**Figure 4** shows the identification of additional/synergistic HLA class II LiTAAs and LiTAPs. (a) Overlap of HLA class II ligand source proteins of primary CLL samples (n=20) and HV PBMC (n=13). (b) Comparative profiling of HLA class II ligand source proteins based on the frequency of HLA restricted representation in CLL and HV PBMC ligandomes. Frequencies [%] of CLL patients/HVs positive for HLA restricted presentation of the respective source protein (x-axis) are indicated on the y-axis. The box on the left-hand side highlights the subset of source proteins showing CLL-exclusive representation in >20% of patients (LiTAAs: ligandome-derived tumor-associated antigens).

(c) HLA class II LiTAAs and corresponding LiTAPs (n=6) functionally evaluated in IFN$\gamma$ ELISPOT assays. Absolute numbers and frequencies of peptide-specific immune recognition by CLL patient PBMC are summarized in the right hand column. (d) Example of HLA class II LiTAPs evaluated in ELISPOT using HV PBMC as a control. PHA was used as positive control. FLNA$_{1669-1683}$ HLA-DR peptide served as negative control. (e) Example of ELISPOT assays using HLA class II LiTAPs (n=6) on PBMC of 3 different CLL patients. Results are shown for immunoreactive LiTAPs. PHA was used as positive control, FLNA$_{1669-1683}$ HLA-DR peptide served as negative control. (f) Overlap analysis of CLL-exclusive HLA class I and HLA class II ligand source proteins for shared/synergistic vaccine targets. (g) Heatmap analysis of the 132 shared HLA class I/II LiTAAs (identified in (d)). The two source proteins showing representation in ≥20% of both, HLA class I and II CLL patient ligandomes are specified.

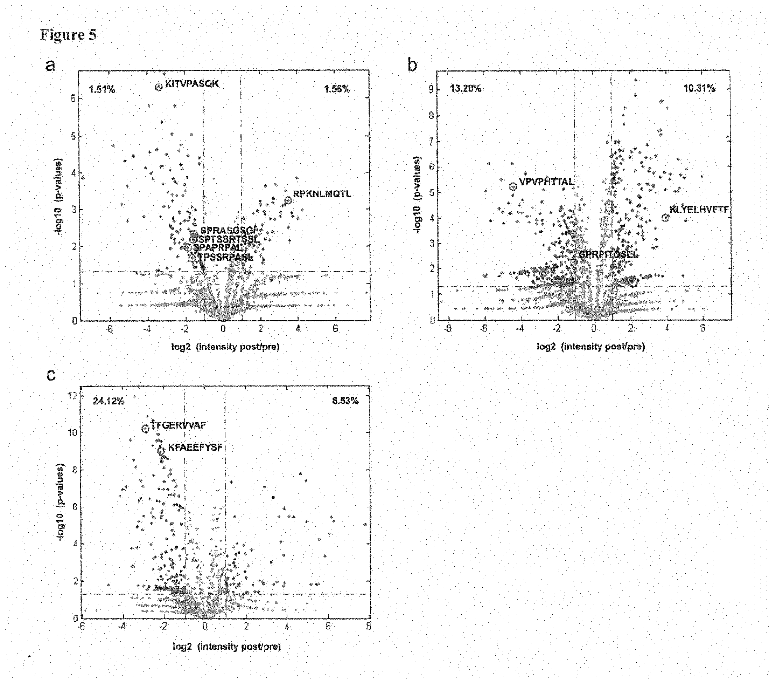

**Figure 5** shows the longitudinal HLA class I ligandome analysis of CLL patients undergoing chemo-/immunotherapy. Volcano-Plots of the relative abundances of ligands in the HLA class I ligandomes of patients after treatment compared to their respective abundance prior to therapy (ratio post therapy/pre therapy). Dashed lines indicate the thresholds for significant changes in abundance (>2-fold ratio, p<0.05), with significantly up-regulated ligands in the upper-right and significantly downregulated ligands in the upper-left. Frequencies of significantly regulated ligands are specified in the respective quadrants. LiTAPs showing significant regulation over the course of therapy are marked in red and their sequences are specified. (a) Analysis of a CLL patient ligandome prior to therapy and 48h/24h after treatment with rituximab/bendamustin (375mg/m$^2$ / 90mg/m$^2$). 1/28 (3.6%) of detectable LiTAPs showed significant changes in abundance. (b) Analysis of a CLL patient ligandome prior to therapy and after the first 7 days of treatment with alemtuzumab (3 doses of alemtuzumab, 10 mg, 20 mg and 30mg on day 1, 3 and 5; ligandome analysis on day 7). 3/24 (12.5%) of detectable LiTAPs showed significant changes in abundance. (c) Analysis of a CLL patient ligandome prior to therapy and 24h after treatment with 300 mg ofatumumab. 2/10 (20.0%) of detectable LiTAPs showed significant changes in abundance.

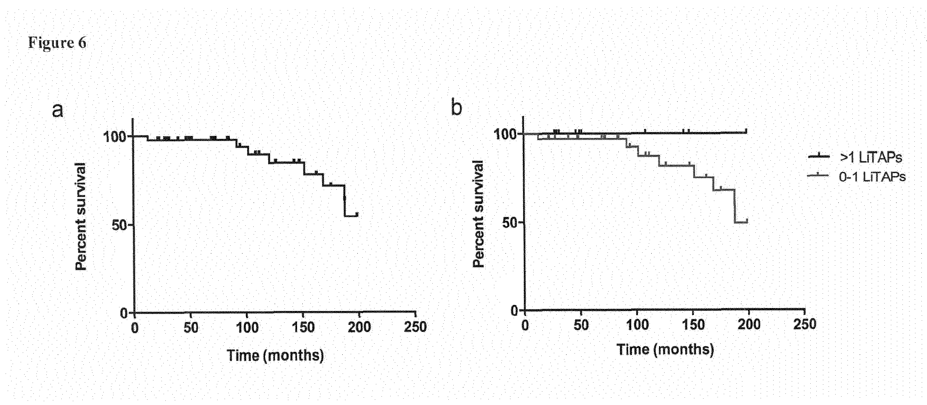

**Figure 6** shows the retrospective survival analysis of CLL patients with respect to their immune recognition of LiTAPs. (a) Kaplan Meier plot of the overall survival of 44 CLL patients. (b) Overall survival of subjects evaluated for LiTAP-specific immune responses grouped as follows: black, CLL patients showing immune responses to >1 LiTAPs (n=10). Red, CLL patients showing immune reactions to 0-1 LiTAPs (n=34).

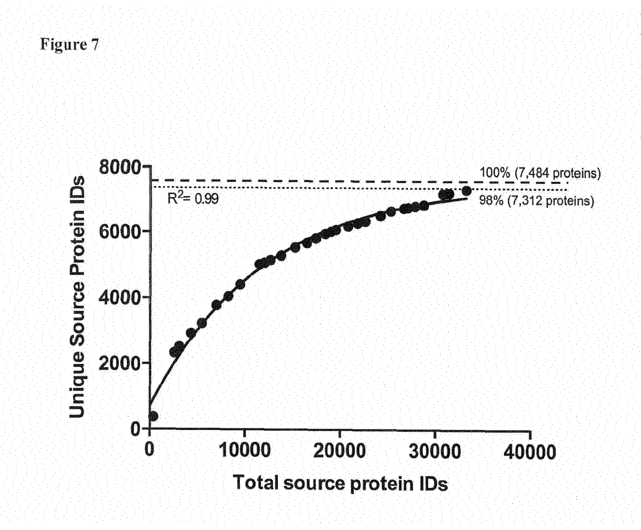

**Figure 7** shows the saturation analysis of HLA class I ligand source protein identifications in CLL patients. Number of unique HLA ligand source protein identifications as a function of total HLA ligand source protein identifications in 30 CLL patients. Exponential regression allowed for the robust calculation (R$^2$=0.9912) of the maximum attainable number of different source protein identifications (dashed line). The dotted line depicts the source proteome coverage achieved in our CLL patient cohort.

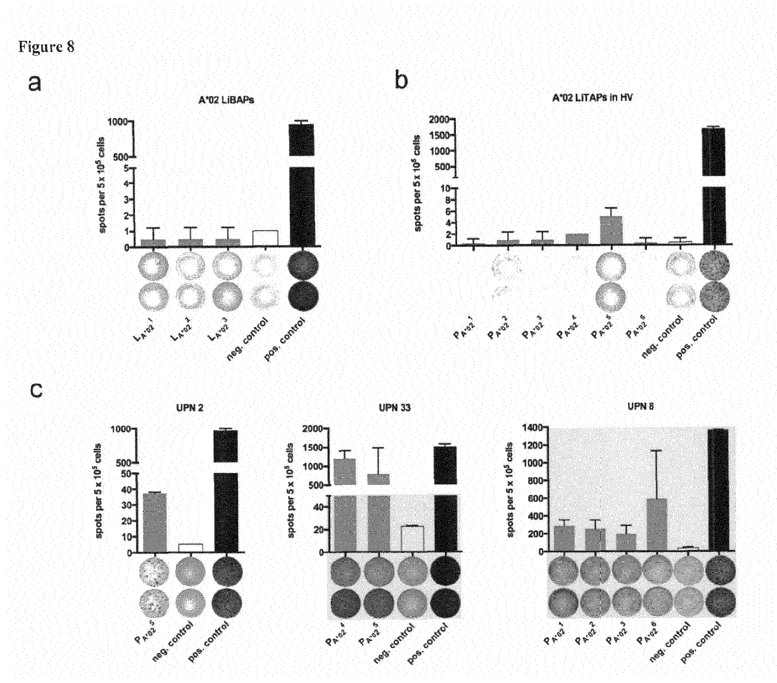

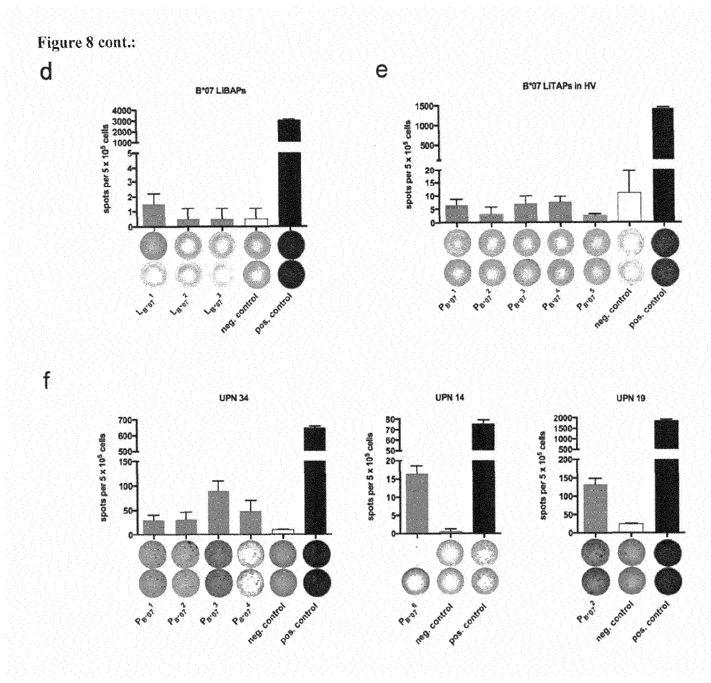

**Figure 8** shows that HLA-A*02 and B*07 LiTAPs are specifically recognized by CLL patient immune responses. (a) Example of HLA-A*02 (n=3) and (d) HLA-B*07 (n=3) benign tissue-derived LiBAPs tested on CLL patient PBMC as internal control for the target selection strategy. EBV epitope mix served as positive control, HIV XX$_{xx-xx}$ A*02 and HIV XX$_{xx-xx}$ HLA-B*07 peptide served as negative control, respectively. (b) Example of HLA-A*02 (n=6) and (e) HLA-B*07 (n=5) LiTAPs evaluated in ELISPOT assays using HV PBMC as a control. Positive and negative controls as described in (a). (c) Example of ELISPOT assays using HLA-A*02 (n=6) and (f) HLA-B*07 (n=5) LiTAPs on PBMC of 3 different HLA-matched CLL patients, each. Results are shown for immunoreactive LiTAPs. Positive and negative controls as described in (a). Abbreviations: LiBAP, ligandome-derived benign tissue-associated peptide; LiTAP, ligandome-derived tumor-associated peptide; HV, healthy volunteer; neg., negative; pos., positive; UPN, uniform patient number.

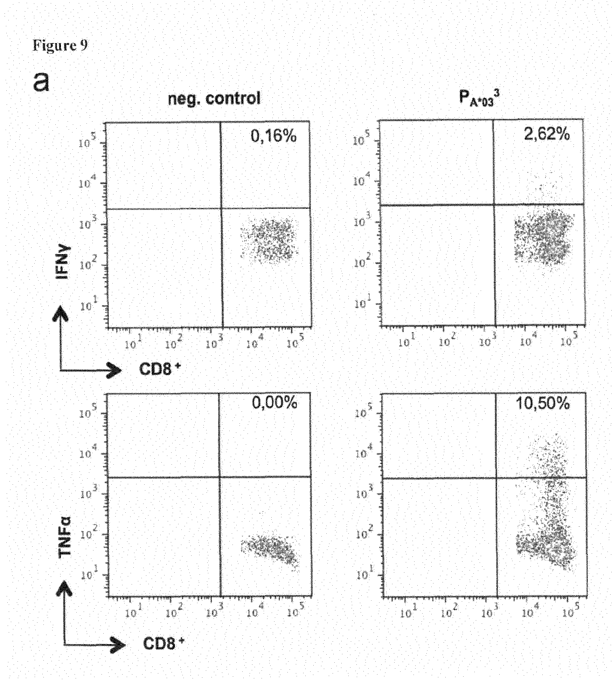

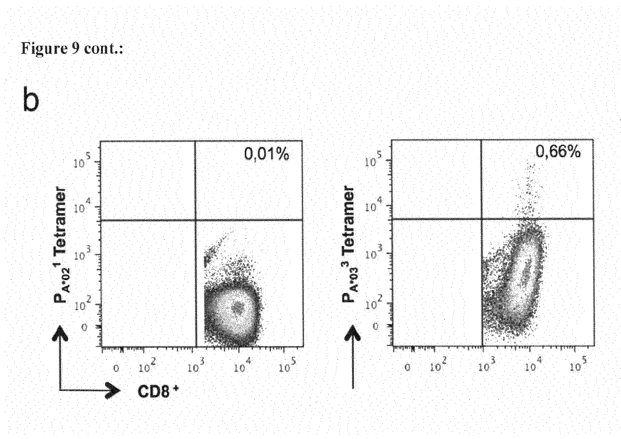

**Figure 9** shows the intracellular cytokine and tetramer staining of HLA-A*03 LiTAP specific CLL patient T cells. (a) Intracellular staining for IFNy and TNFa of P$_{A*03}^3$ (DMXL1$_{1271-1279}$ SSSGLHPPK (SEQ ID NO: 77) stimulated CLL patient PBMC. PMA/ionomycin served as positive control, HIV GAG$_{18-26}$ A*03 peptide as negative control. (b) Tetramer staining of CLL patient CD8$^+$ T cells with P$_{A*03}^3$ (DMXL1$_{1271-1279}$ SSSGLHPPK (SEQ ID NO: 77)) tetramers. As control, tetramer staining with the non-recognized P$_{A*02}^1$ (ABCA6$_{1270-1278}$ ILDEKPVII (SEQ ID NO: 63) in the same patient is shown.

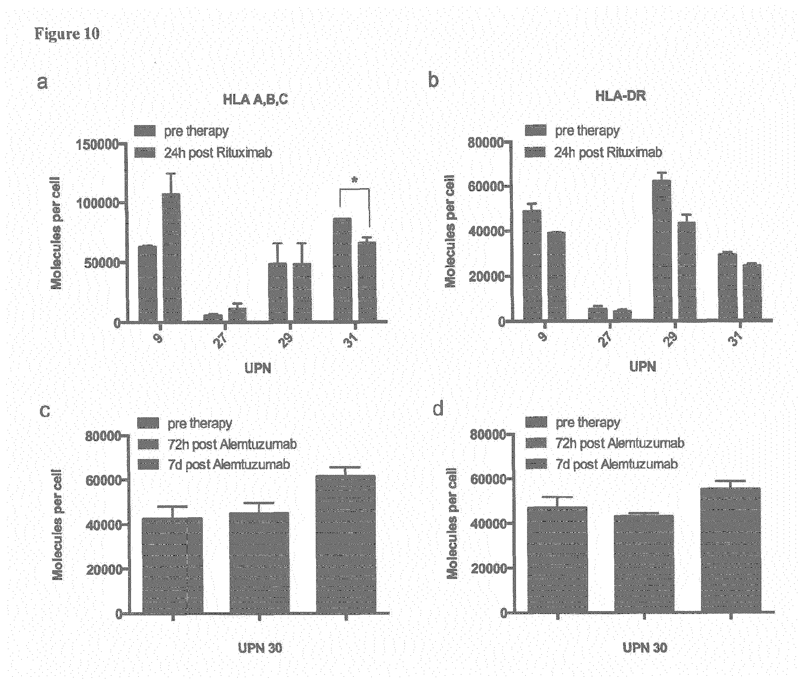

**Figure 10** shows the quantification of HLA surface expression on primary CLL cells from patients undergoing chemo-/immunotherapy. HLA surface expression on CD5$^+$CD19$^+$ CLL cells was quantified by flow cytometry, before and after therapy. Data are expressed as mean ± s.d. of triplicate experiments. (a) HLA class I and (b) HLA class II surface expression on primary CLL cells of 4 patients prior to therapy and 24h after treatment with rituximab. (c) HLA class I and (d) HLA class II surface expression on primary CLL cells of a patient prior to therapy, 72h (10mg) and 7d (60mg) after treatment with alemtuzumab. *P<0.01 Abbreviations: UPN, uniform patient number; h, hour; d, day.

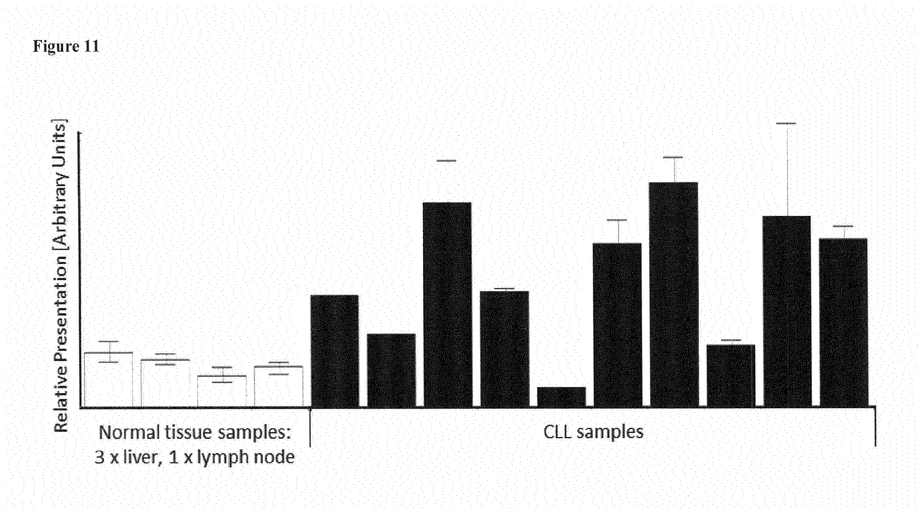

**Figure 11** shows the over-presentation of peptide ILDEKPVII in normal tissues as compared to CLL samples. Shown are only samples on which the peptide was detected. The test panel included 12 CLL samples and the following normal samples: 1 x adipose tissue, 3 x adrenal gland, 6 x artery, 5 x bone marrow, 7 x brain, 3 x breast, 5 x nerve, 13 x colon, 7 x esophagus, 2 x gallbladder, 5 x heart, 12 x kidney, 20 x liver, 44 x lung, 3 x lymph node, 4 x peripheral blood mononuclear cells, 2 x ovary, 6 x pancreas, 1 x peritoneum, 3 x pituitary, 2 x placenta, 3 x pleura, 3 x prostate, 6 x rectum, 7 x salivary gland, 4 x skeletal muscle, 5 x skin, 3 x small intestine, 4 x spleen, 5 x stomach, 4 x testis, 3 x thymus, 3 x thyroid gland, 3 x trachea, 2 x ureter, 5 x urinary bladder, 2 x uterus, 2 x vein.

EXAMPLES

EXAMPLE 1:

**Identification and quantitation of tumor associated peptides presented on the cell surface**

**Tissue samples**

Patients' tumor samples were provided by University of Tübingen, Tübingen, Germany. Written informed consents of all patients had been given. For ligandome analysis, PBMC from CLL patients (>80% CLL cell frequency) as well as PBMC from healthy volunteers (HVs) were isolated by density gradient centrifugation. Informed consent was obtained in accordance with the Helsinki protocol. This study was performed according to the guidelines of the local ethics committee. HLA typing was carried out by the Department of Hematology and Oncology, Tübingen. Samples were stored at -80°C until further use.

**Quantification of HLA surface expression**

For comparison with healthy autologous B lymphocytes, quantification of HLA surface expression was performed in patient samples containing at least 0.5% CD5$^-$ CD19$^+$ normal B cells. HLA surface expression was analyzed using the QIFIKIT® quantitative flow cytometric assay (Dako) according to the manufacturer's instructions. In brief, triplicates of each sample were stained with the pan-HLA class I specific monoclonal antibody (mAb) W6/32, HLA-DR specific mAb L243 (both produced in house) or IgG isotype control (BioLegend), respectively. Surface marker staining was carried out with directly labeled CD3 (BD), CD5 (BD) and CD19 (BD) antibodies. 7-AAD (BioLegend) was added as viability marker immediately prior to flow cytometric analysis on a FACSCanto Analyzer (BD).

**Isolation of HLA peptides from tissue samples**

HLA class I and II molecules were isolated employing standard immunoaffinity purification as described previously. In brief, snap-frozen cell pellets were lysed in 10 mM CHAPS/PBS (AppliChem, St. Louis, MO, USA/Gibco, Carlsbad, CA, USA) containing 1x protease inhibitor (Complete, Roche, Basel, Switzerland). HLA molecules were single-step purified using the pan-HLA class I specific mAb W6/32 and the pan-HLA class II specific mAb Tü39 respectively, covalently linked to CNBr-activated sepharose (GE Healthcare, Chalfont St Giles, UK). HLA:peptide complexes were eluted by repeated addition of 0.2% trifluoroacetic acid (TFA, Merck, Whitehouse Station, NJ, USA). Elution fractions E1-E8 were pooled and free HLA ligands were isolated by ultrafiltration using centrifugal filter units (Amicon, Millipore, Billerica, MA, USA). HLA ligands were extracted and desalted from the filtrate using ZipTip C18 pipette tips (Millipore). Extracted peptides were eluted in 35 μl of 80% acetonitrile (ACN, Merck)/0.2% TFA, centrifuged to complete dryness and resuspended in 25 μl of 1% ACN/0.05% TFA. Samples were stored at -20 °C until analysis by LC-MS/MS.

**Analysis of HLA ligands by LC-MS/MS**

Peptide samples were separated by reversed-phase liquid chromatography (nanoUHPLC, UltiMate 3000 RSLCnano,ThermoFisher, Waltham, MA, USA) and subsequently analyzed in an on-line coupled LTQ Orbitrap XL hybrid mass spectrometer (ThermoFisher). Samples were analyzed in 5 technical replicates. Sample volumes of 5 μl (sample shares of 20%) were injected onto a 75 μm x 2 cm trapping column (Acclaim PepMap RSLC, ThermoFisher) at 4 μl/min for 5.75 min. Peptide separation was subsequently performed at 50°C and a flow rate of 175 nl/min on a 50 μm x 50 cm separation column (Acclaim PepMap RSLC, ThermoFisher) applying a gradient ranging from 2.4-32.0% of ACN over the course of 140 min. Eluting peptides were ionized by nanospray ionization and analyzed in the mass spectrometer implementing a top 5 CID (collision induced dissociation) method generating fragment spectra for the 5 most abundant precursor ions in the survey scans. Resolution was set to 60,000. For HLA class I ligands, the mass range was limited to 400-650 m/z with charge states 2 and 3 permitted for fragmentation. For HLA class II, a mass range of 300-1,500 m/z was analyzed with charge states $\geq$2 allowed for fragmentation.

## Database Search and Spectral Annotation

For data processing, the software Proteome Discoverer (v1.3, ThermoFisher) was used to integrate the search results of the Mascot search engine (Mascot 2.2.04, Matrix Science) against the human proteome as comprised in the Swiss-Prot database (www.uniprot.org, release: September 27th 2013; 20,279 reviewed protein sequences contained). The search combined data of technical replicates and was not restricted by enzymatic specificity. Precursor mass tolerance was set to 5 ppm, fragment mass tolerance to 0.5 Da. Oxidized methionine was allowed as a dynamic modification. False discovery rates (FDR) were determined by the Percolator algorithm based on processing against a decoy database consisting of the shuffled target database. FDR was set at a target value of q$\leq$0.05 (5% FDR). Peptide-spectrum matches (PSMs) with q$\leq$0.05 were filtered according to additional, orthogonal parameters, to ensure spectral quality and validity. Mascot scores were filtered to $\geq$20. For HLA class I, peptide lengths were limited to 8-12 amino acids (aa) of length. For HLA class II, peptides were limited to 12-25 aa length. Protein grouping was disabled, allowing for multiple annotations of peptides (e.g. conserved sequences mapping into multiple proteins). For quality control, yield thresholds of $\geq$300 unique HLA class I ligands and $\geq$100 unique HLA class II ligands per sample were applied. HLA annotation was performed using SYFPEITHI (www.syfpeithi.de) or an extended in-house database.

## Longitudinal analysis of CLL patient ligandomes over the course of therapy

For label-free quantification (LFQ) of the relative HLA ligand abundances over the course of therapy, the injected peptide amounts of paired samples were normalized and LC-MS/MS analysis was performed in 5 technical replicates for each sample.

In brief, relative amounts of substance of paired samples were calculated from average precursor ion intensities determined in dose-finding mass spectrometry runs and adjusted accordingly by dilution. Relative quantification of HLA ligands was performed by calculating the area under the curve of the corresponding precursor extracted ion chromatograms (XIC) using Proteome Discoverer 1.3. The ratios of the mean areas of the individual peptides in the 5 LFQ-MS runs of each sample were calculated and two-tailed *t*-tests were performed using an in-house Matlab script (v8.2, Mathworks).

## Peptide synthesis

The automated peptide synthesizer EPS221 (Abimed) was used to synthesize peptides using the 9-fluorenylmethyl-oxycarbonyl/tert-butyl (Fmoc/tBu) strategy as described. Synthetic peptides were used for validation of LC-MS/MS identifications as well as for functional experiments.

## Amplification of peptide-specific T cells

PBMC from CLL patients and healthy volunteers were cultured in RPMI1640 medium (Gibco) supplemented with 10% pooled human serum (PHS, produced in-house), 100 mM $\beta$-mercaptoethanol (Roth, Karlsruhe, Germany) and 1% penicillin/streptomycin (GE). For CD8$^+$ T cell stimulation, PBMC were thawed and pulsed with 1 $\mu$g/ml per peptide. Peptide-pulsed PBMC (5-6 x 10$^6$ cells/ml) were cultured at 37°C and 5% CO$_2$ for 12 days. On day 0 and day 1.5 ng/ml IL-4 (R&D Systems, Minneapolis, MN, USA) and 5 ng/ml IL-7 (Promokine, Heidelberg, Germany) were added to the culture medium. On days 3, 5, 7 and 9, 2 ng/ml IL-2 (R&D Systems) were added to the culture medium. Peptide-stimulated PBMC were functionally characterized by ELISPOT assays on day 12 and by intracellular cytokine staining on day 13 respectively. For CD4$^+$ T-cell stimulation, culture was performed as described for CD8$^+$T cells with 2 modifications: pulsing was carried out with 10 $\mu$g/ml of HLA class II peptide and no IL-4 and IL-7 was added.

## IFN-$\gamma$ ELISPOT assay

IFN-$\gamma$ ELISPOT assays were carried out as described previously (33). In brief, 96-well nitrocellulose plates (Millipore) were coated with 1 mg/ml IFN-$\gamma$ mAb (Mabtech, Cincinnati, OH, USA) and incubated over night at 4 °C. Plates were blocked with 10% PHS for 2 h at 37 °C. 5 x 10$^5$ cells/well of pre-stimulated PBMC were pulsed with 1 $\mu$g/ml (HLA class I) or 2.5 $\mu$g/ml (HLA class II) peptide and incubated for 24-26 h. Readout was performed according to manufacturer's instructions. Spots were counted using an ImmunoSpot S5 analyzer (CTL, Shaker Heights, OH, USA). T cell responses were considered to be positive when >15 spots/well were counted and the mean spot count per well was at least 3-fold higher than the mean number of spots in the negative control wells (according to the cancer immunoguiding program (CIP) guidelines).

**Intracellular IFN-γ and TNF-α staining**

The frequency and functionality of peptide-specific CD8+ T cells was analyzed by intracellular IFN-γ and TNF-α staining. PBMC were pulsed with 1 μg/ml of individual peptide and incubated in the presence of 10 μg/ml Brefeldin A (Sigma, St. Louis, MO, USA) and 10 μg/ml GolgiStop (BD) for 6-8 h. Cells were labeled using Cytofix/Cytoperm (BD), CD8-PECy7 (Beckman Coulter, Fullerton, CA, USA), CD4-APC (BD Bioscience), TNF-α-PE (Beckman Coulter) and IFN-γ-FITC (BD). Samples were analyzed on a FACS Canto II.
The frequency of peptide-specific CD8+ T cells was determined by staining with anti-CD8 and HLA:peptide-tetramer-PE

**Results**

**Primary CLL cells display no loss or down-regulation of HLA expression compared to autologous normal B cells**

HLA loss or down-regulation in malignancies may pose a major limitation for T cell based immunotherapy. Therefore, as a first step, the inventors determined the HLA expression levels on CD19+CD5+ CLL cells compared to autologous CD19+CD5- B lymphocytes. HLA surface levels were quantified by flow cytometry in a panel of 7 CLL patients. HLA surface expression levels revealed patient-individual heterogeneity with total HLA class I molecule counts ranging from ~42,500-288,500 molecules/cell on CLL cells and ~32,000-256,500 molecules/cell on normal B cells. Patient individual analysis of HLA surface expression in triplicates revealed small, albeit significant differences in expression levels (P<0.01) for 4/7 patients (Fig. 1a). HLA-DR expression ranged from ~29,000-100,500 on CLL cells and ~19,500-79,500 on B cells. Minor differences in HLA-DR levels (P<0.01) were detected for 5/7 patients. Statistical analysis of mean HLA surface expression on CLL cells compared to normal B cells showed no significant differences in HLA class I and II expression (Fig. 1c, d). Taken together, these data demonstrate high levels of HLA class I and II expression on CLL cells without evidence of HLA loss or down-regulation compared to normal B cells.

**LC-MS/MS identifies a vast array of naturally presented HLA class I & II ligands**

Mapping the HLA class I ligandomes of 30 CLL patients, the inventors were able to identify a total of 18,844 different peptides representing 7,377 source proteins, attaining >95% of maximum attainable coverage (Figure 7). The numbers of different peptides identified per patient ranged from 345-2,497 (mean 1,131). Overall, peptides restricted by more than 30 different HLA-A and -B alleles (covering >99% of the Caucasian population_ENREF_27) were identified in this study. In the HV cohort of 30 PBMC donors, a total of 17,322 unique peptides representing 7,180 different source proteins were identified (>90% coverage). The HLA allele distribution in the HV cohort covered 100% of HLA-A and >80% of HLA-B alleles in the CLL patient cohort.
Analysis of the HLA class II ligandomes was performed for 20 CLL patients. A total of 5,059 unique peptides representing 1,486 source proteins was identified. The HLA class II HV cohort of 13 PBMC donors yielded 2,046 different peptides representing 756 source proteins.

**Comparative profiling of HLA class I ligandomes reveals a multitude of CLL-associated antigens**

In order to identify novel CLL-associated antigens, the inventors compared the HLA ligand source proteomes of the CLL and HV cohorts. Overlap analysis of HLA source proteins revealed 2,148 proteins (29.1% of the mapped CLL source proteome) to be exclusively represented in the HLA ligandome of CLL (Fig. 2a). With the aim of designing a broadly applicable off-the-shelf peptide vaccine, the inventors subsequently prioritized the selection of potential targets according to the following criteria:
CLL-exclusivity was defined as paramount criterion, followed by ranking of antigens according to frequency of representation in CLL ligandomes (Fig. 2b). Our platform highlighted 49 source proteins (0.7% of the CLL source proteome) represented by 225 different HLA ligands showing CLL-exclusive representation in ≥20% of CLL patients. Applying the same antigen ranking strategy to HV PBMC exclusive antigens, a set of 71 ligandome-derived benign tissue-associated antigens (LiBAAs) and the 298 corresponding ligands (LiBAPs) were identified for use as internal control in immunological assays.
Apart from broadly represented CLL-LiTAAs suited for the design of off-the-shelf vaccines, a second panel of 2,099 CLL-exclusive antigens with representation frequencies <20% was identified by our platform. These targets lend themselves as repositories for more individualized therapeutic approaches.

**Detection of naturally presented HLA class I ligands derived from established CLL-associated antigens by LC-MS/MS**

Alongside the identification of novel CLL-associated antigens, a secondary approach focused on the ranking of the few established CLL-antigens within the present dataset of naturally presented HLA ligands. The inventors were able to identify 28 different HLA ligands representing 8 described CLL-associated antigens. Of note, only Fibromodulin (FMOD$_{324-333}$, RINEFSISSF, HLA-A*23 (SEQ ID NO: 526) showed CLL-exclusive representation, ranking at #437 of CLL-antigens in the present dataset, due to low frequency of representation in the CLL patient cohort. The remaining seven antigens showed representation, both on CLL and HV PBMC, thus failing to fulfill the paramount criterion of CLL-exclusivity. However, for CD19, CD20, RHAMM and PRAME, CLL-associated overrepresentation of varying degrees was detected (Fig. 2c).

**Comparative ligandome profiling identifies LiTAAs shared among different disease stages and risk strata**

In order to assess the applicability of the novel targets across different stages of disease, the inventors performed subset-specific ligandome profiling comparing patients in disease stages Binet A (n=9), B (n=7) and C (n=14). Overlap analysis of the 2,148 CLL-exclusive source proteins found 550 (25.6%) of them shared among at least two stages, with a core group of 137 proteins (6.1%) represented in patients of all three stages of disease (Fig. 2d). Of note, 45/49 (91.8%) of LiTAAs belong to the core group of shared source proteins represented in all three subsets. Heatmap analysis of the representation frequencies of all 49 LiTAAs across Binet stages A, B and C is shown in Fig. 2e.

Another focus was placed on determining the representation of LiTAAs in the subsets of high-risk patients carrying the 17p13 deletion (del17p, n=5) as compared to patients without this genetic aberration (no del17p, n=25). The inventors found 77.7% of the identified LiTAAs to be represented in both subsets (Fig. 2f). Together, these data support the devised strategy of cohort-comprising analysis of HLA ligandomes for selection of broadly applicable targets.

**Functional characterization of HLA class I LiTAPs reveals CLL-associated immunoreactivity**

In order to evaluate the immunogenicity and specificity of our HLA class I LiTAPs, the inventors next performed 12-day recall IFNγ ELISPOT assays. A panel of 15 LiTAPs (6 A*02, 4 A*03 and 5 B*07 LiTAPs) was implemented for stimulation of HLA-matched PBMC obtained from CLL patients and healthy volunteers (Fig. 3a). The inventors observed IFNy secretion for 14/15 (93.3%) of tested LiTAPs in CLL patients (3/4 A*03 (Fig. 3c), 6/6 A*02 and 5/5 B*07 LiTAPs (Fig. 8 c,f)), but not in healthy controls (0/10, Fig. 3b, Fig. 8 b,e). These findings were confirmed exemplarily for P$_{A*03}$3 (DMXL1$_{1271-1279}$ SSSGLHPPK) by tetramer staining of CD8$^+$ T cells and intracellular cytokine staining for IFNγ and TNFα (Fig. 9 a,b). ELISPOT assays using HLA-matched benign tissue-derived LiBAPs were performed to control for the CLL-specificity of the observed LiTAP-directed immune recognition in CLL patients. The inventors tested a panel of 9 LiBAPs (3 A*02, 3 A*03, 3*B*07) and observed no significant IFNγ secretion in any of the tested CLL patients (0/7 A*03 (Fig. 3d), 0/10 A*02$^+$ and 0/5 B*07 (Fig. 8 a,d)).

For the 14/15 LiTAPs showing immune recognition in 1 or more patients, the inventors calculated the allele-adjusted frequencies of HLA restricted presentation (as detected by LC-MS/MS) and the frequencies of immunoreactivity (as detected by ELISPOT) in CLL patients. Strikingly, a linear correlation of these two parameters was observed (Pearson's r=0.77, R$^2$=0.59, Fig. 3 e). These findings suggest two main points: First, tumor-exclusive representation is prerequisite for immune recognition. Secondly, frequency of immune recognition can be directly deduced from the frequency of HLA restricted presentation for immunoreactive LiTAPs. Together, these data demonstrate the efficacy of our approach identifying immunologically relevant targets for CLL-specific peptide vaccines.

**HLA class II ligandome analysis identifies additional CD4$^+$ T cell epitopes for synergistic vaccine design**

Because of the important indirect and direct roles CD4$^+$ T cells play in anti-cancer immune responses, optimal vaccine design calls for the inclusion of additional HLA class II epitopes. The inventors performed overlap analysis of CLL and HV PBMC ligandomes and identified 937 proteins (63.0% of the identified CLL source proteins) to be exclusively represented in the ligandomes of CLL patients (Fig. 4 a). Applying the same antigen-ranking strategy as described for HLA class I, the inventors identified 73 HLA class II LiTAAs represented by 460 corresponding LiTAPs (Fig. 4 b). Functional characterization of a panel of 7 HLA class II LiTAPs (Fig. 4c) in IFNy ELISPOT assays revealed significant IFNy secretion for 6/7 (85.7%) LiTAPs in CLL patients (Fig. 4e), but not in healthy controls (0/10, Fig. 4d). Next, the inventors performed combined analysis of HLA class I and II ligandomes in order to identify shared, synergistic targets. Overlap analysis of CLL-exclusive source proteins revealed 132 proteins to be represented both in HLA class I and II ligandomes (Fig. 4f). Heatmap analysis identified 2 proteins displaying representation frequencies ≥20% in both ligandomes (B4GALT1 (26.7% class 1/30.0% class II), HLA-DMA (20.0% class I/20% class II), Fig. 4g). Strikingly, one of the class I LiTAPs (HLA-DMA$_{206-214}$, HEIDRYTAI, B*18) was revealed to be completely embedded in the corresponding HLA class II LiTAP (VTHEIDRYTAIAY (SEQ ID No. 924)). Together, the inventors identified a panel of class II LiTAPs, which could be verified as T cell epitopes, as well as an array of potentially synergistic HLA class II ligands covering class I LiTAAs.

**Longitudinal analysis of CLL patient ligandomes under different therapeutic regimens**

The scope of peptide based immunotherapy is maintenance therapy and eradication of MRD. As a consequence, peptide vaccination in CLL would take place after standard chemo-/immunotherapy. Therefore, the inventors analyzed HLA expression and performed ligandome profiling across different time points of CLL patients undergoing different therapeutic regimens.

The inventors quantified HLA class I and II surface expression in 4 patients undergoing rituximab treatment (Rt$_{0h}$, Rt$_{24h}$) and 1 patient receiving alemtuzumab (At$_{0h}$, At$_{72h}$, At$_{7d}$, Fig. 10 a-d). HLA surface expression showed patient-individual heterogeneity with no significant changes in mean HLA class I (Rt$_{0h}$=50,500, Rt$_{24h}$=48,000; At$_{0h}$=42,500, At$_{7d}$=61,500) and HLA class II (Rt$_{0h}$=36,500, Rt$_{24h}$=27,500; At$_{0h}$=47,000, At$_{7d}$=55,500) expression over the course of either therapeutic regimen.

Longitudinal HLA class I ligandome profiling was performed in single patients undergoing rituximab-bendamustin, alemtuzumab or ofatumumab treatment, respectively (Fig. 5a-c). Differential presentation ($\geq$2-fold change, p$\leq$0.05) was observed for 11.1% of HLA class I ligands under rituximab-bendamustin treatment, for 21.6% of ligands under ofatumumab treatment and for 33.6% of ligands under alemtuzumab treatment. Overall, LiTAPs representing 8/49 (16.3%) LiTAAs were revealed to be differentially presented over the course of therapy. Taken together, these data demonstrate stable expression of surface HLA and robust presentation of LiTAPs over the course of different therapies.

**Immune responses against LiTAPs might be associated with improved overall survival of CLL patients**

As a last step, the inventors performed retrospective survival analysis of 33 CLL patients (Fig. 6a) analyzed by ELISPOT assays comparing cases with 0-1 LiTAP-specific (n=23) versus >1 LiTAP-specific (n=10) T cell responses (Fig. 6 b). In the low-responding cohort 6/23 (26.1%) of patients, in the high-responding cohort 0/11 of patients died. Overall survival seems to be prolonged in the cohort showing >1 immune reactions.

EXAMPLE 2

**Synthesis of peptides**

All peptides were synthesized using standard and well-established solid phase peptide synthesis using the Fmoc-strategy. After purification by preparative RP-HPLC, ion-exchange procedure was performed to incorporate physiological compatible counter ions (for example trifluoro-acetate, acetate, ammonium or chloride).

Identity and purity of each individual peptide have been determined by mass spectrometry and analytical RP-HPLC. After ion-exchange procedure the peptides were obtained as white to off-white lyophilizates in purities of 90% to 99.7%.

All TUMAPs are preferably administered as trifluoro-acetate salts or acetate salts, other salt-forms are also possible. For the measurements of example 4, trifluoro-acetate salts of the peptides were used.

EXAMPLE 3

**MHC Binding Assays**

Candidate peptides for T cell based therapies as disclosed were further tested for their MHC binding capacity (affinity). The individual peptide-MHC complexes were produced by peptide-ligand exchange, where a cleavage-sensitive peptide is cleaved, and exchanged with the peptide of interest as analyzed. Only peptide candidates that can effectively bind and stabilize the peptide-receptive MHC molecules prevent dissociation of the MHC complexes. To determine the yield of the exchange reaction, an ELISA was performed based on the detection of the light chain ($\beta$2m) of stabilized MHC complexes. The assay was performed as generally described in Rodenko et al. (Rodenko et al., Nat Protoc. 1 (2006): 1120-1132).

96 well MAXISorp plates (NUNC) were coated over night with 2ug/ml streptavidin in PBS at room temperature, washed 4x and blocked for 1h at 37°C in 2% BSA containing blocking buffer. Refolded HLA-A*0201/MLA-001 monomers served as standards, covering the range of 15-500 ng/ml. Peptide-MHC monomers of the UV-exchange reaction were diluted 100 fold in blocking buffer. Samples were incubated for 1h at 37°C, washed four times, incubated with 2ug/ml HRP conjugated anti-β2m for 1h at 37°C, washed again and detected with TMB solution that is stopped with $NH_2SO_4$. Absorption was measured at 450nm. Candidate peptides that show a high exchange yield (preferably higher than 50%, most preferred higher than 75%) are generally preferred for a generation and production of antibodies or fragments thereof, and/or T cell receptors or fragments thereof, as they show sufficient avidity to the MHC molecules and prevent dissociation of the MHC complexes.

MHC class I binding scores for the peptides as tested were; <20 % = +; 20 % - 49 % = ++; 50 % - 75 %= +++; >= 75 % = ++++

| Seq ID NO. | sequence | Peptide exchange |
|---|---|---|
| 229 | FRVGNVQEL | ++++ |
| 239 | SENAFYLSP | ++++ |

EXAMPLE 4

**In vitro immunogenicity for MHC class I presented peptides**

In order to obtain information regarding the immunogenicity of the TUMAPs as disclosed, the inventors performed investigations using an *in vitro* T-cell priming assay based on repeated stimulations of CD8+ T cells with artificial antigen presenting cells (aAPCs) loaded with peptide/MHC complexes and anti-CD28 antibody. This way the inventors could show immunogenicity for HLA-A*0201 restricted TUMAPs as disclosed, demonstrating that these peptides are T-cell epitopes against which CD8+ precursor T cells exist in humans.

**In vitro priming of CD8+ T cells**

In order to perform *in vitro* stimulations by artificial antigen presenting cells loaded with peptide-MHC complex (pMHC) and anti-CD28 antibody, the inventors first isolated CD8+ T cells from fresh HLA-A*02 leukapheresis products via positive selection using CD8 microbeads (Miltenyi Biotec, Bergisch-Gladbach, Germany) of healthy donors obtained from the University clinics Mannheim, Germany, after informed consent.

PBMCs and isolated CD8+ lymphocytes were incubated in T-cell medium (TCM) until use consisting of RPMI-Glutamax (Invitrogen, Karlsruhe, Germany) supplemented with 10% heat inactivated human AB serum (PAN-Biotech, Aidenbach, Germany), 100 U/ml Penicillin/100 μg/ml Streptomycin (Cambrex, Cologne, Germany), 1 mM sodium pyruvate (CC Pro, Oberdorla, Germany), 20 μg/ml Gentamycin (Cambrex). 2.5 ng/ml IL-7 (PromoCell, Heidelberg, Germany) and 10 U/ml IL-2 (Novartis Pharma, Nürnberg, Germany) were also added to the TCM at this step.

Generation of pMHC/anti-CD28 coated beads, T-cell stimulations and readout was performed in a highly defined *in vitro* system using four different pMHC molecules per stimulation condition and 8 different pMHC molecules per readout condition.

The purified co-stimulatory mouse IgG2a anti human CD28 Ab 9.3 (Jung et al., Proc Natl Acad Sci USA 84 (1987): 4611-4615) was chemically biotinylated using Sulfo-N-hydroxysuccinimidobiotin as recommended by the manufacturer (Perbio, Bonn, Germany). Beads used were 5.6 μm diameter streptavidin coated polystyrene particles (Bangs Laboratories, Illinois, USA).

pMHC used for positive and negative control stimulations were A*0201/MLA-001 (peptide ELAGIGILTV (SEQ ID NO. 1017) from modified Melan-A/MART-1) and A*0201/DDX5-001 (YLLPAIVHI from DDX5, SEQ ID NO. 1018), respectively.

800.000 beads / 200 μl were coated in 96-well plates in the presence of 4 x 12.5 ng different biotin-pMHC, washed and 600 ng biotin anti-CD28 were added subsequently in a volume of 200 μl. Stimulations were initiated in 96-well plates by co-incubating 1x10^6 CD8+ T cells with 2x10^5 washed coated beads in 200 μl TCM supplemented with 5 ng/ml IL-12 (PromoCell) for 3 days at 37°C. Half of the medium was then exchanged by fresh TCM supplemented with 80 U/ml IL-2 and incubating was continued for 4 days at 37°C. This stimulation cycle was performed for a total of three times. For the pMHC multimer readout using 8 different pMHC molecules per condition, a two-dimensional combinatorial coding approach was used as previously described (Andersen et al., Nat.Protoc. 7 (2012): 891-902) with minor modifications encompassing coupling to 5 different fluorochromes. Finally, multimeric analyses were performed by staining the cells with Live/dead near IR dye (Invitrogen, Karlsruhe, Germany), CD8-FITC antibody clone SK1 (BD, Heidelberg, Germany) and fluorescent pMHC multimers. For analysis, a BD LSRII SORP cytometer equipped with appropriate lasers and filters was used. Peptide specific cells were calculated as percentage of total CD8+ cells. Evaluation of multimeric analysis was done using the FlowJo software (Tree Star, Oregon, USA). *In vitro* priming of specific multimer+ CD8+ lymphocytes was detected by by comparing to negative control stimulations. Immunogenicity for a given antigen was detected if at least one evaluable *in vitro* stimulated well of one healthy donor was found to contain a specific CD8+ T-cell line after *in vitro* stimulation (i.e. this well contained at least 1% of specific multimer+ among CD8+ T-cells and the percentage of specific multimer+ cells was at least 10x the median of the negative control stimulations).

*In vitro* immunogenicity for CLL peptides

For tested HLA class I peptides, *in vitro* immunogenicity could be demonstrated by generation of peptide specific T-cell lines. As an exemplary result, peptide KFAEEFYSF (SEQ ID NO. 20) led to *in vitro* T-cell responses in 2 of 5 tested donors.

EXAMPLE 5

**Identification and quantitation of tumor associated peptides presented on the cell surface**

Tissue samples:

In addition to the samples used for identification of peptides, an independent sample set comprising both normal and tumor (CLL) tissues was used for analysis / confirmation of HLA-A*02-associated as disclosed. Written informed consents of all patients had been given before surgery or autopsy. Tissues were shock-frozen immediately after excision and stored until isolation of TUMAPs at -70°C or below.

Isolation of HLA peptides from tissue samples

HLA peptide pools from shock-frozen tissue samples were obtained by immune precipitation from solid tissues according to a slightly modified protocol (Falk et al., Nature 351 (1991): 290-296; Seeger et al., Immunogenetics 49 (1999): 571-576) using the HLA-A*02-specific antibody BB7.2, the HLA-A, -B, C-specific antibody W6/32, CNBr-activated sepharose, acid treatment, and ultrafiltration.

Mass spectrometry analyses

The HLA peptide pools as obtained were separated according to their hydrophobicity by reversed-phase chromatography (nanoAcquity UPLC system, Waters) and the eluting peptides were analyzed in LTQ- velos and fusion hybrid mass spectrometers (ThermoElectron) equipped with an ESI source. Peptide pools were loaded directly onto the analytical fused-silica micro-capillary column (75 $\mu$m i.d. x 250 mm) packed with 1.7 $\mu$m C18 reversed-phase material (Waters) applying a flow rate of 400 nL per minute. Subsequently, the peptides were separated using a two-step 180 minute-binary gradient from 10% to 33% B at a flow rate of 300 nL per minute. The gradient was composed of Solvent A (0.1% formic acid in water) and solvent B (0.1% formic acid in acetonitrile). A gold coated glass capillary (PicoTip, New Objective) was used for introduction into the nanoESI source. The LTQ-Orbitrap mass spectrometers were operated in the data-dependent mode using a TOP5 strategy. In brief, a scan cycle was initiated with a full scan of high mass accuracy in the orbitrap (R = 30 000), which was followed by MS/MS scans also in the orbitrap (R = 7500) on the 5 most abundant precursor ions with dynamic exclusion of previously selected ions. Tandem mass spectra were interpreted by SEQUEST and additional manual control. The identified peptide sequence was assured by comparison of the generated natural peptide fragmentation pattern with the fragmentation pattern of a synthetic sequence-identical reference peptide.

Label-free relative LC-MS quantitation was performed by ion counting i.e. by extraction and analysis of LC-MS features (Mueller et al., Proteomics. 7 (2007): 3470-3480). The method assumes that the peptide's LC-MS signal area correlates with its abundance in the sample. Extracted features were further processed by charge state deconvolution and retention time alignment (Mueller et al., J Proteome.Res 7 (2008): 51-61; Sturm et al., BMC.Bioinformatics. 9 (2008): 163). Finally, all LC-MS features were cross-referenced with the sequence identification results to combine quantitative data of different samples and tissues to peptide presentation profiles. The quantitative data were normalized in a two-tier fashion according to central tendency to account for variation within technical and biological replicates. Thus each identified peptide can be associated with quantitative data allowing relative quantification between samples and tissues. In addition, all quantitative data acquired for peptide candidates was inspected manually to assure data consistency and to verify the accuracy of the automated analysis. For each peptide a presentation profile was calculated showing the mean sample presentation as well as replicate variations. The profiles juxtapose CLL samples to a baseline of normal tissue samples. The presentation profile of an exemplary over-presented peptide is shown in Figure 11.

Cited references

Ding, M. X. et al., Asian Pac.J Cancer Prev. 13 (2012): 5653-5657

Gallardo-Perez, J. C. et al., Biochim.Biophys.Acta 1843 (2014): 1043-1053

Jardim, B. V. et al., Oncol Rep. 30 (2013): 1119-1128

Jevnikar, Z. et al., J Biol.Chem 288 (2013): 2201-2209

Liu, Y. Y. et al., Mol.Cancer 9 (2010): 145

Mayr, C. et al., Blood 105 (2005): 1566-1573

Men, T. et al., Tumour.Biol. 35 (2014): 269-275

Nagai, K. et al., Cancer Med. 3 (2014): 1085-1099

Pallasch, C. P. et al., Blood 112 (2008): 4213-4219

Poeta, M. L. et al., Genes Chromosomes.Cancer 51 (2012): 1133-1143

Teh, M. T. et al., PLoS.One. 7 (2012): e34329

Yi, S. et al., Leuk.Lymphoma 52 (2011): 72-78

Yoon, D. Y. et al., Biochem.Biophys.Res.Commun. 288 (2001): 882-886

Yu, Z. et al., Zhonghua Yi.Xue.Za Zhi. 91 (2011): 1371-1374

Zhang, K. et al., Chin Med.J (Engl.) 126 (2013): 4660-4664

Zhou, H. et al., IUBMB.Life 64 (2012): 889-900

**Claims**

1.  A peptide of between 10 and 30 amino acids in length, comprising an amino acid sequence consisting of SEQ ID NO: 167, or a pharmaceutically acceptable salt thereof.

2.  The peptide according to claim 1, wherein said peptide has the ability to bind to a human major histocompatibility complex (MHC) class-I molecule.

**3.** The peptide according to claim 1 or 2, consisting of the sequence of SEQ ID NO: 167.

**4.** The peptide according to any of claims 1 to 3, wherein said peptide includes non-peptide bonds.

**5.** A fusion protein, comprising

    (a) an amino acid sequence consisting of SEQ ID NO: 167; and
    (b) N-terminal amino acids 1-80 of HLA-DR antigen-associated invariant chain (Ii).

**6.** An antibody that specifically binds to a human major histocompatibility complex (MHC) class I being complexed with the peptide according to any one of claims 1 to 3.

**7.** A T-cell receptor that binds to a human major histocompatibility complex (MHC) class I being complexed with the peptide according to any one of claims 1 to 3.

**8.** A nucleic acid, encoding for (a) the peptide according to any one of claims 1 to 3; (b) the T cell receptor according to claim 7; (c) the fusion protein according to claim 5, or (d) the antibody according to claim 6.

**9.** An expression vector comprising a nucleic acid according to claim 8.

**10.** A host cell comprising the nucleic acid according to claim 8, or the expression vector according to claim 9.

**11.** A method for producing the peptide according to any of claims 1 to 3; the T cell receptor according to claim 7; the fusion protein according to claim 5, or the antibody according to claim 6, said method comprising culturing the host cell according to claim 10, and isolating said peptide, said T cell receptor, said fusion protein, or said antibody from said host cell and/or its culture medium.

**12.** An *in vitro* method for producing activated cytotoxic T lymphocytes (CTL), the method comprising contacting *in vitro* a CTL with antigen loaded human class I MHC molecules expressed on the surface of a suitable antigen-presenting cell for a period of time sufficient to activate said CTL in an antigen specific manner, wherein said antigen is said peptide according to claim 1 or 2.

**13.** An activated cytotoxic T lymphocyte (CTL), produced by the method according to claim 12.

**14.** A pharmaceutical composition comprising at least one active ingredient selected from the group consisting of the peptide according to any of claims 1 to 3, the T cell receptor according to claim 7; the fusion protein according to claim 5, or the antibody according to claim 6, the nucleic acid according to claim 8, the expression vector according to claim 9, the host cell according to claim 10, and the activated cytotoxic T lymphocyte according to claim 13, and a pharmaceutically acceptable carrier.

**15.** The pharmaceutical composition according to claim 14 for use as a medicament that is active against cancer, such as, for example, chronic lymphatic leukemia (CLL), acute myeloid leukemia (AML), adenocarcinoma, and ovary cancer.

**Patentansprüche**

**1.** Peptid mit einer Länge zwischen 10 und 30 Aminosäuren, umfassend eine Aminosäuresequenz bestehend aus SEQ ID No: 167, oder ein pharmazeutisch akzeptables Salz davon.

**2.** Das Peptid gemäß Anspruch 1, wobei das Peptid die Fähigkeit aufweist, an ein Molekül des menschlichen Haupthistokompatibilitätskomplexes (MHC) Klasse-I zu binden.

**3.** Das Peptid nach Anspruch 1 oder 2 bestehend aus der Sequenz mit SEQ ID NO: 167.

**4.** Das Peptid nach einem der Ansprüche 1 bis 3, wobei das Peptid nicht-peptidische Bindungen einschließt.

**5.** Ein Fusionsprotein umfassend

(a) eine Aminosäuresequenz bestehend aus SEQ ID No. 167; und
(b) N-terminale Aminosäuren 1-80 der HLA-DR-Antigen-assoziierten invarianten Kette (Ii).

**6.** Ein Antikörper, der spezifisch an einen menschlichen Haupthistokompatibilitätskomplex (MHC) der Klasse I bindet, der mit dem Peptid nach einem der Ansprüche 1 bis 3 komplexiert ist.

**7.** Ein T-Zell-Rezeptor, der an einen menschlichen Haupthistokompatibilitätskomplex (MHC) der Klasse I bindet, der mit dem Peptid nach einem der Ansprüche 1 bis 3 komplexiert ist.

**8.** Nukleinsäure, die für (a) das Peptid nach einem der Ansprüche 1 bis 3; (b) den T-Zell-Rezeptor nach Anspruch 7; (c) das Fusionsprotein nach Anspruch 5 oder (d) den Antikörper nach Anspruch 6 kodiert.

**9.** Ein Expressionsvektor umfassend eine Nukleinsäure nach Anspruch 8.

**10.** Eine Wirtszelle umfassend die Nukleinsäure nach Anspruch 8 oder den Expressionsvektor nach Anspruch 9.

**11.** Verfahren zur Herstellung des Peptids nach einem der Ansprüche 1 bis 3; des T-Zell-Rezeptors nach Anspruch 7; des Fusionsproteins nach Anspruch 5 oder des Antikörpers nach Anspruch 6, wobei das Verfahren die Kultivierung der Wirtszelle nach Anspruch 10 und die Isolierung des Peptids, des T-Zell-Rezeptors, des Fusionsproteins oder des Antikörpers aus der Wirtszelle und/oder ihrem Kulturmedium umfasst.

**12.** *In vitro*-Verfahren zur Herstellung von aktivierten cytotoxischen T-Lymphocyten (CTL), wobei das Verfahren ein in Kontakt bringen *in vitro* von einem CTL mit Antigen beladenen menschlichen Klasse-I MHC-Molekülen umfasst, die auf der Oberfläche einer geeigneten Antigen-präsentierende Zelle für eine Zeitdauer präsentiert werden, die ausreichend ist, um die CTL auf eine Antigen-spezifische Weise zu aktivieren, wobei das Antigen das Peptid nach Anspruch 1 oder 2 ist.

**13.** Aktivierter zytotoxischer T-Lymphozyt (CTL), der nach dem Verfahren gemäß Anspruch 12 hergestellt wurde.

**14.** Pharmazeutische Zusammensetzung, umfassend mindestens einen Wirkstoff, ausgewählt aus der Gruppe, bestehend aus dem Peptid nach einem der Ansprüche 1 bis 3, dem T-Zell-Rezeptor nach Anspruch 7, dem Fusionsprotein nach Anspruch 5, dem Antikörper nach Anspruch 6, der Nukleinsäure nach Anspruch 8, dem Expressionsvektor nach Anspruch 9, der Wirtszelle nach Anspruch 10 und dem aktivierten zytotoxischen T-Lymphozyten nach Anspruch 13, und einen pharmazeutisch akzeptablen Träger.

**15.** Die pharmazeutische Zusammensetzung nach Anspruch 14 zur Verwendung als Medikament, das gegen Krebs, wie z. B. chronische lymphatische Leukämie (CLL), akute myeloische Leukämie (AML), Adenokarzinom und Eierstockkrebs, wirksam ist.

**Revendications**

**1.** Peptide d'une longueur comprise entre 10 et 30 acides aminés, comprenant une séquence d'acides aminés consistant en SEQ ID n°: 167, ou sel pharmaceutiquement acceptable de ce dernier.

**2.** Peptide conforme à la revendication 1, où ledit peptide a la capacité de se lier à une molécule du complexe majeur d'histocompatibilité (CMH) de classe I.

**3.** Peptide conforme à la revendication 1 ou 2, consistant en la séquence SEQ ID n° 167.

**4.** Peptide conforme à l'une des revendications 1 à 3, où ledit peptide comprend des liaisons non peptidiques.

**5.** Protéine de fusion, comprenant

(a) une séquence d'acides aminés consistant en SEQ ID n° 167 ; et
(b) les acides aminés N-terminaux 1 à 80 de la chaîne invariante (Ii) associée aux antigènes du système HLA-DR.

**6.** Anticorps qui se lie spécifiquement à un complexe majeur d'histocompatibilité (CMH) humain de classe I formant un complexe avec le peptide conforme à l'une des revendications 1 à 3.

7. Récepteur de lymphocytes T qui se lie à un complexe majeur d'histocompatibilité (CMH) humain de classe I formant un complexe avec le peptide conforme à l'une des revendications 1 à 3.

8. Acide nucléique, codant pour (a) le peptide conforme à l'une des revendications 1 à 3, (b) le récepteur de lymphocytes T conforme à la revendication 7, (c) la protéine de fusion conforme à la revendication 5, ou (d) l'anticorps conforme à la revendication 6.

9. Vecteur d'expression comprenant un acide nucléique conforme à la revendication 8.

10. Cellule hôte comprenant l'acide nucléique conforme à la revendication 8 ou le vecteur d'expression conforme à la revendication 9.

11. Procédé de production du peptide conforme à l'une des revendications 1 à 3, du récepteur de lymphocytes T conforme à la revendication 7, de la protéine de fusion conforme à la revendication 5 ou de l'anticorps conforme à la revendication 6, ledit procédé comprenant la mise en culture de la cellule hôte conforme à la revendication 10, et l'isolement dudit peptide, dudit récepteur de lymphocytes T, de ladite protéine de fusion ou dudit anticorps de ladite cellule hôte et/ou de son milieu de culture.

12. Procédé *in vitro* de production de lymphocytes T cytotoxiques (CTL) activés, ce procédé comprenant la mise en contact *in vitro* d'un CTL avec des molécules du CMH de classe I humain chargées d'antigènes exprimées à la surface d'une cellule présentatrice d'antigènes appropriée pendant un temps suffisant pour activer, de manière antigène-spécifique, ledit CTL, où ledit antigène est ledit peptide conforme à la revendication 1 ou 2.

13. Lymphocyte T cytotoxique (CTL) activé, produit par le procédé conforme à la revendication 12.

14. Composition pharmaceutique comprenant au moins un ingrédient actif sélectionné parmi le groupe consistant en : le peptide conforme à l'une des revendications 1 à 3, le récepteur de lymphocytes T conforme à la revendication 7, la protéine de fusion conforme à la revendication 5, l'anticorps conforme à la revendication 6, l'acide nucléique conforme à la revendication 8, le vecteur d'expression conforme à la revendication 9, la cellule hôte conforme à la revendication 10, et le lymphocyte T cytotoxique activé conforme à la revendication 13, et un transporteur pharmaceutiquement acceptable.

15. Composition pharmaceutique conforme à la revendication 14 pour une utilisation en tant que médicament actif contre le cancer, notamment, par exemple, la leucémie lymphoïde chronique (LLC), la leucémie aiguë myéloïde (LAM), l'adénocarcinome et le cancer de l'ovaire.

Figure 1

Figure 2

Figure 2 cont.:

Figure 3

a

| Peptide | Sequence | Source protein | CD8+ T-cell response in CLL |
|---|---|---|---|
| $P_{A^*03}{}^1$ | YGYDNVKEY | CDCA7L | 2/13 (15,3%) |
| $P_{A^*03}{}^2$ | AVFDGAQVTSK | TP53I11 | 3/13 (23,0%) |
| $P_{A^*03}{}^3$ | SSSGLHPPK | DMXL1 | 4/13 (30,8%) |
| $P_{A^*02}{}^1$ | ILDEKPVII | ABCA6 | 2/11 (18,2%) |
| $P_{A^*02}{}^2$ | YLNKEIEEA | CTDP1 | 3/11 (27,3%) |
| $P_{A^*02}{}^3$ | SILEDPPSI | ASUN | 3/11 (27,3%) |
| $P_{A^*02}{}^4$ | DLDVKKMPL | PARP3 | 2/11 (18,2%) |
| $P_{A^*02}{}^5$ | QLLDQVEQI | TMED4 | 3/11 (27,3%) |
| $P_{A^*02}{}^6$ | AAANIIRTL | RASGRF1 | 1/11 (9,1%) |
| $P_{B^*07}{}^1$ | SPRPPLGSSL | KDM2B | 4/6 (66,7%) |
| $P_{B^*07}{}^2$ | APLQRSQSL | TBC1D22A | 4/7 (57,1%) |
| $P_{B^*07}{}^3$ | SPTSSRTSSL | CELSR1 | 3/6 (50%) |
| $P_{B^*07}{}^4$ | KPRQSSPQL | DNMBP | 3/6 (50%) |
| $P_{B^*07}{}^5$ | SASVQRADTSL | ZFAND5 | 4/14 (28,6%) |

b

A*03 LiTAPs in HV

Figure 3 cont.:

Figure 4

Figure 4 cont.:

c

| Peptide | Sequence | Source protein | CD4+ T-cell response in CLL |
|---|---|---|---|
| P_II^1 | LPSQAFEYILYNKG | CTSH | 2/15 (13,3%) |
| P_II^2 | RVEYHFLSPYVSPK | TFRC | 1/15 (6,7%) |
| P_II^3 | NSVIIVDKNGRLV | TFRC | 2/15 (13,3%) |
| P_II^4 | DIMRVNVDKVLERDQKL | VAMP2 | 2/15 (13,3%) |
| P_II^5 | YKAFSSLLASSAVSPE | IL4R | 3/15 (20,0%) |
| P_II^6 | VDKVLERDQKLSELDDR | VAMP2 | 2/15 (13,3%) |

d

HLA class II LiTAPs in HV

e

UPN 19    UPN 18    UPN 9

Figure 4 cont.:

Figure 5

Figure 6

Figure 7

100% (7,484 proteins)

98% (7,312 proteins)

$R^2 = 0.99$

Unique Source Protein IDs

8000

6000

4000

2000

0

Total source protein IDs

0   10000   20000   30000   40000

Figure 8

EP 3 157 549 B1

Figure 8 cont.:

d

B*07 LiBAPs

e

B*07 LiTAPs in HV

f

UPN 34

UPN 14

UPN 19

EP 3 157 549 B1

EP 3 157 549 B1

Figure 9

a

Figure 9 cont.:

b

Figure 10

Figure 11

Relative Presentation [Arbitrary Units]

Normal tissue samples:
3 x liver, 1 x lymph node

CLL samples

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007028574 A
- EP 1760088 B1
- WO 03068201 A
- WO 2004084798 A
- WO 0172768 A
- WO 03070752 A
- US 20100113300 A
- US 20130115191 A
- WO 2004033685 A1
- WO 2004074322 A1
- WO 2012056407 A1
- WO 2013057586 A1
- US 20130096016 A
- US 4816567 A
- WO 9429348 A
- US 4342566 A
- US 4897445 A

- US 4440859 A
- US 4530901 A
- US 4582800 A
- US 4677063 A
- US 4678751 A
- US 4704362 A
- US 4710463 A
- US 4757006 A
- US 4766075 A
- US 4810648 A
- WO 9726328 A
- WO 9518145 A
- US 5849589 A
- US 6406705 B1
- EP 2112253 A
- GB 1411037 A
- US 62014849 B

**Non-patent literature cited in the description**

- *Proc Natl Acad Sci U S A.,* 22 July 2003, vol. 100 (15), 8862-7
- *Clin Cancer Res.,* 01 June 2006, vol. 12, 3435-43
- *J Immunol.,* 01 November 2007, vol. 179 (9), 5829-38
- **DENGJEL J et al.** Unexpected abundance of HLA class II presented peptides in primary renal cell carcinomas. *Clin Cancer Res.,* 15 July 2006, vol. 12, 4163-70
- **COHEN CJ et al.** Recombinant antibodies with MHC-restricted, peptide-specific, T-cell receptor-like specificity: new tools to study antigen presentation and TCR-peptide-MHC interactions. *J Mol Recognit.,* September 2003, vol. 16 (5), 324-32
- **DENKBERG G et al.** Selective targeting of melanoma and APCs using a recombinant antibody with TCR-like specificity directed toward a melanoma differentiation antigen. *J Immunol.,* 01 September 2003, vol. 171 (5), 2197-207
- **COHEN CJ et al.** Direct phenotypic analysis of human MHC class I antigen presentation: visualization, quantitation, and in situ detection of human viral epitopes using peptide-specific, MHC-restricted human recombinant antibodies. *J Immunol.,* 15 April 2003, vol. 170 (8), 4349-61

- **LIDDY N et al.** Monoclonal TCR-redirected tumor cell killing. *Nat Med,* June 2012, vol. 18 (6), 980-987
- **BOULTER JM et al.** Stable, soluble T-cell receptor molecules for crystallization and therapeutics. *Protein Eng,* September 2003, vol. 16 (9), 707-711
- **CARD KF et al.** A soluble single-chain T-cell receptor IL-2 fusion protein retains MHC-restricted peptide specificity and IL-2 bioactivity. *Cancer Immunol Immunother,* April 2004, vol. 53 (4), 345-357
- **WILLCOX BE et al.** Production of soluble alphabeta T-cell receptor heterodimers suitable for biophysical analysis of ligand binding. *Protein Sci,* November 1999, vol. 8 (11), 2418-2423
- **J. PINHEIRO et al.** *The nlme Package: Linear and Nonlinear Mixed Effects Models,* 2007
- **Y. BENJAMINI ; Y. HOCHBERG.** Controlling the False Discovery Rate: A Practical and Powerful Approach to Multiple Testing. *Journal of the Royal Statistical Society,* 1995, vol. 57 (1), 289-300
- **HARLOW ; LANE.** Antibodies: A Laboratory Manual. Cold Spring Harbor Laboratory Press, 1988
- **FONG L et al.** Altered peptide ligand vaccination with Flt3 ligand expanded dendritic cells for tumor immunotherapy. *Proc Natl Acad Sci USA.,* 17 July 2001, vol. 98 (15), 8809-14

- **ZAREMBA S et al.** Identification of an enhancer agonist cytotoxic T lymphocyte peptide from human carcinoembryonic antigen. *Cancer Res.,* 15 October 1997, vol. 57 (20), 4570-7
- **COLOMBETTI S et al.** Impact of orthologous melan-A peptide immunizations on the anti-self melan-A/HLA-A2 T cell cross-reactivity. *J Immunol.,* 01 June 2006, vol. 176 (11), 6560-7
- **APPAY V et al.** Decreased specific CD8+ T cell cross-reactivity of antigen recognition following vaccination with Melan-A peptide. *Eur J Immunol.,* July 2006, vol. 36 (7), 1805-14
- **GODKIN A et al.** Use of eluted peptide sequence data to identify the binding characteristics of peptides to the insulin-dependent diabetes susceptibility allele HLA-DQ8 (DQ 3.2). *Int Immunol.,* June 1997, vol. 9 (6), 905-11
- **RAMMENSEE H. et al.** SYFPEITHI: database for MHC ligands and peptide motifs. *Immunogenetics,* November 1999, vol. 50 (3-4), 213-9
- **MEZIERE et al.** *J. Immunol.,* 1997, vol. 159, 3230-3237
- **LUKAS et al.** Solid-phase peptide synthesis under continuous-flow conditions. *Proc Natl Acad Sci U S A.,* 1981, vol. 78 (5), 2791-2795
- **SAIKI RK et al.** Diagnosis of sickle cell anemia and beta-thalassemia with enzymatically amplified DNA and nonradioactive allele-specific oligonucleotide probes. *N Engl J Med.,* 01 September 1988, vol. 319 (9), 537-41
- **PAULINA BALBÁS ; ARGELIA LORENCE.** Methods in Molecular Biology Recombinant Gene Expression, Reviews and Protocols
- **COHEN et al.** *Proc. Natl. Acad. Sci. USA,* 1972, vol. 69, 2110
- **SAMBROOK et al.** Molecular Cloning, A Laboratory Manual. Cold Spring Harbor Laboratory, 1989
- **SHERMAN et al.** Methods In Yeast Genetics, A Laboratory Manual. Cold Spring Harbor, 1986
- The method of Beggs. *Nature,* 1978, vol. 275, 104-109
- **SMALL EJ et al.** Placebo-controlled phase III trial of immunologic therapy with sipuleucel-T (APC8015) in patients with metastatic, asymptomatic hormone refractory prostate cancer. *J Clin Oncol.,* 01 July 2006, vol. 24 (19), 3089-94
- **RINI et al.** Combination immunotherapy with prostatic acid phosphatase pulsed antigen-presenting cells (provenge) plus bevacizumab in patients with serologic progression of prostate cancer after definitive local therapy. *Cancer.,* 01 July 2006, vol. 107 (1), 67-74
- **WALTER et al.** *Nature Medicine,* 2012, vol. 18, 1254-1261
- **LJUNGGREN, H.-G. ; K. KARRE.** *J. Exp. Med.,* 1985, vol. 162, 1745
- **PLEBANSKI et al.** Induction of peptide-specific primary cytotoxic T lymphocyte responses from human peripheral blood. *Eur J Immunol.,* June 1995, vol. 25 (6), 1783-7
- **S. WALTER et al.** Cutting edge: predetermined avidity of human CD8 T cells expanded on calibrated MHC/anti-CD28-coated microspheres. *J Immunol.,* 15 November 2003, vol. 171 (10), 4974-8
- **PORTA et al.** Development of cowpea mosaic virus as a high-yielding system for the presentation of foreign peptides. *Virology,* 01 August 1994, vol. 202 (2), 949-55
- **GATTINONI L et al.** Adoptive immunotherapy for cancer: building on success. *Nat Rev Immunol.,* May 2006, vol. 6 (5), 383-93
- **MORGAN RA et al.** Cancer regression in patients after transfer of genetically engineered lymphocytes. *Science,* 06 October 2006, vol. 314 (5796), 126-9
- **PASCOLO et al.** Human peripheral blood mononuclear cells transfected with messenger RNA stimulate antigen-specific cytotoxic T-lymphocytes in vitro. *Cell Mol Life Sci.,* August 2005, vol. 62 (15), 1755-62
- **ALLISON ; KRUMMEL.** The Yin and Yang of T cell costimulation. *Science,* 10 November 1995, vol. 270 (5238), 932-3
- **GABRILOVICH.** Production of vascular endothelial growth factor by human tumors inhibits the functional maturation of dendritic cells. *Nat Med.,* October 1996, vol. 2 (10), 1096-103
- **A. KIBBE.** Handbook of Pharmaceutical Excipients. American Pharmaceutical Association, 2000
- **RODENKO et al.** *Nat Protoc.,* 2006, vol. 1, 1120-1132
- **JUNG et al.** *Proc Natl Acad Sci USA,* 1987, vol. 84, 4611-4615
- **ANDERSEN et al.** *Nat.Protoc.,* 2012, vol. 7, 891-902
- **FALK et al.** *Nature,* 1991, vol. 351, 290-296
- **SEEGER et al.** *Immunogenetics,* 1999, vol. 49, 571-576
- **MUELLER et al.** *Proteomics,* 2007, vol. 7, 3470-3480
- **MUELLER et al.** *J Proteome.Res,* 2008, vol. 7, 51-61
- **STURM et al.** *BMC.Bioinformatics.,* 2008, vol. 9, 163
- **DING, M. X. et al.** *Asian Pac.J Cancer Prev.,* 2012, vol. 13, 5653-5657
- **GALLARDO-PEREZ, J. C. et al.** *Biochim.Biophys.Acta,* 2014, vol. 1843, 1043-1053
- **JARDIM, B. V. et al.** *Oncol Rep.,* 2013, vol. 30, 1119-1128
- **JEVNIKAR, Z. et al.** *J Biol.Chem,* 2013, vol. 288, 2201-2209
- **LIU, Y. Y. et al.** *Mol.Cancer,* 2010, vol. 9, 145

- **MAYR, C. et al.** *Blood,* 2005, vol. 105, 1566-1573
- **MEN, T. et al.** *Tumour.Biol.,* 2014, vol. 35, 269-275
- **NAGAI, K. et al.** *Cancer Med.,* 2014, vol. 3, 1085-1099
- **PALLASCH, C. P. et al.** *Blood,* 2008, vol. 112, 4213-4219
- **POETA, M. L. et al.** Genes Chromosomes. *Cancer,* 2012, vol. 51, 1133-1143
- **TEH, M. T. et al.** *PLoS.One.,* 2012, vol. 7, e34329
- **YI, S. et al.** *Leuk.Lymphoma,* 2011, vol. 52, 72-78
- **YOON, D. Y. et al.** *Biochem.Biophys.Res.Commun.,* 2001, vol. 288, 882-886
- **YU, Z. et al.** *Zhonghua Yi.Xue.Za Zhi.,* 2011, vol. 91, 1371-1374
- **ZHANG, K. et al.** *Chin Med.J (Engl.),* 2013, vol. 126, 4660-4664
- **ZHOU, H. et al.** *IUBMB.Life,* 2012, vol. 64, 889-900

SEQUENCE LISTING

<110> immatics biotechnologies GmbH

<120> Novel immunotherapy against several tumors of the blood, in particular chronic lymphoid leukemia (CLL)

<130> I32605WO

<150> GB 1411037.3
<151> 2014-06-20

<150> US 62/014,849
<151> 2014-06-20

<160> 1018

<170> PatentIn version 3.5

<210> 1

<210> 1
<211> 10
<212> PRT
<213> Homo sapiens

<400> 1

```
Ala Glu His Pro Asn Val Thr Leu Thr Ile
1               5                   10
```

<210> 2
<211> 10
<212> PRT
<213> Homo sapiens

<400> 2

```
Phe Leu Ala Glu His Pro Asn Val Thr Leu
1               5                   10
```

<210> 3
<211> 9
<212> PRT
<213> Homo sapiens

<400> 3

```
Ile Leu Tyr Gly Arg Ser Tyr Thr Trp
1               5
```

<210> 4
<211> 9
<212> PRT
<213> Homo sapiens

<400> 4

```
Glu Val Ala Glu Phe Leu Ala Arg His
1               5
```

<210> 5
<211> 9
<212> PRT
<213> Homo sapiens

<400> 5

```
Arg His Ser Asn Val Asn Leu Thr Ile
1               5
```

<210> 6
<211> 9
<212> PRT
<213> Homo sapiens

<400> 6

```
His Pro Asp Asn Val Lys Leu Phe Leu
1               5
```

<210> 7
<211> 9
<212> PRT
<213> Homo sapiens

<400> 7

Ile Ser Asp Thr Gly Glu Leu Lys Leu
1               5

<210> 8
<211> 10
<212> PRT
<213> Homo sapiens

<400> 8

Lys Val Asn Gly Lys Leu Val Ala Leu Lys
1               5                   10

<210> 9
<211> 9
<212> PRT
<213> Homo sapiens

<400> 9

Asn Arg Leu Ser Ala Gln Ala Ala Leu
1               5

<210> 10
<211> 9
<212> PRT
<213> Homo sapiens

<400> 10

Thr Pro Phe Thr Ala Ile Arg Glu Ala
1               5

<210> 11
<211> 9
<212> PRT
<213> Homo sapiens

<400> 11

Phe Gly Leu Ala Arg Ala Lys Ser Val
1               5

<210> 12
<211> 9
<212> PRT
<213> Homo sapiens

<400> 12

```
Lys Ile Ala Asp Phe Gly Leu Ala Arg
1               5
```

<210> 13
<211> 9
<212> PRT
<213> Homo sapiens

<400> 13

```
Ala Ala Ala Asn Ile Ile Arg Thr Leu
1               5
```

<210> 14
<211> 10
<212> PRT
<213> Homo sapiens

<400> 14

```
Gly Arg Phe Lys Asn Leu Arg Glu Ala Leu
1               5                   10
```

<210> 15
<211> 9
<212> PRT
<213> Homo sapiens

<400> 15

```
Met Ser Pro Phe Ser Lys Ala Thr Leu
1               5
```

<210> 16
<211> 11
<212> PRT
<213> Homo sapiens

<400> 16

```
Gln Glu Asp Pro Gly Asp Asn Gln Ile Thr Leu
1               5                   10
```

<210> 17
<211> 8
<212> PRT
<213> Homo sapiens

<400> 17

```
Ser Pro Phe Ser Lys Ala Thr Leu
1               5
```

<210> 18
<211> 8
<212> PRT
<213> Homo sapiens

<400> 18

```
Asp Ala Leu Leu Lys Arg Thr Met
1               5
```

<210> 19
<211> 9
<212> PRT
<213> Homo sapiens

<400> 19

```
Gly Glu Asp Val Arg Ser Ala Leu Leu
1               5
```

<210> 20
<211> 9
<212> PRT
<213> Homo sapiens

<400> 20

```
Lys Phe Ala Glu Glu Phe Tyr Ser Phe
1               5
```

<210> 21
<211> 9
<212> PRT
<213> Homo sapiens

<400> 21

```
Tyr Gly Tyr Asp Asn Val Lys Glu Tyr
1               5
```

<210> 22
<211> 10
<212> PRT
<213> Homo sapiens

<400> 22

```
Leu Glu Val Glu Glu Arg Thr Lys Pro Val
1               5                   10
```

<210> 23
<211> 11
<212> PRT
<213> Homo sapiens

<400> 23

```
Arg Asp Ser Pro Ile Asn Ala Asn Leu Arg Tyr
1               5                   10
```

<210> 24
<211> 8
<212> PRT

<213> Homo sapiens

<400> 24

Arg Pro Phe Val Ile Val Thr Ala
1                   5

<210> 25
<211> 9
<212> PRT
<213> Homo sapiens

<400> 25

Arg Pro Ile Ile Asn Thr Pro Met Val
1                   5

<210> 26
<211> 10
<212> PRT
<213> Homo sapiens

<400> 26

Ser Pro Thr Ser Ser Arg Thr Ser Ser Leu
1                   5                   10

<210> 27
<211> 9
<212> PRT
<213> Homo sapiens

<400> 27

Ala Thr Ser Ala Pro Leu Val Ser Arg

1                   5

<210> 28
<211> 10
<212> PRT
<213> Homo sapiens

<400> 28

Ala Glu Leu Arg Ser Thr Ala Ser Leu Leu
1                   5                   10

<210> 29
<211> 11
<212> PRT
<213> Homo sapiens

<400> 29

```
Ala Pro Ala Ser Ser His Glu Arg Ala Ser Met
1                   5                   10
```

<210> 30
<211> 9
<212> PRT
<213> Homo sapiens

<400> 30

```
Ala Ser Arg Gln Ala Pro Pro His Ile
1                   5
```

<210> 31
<211> 10
<212> PRT
<213> Homo sapiens

<400> 31

```
Ala Val Lys Lys Asn Pro Gly Ile Ala Ala
1                   5                   10
```

<210> 32
<211> 10
<212> PRT
<213> Homo sapiens

<400> 32

```
Glu Glu His Leu Glu Ser His Lys Lys Tyr
1                   5                   10
```

<210> 33
<211> 9
<212> PRT
<213> Homo sapiens

<400> 33

```
Gly Glu Phe Thr Ser Ala Arg Ala Val
1                   5
```

<210> 34
<211> 10
<212> PRT
<213> Homo sapiens

<400> 34

```
Gly Gln Ser Thr Pro Arg Leu Phe Ser Ile
1                   5                   10
```

<210> 35
<211> 8
<212> PRT
<213> Homo sapiens

<400> 35

Leu Val Asp Asp Pro Leu Glu Tyr
1               5

<210> 36
<211> 9
<212> PRT
<213> Homo sapiens

<400> 36

Arg Pro Lys Asn Leu Met Gln Thr Leu
1               5

<210> 37
<211> 9
<212> PRT
<213> Homo sapiens

<400> 37

Arg Gln Ala Pro Pro His Ile Glu Leu
1               5

<210> 38
<211> 10
<212> PRT
<213> Homo sapiens

<400> 38

Ser Glu Ala Ala Glu Leu Arg Ser Thr Ala
1               5                       10

<210> 39
<211> 9
<212> PRT
<213> Homo sapiens

<400> 39

Ala Ala Val Arg Ile Gly Ser Val Leu
1               5

<210> 40
<211> 9
<212> PRT
<213> Homo sapiens

<400> 40

Glu Arg Ala Gly Val Val Arg Glu Leu
1               5

<210> 41
<211> 10
<212> PRT

<213> Homo sapiens

<400> 41

Gly Ala Ala Val Arg Ile Gly Ser Val Leu
1               5                   10

<210> 42
<211> 10
<212> PRT
<213> Homo sapiens

<400> 42

Lys Leu Tyr Glu Leu His Val Phe Thr Phe
1               5                   10

<210> 43
<211> 9
<212> PRT
<213> Homo sapiens

<400> 43

Leu Tyr Glu Leu His Val Phe Thr Phe
1               5

<210> 44
<211> 9
<212> PRT
<213> Homo sapiens

<400> 44

Tyr Leu Asn Lys Glu Ile Glu Glu Ala
1               5

<210> 45
<211> 9
<212> PRT
<213> Homo sapiens

<400> 45

Asp Glu Leu Pro Lys Phe His Gln Tyr
1               5

<210> 46
<211> 10
<212> PRT
<213> Homo sapiens

<400> 46

Asp Val Thr Gly Gln Phe Pro Ser Ser Phe
1               5                   10

<210> 47

<211> 9
<212> PRT
<213> Homo sapiens

<400> 47

Glu His Ser Arg Val Leu Gln Gln Leu
1                   5

<210> 48
<211> 8
<212> PRT
<213> Homo sapiens

<400> 48

Ile Lys Val Ser Lys Gln Leu Leu
1                   5

<210> 49
<211> 9
<212> PRT
<213> Homo sapiens

<400> 49

Lys Pro Arg Gln Ser Ser Pro Gln Leu
1                   5

<210> 50
<211> 9
<212> PRT
<213> Homo sapiens

<400> 50

Lys Gln Leu Leu Ala Ala Leu Glu Ile
1                   5

<210> 51
<211> 9
<212> PRT
<213> Homo sapiens

<400> 51

Arg Arg Lys Asp Leu Val Leu Lys Tyr
1                   5

<210> 52
<211> 11
<212> PRT
<213> Homo sapiens

<400> 52

Arg Thr Arg Asp Tyr Ala Ser Leu Pro Pro Lys
1                   5                   10

<210> 53
<211> 10
<212> PRT
<213> Homo sapiens

<400> 53

```
Ala Pro Gly Ser Val Leu Pro Arg Ala Leu
1               5                   10
```

<210> 54
<211> 8
<212> PRT
<213> Homo sapiens

<400> 54

```
Asp Ile Lys Glu His Pro Leu Leu
1               5
```

<210> 55
<211> 9
<212> PRT
<213> Homo sapiens

<400> 55

```
Asp Ser Ala Gly Pro Gln Asp Ala Arg
1               5
```

<210> 56
<211> 9
<212> PRT
<213> Homo sapiens

<400> 56

```
Phe Gln Tyr Ala Lys Glu Ser Tyr Ile
1               5
```

<210> 57
<211> 9
<212> PRT
<213> Homo sapiens

<400> 57

```
Lys Val Leu Ser Trp Pro Phe Leu Met
1               5
```

<210> 58
<211> 9
<212> PRT
<213> Homo sapiens

<400> 58

Leu Glu Asn Asp Gln Ser Leu Ser Phe
1               5

<210> 59
<211> 8
<212> PRT
<213> Homo sapiens


<400> 59

Ser Pro Ser Arg Gln Pro Gln Val
1               5

<210> 60
<211> 9
<212> PRT
<213> Homo sapiens


<400> 60

Ser Arg His Gln Ser Phe Thr Thr Lys
1               5

<210> 61
<211> 9
<212> PRT
<213> Homo sapiens


<400> 61

Ser Ser His Asn Ala Ser Lys Thr Leu
1               5

<210> 62
<211> 9
<212> PRT
<213> Homo sapiens


<400> 62

Glu Glu Ile Asp Thr Thr Met Arg Trp
1               5

<210> 63
<211> 9
<212> PRT
<213> Homo sapiens


<400> 63

Ile Leu Asp Glu Lys Pro Val Ile Ile
1               5

<210> 64
<211> 9
<212> PRT
<213> Homo sapiens

<400> 64

```
Leu Pro Gln Glu Pro Arg Thr Ser Leu
1               5
```

<210> 65
<211> 8
<212> PRT
<213> Homo sapiens

<400> 65

```
Leu Thr Tyr Lys Leu Pro Val Ala
1               5
```

<210> 66
<211> 10
<212> PRT
<213> Homo sapiens

<400> 66

```
Asn Glu Met Glu Leu Ala His Ser Ser Phe
1               5                   10
```

<210> 67
<211> 10
<212> PRT
<213> Homo sapiens

<400> 67

```
Arg Glu Phe Pro Glu Ala Asn Phe Glu Leu
1               5                   10
```

<210> 68
<211> 9
<212> PRT
<213> Homo sapiens

<400> 68

```
Thr His His Ile Pro Asp Ala Lys Leu
1               5
```

<210> 69
<211> 9
<212> PRT
<213> Homo sapiens

<400> 69

```
Thr Val Lys Glu Asn Leu Ser Leu Phe
1               5
```

<210> 70
<211> 8
<212> PRT

<213> Homo sapiens

<400> 70

```
Val Leu Leu Lys Lys Ala Val Leu
1               5
```

<210> 71
<211> 9
<212> PRT
<213> Homo sapiens

<400> 71

```
His Leu Lys Ser Ile Pro Val Ser Leu
1               5
```

<210> 72
<211> 9
<212> PRT
<213> Homo sapiens

<400> 72

```
Lys Val Trp Tyr Asn Val Glu Asn Trp
1               5
```

<210> 73
<211> 9
<212> PRT
<213> Homo sapiens

<400> 73

```
Leu Pro Ala Tyr Arg Ala Gln Leu Leu
1               5
```

<210> 74
<211> 9
<212> PRT
<213> Homo sapiens

<400> 74

```
Leu Ser Glu Gln Thr Ser Val Pro Leu
1               5
```

<210> 75
<211> 9
<212> PRT
<213> Homo sapiens

<400> 75

```
Ser Leu Asn Gln Trp Leu Val Ser Phe
1               5
```

<210> 76

<211> 9
<212> PRT
<213> Homo sapiens

<400> 76

```
Ser Met Thr Ser Leu Ala Gln Lys Ile
1               5
```

<210> 77
<211> 9
<212> PRT
<213> Homo sapiens

<400> 77

```
Ser Ser Ser Gly Leu His Pro Pro Lys
1               5
```

<210> 78
<211> 9
<212> PRT
<213> Homo sapiens

<400> 78

```
Asp Leu Asp Val Lys Lys Met Pro Leu
1               5
```

<210> 79
<211> 9
<212> PRT
<213> Homo sapiens

<400> 79

```
Phe Tyr Thr Val Ile Pro His Asn Phe
1               5
```

<210> 80
<211> 10
<212> PRT
<213> Homo sapiens

<400> 80

```
His His Ile Asn Thr Asp Asn Pro Ser Leu
1               5                   10
```

<210> 81
<211> 9
<212> PRT
<213> Homo sapiens

<400> 81

```
Arg Val Gly Glu Val Gly Gln Ser Lys
1               5
```

<210> 82
<211> 11
<212> PRT
<213> Homo sapiens

<400> 82

```
Ala Val Phe Asp Gly Ala Gln Val Thr Ser Lys
1               5               10
```

<210> 83
<211> 9
<212> PRT
<213> Homo sapiens

<400> 83

```
Ser Gln Thr Asp Leu Val Ser Arg Leu
1               5
```

<210> 84
<211> 9
<212> PRT
<213> Homo sapiens

<400> 84

```
Val Pro Val Pro His Thr Thr Ala Leu
1               5
```

<210> 85
<211> 9
<212> PRT
<213> Homo sapiens

<400> 85

```
Tyr Gln Val Leu Asp Val Gln Arg Tyr
1               5
```

<210> 86
<211> 10
<212> PRT
<213> Homo sapiens

<400> 86

```
Ala Pro Phe Gln Gly Asp Gln Arg Ser Leu
1               5               10
```

<210> 87
<211> 9
<212> PRT
<213> Homo sapiens

<400> 87

Asp Val Ala Glu Pro Tyr Lys Val Tyr
1               5

<210> 88
<211> 10
<212> PRT
<213> Homo sapiens

<400> 88

Ile Val Ser Gly Gln Pro Gly Thr Gln Lys
1               5                    10

<210> 89
<211> 9
<212> PRT
<213> Homo sapiens

<400> 89

Thr Pro Glu Gln Gln Ala Ala Ile Leu
1               5

<210> 90
<211> 9
<212> PRT
<213> Homo sapiens

<400> 90

Val Glu Leu Phe Arg Thr Ala Tyr Phe

                1               5

<210> 91
<211> 9
<212> PRT
<213> Homo sapiens

<400> 91

Glu His Ala Asp Asp Asp Pro Ser Leu
1               5

<210> 92
<211> 10
<212> PRT
<213> Homo sapiens

<400> 92

Ser Glu Glu Ser Val Lys Ser Thr Thr Leu
1               5                    10

<210> 93
<211> 10

<212> PRT
<213> Homo sapiens

<400> 93

```
Ser Pro Arg Pro Pro Leu Gly Ser Ser Leu
1               5                   10
```

<210> 94
<211> 8
<212> PRT
<213> Homo sapiens

<400> 94

```
Ser Pro Trp Trp Arg Ser Ser Leu
1               5
```

<210> 95
<211> 10
<212> PRT
<213> Homo sapiens

<400> 95

```
Val Tyr Thr Pro Val Asp Ser Leu Val Phe
1               5                   10
```

<210> 96
<211> 9
<212> PRT
<213> Homo sapiens

<400> 96

```
Ala Pro Leu Gln Arg Ser Gln Ser Leu
1               5
```

<210> 97
<211> 8
<212> PRT
<213> Homo sapiens

<400> 97

```
Asp Glu Val His Gln Asp Thr Tyr
1               5
```

<210> 98
<211> 9
<212> PRT
<213> Homo sapiens

<400> 98

```
Leu Pro His Ser Ala Thr Val Thr Leu
1               5
```

<210> 99
<211> 9
<212> PRT
<213> Homo sapiens

<400> 99

Ser Glu Ala Pro Glu Ala Pro Leu Leu
1               5

<210> 100
<211> 9
<212> PRT
<213> Homo sapiens

<400> 100

Ser Pro Arg Ala Ser Gly Ser Gly Leu
1               5

<210> 101
<211> 9
<212> PRT
<213> Homo sapiens

<400> 101

Val Val Gly Pro Ala Ala Glu Ala Lys
1               5

<210> 102
<211> 9
<212> PRT
<213> Homo sapiens

<400> 102

Phe Ser Ile Thr Lys Ser Val Glu Leu
1               5

<210> 103
<211> 9
<212> PRT
<213> Homo sapiens

<400> 103

Gly Gln Thr Lys Asn Asp Leu Val Val
1               5

<210> 104
<211> 8
<212> PRT
<213> Homo sapiens

<400> 104

Leu Ser Gln Glu Val Cys Arg Asp
1               5

<210> 105
<211> 9
<212> PRT
<213> Homo sapiens

<400> 105

Arg Asp Ile Gln Ser Pro Glu Gln Ile
1               5

<210> 106
<211> 9
<212> PRT
<213> Homo sapiens

<400> 106

Arg Glu Asp Asn Ser Ser Asn Ser Leu
1               5

<210> 107
<211> 8
<212> PRT
<213> Homo sapiens

<400> 107

Thr Glu His Gln Glu Pro Gly Leu
1               5

<210> 108
<211> 9
<212> PRT
<213> Homo sapiens

<400> 108

Thr Lys Asn Asp Leu Val Val Ser Leu
1               5

<210> 109
<211> 9
<212> PRT
<213> Homo sapiens

<400> 109

Ala Glu Glu Ala Gly Gly Thr Arg Leu
1               5

<210> 110
<211> 8
<212> PRT
<213> Homo sapiens

<400> 110

```
Glu Asn Val Asn Lys Lys Asp Tyr
1               5
```

<210> 111
<211> 10
<212> PRT
<213> Homo sapiens

<400> 111

```
Gly Leu Asp Pro Asn Lys Pro Pro Glu Leu
1               5               10
```

<210> 112
<211> 12
<212> PRT
<213> Homo sapiens

<400> 112

```
Arg Pro Ala Gly Glu Pro Tyr Asn Arg Lys Thr Leu
1               5               10
```

<210> 113
<211> 11
<212> PRT
<213> Homo sapiens

<400> 113

```
Ser Ala Ser Val Gln Arg Ala Asp Thr Ser Leu
1               5               10
```

<210> 114
<211> 9
<212> PRT
<213> Homo sapiens

<400> 114

```
Tyr Gly Asn Pro Arg Thr Asn Gly Met
1               5
```

<210> 115
<211> 9
<212> PRT
<213> Homo sapiens

<400> 115

```
Leu Ile Arg Pro Val Ser Ala Ser Phe
1               5
```

<210> 116
<211> 11
<212> PRT

<213> Homo sapiens

<400> 116

Ser Pro Val Asn Ser Ser Lys Gln Pro Ser Tyr
1               5               10

<210> 117
<211> 10
<212> PRT
<213> Homo sapiens

<400> 117

Gln Leu Phe Ser Tyr Ala Ile Leu Gly Phe
1               5               10

<210> 118
<211> 9
<212> PRT
<213> Homo sapiens

<400> 118

Asp Glu His Leu Leu Ile Gln His Tyr
1               5

<210> 119
<211> 9
<212> PRT
<213> Homo sapiens

<400> 119

Lys Gln Val Ala Ser Ser Thr Gly Phe
1               5

<210> 120
<211> 11
<212> PRT
<213> Homo sapiens

<400> 120

Arg Asp Phe Gly Pro Ala Ser Gln His Phe Leu
1               5               10

<210> 121
<211> 9
<212> PRT
<213> Homo sapiens

<400> 121

Arg Gln Leu Gly Glu Val Ala Ser Phe
1               5

<210> 122

<211> 10
<212> PRT
<213> Homo sapiens

<400> 122

```
Thr Glu Ala Glu Thr Thr Ala Asn Val Leu
1               5                   10
```

<210> 123
<211> 9
<212> PRT
<213> Homo sapiens

<400> 123

```
Gly Tyr Leu Pro Val Gln Thr Val Leu
1               5
```

<210> 124
<211> 9
<212> PRT
<213> Homo sapiens

<400> 124

```
Gly Gln Lys Glu Ala Leu Leu Lys Tyr
1               5
```

<210> 125
<211> 9
<212> PRT
<213> Homo sapiens

<400> 125

```
Lys Pro Ser Glu Glu Arg Lys Thr Ile
1               5
```

<210> 126
<211> 9
<212> PRT
<213> Homo sapiens

<400> 126

```
Lys Gln Thr Pro Lys Val Leu Val Val
1               5
```

<210> 127
<211> 9
<212> PRT
<213> Homo sapiens

<400> 127

```
Ser Val Ile Gln His Val Gln Ser Phe
1               5
```

<210> 128
<211> 9
<212> PRT
<213> Homo sapiens

<400> 128

```
Thr Pro Ile Glu Arg Ile Pro Tyr Leu
1               5
```

<210> 129
<211> 9
<212> PRT
<213> Homo sapiens

<400> 129

```
Ala Glu Val Glu Lys Asn Glu Thr Val
1               5
```

<210> 130
<211> 9
<212> PRT
<213> Homo sapiens

<400> 130

```
Glu Val Lys Glu Glu Ile Pro Leu Val
1               5
```

<210> 131
<211> 9
<212> PRT
<213> Homo sapiens

<400> 131

```
Lys Pro Thr Ser Ala Arg Ser Gly Leu
1               5
```

<210> 132
<211> 10
<212> PRT
<213> Homo sapiens

<400> 132

```
Lys Tyr Ile Glu Thr Thr Pro Leu Thr Ile
1               5                   10
```

<210> 133
<211> 9
<212> PRT
<213> Homo sapiens

<400> 133

```
                              Ser Glu Ile Lys Thr Ser Ile Glu Val
                              1               5
```

<210> 134
<211> 9
<212> PRT
<213> Homo sapiens


<400> 134

```
                              Ser Val Lys Pro Thr Ser Ala Thr Lys
                              1               5
```

<210> 135
<211> 9
<212> PRT
<213> Homo sapiens


<400> 135

```
                              Tyr Pro Asn Lys Gly Val Gly Gln Ala
                              1               5
```

<210> 136
<211> 9
<212> PRT
<213> Homo sapiens


<400> 136

```
                              Ile Ser Met Lys Ile Leu Asn Ser Leu
                              1               5
```

<210> 137
<211> 10
<212> PRT
<213> Homo sapiens


<400> 137

```
                            Lys Thr Ile Ala Phe Leu Leu Pro Met Phe
                            1               5                   10
```

<210> 138
<211> 8
<212> PRT
<213> Homo sapiens


<400> 138

```
                              Arg Asp Ser Ile Ile Asn Asp Phe
                              1               5
```

<210> 139
<211> 10
<212> PRT
<213> Homo sapiens

<400> 139

```
Ser Val Lys Gly Gly Gly Gly Asn Glu Lys
1               5                   10
```

<210> 140
<211> 9
<212> PRT
<213> Homo sapiens

<400> 140

```
Gly Ile Ala Lys Thr Gly Ser Gly Lys
1               5
```

<210> 141
<211> 10
<212> PRT
<213> Homo sapiens

<400> 141

```
Ala Glu Thr Thr Asp Asn Val Phe Thr Leu
1               5                   10
```

<210> 142
<211> 9
<212> PRT
<213> Homo sapiens

<400> 142

```
Ser Glu Tyr Gln Arg Phe Ala Val Met
1               5
```

<210> 143
<211> 9
<212> PRT
<213> Homo sapiens

<400> 143

```
Thr Phe Gly Glu Arg Val Val Ala Phe
1               5
```

<210> 144
<211> 8
<212> PRT
<213> Homo sapiens

<400> 144

```
Asn Glu Asn Leu Val Glu Arg Phe
1               5
```

<210> 145
<211> 9
<212> PRT

<213> Homo sapiens

<400> 145

Lys Ile Thr Val Pro Ala Ser Gln Lys
1               5

<210> 146
<211> 10
<212> PRT
<213> Homo sapiens

<400> 146

Lys Ile Thr Val Pro Ala Ser Gln Lys Leu
1               5                   10

<210> 147
<211> 10
<212> PRT
<213> Homo sapiens

<400> 147

Val Pro Ala Ser Gln Lys Leu Arg Gln Leu
1               5                   10

<210> 148
<211> 9
<212> PRT
<213> Homo sapiens

<400> 148

His Val Gly Tyr Thr Leu Ser Tyr Lys
1               5

<210> 149
<211> 10
<212> PRT
<213> Homo sapiens

<400> 149

Lys Leu Pro Leu Pro Leu Pro Pro Arg Leu
1               5                   10

<210> 150
<211> 9
<212> PRT
<213> Homo sapiens

<400> 150

Lys Pro Ile Glu Pro Arg Arg Glu Leu
1               5

<210> 151

<211> 9
<212> PRT
<213> Homo sapiens

<400> 151

```
                        Ser His Ser His Val Gly Tyr Thr Leu
                        1                   5
```

<210> 152
<211> 9
<212> PRT
<213> Homo sapiens

<400> 152

```
                        Ala Pro Ser Glu Tyr Arg Tyr Thr Leu
                        1                   5
```

<210> 153
<211> 10
<212> PRT
<213> Homo sapiens

<400> 153

```
                Ala Pro Ser Glu Tyr Arg Tyr Thr Leu Leu

                1                   5                   10
```

<210> 154
<211> 9
<212> PRT
<213> Homo sapiens

<400> 154

```
                        Glu Ile Phe Gln Asn Glu Val Ala Arg
                        1                   5
```

<210> 155
<211> 9
<212> PRT
<213> Homo sapiens

<400> 155

```
                        Lys Asp Val Leu Ile Pro Gly Lys Leu
                        1                   5
```

<210> 156
<211> 9
<212> PRT
<213> Homo sapiens

<400> 156

```
Val Pro Leu Val Arg Glu Ile Thr Phe
1               5
```

<210> 157
<211> 9
<212> PRT
<213> Homo sapiens

<400> 157

```
Asp Pro Asn Pro Asn Phe Glu Lys Phe
1               5
```

<210> 158
<211> 9
<212> PRT
<213> Homo sapiens

<400> 158

```
Ile Gln Ala Pro Leu Ser Trp Glu Leu
1               5
```

<210> 159
<211> 10
<212> PRT
<213> Homo sapiens

<400> 159

```
Val Ile Tyr Asn Glu Gln Met Ala Ser Lys
1               5                   10
```

<210> 160
<211> 9
<212> PRT
<213> Homo sapiens

<400> 160

```
Val Leu Arg Pro Gly Gly Ala Phe Tyr
1               5
```

<210> 161
<211> 9
<212> PRT
<213> Homo sapiens

<400> 161

```
Glu Asp Pro Asp Gln Asp Ile Leu Ile
1               5
```

<210> 162
<211> 9
<212> PRT
<213> Homo sapiens

<400> 162

```
His Gly Asn Leu Arg Glu Leu Ala Leu
1               5
```

<210> 163
<211> 9
<212> PRT
<213> Homo sapiens

<400> 163

```
Lys Leu Tyr Pro Thr Leu Val Ile Arg
1               5
```

<210> 164
<211> 9
<212> PRT
<213> Homo sapiens

<400> 164

```
Ser Glu Glu Thr Phe Arg Phe Glu Leu
1               5
```

<210> 165
<211> 9
<212> PRT
<213> Homo sapiens

<400> 165

```
Glu Leu Asn Lys Leu Leu Glu Glu Ile
1               5
```

<210> 166
<211> 9
<212> PRT
<213> Homo sapiens

<400> 166

```
Ile Pro Phe Ser Asn Pro Arg Val Leu
1               5
```

<210> 167
<211> 10
<212> PRT
<213> Homo sapiens

<400> 167

```
Leu Leu Asp Glu Gly Ala Lys Leu Leu Tyr
1               5                   10
```

<210> 168
<211> 9
<212> PRT

<213> Homo sapiens

<400> 168

```
                              Ser Pro Ala Asp Ala His Arg Asn Leu
                              1               5
```

<210> 169
<211> 9
<212> PRT
<213> Homo sapiens

<400> 169

```
                              Ala Glu Leu Glu Arg Gln Ala Val Leu
                              1               5
```

<210> 170
<211> 9
<212> PRT
<213> Homo sapiens

<400> 170

```
                              Gly Arg Val Pro Gly Pro Leu Ser Leu
                              1               5
```

<210> 171
<211> 9
<212> PRT
<213> Homo sapiens

<400> 171

```
                              Ser Asp Leu Ala Arg Leu Ile Leu Leu
                              1               5
```

<210> 172
<211> 9
<212> PRT
<213> Homo sapiens

<400> 172

```
                              Thr Pro Ile Arg Glu Gln His Val Leu
                              1               5
```

<210> 173
<211> 8
<212> PRT
<213> Homo sapiens

<400> 173

```
                               Ala Pro Arg Lys Gly Asn Thr Leu
                               1               5
```

<210> 174

<211> 10
<212> PRT
<213> Homo sapiens

<400> 174

```
Glu Glu Glu Glu Ala Leu Gln Lys Lys Phe
1               5                   10
```

<210> 175
<211> 8
<212> PRT
<213> Homo sapiens

<400> 175

```
Lys Glu Asn Leu Val Asp Gly Phe
1               5
```

<210> 176
<211> 10
<212> PRT
<213> Homo sapiens

<400> 176

```
Val Tyr Lys Glu Asn Leu Val Asp Gly Phe
1               5                   10
```

<210> 177
<211> 9
<212> PRT
<213> Homo sapiens

<400> 177

```
Thr Leu Leu Val Val Val Pro Lys Leu
1               5
```

<210> 178
<211> 9
<212> PRT
<213> Homo sapiens

<400> 178

```
His Glu Ile Asp Arg Tyr Thr Ala Ile
1               5
```

<210> 179
<211> 9
<212> PRT
<213> Homo sapiens

<400> 179

```
Val Phe Thr Leu Lys Pro Leu Glu Phe
1               5
```

<210> 180
<211> 8
<212> PRT
<213> Homo sapiens

<400> 180

Tyr Trp Val Pro Arg Asn Ala Leu
1               5

<210> 181
<211> 9
<212> PRT
<213> Homo sapiens

<400> 181

Ile Gly Val Glu His Val Val Val Tyr
1               5

<210> 182
<211> 8
<212> PRT
<213> Homo sapiens

<400> 182

Arg Asp Lys Pro His Val Asn Val
1               5

<210> 183
<211> 9
<212> PRT
<213> Homo sapiens

<400> 183

Ala Asp Val Leu Lys Val Glu Val Phe
1               5

<210> 184
<211> 8
<212> PRT
<213> Homo sapiens

<400> 184

Ile Pro Val Val His Ala Ser Ile
1               5

<210> 185
<211> 9
<212> PRT
<213> Homo sapiens

<400> 185

Arg Asp Ser Leu Ile Asp Ser Leu Thr
1                   5

<210> 186
<211> 11
<212> PRT
<213> Homo sapiens

<400> 186

Thr Val Ala Asp Gln Val Leu Val Gly Ser Tyr
1                   5                   10

<210> 187
<211> 9
<212> PRT
<213> Homo sapiens

<400> 187

Ala Ala Asp Thr Glu Arg Leu Ala Leu
1                   5

<210> 188
<211> 9
<212> PRT
<213> Homo sapiens

<400> 188

Asp Met Lys Ala Lys Val Ala Ser Leu
1                   5

<210> 189
<211> 9
<212> PRT
<213> Homo sapiens

<400> 189

His Val Leu Glu Glu Val Gln Gln Val
1                   5

<210> 190
<211> 9
<212> PRT
<213> Homo sapiens

<400> 190

Lys Glu Ala Ala Asp Thr Glu Arg Leu
1                   5

<210> 191
<211> 9
<212> PRT
<213> Homo sapiens

<400> 191

```
Arg Ile Ser Glu Val Leu Gln Lys Leu
1               5
```

<210> 192
<211> 9
<212> PRT
<213> Homo sapiens

<400> 192

```
Thr Glu Val Arg Glu Leu Val Ser Leu
1               5
```

<210> 193
<211> 10
<212> PRT
<213> Homo sapiens

<400> 193

```
Ala Ile Arg Ser Gly Glu Ala Ala Ala Lys
1               5                   10
```

<210> 194
<211> 9
<212> PRT
<213> Homo sapiens

<400> 194

```
Ala Pro Asn Pro Ala Pro Lys Glu Leu
1               5
```

<210> 195
<211> 8
<212> PRT
<213> Homo sapiens

<400> 195

```
Arg Gln Ser Leu Leu Thr Ala Ile
1               5
```

<210> 196
<211> 9
<212> PRT
<213> Homo sapiens

<400> 196

```
Ser Pro Glu Gln Thr Leu Ser Pro Leu
1               5
```

<210> 197
<211> 9
<212> PRT

<213> Homo sapiens

<400> 197

Thr Glu His Gln Val Pro Ser Ser Val
1                   5

<210> 198
<211> 9
<212> PRT
<213> Homo sapiens

<400> 198

Thr Thr Tyr Lys Ile Val Pro Pro Lys
1                   5

<210> 199
<211> 9
<212> PRT
<213> Homo sapiens

<400> 199

Gln Leu Leu Asp Gln Val Glu Gln Ile
1                   5

<210> 200
<211> 9
<212> PRT
<213> Homo sapiens

<400> 200

Asp Glu Thr Met Val Ile Gly Asn Tyr
1                   5

<210> 201
<211> 11
<212> PRT
<213> Homo sapiens

<400> 201

Arg Gln Tyr Gly Ser Glu Gly Arg Phe Thr Phe
1                   5                   10

<210> 202
<211> 8
<212> PRT
<213> Homo sapiens

<400> 202

Ser Pro Ala Pro Arg Thr Ala Leu
1                   5

<210> 203

<211> 10
<212> PRT
<213> Homo sapiens

<400> 203

```
Gly Pro Arg Pro Ile Thr Gln Ser Glu Leu
1               5                   10
```

<210> 204
<211> 9
<212> PRT
<213> Homo sapiens

<400> 204

```
Lys Pro Glu Pro Val Asp Lys Val Ala
1               5
```

<210> 205
<211> 9
<212> PRT
<213> Homo sapiens

<400> 205

```
Thr Pro Ser Ser Arg Pro Ala Ser Leu
1               5
```

<210> 206
<211> 9
<212> PRT
<213> Homo sapiens

<400> 206

```
Asp Glu Thr Gln Val Arg Ser Leu Tyr
1               5
```

<210> 207
<211> 11
<212> PRT
<213> Homo sapiens

<400> 207

```
Lys Glu Glu Glu Thr Asn Ser Val Ala Thr Leu
1               5                   10
```

<210> 208
<211> 9
<212> PRT
<213> Homo sapiens

<400> 208

```
Leu Glu Gln Lys Val Val Glu Leu Tyr
1               5
```

<210> 209
<211> 11
<212> PRT
<213> Homo sapiens

<400> 209

```
Asn Pro Ile Ser Asn Ala Val Leu Asn Glu Tyr
1               5                   10
```

<210> 210
<211> 8
<212> PRT
<213> Homo sapiens

<400> 210

```
Ser Ile Lys Glu Lys Ser Ser Leu
1               5
```

<210> 211
<211> 11
<212> PRT
<213> Homo sapiens

<400> 211

```
Thr Glu Ile Thr Glu Ile Ser Thr Pro Ser Leu
1               5                   10
```

<210> 212
<211> 9
<212> PRT
<213> Homo sapiens

<400> 212

```
Gly Arg Leu Asn Ser Val Asn Asn Arg
1               5
```

<210> 213
<211> 9
<212> PRT
<213> Homo sapiens

<400> 213

```
Ser Ile Leu Glu Asp Pro Pro Ser Ile
1               5
```

<210> 214
<211> 9
<212> PRT
<213> Homo sapiens

<400> 214

```
Thr Pro Arg Thr Asn Asn Ile Glu Leu
1               5
```

<210> 215
<211> 9
<212> PRT
<213> Homo sapiens

<400> 215

```
Asp Ala Met Lys Arg Val Glu Glu Ile
1               5
```

<210> 216
<211> 9
<212> PRT
<213> Homo sapiens

<400> 216

```
Asp Ile Lys Glu Val Lys Gln Asn Ile


            1               5
```

<210> 217
<211> 9
<212> PRT
<213> Homo sapiens

<400> 217

```
Gly Pro Ile Tyr Pro Gly His Gly Met
1               5
```

<210> 218
<211> 9
<212> PRT
<213> Homo sapiens

<400> 218

```
Gly Asp Tyr Gly Arg Ala Phe Asn Leu
1               5
```

<210> 219
<211> 9
<212> PRT
<213> Homo sapiens

<400> 219

```
Thr Arg His Lys Ile Val His Thr Lys
1               5
```

<210> 220
<211> 10

<212> PRT
<213> Homo sapiens

<400> 220

Arg Ile His Thr Gly Glu Lys Pro Tyr Lys
1               5                   10

<210> 221
<211> 9
<212> PRT
<213> Homo sapiens

<400> 221

Lys Ala Phe Asn Trp Phe Ser Thr Leu
1               5

<210> 222
<211> 9
<212> PRT
<213> Homo sapiens

<400> 222

Gln Ser Thr Gln Arg Ser Leu Ala Leu
1               5

<210> 223
<211> 11
<212> PRT
<213> Homo sapiens

<400> 223

Arg Asp Leu Gln Met Asn Gln Ala Leu Arg Phe
1               5                   10

<210> 224
<211> 10
<212> PRT
<213> Homo sapiens

<400> 224

Arg Glu Leu Glu Ser Gln Leu His Val Leu
1               5                   10

<210> 225
<211> 9
<212> PRT
<213> Homo sapiens

<400> 225

Ser Glu Ala Glu Lys Leu Thr Leu Val
1               5

<210> 226
<211> 9
<212> PRT
<213> Homo sapiens

<400> 226

```
Ala Ala Ala Lys Pro Val Ala Thr Lys
1               5
```

<210> 227
<211> 10
<212> PRT
<213> Homo sapiens

<400> 227

```
Ala Thr Tyr His Gly Ser Phe Ser Thr Lys
1               5                   10
```

<210> 228
<211> 9
<212> PRT
<213> Homo sapiens

<400> 228

```
Phe Met Tyr Asp Arg Pro Leu Arg Leu
1               5
```

<210> 229
<211> 9
<212> PRT
<213> Homo sapiens

<400> 229

```
Phe Arg Val Gly Asn Val Gln Glu Leu
1               5
```

<210> 230
<211> 9
<212> PRT
<213> Homo sapiens

<400> 230

```
Gly Val Ala Pro Phe Thr Ile Ala Arg
1               5
```

<210> 231
<211> 11
<212> PRT
<213> Homo sapiens

<400> 231

Lys Met Lys Pro Leu Asp Gly Ser Ala Leu Tyr
1                   5                   10

<210> 232
<211> 9
<212> PRT
<213> Homo sapiens

<400> 232

Lys Pro Ala Pro Ala Lys Pro Val Ala
1                   5

<210> 233
<211> 9
<212> PRT
<213> Homo sapiens

<400> 233

Lys Pro Val Ala Ala Lys Pro Ala Ala
1                   5

<210> 234
<211> 10
<212> PRT
<213> Homo sapiens

<400> 234

Lys Gln Phe Gly Val Ala Pro Phe Thr Ile
1                   5                   10

<210> 235
<211> 9
<212> PRT
<213> Homo sapiens

<400> 235

Gln Glu Glu Leu Val Lys Ile Ser Leu
1                   5

<210> 236
<211> 10
<212> PRT
<213> Homo sapiens

<400> 236

Arg Gln Leu Gly Thr Val Gln Gln Val Ile
1                   5                   10

<210> 237
<211> 9
<212> PRT
<213> Homo sapiens

<400> 237

```
Arg Gln Leu Ile Asn Ala Leu Gln Ile
1               5
```

<210> 238
<211> 12
<212> PRT
<213> Homo sapiens

<400> 238

```
Arg Val Ile Gly Gly Leu Leu Ala Gly Gln Thr Tyr
1             5                   10
```

<210> 239
<211> 9
<212> PRT
<213> Homo sapiens

<400> 239

```
Ser Glu Asn Ala Phe Tyr Leu Ser Pro
1               5
```

<210> 240
<211> 9
<212> PRT
<213> Homo sapiens

<400> 240

```
Ser Gln Ala Pro Val Leu Asp Ala Ile
1               5
```

<210> 241
<211> 9
<212> PRT
<213> Homo sapiens

<400> 241

```
Ser Thr Arg Tyr Pro Pro Pro Ala Val
1               5
```

<210> 242
<211> 9
<212> PRT
<213> Homo sapiens

<400> 242

```
Thr Glu Asp Thr Leu Lys Val Tyr Leu
1               5
```

<210> 243
<211> 9
<212> PRT

<213> Homo sapiens

<400> 243

Val Ala Ala Lys Pro Val Ala Thr Lys
1                   5

<210> 244
<211> 8
<212> PRT
<213> Homo sapiens

<400> 244

Val Gln Arg Val Val Glu Ser Leu
1                   5

<210> 245
<211> 9
<212> PRT
<213> Homo sapiens

<400> 245

Val Arg Asn Pro Ser Val Val Val Lys
1                   5

<210> 246
<211> 9
<212> PRT
<213> Homo sapiens

<400> 246

Gly Glu Ser Glu Val Ala Ile Lys Ile
1                   5

<210> 247
<211> 10
<212> PRT
<213> Homo sapiens

<400> 247

Leu Ile Tyr Ser Val Gly Leu Leu Leu Ala
1                   5                   10

<210> 248
<211> 9
<212> PRT
<213> Homo sapiens

<400> 248

Ser Ala Tyr Pro His Gln Leu Ser Phe
1                   5

<210> 249

<211> 9
<212> PRT
<213> Homo sapiens

<400> 249

```
Ser Val Ile Gly Val Phe Ile Thr Lys
1               5
```

<210> 250
<211> 10
<212> PRT
<213> Homo sapiens

<400> 250

```
Ala Glu Leu Gly Asn Ser Val Gln Leu Ile
1               5                   10
```

<210> 251
<211> 8
<212> PRT
<213> Homo sapiens

<400> 251

```
Ala Asn Met Thr Val Thr Arg Ile
1               5
```

<210> 252
<211> 9
<212> PRT
<213> Homo sapiens

<400> 252

```
Ala Arg Ile Ser Asn Val Glu Phe Tyr
1               5
```

<210> 253
<211> 9
<212> PRT
<213> Homo sapiens

<400> 253

```
Ala Val Phe Ile Gly Asn Gln Gln Phe
1               5
```

<210> 254
<211> 9
<212> PRT
<213> Homo sapiens

<400> 254

```
Asp Ile Glu Leu Gln Ala Glu Asn Ile
1               5
```

<210> 255
<211> 9
<212> PRT
<213> Homo sapiens

<400> 255

```
Asp Ser Tyr Thr Val Arg Val Ser Val
1               5
```

<210> 256
<211> 9
<212> PRT
<213> Homo sapiens

<400> 256

```
Asp Val Lys Ile Phe Val Asn Thr Ile
1               5
```

<210> 257
<211> 9
<212> PRT
<213> Homo sapiens

<400> 257

```
Glu Ile Ile Pro Lys Tyr Gly Ser Ile
1               5
```

<210> 258
<211> 9
<212> PRT
<213> Homo sapiens

<400> 258

```
Glu Gln Ser Lys Ile Phe Ile His Arg
1               5
```

<210> 259
<211> 9
<212> PRT
<213> Homo sapiens

<400> 259

```
Phe Val Asp Val Gly Leu Tyr Gln Tyr
1               5
```

<210> 260
<211> 9
<212> PRT
<213> Homo sapiens

<400> 260

```
Gly His Thr Ser Thr Ile Ser Thr Leu
1               5
```

<210> 261
<211> 9
<212> PRT
<213> Homo sapiens

<400> 261

```
Gly Arg Ile Glu Tyr Val Glu Val Phe
1               5
```

<210> 262
<211> 9
<212> PRT
<213> Homo sapiens

<400> 262

```
Gly Thr Ser Ile Ile Pro Phe Gln Lys
1               5
```

<210> 263
<211> 9
<212> PRT
<213> Homo sapiens

<400> 263

```
His Pro Phe Leu Arg Gly Ile Gly Tyr
1               5
```

<210> 264
<211> 8
<212> PRT
<213> Homo sapiens

<400> 264

```
Ile Pro Val Glu Ile His Thr Ala
1               5
```

<210> 265
<211> 9
<212> PRT
<213> Homo sapiens

<400> 265

```
Lys Ile Phe Val Asn Thr Ile Ala Tyr
1               5
```

<210> 266
<211> 10
<212> PRT
<213> Homo sapiens

<400> 266

Leu Pro Glu Asp Lys Val Arg Ile Ala Tyr
1               5                   10

<210> 267
<211> 9
<212> PRT
<213> Homo sapiens

<400> 267

Leu Pro Phe Ser Glu Gly Leu Thr Val
1               5

<210> 268
<211> 9
<212> PRT
<213> Homo sapiens

<400> 268

Leu Pro Trp Ala Asn Lys Val Thr Ile
1               5

<210> 269
<211> 8
<212> PRT
<213> Homo sapiens

<400> 269

Pro Trp Ala Asn Lys Val Thr Ile
1               5

<210> 270
<211> 9
<212> PRT
<213> Homo sapiens

<400> 270

Gln Ala Tyr Asn Arg Ala Val Thr Ile
1               5

<210> 271
<211> 9
<212> PRT
<213> Homo sapiens

<400> 271

Arg Ser Phe Pro Gln Lys Met Ala Tyr
1               5

<210> 272
<211> 9
<212> PRT

<213> Homo sapiens

<400> 272

Arg Tyr Pro Ile His Trp His Leu Leu
1               5

<210> 273
<211> 9
<212> PRT
<213> Homo sapiens

<400> 273

Ser Pro Gln Asn Leu Arg Leu Met Leu
1               5

<210> 274
<211> 9
<212> PRT
<213> Homo sapiens

<400> 274

Ser Tyr Phe Ser Ser Pro Thr Gln Arg
1               5

<210> 275
<211> 8
<212> PRT
<213> Homo sapiens

<400> 275

Val Gln Ile Lys Ser Ser Leu Ile
1               5

<210> 276
<211> 9
<212> PRT
<213> Homo sapiens

<400> 276

Val Tyr Ile Gly His Thr Ser Thr Ile
1               5

<210> 277
<211> 10
<212> PRT
<213> Homo sapiens

<400> 277

Tyr His Val Pro Gly Thr Gly Glu Ser Tyr
1               5               10

<210> 278

<211> 9
<212> PRT
<213> Homo sapiens

<400> 278

```
Ala Thr Asn Gly Asp Leu Ala Ser Arg
1               5
```

<210> 279
<211> 11
<212> PRT
<213> Homo sapiens

<400> 279

```
Gly Leu His Ala Glu Val Thr Gly Val Gly Tyr

1               5               10
```

<210> 280
<211> 9
<212> PRT
<213> Homo sapiens

<400> 280

```
His Val Ser Ser Thr Ser Ser Ser Phe
1               5
```

<210> 281
<211> 9
<212> PRT
<213> Homo sapiens

<400> 281

```
Leu Gln Ala Asp Leu Gln Asn Gly Leu
1               5
```

<210> 282
<211> 9
<212> PRT
<213> Homo sapiens

<400> 282

```
Ser Glu Leu Pro Val Ser Glu Val Ala
1               5
```

<210> 283
<211> 9
<212> PRT
<213> Homo sapiens

<400> 283

```
Ser Gln Thr Lys Ser Val Phe Glu Ile
1               5
```

<210> 284
<211> 9
<212> PRT
<213> Homo sapiens

<400> 284

```
Thr His Ile Phe Thr Ser Asp Gly Leu
1               5
```

<210> 285
<211> 9
<212> PRT
<213> Homo sapiens

<400> 285

```
Val Ile Tyr Phe Pro Pro Leu Gln Lys
1               5
```

<210> 286
<211> 9
<212> PRT
<213> Homo sapiens

<400> 286

```
Tyr Pro Phe Ser Ser Glu Gln Lys Trp
1               5
```

<210> 287
<211> 9
<212> PRT
<213> Homo sapiens

<400> 287

```
Gly Gln Tyr Phe Gly Glu Leu Ala Leu
1               5
```

<210> 288
<211> 9
<212> PRT
<213> Homo sapiens

<400> 288

```
Arg Ile Ile Val Lys Asn Asn Ala Lys
1               5
```

<210> 289
<211> 10
<212> PRT
<213> Homo sapiens

<400> 289

```
Arg Arg Ile Ile Val Lys Asn Asn Ala Lys
1               5                   10
```

<210> 290
<211> 9
<212> PRT
<213> Homo sapiens

<400> 290

```
Ser Phe Gly Glu Leu Ala Leu Met Tyr
1               5
```

<210> 291
<211> 9
<212> PRT
<213> Homo sapiens

<400> 291

```
Ala Phe Asn Ala Pro Val Ile Asn Arg
1               5
```

<210> 292
<211> 9
<212> PRT
<213> Homo sapiens

<400> 292

```
Ile Met Lys Arg Asn Ile Ala Thr Tyr
1               5
```

<210> 293
<211> 9
<212> PRT
<213> Homo sapiens

<400> 293

```
Lys Val Val Asp Val Ile Gly Thr Lys
1               5
```

<210> 294
<211> 9
<212> PRT
<213> Homo sapiens

<400> 294

```
Leu Pro Phe Leu Lys Ser Leu Glu Phe
1               5
```

<210> 295
<211> 11
<212> PRT

<213> Homo sapiens

<400> 295

```
Arg Leu Lys Val Val Asp Val Ile Gly Thr Lys
1               5               10
```

<210> 296
<211> 9
<212> PRT
<213> Homo sapiens

<400> 296

```
Thr Pro Arg Ala Ala Thr Ile Thr Ala
1               5
```

<210> 297
<211> 9
<212> PRT
<213> Homo sapiens

<400> 297

```
Lys Pro Ser Glu Lys Ile Gln Val Leu
1               5
```

<210> 298
<211> 9
<212> PRT
<213> Homo sapiens

<400> 298

```
Val Pro Tyr Pro Val Thr Thr Thr Val
1               5
```

<210> 299
<211> 9
<212> PRT
<213> Homo sapiens

<400> 299

```
Ala Ser Phe Pro Pro Phe Val Glu Lys
1               5
```

<210> 300
<211> 9
<212> PRT
<213> Homo sapiens

<400> 300

```
Ala Phe Ile His Ile Ser Thr Ala Tyr
1               5
```

<210> 301

<211> 10
<212> PRT
<213> Homo sapiens

<400> 301

```
Ala Thr Phe Glu Lys Ile Pro Phe Glu Arg
1               5                   10
```

<210> 302
<211> 9
<212> PRT
<213> Homo sapiens

<400> 302

```
Lys Leu Phe Glu Lys Val Lys Glu Val
1               5
```

<210> 303
<211> 9
<212> PRT
<213> Homo sapiens

<400> 303

```
Ser Gln Met Pro Lys Leu Glu Ala Phe
1               5
```

<210> 304
<211> 9
<212> PRT
<213> Homo sapiens

<400> 304

```
Ala Val Leu Gly Gln His His Asn Tyr
1               5
```

<210> 305
<211> 8
<212> PRT
<213> Homo sapiens

<400> 305

```
Gly Pro Pro Ala His Lys Pro Arg
1               5
```

<210> 306
<211> 9
<212> PRT
<213> Homo sapiens

<400> 306

```
Arg Val Tyr Asp Val Leu Val Leu Lys
1               5
```

<210> 307
<211> 9
<212> PRT
<213> Homo sapiens

<400> 307

```
Leu Pro Arg Pro Gln Gly Ile Thr Val
1                   5
```

<210> 308
<211> 9
<212> PRT
<213> Homo sapiens

<400> 308

```
Val Leu Tyr Val Gly Ser Lys Thr Lys
1                   5
```

<210> 309
<211> 9
<212> PRT
<213> Homo sapiens

<400> 309

```
Lys Thr Lys Glu Gln Val Thr Asn Val
1                   5
```

<210> 310
<211> 10
<212> PRT
<213> Homo sapiens

<400> 310

```
Met Pro Val Asp Pro Asp Asn Glu Ala Tyr
1               5                   10
```

<210> 311
<211> 9
<212> PRT
<213> Homo sapiens

<400> 311

```
Ala Glu Lys Thr Lys Gln Gly Val Ala
1                   5
```

<210> 312
<211> 9
<212> PRT
<213> Homo sapiens

<400> 312

Asp Ile Ala Asp Phe Phe Thr Thr Arg
1                   5

<210> 313
<211> 9
<212> PRT
<213> Homo sapiens


<400> 313

His Ser Tyr Leu Gln Arg Gln Ser Val
1                   5

<210> 314
<211> 9
<212> PRT
<213> Homo sapiens


<400> 314

Lys Glu Val Thr Leu Ile Glu Glu Leu
1                   5

<210> 315
<211> 9
<212> PRT
<213> Homo sapiens


<400> 315

Arg Glu Asp Gly Pro Gly Val Ala Leu
1                   5

<210> 316
<211> 9
<212> PRT
<213> Homo sapiens


<400> 316

Arg Glu Asp Pro Leu Pro Pro Gly Leu
1                   5

<210> 317
<211> 11
<212> PRT
<213> Homo sapiens


<400> 317

Ser Leu Phe Gly Gly Ser Gln Gly Leu Arg Lys
1               5                   10

<210> 318
<211> 9
<212> PRT
<213> Homo sapiens

<400> 318

```
        Ala Glu Phe Gln Arg Leu Lys Gln Ala
        1               5
```

<210> 319
<211> 10
<212> PRT
<213> Homo sapiens

<400> 319

```
        Glu Val Ile Asp Gly Val Pro Gly Lys Trp
        1               5                   10
```

<210> 320
<211> 9
<212> PRT
<213> Homo sapiens

<400> 320

```
        Ile Pro Lys Ala Pro Gly Lys Ile Ile
        1               5
```

<210> 321
<211> 9
<212> PRT
<213> Homo sapiens

<400> 321

```
        Ser His Asn Gly Ser Ala Ile Arg Tyr
        1               5
```

<210> 322
<211> 10
<212> PRT
<213> Homo sapiens

<400> 322

```
        Thr Glu Val Thr Val Val Gly Asp Lys Leu
        1               5                   10
```

<210> 323
<211> 9
<212> PRT
<213> Homo sapiens

<400> 323

```
        Tyr Ala Ser Val Val Val Lys Arg Tyr
        1               5
```

<210> 324
<211> 9
<212> PRT

<213> Homo sapiens

<400> 324

```
Ala Thr Asp Leu Ala Leu Tyr Ile Lys
1               5
```

<210> 325
<211> 9
<212> PRT
<213> Homo sapiens

<400> 325

```
Ala Tyr His Asn Trp Arg His Ala Phe
1               5
```

<210> 326
<211> 9
<212> PRT
<213> Homo sapiens

<400> 326

```
Glu Pro Leu Asn Ile Lys Asp Ala Tyr
1               5
```

<210> 327
<211> 9
<212> PRT
<213> Homo sapiens

<400> 327

```
Lys Ile Ala Ala Thr Ile Ile Ser Phe
1               5
```

<210> 328
<211> 9
<212> PRT
<213> Homo sapiens

<400> 328

```
Lys Ile Phe Leu His Ile His Gly Leu
1               5
```

<210> 329
<211> 8
<212> PRT
<213> Homo sapiens

<400> 329

```
Leu Glu Val Ile Leu Lys Lys Ile
1               5
```

<210> 330

<211> 9
<212> PRT
<213> Homo sapiens

<400> 330

Ser Glu His Pro Leu Ala Gln Leu Tyr
1               5

<210> 331
<211> 9
<212> PRT
<213> Homo sapiens

<400> 331

Val Pro Ser Ala Gln Thr Leu Lys Ile
1               5

<210> 332
<211> 8
<212> PRT
<213> Homo sapiens

<400> 332

Ala Glu Tyr Arg Ser Tyr Val Ala
1               5

<210> 333
<211> 10
<212> PRT
<213> Homo sapiens

<400> 333

Ala Leu Ala Pro Gly Arg Gly Thr Leu Tyr
1               5               10

<210> 334
<211> 9
<212> PRT
<213> Homo sapiens

<400> 334

Gly Pro Arg Gly Thr Gln Ala Ala Leu
1               5

<210> 335
<211> 9
<212> PRT
<213> Homo sapiens

<400> 335

Ile Glu Asp Pro Gly Thr Leu His Ile
1               5

<210> 336
<211> 10
<212> PRT
<213> Homo sapiens

<400> 336

```
        Ile Glu Asp Pro Gly Thr Leu His Ile Trp
        1               5                   10
```

<210> 337
<211> 9
<212> PRT
<213> Homo sapiens

<400> 337

```
        Arg Pro Ile Pro Ile Ala Val Lys Tyr
        1               5
```

<210> 338
<211> 8
<212> PRT
<213> Homo sapiens

<400> 338

```
        Val Glu Lys Leu Leu Thr Asn Trp
        1               5
```

<210> 339
<211> 11
<212> PRT
<213> Homo sapiens

<400> 339

```
        Phe Leu Asp Pro Asp Ile Gly Gly Val Ala Val
        1               5                   10
```

<210> 340
<211> 10
<212> PRT
<213> Homo sapiens

<400> 340

```
        His Thr Ala Pro Pro Glu Asn Lys Thr Trp
        1               5                   10
```

<210> 341
<211> 10
<212> PRT
<213> Homo sapiens

<400> 341

```
Leu Leu Asp Thr Pro Val Lys Thr Gln Tyr
1               5               10
```

<210> 342
<211> 9
<212> PRT
<213> Homo sapiens

<400> 342

```
Asn Ala Val Lys Asp Phe Thr Ser Phe
          1               5
```

<210> 343
<211> 9
<212> PRT
<213> Homo sapiens

<400> 343

```
Ser Gly Leu Leu Gln Ile Lys Lys Leu
1               5
```

<210> 344
<211> 9
<212> PRT
<213> Homo sapiens

<400> 344

```
Tyr His Asp Lys Asn Ile Val Leu Leu
1               5
```

<210> 345
<211> 9
<212> PRT
<213> Homo sapiens

<400> 345

```
Ser Val Asp Pro Lys Asn Tyr Pro Lys
1               5
```

<210> 346
<211> 9
<212> PRT
<213> Homo sapiens

<400> 346

```
Ala Val Gly Leu Val Leu Pro Ala Lys
1               5
```

<210> 347
<211> 10

<212> PRT
<213> Homo sapiens

<400> 347

```
Ala Val Gly Leu Val Leu Pro Ala Lys Leu
1               5                   10
```

<210> 348
<211> 9
<212> PRT
<213> Homo sapiens

<400> 348

```
Ala Leu Leu Glu Val Leu Ser Gln Lys
1               5
```

<210> 349
<211> 9
<212> PRT
<213> Homo sapiens

<400> 349

```
His Glu Lys Gln Asp Thr Leu Val Ala
1               5
```

<210> 350
<211> 9
<212> PRT
<213> Homo sapiens

<400> 350

```
Lys Glu Leu Glu Leu Gln Ile Gly Met
1               5
```

<210> 351
<211> 9
<212> PRT
<213> Homo sapiens

<400> 351

```
Met Tyr Ser Asp Val Trp Lys Gln Leu
1               5
```

<210> 352
<211> 10
<212> PRT
<213> Homo sapiens

<400> 352

```
Arg Glu Leu Gln Asp Glu Lys Ala Glu Leu
1               5                   10
```

177

<210> 353
<211> 9
<212> PRT
<213> Homo sapiens

<400> 353

```
                    Arg Ile Thr Asp Val Leu Asp Gln Lys
                    1               5
```

<210> 354
<211> 9
<212> PRT
<213> Homo sapiens

<400> 354

```
                    Glu Val Ile Lys Ile Thr Gly Leu Lys
                    1               5
```

<210> 355
<211> 9
<212> PRT
<213> Homo sapiens

<400> 355

```
                    His His Val Asp Ile Thr Lys Lys Leu
                    1               5
```

<210> 356
<211> 9
<212> PRT
<213> Homo sapiens

<400> 356

```
                    Leu Pro Phe Asn Val Lys Val Ser Val
                    1               5
```

<210> 357
<211> 8
<212> PRT
<213> Homo sapiens

<400> 357

```
                     Thr Leu Pro Arg Val Leu Glu Ile
                     1               5
```

<210> 358
<211> 9
<212> PRT
<213> Homo sapiens

<400> 358

Thr Val Asp Leu Pro Lys Ser Pro Lys
1               5

<210> 359
<211> 9
<212> PRT
<213> Homo sapiens

<400> 359

Ala Glu His Gly Leu Leu Leu Thr Ala
1               5

<210> 360
<211> 9
<212> PRT
<213> Homo sapiens

<400> 360

Ala Gln Ala Gly Ala Leu Leu Gln Val
1               5

<210> 361
<211> 8
<212> PRT
<213> Homo sapiens

<400> 361

Asp Gly Gly Phe Val Leu Lys Val
1               5

<210> 362
<211> 9
<212> PRT
<213> Homo sapiens

<400> 362

Ile Val Tyr Pro Ser Gly Lys Val Tyr
1               5

<210> 363
<211> 9
<212> PRT
<213> Homo sapiens

<400> 363

Lys Leu Asp Asn Gln Val Ser Lys Val
1               5

<210> 364
<211> 9
<212> PRT
<213> Homo sapiens

<400> 364

```
                    Ser Glu Asn Val Lys Leu Phe Ser Ala
                    1               5
```

<210> 365
<211> 8
<212> PRT
<213> Homo sapiens

<400> 365

```
                      Val Gln Lys Leu Gln Asn Ile Ile
                      1               5
```

<210> 366
<211> 9
<212> PRT
<213> Homo sapiens

<400> 366

```
                    Phe Ser Thr Pro His Gly Leu Glu Val
                    1               5
```

<210> 367
<211> 9
<212> PRT
<213> Homo sapiens

<400> 367

```
                    Lys Arg Phe His Gln Lys Ser Asp Met
                    1               5
```

<210> 368
<211> 9
<212> PRT
<213> Homo sapiens

<400> 368

```
                    Lys Thr Phe Gly His Ala Val Ser Leu
                    1               5
```

<210> 369
<211> 9
<212> PRT
<213> Homo sapiens

<400> 369

```
                    Ser Ser Asn Leu Ile Thr His Ser Arg
                    1               5
```

<210> 370
<211> 10
<212> PRT

<213> Homo sapiens

<400> 370

```
                    Gly Val Ile Asp Gly His Ile Tyr Ala Val
                    1               5                   10
```

<210> 371
<211> 11
<212> PRT
<213> Homo sapiens

<400> 371

```
                    Ile Glu Pro Ala Lys Glu Thr Thr Thr Asn Val
                    1               5                   10
```

<210> 372
<211> 9
<212> PRT
<213> Homo sapiens

<400> 372

```
                    Asn Ala Pro Pro Ser Glu Val Leu Leu
                    1               5
```

<210> 373
<211> 12
<212> PRT
<213> Homo sapiens

<400> 373

```
                Ser Ile Glu Pro Ala Lys Glu Thr Thr Thr Asn Val
                1               5                   10
```

<210> 374
<211> 9
<212> PRT
<213> Homo sapiens

<400> 374

```
                    Ala Gln Ser Gln His Asn Gln Ser Leu
                    1               5
```

<210> 375
<211> 9
<212> PRT
<213> Homo sapiens

<400> 375

```
                    Ala Gln Ser Arg Thr Asn Pro Gln Val
                    1               5
```

<210> 376

<211> 9
<212> PRT
<213> Homo sapiens

<400> 376

```
Lys Met His Asp Lys Val Phe Ala Tyr
1               5
```

<210> 377
<211> 9
<212> PRT
<213> Homo sapiens

<400> 377

```
Thr Ala Lys Ala Pro Leu Ser Thr Val
1               5
```

<210> 378
<211> 8
<212> PRT
<213> Homo sapiens

<400> 378

```
Ile Pro Thr Arg Thr Val Ala Ile
1               5
```

<210> 379
<211> 8
<212> PRT
<213> Homo sapiens

<400> 379

```
Asn His Asp Arg Lys His Ala Val
1               5
```

<210> 380
<211> 9
<212> PRT
<213> Homo sapiens

<400> 380

```
Asn Asn His Asp Arg Lys His Ala Val
1               5
```

<210> 381
<211> 9
<212> PRT
<213> Homo sapiens

<400> 381

```
Thr Pro Gly Gly Thr Arg Ile Ile Tyr
1               5
```

<210> 382
<211> 9
<212> PRT
<213> Homo sapiens

<400> 382

```
            Glu His Trp Pro Ser Pro Glu Thr Phe
            1               5
```

<210> 383
<211> 9
<212> PRT
<213> Homo sapiens

<400> 383

```
            Glu Ile Ile Thr Asn Thr Leu Ser Phe
            1               5
```

<210> 384
<211> 10
<212> PRT
<213> Homo sapiens

<400> 384

```
            Glu Val Arg Gly Ala Leu Met Ser Ala Phe
            1               5                   10
```

<210> 385
<211> 9
<212> PRT
<213> Homo sapiens

<400> 385

```
            Ile Pro Arg Pro Ile Leu Val Leu Leu
            1               5
```

<210> 386
<211> 9
<212> PRT
<213> Homo sapiens

<400> 386

```
            Leu Pro Asn Lys Asn Arg Asp Glu Leu
            1               5
```

<210> 387
<211> 9
<212> PRT
<213> Homo sapiens

<400> 387

```
                    Gln Arg Ile Pro Ala Gly Ala Val Leu
                    1                   5
```

<210> 388
<211> 11
<212> PRT
<213> Homo sapiens

<400> 388

```
                    Ala Glu Gly Pro Ala Gly Gly Phe Met Val Val
                    1                   5                   10
```

<210> 389
<211> 9
<212> PRT
<213> Homo sapiens

<400> 389

```
                    Ala Tyr Tyr Arg Asp Ala Glu Ala Tyr
                    1                   5
```

<210> 390
<211> 9
<212> PRT
<213> Homo sapiens

<400> 390

```
                    Gln Val Asn Arg Pro Leu Thr Met Arg
                    1                   5
```

<210> 391
<211> 9
<212> PRT
<213> Homo sapiens

<400> 391

```
                    Arg His Ser Pro Val Phe Gln Val Tyr
                    1                   5
```

<210> 392
<211> 9
<212> PRT
<213> Homo sapiens

<400> 392

```
                    Ser Leu Pro Val Pro Asn Ser Ala Tyr
                    1                   5
```

<210> 393
<211> 11
<212> PRT
<213> Homo sapiens

<400> 393

```
                    Thr Leu Gly Pro Pro Gly Thr Ala His Leu Tyr
                    1               5                   10
```

<210> 394
<211> 11
<212> PRT
<213> Homo sapiens

<400> 394

```
                    Ile Glu Pro Ala Lys Glu Thr Thr Thr Asn Val
                    1               5                   10
```

<210> 395
<211> 9
<212> PRT
<213> Homo sapiens

<400> 395

```
                    Asn Ala Pro Pro Ser Glu Val Leu Leu
                    1               5
```

<210> 396
<211> 12
<212> PRT
<213> Homo sapiens

<400> 396

```
                Ser Ile Glu Pro Ala Lys Glu Thr Thr Thr Asn Val
                1               5                       10
```

<210> 397
<211> 9
<212> PRT
<213> Homo sapiens

<400> 397

```
                    Asp Leu Tyr Ser Gly Leu Asn Gln Arg
                    1               5
```

<210> 398
<211> 9
<212> PRT
<213> Homo sapiens

<400> 398

```
                    Lys Ala Lys Ala Lys Pro Val Thr Arg
                    1               5
```

<210> 399
<211> 10
<212> PRT

<213> Homo sapiens

<400> 399

Ala Val Leu Asp Lys Ala Met Lys Ala Lys
1               5                   10

<210> 400
<211> 9
<212> PRT
<213> Homo sapiens

<400> 400

Leu Glu Leu Ser Thr Pro Leu Lys Ile
1               5

<210> 401
<211> 9
<212> PRT
<213> Homo sapiens

<400> 401

Leu Pro Leu Asn Leu Asp Thr Lys Tyr
1               5

<210> 402
<211> 9
<212> PRT
<213> Homo sapiens

<400> 402

Thr Val Ile Tyr Arg Ile Gln Ala Leu
1               5

<210> 403
<211> 9
<212> PRT
<213> Homo sapiens

<400> 403

Asp Ala His Ile Tyr Leu Asn His Ile
1               5

<210> 404
<211> 10
<212> PRT
<213> Homo sapiens

<400> 404

Asn His Ile Glu Pro Leu Lys Ile Gln Leu
1               5                   10

<210> 405

<211> 9
<212> PRT
<213> Homo sapiens

<400> 405

Ala Tyr Arg Pro Ala Val His Pro Arg

1               5

<210> 406
<211> 9
<212> PRT
<213> Homo sapiens

<400> 406

Leu Arg Ala Pro Leu Glu His Glu Leu
1               5

<210> 407
<211> 9
<212> PRT
<213> Homo sapiens

<400> 407

Arg Leu Phe Met Val Leu Leu Leu Lys
1               5

<210> 408
<211> 9
<212> PRT
<213> Homo sapiens

<400> 408

Arg Ser Pro Asp Val Leu Lys Asp Phe
1               5

<210> 409
<211> 9
<212> PRT
<213> Homo sapiens

<400> 409

Glu Thr Ala Pro Gly Val His Lys Arg
1               5

<210> 410
<211> 9
<212> PRT
<213> Homo sapiens

<400> 410

```
                    Leu Tyr His Gly Tyr Ile Tyr Thr Tyr
                    1               5
```

<210> 411
<211> 9
<212> PRT
<213> Homo sapiens

<400> 411

```
                    Gly Gln His Val Ala Thr Gln His Phe
                    1               5
```

<210> 412
<211> 8
<212> PRT
<213> Homo sapiens

<400> 412

```
                      Leu Asn Gly Gln Leu Pro Asn Leu
                      1               5
```

<210> 413
<211> 11
<212> PRT
<213> Homo sapiens

<400> 413

```
                    Leu Pro Phe Pro Asp Glu Thr His Glu Arg Tyr
                    1               5                   10
```

<210> 414
<211> 9
<212> PRT
<213> Homo sapiens

<400> 414

```
                    Leu Pro His Asn Thr His Arg Val Val
                    1               5
```

<210> 415
<211> 9
<212> PRT
<213> Homo sapiens

<400> 415

```
                    Val Val Phe Asp Ser Pro Arg Asn Arg
                    1               5
```

<210> 416
<211> 8
<212> PRT
<213> Homo sapiens

<400> 416

```
                    Tyr Pro Leu Gly Arg Ile Leu Ile
                    1               5
```

<210> 417
<211> 9
<212> PRT
<213> Homo sapiens

<400> 417

```
                    Lys Glu Phe Ala Glu Phe Val Thr Ser
                    1               5
```

<210> 418
<211> 9
<212> PRT
<213> Homo sapiens

<400> 418

```
                    Val Met Leu Asp Val Pro Ile Arg Leu
                    1               5
```

<210> 419
<211> 9
<212> PRT
<213> Homo sapiens

<400> 419

```
                    Val Pro Met Thr Pro Leu Arg Thr Val
                    1               5
```

<210> 420
<211> 9
<212> PRT
<213> Homo sapiens

<400> 420

```
                    Gln Ile Asp Tyr Lys Thr Leu Val Leu
                    1               5
```

<210> 421
<211> 9
<212> PRT
<213> Homo sapiens

<400> 421

```
                    Val Glu Asp Pro Thr Ile Val Arg Ile
                    1               5
```

<210> 422
<211> 9
<212> PRT

<213> Homo sapiens

<400> 422

Ile Pro Tyr Gln Asp Leu Pro His Leu
1                 5

<210> 423
<211> 10
<212> PRT
<213> Homo sapiens

<400> 423

Asp Thr Pro Phe Leu Thr Gly His Gly Arg
1                 5                    10

<210> 424
<211> 8
<212> PRT
<213> Homo sapiens

<400> 424

Glu Phe Tyr Arg Ala Leu Tyr Ile
1                 5

<210> 425
<211> 10
<212> PRT
<213> Homo sapiens

<400> 425

Arg Tyr Tyr Pro Gln Ile Leu Thr Asn Lys
1                 5                    10

<210> 426
<211> 8
<212> PRT
<213> Homo sapiens

<400> 426

Lys Ala Tyr Glu Arg His Val Leu
1                 5

<210> 427
<211> 9
<212> PRT
<213> Homo sapiens

<400> 427

Leu Pro Ser Pro Glu Phe His Asp Tyr
1                 5

<210> 428

<211> 9
<212> PRT
<213> Homo sapiens

<400> 428

```
Ser Leu Tyr Ala His Pro Ile Glu His
1               5
```

<210> 429
<211> 9
<212> PRT
<213> Homo sapiens

<400> 429

```
Leu Val Arg Glu Pro Gly Ser Gln Ala
1               5
```

<210> 430
<211> 10
<212> PRT
<213> Homo sapiens

<400> 430

```
Arg Leu Ala Gly Pro Gly Ser Glu Lys Tyr
1               5                   10
```

<210> 431
<211> 10
<212> PRT
<213> Homo sapiens

<400> 431

```
Ser Pro Gly Ala Gly Arg Asn Ser Val Leu
1               5                   10
```

<210> 432
<211> 11
<212> PRT
<213> Homo sapiens

<400> 432

```
Ser Val Gln Ser Asp Gln Gly Tyr Ile Ser Arg
1               5                   10
```

<210> 433
<211> 9
<212> PRT
<213> Homo sapiens

<400> 433

```
Gly Val Arg Pro Pro Ala Pro Ser Leu
1               5
```

<210> 434
<211> 9
<212> PRT
<213> Homo sapiens

<400> 434

```
Ile Phe Ser Glu Lys Pro Val Phe Val
1               5
```

<210> 435
<211> 9
<212> PRT
<213> Homo sapiens

<400> 435

```
Lys Ala Ser Asn Leu Leu Leu Gly Phe
1               5
```

<210> 436
<211> 9
<212> PRT
<213> Homo sapiens

<400> 436

```
Lys Arg Tyr Ile Phe Ala Asp Ala Tyr
1               5
```

<210> 437
<211> 9
<212> PRT
<213> Homo sapiens

<400> 437

```
Arg Asn Leu Gln Leu Ser Leu Pro Arg
1               5
```

<210> 438
<211> 9
<212> PRT
<213> Homo sapiens

<400> 438

```
Glu Ala Ser Glu Pro Val Ala Leu Arg
1               5
```

<210> 439
<211> 9
<212> PRT
<213> Homo sapiens

<400> 439

```
                              Arg Pro Lys Val Pro Asp Gln Ser Val
                              1               5
```

<210> 440
<211> 9
<212> PRT
<213> Homo sapiens


<400> 440

```
                              Val Leu Tyr Glu Asn Ala Leu Lys Leu
                              1               5
```

<210> 441
<211> 10
<212> PRT
<213> Homo sapiens


<400> 441

```
                          Glu Val Leu Asp Lys Ser Gln Thr Asn Tyr
                          1               5                   10
```

<210> 442
<211> 9
<212> PRT
<213> Homo sapiens


<400> 442

```
                              Met Pro Ser Pro Ile Pro Ala Lys Tyr
                              1               5
```

<210> 443
<211> 9
<212> PRT
<213> Homo sapiens


<400> 443

```
                              Tyr Gly Ile Glu Asn Phe Thr Ser Val
                              1               5
```

<210> 444
<211> 9
<212> PRT
<213> Homo sapiens


<400> 444

```
                              Ala Arg Ala Ala Gln Val Phe Phe Leu
                              1               5
```

<210> 445
<211> 9
<212> PRT
<213> Homo sapiens

<400> 445

```
Glu His Ile Val Pro Asn Ala Glu Leu
1               5
```

<210> 446
<211> 9
<212> PRT
<213> Homo sapiens

<400> 446

```
Glu Ala Phe Glu Phe Val Lys Gln Arg
1               5
```

<210> 447
<211> 9
<212> PRT
<213> Homo sapiens

<400> 447

```
Asn His Phe Glu Gly His Tyr Gln Tyr
1               5
```

<210> 448
<211> 9
<212> PRT
<213> Homo sapiens

<400> 448

```
Asp Ala Tyr Pro Lys Asn Pro His Leu
1               5
```

<210> 449
<211> 9
<212> PRT
<213> Homo sapiens

<400> 449

```
Asp Val Asn Ile Lys Ser Thr Glu Arg
1               5
```

<210> 450
<211> 9
<212> PRT
<213> Homo sapiens

<400> 450

```
His Ile Asn Ser Ile Lys Ser Val Phe
1               5
```

<210> 451
<211> 9
<212> PRT

<213> Homo sapiens

<400> 451

```
                              Tyr Glu Ser Glu Lys Val Gly Val Ala
                              1               5
```

<210> 452
<211> 9
<212> PRT
<213> Homo sapiens

<400> 452

```
                              Glu Asn Ala Pro Thr Thr Val Ser Arg
                              1               5
```

<210> 453
<211> 10
<212> PRT
<213> Homo sapiens

<400> 453

```
                            Arg Phe Pro His Leu Leu Ala His Thr Tyr
                            1               5                   10
```

<210> 454
<211> 9
<212> PRT
<213> Homo sapiens

<400> 454

```
                              Thr Leu Asp Gly Ser Leu His Ala Val
                              1               5
```

<210> 455
<211> 11
<212> PRT
<213> Homo sapiens

<400> 455

```
                            Arg Thr Val Leu Lys Asn Leu Ser Leu Leu Lys
                            1               5                   10
```

<210> 456
<211> 8
<212> PRT
<213> Homo sapiens

<400> 456

```
                              Phe Glu Ala Lys Val Gln Ala Ile
                              1               5
```

<210> 457

<211> 9
<212> PRT
<213> Homo sapiens

<400> 457

```
Phe Phe Glu Ala Lys Val Gln Ala Ile
1               5
```

<210> 458
<211> 8
<212> PRT
<213> Homo sapiens

<400> 458

```
Lys Glu Leu Gln Ser Thr Phe Lys
1               5
```

<210> 459
<211> 10
<212> PRT
<213> Homo sapiens

<400> 459

```
Asn Val Ser Ser Arg Phe Glu Glu Glu Ile
1               5                   10
```

<210> 460
<211> 10
<212> PRT
<213> Homo sapiens

<400> 460

```
Glu Val Trp Asn Asn Leu Gly Thr Thr Lys
1               5                   10
```

<210> 461
<211> 9
<212> PRT
<213> Homo sapiens

<400> 461

```
Met Ile Phe Arg Ser Gly Ser Leu Ile
1               5
```

<210> 462
<211> 9
<212> PRT
<213> Homo sapiens

<400> 462

```
Asn His Ala Leu Pro Leu Pro Gly Phe
1               5
```

<210> 463
<211> 10
<212> PRT
<213> Homo sapiens

<400> 463

```
          Ala Ser Val Phe Gly Thr Met Pro Leu Lys
          1               5                   10
```

<210> 464
<211> 10
<212> PRT
<213> Homo sapiens

<400> 464

```
          Arg Glu Phe Pro Asp Arg Leu Val Gly Tyr
          1               5                   10
```

<210> 465
<211> 9
<212> PRT
<213> Homo sapiens

<400> 465

```
          Ser Val Phe Gly Thr Met Pro Leu Lys
          1               5
```

<210> 466
<211> 9
<212> PRT
<213> Homo sapiens

<400> 466

```
          Asp Glu Met Arg Phe Val Thr Gln Ile
          1               5
```

<210> 467
<211> 10
<212> PRT
<213> Homo sapiens

<400> 467

```
          Glu Thr Val His Phe Ala Thr Thr Gln Trp
          1               5                   10
```

<210> 468
<211> 8
<212> PRT
<213> Homo sapiens

<400> 468

Leu Pro Pro Pro Ala Thr Gln Ile

1                    5

<210> 469
<211> 8
<212> PRT
<213> Homo sapiens

<400> 469

Leu Ala Arg Asp Leu Tyr Ala Phe
1                    5

<210> 470
<211> 10
<212> PRT
<213> Homo sapiens

<400> 470

Leu Pro Gly Ile Gly Leu Ser Thr Ser Leu
1                    5                    10

<210> 471
<211> 8
<212> PRT
<213> Homo sapiens

<400> 471

Met Glu Val Ile Leu Pro Met Leu
1                    5

<210> 472
<211> 9
<212> PRT
<213> Homo sapiens

<400> 472

Ala Ile Leu Asp Tyr Ile Leu Ala Lys
1                    5

<210> 473
<211> 9
<212> PRT
<213> Homo sapiens

<400> 473

Lys Ile Ala Ser Gln Leu Ser Lys Leu
1                    5

<210> 474
<211> 9

<212> PRT
<213> Homo sapiens

<400> 474

```
Lys Val Thr Ser Thr Thr Thr Val Lys
1               5
```

<210> 475
<211> 9
<212> PRT
<213> Homo sapiens

<400> 475

```
Tyr Asn Thr Leu Leu Pro Tyr Thr Phe
1               5
```

<210> 476
<211> 9
<212> PRT
<213> Homo sapiens

<400> 476

```
Phe Leu Asp Pro Arg Pro Leu Thr Val
1               5
```

<210> 477
<211> 9
<212> PRT
<213> Homo sapiens

<400> 477

```
Ser Ala Phe Ala Asp Arg Pro Ala Phe
1               5
```

<210> 478
<211> 9
<212> PRT
<213> Homo sapiens

<400> 478

```
Ala Ala Val Pro Val Ile Ile Ser Arg
1               5
```

<210> 479
<211> 9
<212> PRT
<213> Homo sapiens

<400> 479

```
Glu Glu Ile Gly Lys Val Ala Ala Ala
1               5
```

<210> 480
<211> 9
<212> PRT
<213> Homo sapiens

<400> 480

```
        Phe Leu Lys Asp Leu Val Ala Ser Val
        1               5
```

<210> 481
<211> 9
<212> PRT
<213> Homo sapiens

<400> 481

```
        Val Ile Ile Ser Arg Ala Leu Glu Leu
        1               5
```

<210> 482
<211> 12
<212> PRT
<213> Homo sapiens

<400> 482

```
        Ala Pro Arg Thr Thr Gly Thr Pro Arg Thr Ser Leu
        1               5                   10
```

<210> 483
<211> 10
<212> PRT
<213> Homo sapiens

<400> 483

```
        Glu Ser Val Gly Gly Ser Pro Gln Thr Lys
        1               5                   10
```

<210> 484
<211> 8
<212> PRT
<213> Homo sapiens

<400> 484

```
        Ile Pro Lys Asp Lys Ala Ile Leu
        1               5
```

<210> 485
<211> 9
<212> PRT
<213> Homo sapiens

<400> 485

```
                    Leu Pro Ala Tyr Gly Arg Thr Thr Leu
                    1               5
```

<210> 486
<211> 9
<212> PRT
<213> Homo sapiens

<400> 486

```
                    His Gln Ala Ala Ile Val Ser Lys Ile
                    1               5
```

<210> 487
<211> 8
<212> PRT
<213> Homo sapiens

<400> 487

```
                      Gln Ala Ala Ile Val Ser Lys Ile
                      1               5
```

<210> 488
<211> 9
<212> PRT
<213> Homo sapiens

<400> 488

```
                    Arg Gln Lys Met Pro Glu Asp Gly Leu
                    1               5
```

<210> 489
<211> 9
<212> PRT
<213> Homo sapiens

<400> 489

```
                    Ser Val Gln Lys Ser Ser Gly Val Lys
                    1               5
```

<210> 490
<211> 11
<212> PRT
<213> Homo sapiens

<400> 490

```
                Asp Ser Ile Gly Ser Thr Val Ser Ser Glu Arg
                1               5                   10
```

<210> 491
<211> 11
<212> PRT
<213> Homo sapiens

<400> 491

```
Leu Pro Tyr Asn Asn Lys Asp Arg Asp Ala Leu
1               5                   10
```

<210> 492
<211> 9
<212> PRT
<213> Homo sapiens

<400> 492

```
Ile Tyr Asp Glu Ile Gln Gln Glu Met
1               5
```

<210> 493
<211> 9
<212> PRT
<213> Homo sapiens

<400> 493

```
Ala Gln Ala Lys Gly Leu Ile Gln Val
1               5
```

<210> 494
<211> 9
<212> PRT
<213> Homo sapiens

<400> 494

```
Glu Val Ser Ser Glu Ile Tyr Gln Trp
1               5
```

<210> 495
<211> 9
<212> PRT
<213> Homo sapiens

<400> 495

```
Lys Trp Asn Pro Val Pro Leu Ser Tyr
1               5
```

<210> 496
<211> 9
<212> PRT
<213> Homo sapiens

<400> 496

```
Asn Arg Leu Leu Ala Gln Gln Ser Leu
1               5
```

<210> 497
<211> 9
<212> PRT

<213> Homo sapiens

<400> 497

Ala Pro Arg Pro Val Ala Val Ala Val
1               5

<210> 498
<211> 10
<212> PRT
<213> Homo sapiens

<400> 498

Phe Tyr Arg Glu Thr Val Gln Val Gly Arg
1               5                   10

<210> 499
<211> 9
<212> PRT
<213> Homo sapiens

<400> 499

Leu Leu Ala Pro Arg Pro Val Ala Val
1               5

<210> 500
<211> 9
<212> PRT
<213> Homo sapiens

<400> 500

Gly Leu Ala Ala Leu Val Ile Leu Lys
1               5

<210> 501
<211> 9
<212> PRT
<213> Homo sapiens

<400> 501

Lys Ile Gln Glu Val Phe Ser Ser Tyr
1               5

<210> 502
<211> 9
<212> PRT
<213> Homo sapiens

<400> 502

Ala Ser Leu Asp Lys Phe Leu Ser His
1               5

<210> 503

<211> 9
<212> PRT
<213> Homo sapiens

<400> 503

```
Ala Leu Tyr Ala Thr Lys Thr Leu Arg
1               5
```

<210> 504
<211> 8
<212> PRT
<213> Homo sapiens

<400> 504

```
Met Glu Tyr Val Ile Ser Arg Ile
1               5
```

<210> 505
<211> 9
<212> PRT
<213> Homo sapiens

<400> 505

```
Val Pro Val Gly Arg Gln Pro Ile Ile
1               5
```

<210> 506
<211> 10
<212> PRT
<213> Homo sapiens

<400> 506

```
Lys Leu Leu Ile Gly Val Ile Ala Ala Val
1               5                   10
```

<210> 507
<211> 8
<212> PRT
<213> Homo sapiens

<400> 507

```
Leu Pro Ser Leu Ile Lys Leu Asp
1               5
```

<210> 508
<211> 8
<212> PRT
<213> Homo sapiens

<400> 508

```
Pro Ser Leu Ile Lys Leu Asp Leu
1               5
```

<210> 509
<211> 9
<212> PRT
<213> Homo sapiens

<400> 509

```
Ala Arg Asn Lys Glu Leu Ile Gly Lys
1               5
```

<210> 510
<211> 9
<212> PRT
<213> Homo sapiens

<400> 510

```
Ala Val Lys Ser Asn Ala Ala Ala Tyr
1               5
```

<210> 511
<211> 9
<212> PRT
<213> Homo sapiens

<400> 511

```
Glu Val Ile Ile Pro His Ser Gly Trp
1               5
```

<210> 512
<211> 9
<212> PRT
<213> Homo sapiens

<400> 512

```
Ser Val Lys Glu Gln Glu Ala Gln Phe
1               5
```

<210> 513
<211> 10
<212> PRT
<213> Homo sapiens

<400> 513

```
Ala Pro Arg Gly Leu Glu Pro Ile Ala Ile
1               5                   10
```

<210> 514
<211> 9
<212> PRT
<213> Homo sapiens

<400> 514

Gly Arg Phe Gly Gly Val Ile Thr Ile
1                   5

<210> 515
<211> 9
<212> PRT
<213> Homo sapiens

<400> 515

Pro Val Ala Gly Phe Phe Ile Asn Arg
1                   5

<210> 516
<211> 9
<212> PRT
<213> Homo sapiens

<400> 516

Thr Pro Lys Thr Pro Ser Arg Asp Ala
1                   5

<210> 517
<211> 10
<212> PRT
<213> Homo sapiens

<400> 517

Val Leu Phe Gly Gly Lys Val Ser Gly Ala
1                   5                   10

<210> 518
<211> 9
<212> PRT
<213> Homo sapiens

<400> 518

Ala Glu His Ile Glu Ser Arg Thr Leu
1                   5

<210> 519
<211> 9
<212> PRT
<213> Homo sapiens

<400> 519

Asp Gln Tyr Pro Tyr Leu Lys Ser Val
1                   5

<210> 520
<211> 9
<212> PRT
<213> Homo sapiens

<400> 520

```
Ile Ala Arg Asn Leu Thr Gln Gln Leu
1               5
```

<210> 521
<211> 9
<212> PRT
<213> Homo sapiens

<400> 521

```
Ile Glu Ser Arg Thr Leu Ala Ile Ala
1               5
```

<210> 522
<211> 10
<212> PRT
<213> Homo sapiens

<400> 522

```
Met Thr Ser Ala Leu Pro Ile Ile Gln Lys
1               5                   10
```

<210> 523
<211> 12
<212> PRT
<213> Homo sapiens

<400> 523

```
Ser Leu Leu Thr Ser Ser Lys Gly Gln Leu Gln Lys
1               5                   10
```

<210> 524
<211> 9
<212> PRT
<213> Homo sapiens

<400> 524

```
Thr Ser Ala Leu Pro Ile Ile Gln Lys
1               5
```

<210> 525
<211> 9
<212> PRT
<213> Homo sapiens

<400> 525

```
Val Arg Leu Gly Ser Leu Ser Thr Lys
1               5
```

<210> 526
<211> 10
<212> PRT

<213> Homo sapiens

<400> 526

Arg Ile Asn Glu Phe Ser Ile Ser Ser Phe
1               5                   10

<210> 527
<211> 9
<212> PRT
<213> Homo sapiens

<400> 527

Asp Glu Lys Gln Gln His Ile Val Tyr
1               5

<210> 528
<211> 9
<212> PRT
<213> Homo sapiens

<400> 528

Asp Glu Val Tyr Gln Val Thr Val Tyr
1               5

<210> 529
<211> 9
<212> PRT
<213> Homo sapiens

<400> 529

Gly Glu Ile Ser Glu Lys Ala Lys Leu
1               5

<210> 530
<211> 9
<212> PRT
<213> Homo sapiens

<400> 530

Tyr Thr Met Lys Glu Val Leu Phe Tyr
1               5

<210> 531
<211> 9
<212> PRT
<213> Homo sapiens

<400> 531

Ser Gln Leu Thr Thr Leu Ser Phe Tyr

1                    5

<210> 532
<211> 8
<212> PRT
<213> Homo sapiens

<400> 532

Leu Glu Lys Gln Leu Ile Glu Leu
1                    5

<210> 533
<211> 9
<212> PRT
<213> Homo sapiens

<400> 533

Glu Leu Thr Leu Gly Glu Phe Leu Lys
1                    5

<210> 534
<211> 8
<212> PRT
<213> Homo sapiens

<400> 534

Leu Thr Leu Gly Glu Phe Leu Lys
1                    5

<210> 535
<211> 9
<212> PRT
<213> Homo sapiens

<400> 535

Leu Thr Leu Gly Glu Phe Leu Lys Leu
1                    5

<210> 536
<211> 8
<212> PRT
<213> Homo sapiens

<400> 536

Thr Leu Gly Glu Phe Leu Lys Leu
1                    5

<210> 537
<211> 8
<212> PRT
<213> Homo sapiens

<400> 537

Ile Thr Ala Arg Pro Val Leu Trp
1                   5

<210> 538
<211> 10
<212> PRT
<213> Homo sapiens

<400> 538

Lys Leu Met Ser Pro Lys Leu Tyr Val Trp
1                   5                   10

<210> 539
<211> 9
<212> PRT
<213> Homo sapiens

<400> 539

Lys Val Ser Ala Val Thr Leu Ala Tyr
1                   5

<210> 540
<211> 10
<212> PRT
<213> Homo sapiens

<400> 540

Val Glu Gly Ser Gly Glu Leu Phe Arg Trp
1                   5                   10

<210> 541
<211> 8
<212> PRT
<213> Homo sapiens

<400> 541

Arg Pro Lys Ser Asn Ile Val Leu
1                   5

<210> 542
<211> 9
<212> PRT
<213> Homo sapiens

<400> 542

Arg Pro Lys Ser Asn Ile Val Leu Leu
1                   5

<210> 543
<211> 14
<212> PRT

<213> Homo sapiens

<400> 543

```
        Gly Glu Pro Leu Ser Tyr Thr Arg Phe Ser Leu Ala Arg Gln
        1               5                   10
```

<210> 544
<211> 16
<212> PRT
<213> Homo sapiens

<400> 544

```
     Gly Glu Pro Leu Ser Tyr Thr Arg Phe Ser Leu Ala Arg Gln Val Asp
     1               5                   10                  15
```

<210> 545
<211> 17
<212> PRT
<213> Homo sapiens

<400> 545

```
     Gly Glu Pro Leu Ser Tyr Thr Arg Phe Ser Leu Ala Arg Gln Val Asp
     1               5                   10                  15

     Gly
```

<210> 546
<211> 17
<212> PRT
<213> Homo sapiens

<400> 546

```
     Gly Gly Glu Pro Leu Ser Tyr Thr Arg Phe Ser Leu Ala Arg Gln Val
     1               5                   10                  15

     Asp
```

<210> 547
<211> 18
<212> PRT
<213> Homo sapiens

<400> 547

```
     Gly Gly Glu Pro Leu Ser Tyr Thr Arg Phe Ser Leu Ala Arg Gln Val
     1               5                   10                  15

     Asp Gly
```

<210> 548
<211> 16

<212> PRT
<213> Homo sapiens

<400> 548

```
Asn Pro Gly Gly Tyr Val Ala Tyr Ser Lys Ala Ala Thr Val Thr Gly
1               5                   10                  15
```

<210> 549
<211> 17
<212> PRT
<213> Homo sapiens

<400> 549

```
Asn Pro Gly Gly Tyr Val Ala Tyr Ser Lys Ala Ala Thr Val Thr Gly
1               5                   10                  15

Lys
```

<210> 550
<211> 18
<212> PRT
<213> Homo sapiens

<400> 550

```
Asn Pro Gly Gly Tyr Val Ala Tyr Ser Lys Ala Ala Thr Val Thr Gly
1               5                   10                  15

Lys Leu
```

<210> 551
<211> 12
<212> PRT
<213> Homo sapiens

<400> 551

```
Asn Ser Val Ile Ile Val Asp Lys Asn Gly Arg Leu
1               5                   10
```

<210> 552
<211> 13
<212> PRT
<213> Homo sapiens

<400> 552

```
Asn Ser Val Ile Ile Val Asp Lys Asn Gly Arg Leu Val
1               5                   10
```

<210> 553
<211> 14
<212> PRT
<213> Homo sapiens

<400> 553

```
        Asn Ser Val Ile Ile Val Asp Lys Asn Gly Arg Leu Val Tyr
        1               5                   10
```

<210> 554
<211> 14
<212> PRT
<213> Homo sapiens

<400> 554

```
        Arg Val Glu Tyr His Phe Leu Ser Pro Tyr Val Ser Pro Lys
        1               5                   10
```

<210> 555
<211> 15
<212> PRT
<213> Homo sapiens

<400> 555

```
        Arg Val Glu Tyr His Phe Leu Ser Pro Tyr Val Ser Pro Lys Glu
        1               5                   10                  15
```

<210> 556
<211> 18
<212> PRT
<213> Homo sapiens

<400> 556

```
        Arg Val Glu Tyr His Phe Leu Ser Pro Tyr Val Ser Pro Lys Glu Ser
        1               5                   10                  15

        Pro Phe
```

<210> 557
<211> 15
<212> PRT
<213> Homo sapiens

<400> 557

```
        Ser Pro Phe Arg His Val Phe Trp Gly Ser Gly Ser His Thr Leu
        1               5                   10                  15
```

<210> 558
<211> 12
<212> PRT
<213> Homo sapiens

<400> 558

```
            Ser Val Ile Ile Val Asp Lys Asn Gly Arg Leu Val
            1               5                   10
```

<210> 559
<211> 13
<212> PRT
<213> Homo sapiens

<400> 559

```
        Val Glu Tyr His Phe Leu Ser Pro Tyr Val Ser Pro Lys
        1                   5                   10
```

<210> 560
<211> 14
<212> PRT
<213> Homo sapiens

<400> 560

```
        Val Glu Tyr His Phe Leu Ser Pro Tyr Val Ser Pro Lys Glu
        1                   5                   10
```

<210> 561
<211> 14
<212> PRT
<213> Homo sapiens

<400> 561

```
        Leu Pro Ser Gln Ala Phe Glu Tyr Ile Leu Tyr Asn Lys Gly
        1                   5                   10
```

<210> 562
<211> 15
<212> PRT
<213> Homo sapiens

<400> 562

```
        Leu Pro Ser Gln Ala Phe Glu Tyr Ile Leu Tyr Asn Lys Gly Ile
        1                   5                   10                  15
```

<210> 563
<211> 16
<212> PRT
<213> Homo sapiens

<400> 563

```
        Leu Pro Ser Gln Ala Phe Glu Tyr Ile Leu Tyr Asn Lys Gly Ile Met
        1                   5                   10                  15
```

<210> 564
<211> 17
<212> PRT
<213> Homo sapiens

<400> 564

Leu Pro Ser Gln Ala Phe Glu Tyr Ile Leu Tyr Asn Lys Gly Ile Met
1               5                   10                  15

Gly

<210> 565
<211> 13
<212> PRT
<213> Homo sapiens

<400> 565

Met Asn Gly Tyr Phe Leu Ile Glu Arg Gly Lys Asn Met
1               5                   10

<210> 566
<211> 12
<212> PRT
<213> Homo sapiens

<400> 566

Asn Gly Tyr Phe Leu Ile Glu Arg Gly Lys Asn Met
1               5                   10

<210> 567
<211> 13
<212> PRT
<213> Homo sapiens

<400> 567

Pro Ser Gln Ala Phe Glu Tyr Ile Leu Tyr Asn Lys Gly
1               5                   10

<210> 568
<211> 14
<212> PRT
<213> Homo sapiens

<400> 568

Pro Ser Gln Ala Phe Glu Tyr Ile Leu Tyr Asn Lys Gly Ile
1               5                   10

<210> 569
<211> 15
<212> PRT
<213> Homo sapiens

<400> 569

Pro Ser Gln Ala Phe Glu Tyr Ile Leu Tyr Asn Lys Gly Ile Met
1               5                   10                  15

<210> 570
<211> 13

<212> PRT
<213> Homo sapiens

<400> 570

```
Glu Gly Val Gln Tyr Ser Tyr Ser Leu Phe His Leu Met
1               5                   10
```

<210> 571
<211> 14
<212> PRT
<213> Homo sapiens

<400> 571

```
Glu Gly Val Gln Tyr Ser Tyr Ser Leu Phe His Leu Met Leu
1               5                   10
```

<210> 572
<211> 12
<212> PRT
<213> Homo sapiens

<400> 572

```
Gly Val Gln Tyr Ser Tyr Ser Leu Phe His Leu Met
1               5                   10
```

<210> 573
<211> 13
<212> PRT
<213> Homo sapiens

<400> 573

```
Gly Val Gln Tyr Ser Tyr Ser Leu Phe His Leu Met Leu
1               5                   10
```

<210> 574
<211> 15
<212> PRT
<213> Homo sapiens

<400> 574

```
Ser Ile Ile Ser Ile His Pro Lys Ile Gln Glu His Gln Pro Arg
1               5                   10                  15
```

<210> 575
<211> 13
<212> PRT
<213> Homo sapiens

<400> 575

```
Ser Ser Ile Arg Thr Ser Thr Asn Ser Gln Val Asp Lys
1               5                   10
```

<210> 576
<211> 12
<212> PRT
<213> Homo sapiens

<400> 576

```
Val Leu Val Gly Tyr Lys Ala Val Tyr Arg Ile Ser
1               5               10
```

<210> 577
<211> 14
<212> PRT
<213> Homo sapiens

<400> 577

```
Tyr Ser Ser Ile Arg Thr Ser Thr Asn Ser Gln Val Asp Lys
1               5               10
```

<210> 578
<211> 18
<212> PRT
<213> Homo sapiens

<400> 578

```
Gly Gly Gly Tyr Gly Ser Gly Gly Gly Ser Gly Gly Tyr Gly Ser Arg
1               5               10                  15

Arg Phe
```

<210> 579
<211> 12
<212> PRT
<213> Homo sapiens

<400> 579

```
Gly Gly Ser Phe Gly Gly Arg Ser Ser Gly Ser Pro
1               5               10
```

<210> 580
<211> 13
<212> PRT
<213> Homo sapiens

<400> 580

```
Lys Gly Gly Ser Phe Gly Gly Arg Ser Ser Gly Ser Pro
1               5               10
```

<210> 581
<211> 25
<212> PRT
<213> Homo sapiens

<400> 581

```
Ser Gly Gln Gln Gln Ser Asn Tyr Gly Pro Met Lys Gly Gly Ser Phe
1               5               10              15

Gly Gly Arg Ser Ser Gly Ser Pro Tyr
        20              25
```

<210> 582
<211> 23
<212> PRT
<213> Homo sapiens

<400> 582

```
Ser Gly Ser Pro Tyr Gly Gly Gly Tyr Gly Ser Gly Gly Gly Ser Gly
1               5               10              15

Gly Tyr Gly Ser Arg Arg Phe
        20
```

<210> 583
<211> 21
<212> PRT
<213> Homo sapiens

<400> 583

```
Ser Pro Tyr Gly Gly Gly Tyr Gly Ser Gly Gly Gly Ser Gly Gly Tyr
1               5               10              15

Gly Ser Arg Arg Phe
        20
```

<210> 584
<211> 19
<212> PRT
<213> Homo sapiens

<400> 584

```
Tyr Gly Gly Gly Tyr Gly Ser Gly Gly Gly Ser Gly Gly Tyr Gly Ser
1               5               10              15

                    Arg Arg Phe
```

<210> 585
<211> 12
<212> PRT
<213> Homo sapiens

<400> 585

Gly Asn Arg Ile Asn Glu Phe Ser Ile Ser Ser Phe
1                   5                   10

<210> 586
<211> 14
<212> PRT
<213> Homo sapiens

<400> 586

His Gly Asn Gln Ile Thr Ser Asp Lys Val Gly Arg Lys Val
1                   5                   10

<210> 587
<211> 14
<212> PRT
<213> Homo sapiens

<400> 587

Ile Pro Pro Val Asn Thr Asn Leu Glu Asn Leu Tyr Leu Gln
1                   5                   10

<210> 588
<211> 13
<212> PRT
<213> Homo sapiens

<400> 588

Leu Gln Val Leu Arg Leu Asp Gly Asn Glu Ile Lys Arg
1                   5                   10

<210> 589
<211> 14
<212> PRT
<213> Homo sapiens

<400> 589

Leu Gln Val Leu Arg Leu Asp Gly Asn Glu Ile Lys Arg Ser
1                   5                   10

<210> 590
<211> 15
<212> PRT
<213> Homo sapiens

<400> 590

Leu Gln Val Leu Arg Leu Asp Gly Asn Glu Ile Lys Arg Ser Ala
1                   5                   10                  15

<210> 591
<211> 16
<212> PRT
<213> Homo sapiens

<400> 591

```
        Leu Arg Glu Leu His Leu Asp His Asn Gln Ile Ser Arg Val Pro Asn
        1               5                   10                  15
```

<210> 592
<211> 14
<212> PRT
<213> Homo sapiens

<400> 592

```
        Leu Tyr Val Arg Leu Ser His Asn Ser Leu Thr Asn Asn Gly
        1               5                   10
```

<210> 593
<211> 14
<212> PRT
<213> Homo sapiens

<400> 593

```
        Val Pro Ser Arg Met Lys Tyr Val Tyr Phe Gln Asn Asn Gln
        1               5                   10
```

<210> 594
<211> 16
<212> PRT
<213> Homo sapiens

<400> 594

```
        Val Pro Ser Arg Met Lys Tyr Val Tyr Phe Gln Asn Asn Gln Ile Thr
        1               5                   10                  15
```

<210> 595
<211> 17
<212> PRT
<213> Homo sapiens

<400> 595

```
        Val Pro Ser Arg Met Lys Tyr Val Tyr Phe Gln Asn Asn Gln Ile Thr
        1               5                   10                  15

        Ser
```

<210> 596
<211> 12
<212> PRT
<213> Homo sapiens

<400> 596

```
            Trp Ile Ala Leu His Gly Asn Gln Ile Thr Ser Asp
            1               5                   10
```

<210> 597
<211> 13
<212> PRT
<213> Homo sapiens

<400> 597

```
Trp Ile Ala Leu His Gly Asn Gln Ile Thr Ser Asp Lys
1               5                   10
```

<210> 598
<211> 15
<212> PRT
<213> Homo sapiens

<400> 598

```
Ala Asp Asp Asn Val Ser Phe Arg Trp Glu Ala Leu Gly Asn Thr
1               5                   10                  15
```

<210> 599
<211> 16
<212> PRT
<213> Homo sapiens

<400> 599

```
Ala Asp Asp Asn Val Ser Phe Arg Trp Glu Ala Leu Gly Asn Thr Leu
1               5                   10                  15
```

<210> 600
<211> 17
<212> PRT
<213> Homo sapiens

<400> 600

```
Asp Ala Asp Asp Asn Val Ser Phe Arg Trp Glu Ala Leu Gly Asn Thr
1               5                   10                  15

Leu
```

<210> 601
<211> 14
<212> PRT
<213> Homo sapiens

<400> 601

```
Asp Asp Asn Val Ser Phe Arg Trp Glu Ala Leu Gly Asn Thr
1               5                   10
```

<210> 602
<211> 15
<212> PRT
<213> Homo sapiens

<400> 602

     Asp Asp Asn Val Ser Phe Arg Trp Glu Ala Leu Gly Asn Thr Leu
     1            5              10            15

<210> 603
<211> 13
<212> PRT
<213> Homo sapiens

<400> 603

     Asp Asn Val Ser Phe Arg Trp Glu Ala Leu Gly Asn Thr
     1            5              10

<210> 604
<211> 14
<212> PRT
<213> Homo sapiens

<400> 604

     Asp Asn Val Ser Phe Arg Trp Glu Ala Leu Gly Asn Thr Leu
     1            5              10

<210> 605
<211> 15
<212> PRT
<213> Homo sapiens

<400> 605

     Asp Asn Val Ser Phe Arg Trp Glu Ala Leu Gly Asn Thr Leu Ser
     1            5              10            15

<210> 606
<211> 16
<212> PRT
<213> Homo sapiens

<400> 606

     Asp Thr Gly Ser Tyr Arg Ala Gln Ile Ser Thr Lys Thr Ser Ala Lys
     1            5              10            15

<210> 607
<211> 17
<212> PRT
<213> Homo sapiens

<400> 607

     Asp Thr Gly Ser Tyr Arg Ala Gln Ile Ser Thr Lys Thr Ser Ala Lys
     1            5              10            15

     Leu

<210> 608
<211> 13
<212> PRT
<213> Homo sapiens

<400> 608

```
Asp Thr Ile Thr Ile Tyr Ser Thr Ile Asn His Ser Lys
1               5                   10
```

<210> 609
<211> 17
<212> PRT
<213> Homo sapiens

<400> 609

```
Glu Asp Thr Gly Ser Tyr Arg Ala Gln Ile Ser Thr Lys Thr Ser Ala
1               5                   10                  15

Lys
```

<210> 610
<211> 15
<212> PRT
<213> Homo sapiens

<400> 610

```
Glu Asn Asp Thr Ile Thr Ile Tyr Ser Thr Ile Asn His Ser Lys
1               5                   10                  15
```

<210> 611
<211> 20
<212> PRT
<213> Homo sapiens

<400> 611

```
Glu Asn Asp Thr Ile Thr Ile Tyr Ser Thr Ile Asn His Ser Lys Glu
1               5                   10                  15

Ser Lys Pro Thr
                20
```

<210> 612
<211> 14
<212> PRT
<213> Homo sapiens

<400> 612

```
Gly Ser Tyr Arg Ala Gln Ile Ser Thr Lys Thr Ser Ala Lys
1               5                   10
```

<210> 613
<211> 12

<212> PRT
<213> Homo sapiens

<400> 613

```
          Asn Asp Thr Ile Thr Ile Tyr Ser Thr Ile Asn His
          1               5                   10
```

<210> 614
<211> 13
<212> PRT
<213> Homo sapiens

<400> 614

```
          Asn Asp Thr Ile Thr Ile Tyr Ser Thr Ile Asn His Ser
          1               5                   10
```

<210> 615
<211> 14
<212> PRT
<213> Homo sapiens

<400> 615

```
          Asn Asp Thr Ile Thr Ile Tyr Ser Thr Ile Asn His Ser Lys
          1               5                   10
```

<210> 616
<211> 13
<212> PRT
<213> Homo sapiens

<400> 616

```
          Asn Val Ser Phe Arg Trp Glu Ala Leu Gly Asn Thr Leu
          1               5                   10
```

<210> 617
<211> 18
<212> PRT
<213> Homo sapiens

<400> 617

```
          Ser Pro Thr Asn Asn Thr Val Tyr Ala Ser Val Thr His Ser Asn Arg
          1               5                   10                  15

          Glu Thr
```

<210> 618
<211> 15
<212> PRT
<213> Homo sapiens

<400> 618

```
        Thr Gly Ser Tyr Arg Ala Gln Ile Ser Thr Lys Thr Ser Ala Lys
        1               5                   10                  15
```

<210> 619
<211> 18
<212> PRT
<213> Homo sapiens

<400> 619

```
        Thr Pro Arg Glu Asn Asp Thr Ile Thr Ile Tyr Ser Thr Ile Asn His
        1               5                   10                  15
```

```
        Ser Lys
```

<210> 620
<211> 23
<212> PRT
<213> Homo sapiens

<400> 620

```
        Thr Pro Arg Glu Asn Asp Thr Ile Thr Ile Tyr Ser Thr Ile Asn His
        1               5                   10                  15
```

```
        Ser Lys Glu Ser Lys Pro Thr
                    20
```

<210> 621
<211> 12
<212> PRT
<213> Homo sapiens

<400> 621

```
            Val Ser Phe Arg Trp Glu Ala Leu Gly Asn Thr Leu
            1               5                   10
```

<210> 622
<211> 15
<212> PRT
<213> Homo sapiens

<400> 622

```
        Ala Pro Ile His Phe Thr Ile Glu Lys Leu Glu Leu Asn Glu Lys
        1               5                   10                  15
```

<210> 623
<211> 13
<212> PRT
<213> Homo sapiens

<400> 623

Asp Ala Gln Phe Glu Val Ile Lys Gly Gln Thr Ile Glu
1               5                   10

<210> 624
<211> 15
<212> PRT
<213> Homo sapiens

<400> 624

Asp Ala Gln Phe Glu Val Ile Lys Gly Gln Thr Ile Glu Val Arg
1               5                   10                  15

<210> 625
<211> 15
<212> PRT
<213> Homo sapiens

<400> 625

Glu Ser Tyr Phe Ile Pro Glu Val Arg Ile Tyr Asp Ser Gly Thr
1               5                   10                  15

<210> 626
<211> 12
<212> PRT
<213> Homo sapiens

<400> 626

Ile Pro Glu Val Arg Ile Tyr Asp Ser Gly Thr Tyr
1               5                   10

<210> 627
<211> 14
<212> PRT
<213> Homo sapiens

<400> 627

Lys Asp Lys Ala Ile Val Ala His Asn Arg His Gly Asn Lys
1               5                   10

<210> 628
<211> 15
<212> PRT
<213> Homo sapiens

<400> 628

Lys Asp Lys Ala Ile Val Ala His Asn Arg His Gly Asn Lys Ala
1               5                   10                  15

<210> 629
<211> 14
<212> PRT
<213> Homo sapiens

<400> 629

```
        Asn Phe Val Ile Leu Glu Phe Pro Val Glu Glu Gln Asp Arg
        1               5                   10
```

<210> 630
<211> 15
<212> PRT
<213> Homo sapiens

<400> 630

```
        Ser Gln Pro Arg Ile Ser Tyr Asp Ala Gln Phe Glu Val Ile Lys
        1               5                   10                  15
```

<210> 631
<211> 16
<212> PRT
<213> Homo sapiens

<400> 631

```
        Ser Gln Pro Arg Ile Ser Tyr Asp Ala Gln Phe Glu Val Ile Lys Gly
        1               5                   10                  15
```

<210> 632
<211> 14
<212> PRT
<213> Homo sapiens

<400> 632

```
        Tyr Asp Ala Gln Phe Glu Val Ile Lys Gly Gln Thr Ile Glu
        1               5                   10
```

<210> 633
<211> 14
<212> PRT
<213> Homo sapiens

<400> 633

```
        Gly Asn Pro Ala Tyr Arg Ser Phe Ser Asn Ser Leu Ser Gln
        1               5                   10
```

<210> 634
<211> 16
<212> PRT
<213> Homo sapiens

<400> 634

```
        Gly Pro Pro Gly Glu Ala Gly Tyr Lys Ala Phe Ser Ser Leu Leu Ala
        1               5                   10                  15
```

<210> 635
<211> 18
<212> PRT

227

<213> Homo sapiens

<400> 635

```
        Gly Pro Pro Gly Glu Ala Gly Tyr Lys Ala Phe Ser Ser Leu Leu Ala
        1               5               10                  15

        Ser Ser
```

<210> 636
<211> 19
<212> PRT
<213> Homo sapiens

<400> 636

```
        Gly Pro Pro Gly Glu Ala Gly Tyr Lys Ala Phe Ser Ser Leu Leu Ala
        1               5               10                  15

        Ser Ser Ala
```

<210> 637
<211> 23
<212> PRT
<213> Homo sapiens

<400> 637

```
        Gly Pro Pro Gly Glu Ala Gly Tyr Lys Ala Phe Ser Ser Leu Leu Ala
        1               5               10                  15

        Ser Ser Ala Val Ser Pro Glu
                        20
```

<210> 638
<211> 24
<212> PRT
<213> Homo sapiens

<400> 638

```
        Gly Pro Pro Gly Glu Ala Gly Tyr Lys Ala Phe Ser Ser Leu Leu Ala
        1               5               10                  15

        Ser Ser Ala Val Ser Pro Glu Lys
                        20
```

<210> 639
<211> 16
<212> PRT
<213> Homo sapiens

<400> 639

```
        Gly Tyr Lys Ala Phe Ser Ser Leu Leu Ala Ser Ser Ala Val Ser Pro
        1               5                   10                  15
```

<210> 640
<211> 17
<212> PRT
<213> Homo sapiens

<400> 640

```
        Gly Tyr Lys Ala Phe Ser Ser Leu Leu Ala Ser Ser Ala Val Ser Pro
        1               5                   10                  15
```

```
        Glu
```

<210> 641
<211> 15
<212> PRT
<213> Homo sapiens

<400> 641

```
        Lys Ala Phe Ser Ser Leu Leu Ala Ser Ser Ala Val Ser Pro Glu
        1               5                   10                  15
```

<210> 642
<211> 13
<212> PRT
<213> Homo sapiens

<400> 642

```
        Asn Pro Ala Tyr Arg Ser Phe Ser Asn Ser Leu Ser Gln
        1               5                   10
```

<210> 643
<211> 15
<212> PRT
<213> Homo sapiens

<400> 643

```
        Ser Arg Asp Asp Phe Gln Glu Gly Arg Glu Gly Ile Val Ala Arg
        1               5                   10                  15
```

<210> 644
<211> 13
<212> PRT
<213> Homo sapiens

<400> 644

```
        Ser Ser Ser Ser Phe His Pro Ala Pro Gly Asn Ala Gln
        1               5                   10
```

<210> 645
<211> 12

<212> PRT
<213> Homo sapiens

<400> 645

Val Ala Arg Leu Thr Glu Ser Leu Phe Leu Asp Leu
1               5                   10

<210> 646
<211> 14
<212> PRT
<213> Homo sapiens

<400> 646

Val Ala Arg Leu Thr Glu Ser Leu Phe Leu Asp Leu Leu Gly
1               5                   10

<210> 647
<211> 13
<212> PRT
<213> Homo sapiens

<400> 647

Val Ile Ala Gly Asn Pro Ala Tyr Arg Ser Phe Ser Asn
1               5                   10

<210> 648
<211> 19
<212> PRT
<213> Homo sapiens

<400> 648

Val Pro Gln Pro Glu Pro Glu Thr Trp Glu Gln Ile Leu Arg Arg Asn
1               5                   10                  15

Val Leu Gln

<210> 649
<211> 14
<212> PRT
<213> Homo sapiens

<400> 649

Tyr Lys Ala Phe Ser Ser Leu Leu Ala Ser Ser Ala Val Ser
1               5                   10

<210> 650
<211> 15
<212> PRT
<213> Homo sapiens

<400> 650

```
        Tyr Lys Ala Phe Ser Ser Leu Leu Ala Ser Ser Ala Val Ser Pro
        1               5                   10                  15
```

<210> 651
<211> 16
<212> PRT
<213> Homo sapiens


<400> 651

```
        Tyr Lys Ala Phe Ser Ser Leu Leu Ala Ser Ser Ala Val Ser Pro Glu
        1               5                   10                  15
```

<210> 652
<211> 17
<212> PRT
<213> Homo sapiens


<400> 652

```
        Gly Asn Gln Val Phe Ser Tyr Thr Ala Asn Lys Glu Ile Arg Thr Asp
        1               5                   10                  15
```

```
        Asp
```

<210> 653
<211> 14
<212> PRT
<213> Homo sapiens


<400> 653

```
            Ile Glu Glu Ile Val Leu Val Asp Asp Ala Ser Glu Arg Asp
            1               5                   10
```

<210> 654
<211> 15
<212> PRT
<213> Homo sapiens


<400> 654

```
            Ile Glu Glu Ile Val Leu Val Asp Asp Ala Ser Glu Arg Asp Phe
            1               5                   10                  15
```

<210> 655
<211> 13
<212> PRT
<213> Homo sapiens


<400> 655

```
            Leu Glu Asn Ile Tyr Pro Asp Ser Gln Ile Pro Arg His
            1               5                   10
```

<210> 656
<211> 14

<212> PRT
<213> Homo sapiens

<400> 656

Leu Glu Asn Ile Tyr Pro Asp Ser Gln Ile Pro Arg His Tyr
1               5                   10

<210> 657
<211> 13
<212> PRT
<213> Homo sapiens

<400> 657

Asn Gln Val Phe Ser Tyr Thr Ala Asn Lys Glu Ile Arg
1               5                   10

<210> 658
<211> 14
<212> PRT
<213> Homo sapiens

<400> 658

Asn Gln Val Phe Ser Tyr Thr Ala Asn Lys Glu Ile Arg Thr
1               5                   10

<210> 659
<211> 16
<212> PRT
<213> Homo sapiens

<400> 659

Asn Gln Val Phe Ser Tyr Thr Ala Asn Lys Glu Ile Arg Thr Asp Asp
1               5                   10                  15

<210> 660
<211> 15
<212> PRT
<213> Homo sapiens

<400> 660

Val His Ser Val Ile Asn Arg Ser Pro Arg His Met Ile Glu Glu
1               5                   10                  15

<210> 661
<211> 12
<212> PRT
<213> Homo sapiens

<400> 661

Glu Tyr Val Ser Leu Tyr His Gln Pro Ala Ala Met
1               5                   10

<210> 662
<211> 16
<212> PRT
<213> Homo sapiens

<400> 662

```
Ile Lys Ala Glu Tyr Lys Gly Arg Val Thr Leu Lys Gln Tyr Pro Arg
1               5                   10                  15
```

<210> 663
<211> 15
<212> PRT
<213> Homo sapiens

<400> 663

```
Leu Asn Val His Ser Glu Tyr Glu Pro Ser Trp Glu Glu Gln Pro
1               5                   10                  15
```

<210> 664
<211> 14
<212> PRT
<213> Homo sapiens

<400> 664

```
Leu Pro Tyr Leu Phe Gln Met Pro Ala Tyr Ala Ser Ser Ser
1               5                   10
```

<210> 665
<211> 15
<212> PRT
<213> Homo sapiens

<400> 665

```
Leu Pro Tyr Leu Phe Gln Met Pro Ala Tyr Ala Ser Ser Ser Lys
1               5                   10                  15
```

<210> 666
<211> 12
<212> PRT
<213> Homo sapiens

<400> 666

```
Asn Phe Ile Lys Ala Glu Tyr Lys Gly Arg Val Thr
1               5                   10
```

<210> 667
<211> 13
<212> PRT
<213> Homo sapiens

<400> 667

EP 3 157 549 B1

Thr Asn Phe Ile Lys Ala Glu Tyr Lys Gly Arg Val Thr
1               5                   10

<210> 668
<211> 14
<212> PRT
<213> Homo sapiens

<400> 668

Thr Thr Asn Phe Ile Lys Ala Glu Tyr Lys Gly Arg Val Thr
1               5                   10

<210> 669
<211> 17
<212> PRT
<213> Homo sapiens

<400> 669

Val Thr Leu Asn Val His Ser Glu Tyr Glu Pro Ser Trp Glu Glu Gln
1               5                   10                  15

Pro

<210> 670
<211> 19
<212> PRT
<213> Homo sapiens

<400> 670

Tyr Pro Arg Lys Asn Leu Phe Leu Val Glu Val Thr Gln Leu Thr Glu
1               5                   10                  15

Ser Asp Ser

<210> 671
<211> 20
<212> PRT
<213> Homo sapiens

<400> 671

Tyr Pro Arg Lys Asn Leu Phe Leu Val Glu Val Thr Gln Leu Thr Glu
1               5                   10                  15

Ser Asp Ser Gly
            20

<210> 672
<211> 12
<212> PRT
<213> Homo sapiens

<400> 672

```
        Ala Asp Leu Ser Ser Phe Lys Ser Gln Glu Leu Asn
        1               5                   10
```

<210> 673
<211> 14
<212> PRT
<213> Homo sapiens

<400> 673

```
        Ala Asp Leu Ser Ser Phe Lys Ser Gln Glu Leu Asn Glu Arg
        1               5                   10
```

<210> 674
<211> 15
<212> PRT
<213> Homo sapiens

<400> 674

```
        Ala Asp Leu Ser Ser Phe Lys Ser Gln Glu Leu Asn Glu Arg Asn
        1               5                   10                  15
```

<210> 675
<211> 16
<212> PRT
<213> Homo sapiens

<400> 675

```
        Ala Asp Leu Ser Ser Phe Lys Ser Gln Glu Leu Asn Glu Arg Asn Glu
        1               5                   10                  15
```

<210> 676
<211> 17
<212> PRT
<213> Homo sapiens

<400> 676

```
        Ala Asp Leu Ser Ser Phe Lys Ser Gln Glu Leu Asn Glu Arg Asn Glu
        1               5                   10                  15

        Ala
```

<210> 677
<211> 19
<212> PRT
<213> Homo sapiens

<400> 677

```
Ala Glu Gln Gln Arg Leu Lys Ser Gln Asp Leu Glu Leu Ser Trp Asn
1               5                   10                  15

Leu Asn Gly
```

<210> 678
<211> 15
<212> PRT
<213> Homo sapiens

<400> 678

```
Glu Gln Gln Arg Leu Lys Ser Gln Asp Leu Glu Leu Ser Trp Asn
1               5                   10                  15
```

<210> 679
<211> 14
<212> PRT
<213> Homo sapiens

<400> 679

```
Ile Ser Gln Glu Leu Glu Glu Leu Arg Ala Glu Gln Gln Arg
1               5                   10
```

<210> 680
<211> 16
<212> PRT
<213> Homo sapiens

<400> 680

```
Ile Ser Gln Glu Leu Glu Glu Leu Arg Ala Glu Gln Gln Arg Leu Lys
1               5                   10                  15
```

<210> 681
<211> 12
<212> PRT
<213> Homo sapiens

<400> 681

```
Lys Gly Thr Lys Gln Trp Val His Ala Arg Tyr Ala
1               5                   10
```

<210> 682
<211> 15
<212> PRT
<213> Homo sapiens

<400> 682

```
Gln Ala Asp Leu Ser Ser Phe Lys Ser Gln Glu Leu Asn Glu Arg
1               5                   10                  15
```

<210> 683
<211> 15

<212> PRT
<213> Homo sapiens

<400> 683

Ser Trp Asn Leu Asn Gly Leu Gln Ala Asp Leu Ser Ser Phe Lys
1               5                   10                  15

<210> 684
<211> 14
<212> PRT
<213> Homo sapiens

<400> 684

Thr Gly Ser Trp Ile Gly Leu Arg Asn Leu Asp Leu Lys Gly
1               5                   10

<210> 685
<211> 23
<212> PRT
<213> Homo sapiens

<400> 685

Phe Gly Asn Tyr Asn Asn Gln Ser Ser Asn Phe Gly Pro Met Lys Gly
1               5                   10                  15

Gly Asn Phe Gly Gly Arg Ser
                20

<210> 686
<211> 23
<212> PRT
<213> Homo sapiens

<400> 686

Phe Gly Pro Met Lys Gly Gly Asn Phe Gly Gly Arg Ser Ser Gly Pro
1               5                   10                  15

Tyr Gly Gly Gly Gly Gln Tyr
                20

<210> 687
<211> 15
<212> PRT
<213> Homo sapiens

<400> 687

Gly Pro Met Lys Gly Gly Asn Phe Gly Gly Arg Ser Ser Gly Pro
1               5                   10                  15

<210> 688
<211> 13

<212> PRT
<213> Homo sapiens

<400> 688

Gly Pro Tyr Gly Gly Gly Gly Gln Tyr Phe Ala Lys Pro
1               5                   10

<210> 689
<211> 12
<212> PRT
<213> Homo sapiens

<400> 689

Lys Gly Gly Asn Phe Gly Gly Arg Ser Ser Gly Pro
1               5                   10

<210> 690
<211> 15
<212> PRT
<213> Homo sapiens

<400> 690

Asn Asp Phe Gly Asn Tyr Asn Asn Gln Ser Ser Asn Phe Gly Pro
1               5                   10                  15

<210> 691
<211> 14
<212> PRT
<213> Homo sapiens

<400> 691

Ser Gly Pro Tyr Gly Gly Gly Gly Gln Tyr Phe Ala Lys Pro
1               5                   10

<210> 692
<211> 15
<212> PRT
<213> Homo sapiens

<400> 692

Asp Ala Gly Ser Tyr Lys Ala Gln Ile Asn Gln Arg Asn Phe Glu
1               5                   10                  15

<210> 693
<211> 17
<212> PRT
<213> Homo sapiens

<400> 693

```
          Asp Ala Gly Ser Tyr Lys Ala Gln Ile Asn Gln Arg Asn Phe Glu Val
          1               5                   10                  15

          Thr
```

<210> 694
<211> 14
<212> PRT
<213> Homo sapiens

<400> 694

```
          Asp Gly Glu Leu Ile Arg Thr Gln Pro Gln Arg Leu Pro Gln
          1               5                   10
```

<210> 695
<211> 13
<212> PRT
<213> Homo sapiens

<400> 695

```
          Gly Glu Leu Ile Arg Thr Gln Pro Gln Arg Leu Pro Gln
          1               5                   10
```

<210> 696
<211> 16
<212> PRT
<213> Homo sapiens

<400> 696

```
          Asn Pro Ser Asp Gly Glu Leu Ile Arg Thr Gln Pro Gln Arg Leu Pro
          1               5                   10                  15
```

<210> 697
<211> 17
<212> PRT
<213> Homo sapiens

<400> 697

```
          Asn Pro Ser Asp Gly Glu Leu Ile Arg Thr Gln Pro Gln Arg Leu Pro
          1               5                   10                  15


          Gln
```

<210> 698
<211> 18
<212> PRT
<213> Homo sapiens

<400> 698

```
Asn Pro Ser Asp Gly Glu Leu Ile Arg Thr Gln Pro Gln Arg Leu Pro
1               5               10              15

Gln Leu
```

<210> 699
<211> 15
<212> PRT
<213> Homo sapiens

<400> 699

```
Ala Ser Asn Asp Met Tyr His Ser Arg Ala Leu Gln Val Val Arg
1               5               10              15
```

<210> 700
<211> 16
<212> PRT
<213> Homo sapiens

<400> 700

```
Ala Ser Asn Asp Met Tyr His Ser Arg Ala Leu Gln Val Val Arg Ala
1               5               10              15
```

<210> 701
<211> 15
<212> PRT
<213> Homo sapiens

<400> 701

```
Glu Gly Val Arg Arg Ala Leu Asp Phe Ala Val Gly Glu Tyr Asn
1               5               10              15
```

<210> 702
<211> 16
<212> PRT
<213> Homo sapiens

<400> 702

```
Glu Gly Val Arg Arg Ala Leu Asp Phe Ala Val Gly Glu Tyr Asn Lys
1               5               10              15
```

<210> 703
<211> 14
<212> PRT
<213> Homo sapiens

<400> 703

```
Ser Asn Asp Met Tyr His Ser Arg Ala Leu Gln Val Val Arg
1               5               10
```

<210> 704
<211> 13

<212> PRT
<213> Homo sapiens

<400> 704

```
Val Gly Glu Tyr Asn Lys Ala Ser Asn Asp Met Tyr His
1               5                   10
```

<210> 705
<211> 13
<212> PRT
<213> Homo sapiens

<400> 705

```
Val Arg Ala Arg Lys Gln Ile Val Ala Gly Val Asn Tyr
1               5                   10
```

<210> 706
<211> 18
<212> PRT
<213> Homo sapiens

<400> 706

```
Val Arg Arg Ala Leu Asp Phe Ala Val Gly Glu Tyr Asn Lys Ala Ser
1               5                   10                  15

Asn Asp
```

<210> 707
<211> 13
<212> PRT
<213> Homo sapiens

<400> 707

```
Val Val Arg Ala Arg Lys Gln Ile Val Ala Gly Val Asn
1               5                   10
```

<210> 708
<211> 14
<212> PRT
<213> Homo sapiens

<400> 708

```
Val Val Arg Ala Arg Lys Gln Ile Val Ala Gly Val Asn Tyr
1               5                   10
```

<210> 709
<211> 14
<212> PRT
<213> Homo sapiens

<400> 709

```
Ala Pro Leu Glu Gly Ala Arg Phe Ala Leu Val Arg Glu Asp
1               5                   10
```

<210> 710
<211> 16
<212> PRT
<213> Homo sapiens


<400> 710

```
Ala Pro Val Glu Leu Ile Leu Ser Asp Glu Thr Leu Pro Ala Pro Glu
1               5                   10                  15
```

<210> 711
<211> 13
<212> PRT
<213> Homo sapiens


<400> 711

```
Glu Leu Ile Leu Ser Asp Glu Thr Leu Pro Ala Pro Glu
1               5                   10
```

<210> 712
<211> 14
<212> PRT
<213> Homo sapiens


<400> 712

```
Leu Ala Pro Leu Glu Gly Ala Arg Phe Ala Leu Val Arg Glu
1               5                   10
```

<210> 713
<211> 15
<212> PRT
<213> Homo sapiens


<400> 713

```
Leu Ala Pro Leu Glu Gly Ala Arg Phe Ala Leu Val Arg Glu Asp
1               5                   10                  15
```

<210> 714
<211> 16
<212> PRT
<213> Homo sapiens


<400> 714

```
Arg Gly Glu Lys Glu Leu Leu Val Pro Arg Ser Ser Thr Ser Pro Asp
1               5                   10                  15
```

<210> 715
<211> 16
<212> PRT
<213> Homo sapiens

<400> 715

```
        Ala Ser Lys Thr Phe Thr Thr Gln Glu Thr Ile Thr Asn Ala Glu Thr
        1               5                   10                  15
```

<210> 716
<211> 15
<212> PRT
<213> Homo sapiens

<400> 716

```
        Asp Gln His Phe Arg Thr Thr Pro Leu Glu Lys Asn Ala Pro Val
        1               5                   10                  15
```

<210> 717
<211> 14
<212> PRT
<213> Homo sapiens

<400> 717

```
        Asn Thr Pro Ile Leu Val Asp Gly Lys Asp Val Met Pro Glu
        1               5                   10
```

<210> 718
<211> 15
<212> PRT
<213> Homo sapiens

<400> 718

```
        Asn Thr Pro Ile Leu Val Asp Gly Lys Asp Val Met Pro Glu Val
        1               5                   10                  15
```

<210> 719
<211> 16
<212> PRT
<213> Homo sapiens

<400> 719

```
        Asn Thr Pro Ile Leu Val Asp Gly Lys Asp Val Met Pro Glu Val Asn
        1               5                   10                  15
```

<210> 720
<211> 15
<212> PRT
<213> Homo sapiens

<400> 720

```
        Ser Asn Thr Pro Ile Leu Val Asp Gly Lys Asp Val Met Pro Glu
        1               5                   10                  15
```

<210> 721
<211> 17
<212> PRT

<213> Homo sapiens

<400> 721

```
Ser Asn Thr Pro Ile Leu Val Asp Gly Lys Asp Val Met Pro Glu Val
1               5               10              15
```

```
Asn
```

<210> 722
<211> 12
<212> PRT
<213> Homo sapiens

<400> 722

```
Thr Pro Ile Leu Val Asp Gly Lys Asp Val Met Pro
1               5               10
```

<210> 723
<211> 13
<212> PRT
<213> Homo sapiens

<400> 723

```
Thr Pro Ile Leu Val Asp Gly Lys Asp Val Met Pro Glu

1               5                       10
```

<210> 724
<211> 14
<212> PRT
<213> Homo sapiens

<400> 724

```
Thr Pro Ile Leu Val Asp Gly Lys Asp Val Met Pro Glu Val
1               5               10
```

<210> 725
<211> 15
<212> PRT
<213> Homo sapiens

<400> 725

```
Thr Pro Ile Leu Val Asp Gly Lys Asp Val Met Pro Glu Val Asn
1               5               10              15
```

<210> 726
<211> 15
<212> PRT
<213> Homo sapiens

<400> 726

```
Gly Pro Leu Lys Phe Leu His Gln Asp Ile Asp Ser Gly Gln Gly
1               5                   10                  15
```

<210> 727
<211> 17
<212> PRT
<213> Homo sapiens

<400> 727

```
Gly Pro Leu Lys Phe Leu His Gln Asp Ile Asp Ser Gly Gln Gly Ile
1               5                   10                  15

Arg
```

<210> 728
<211> 13
<212> PRT
<213> Homo sapiens

<400> 728

```
Leu Gly Asp Ile Tyr Phe Lys Leu Phe Arg Ala Ser Gly
1               5                   10
```

<210> 729
<211> 15
<212> PRT
<213> Homo sapiens

<400> 729

```
Thr Gly His Leu Phe Asp Leu Ser Ser Leu Ser Gly Arg Ala Gly
1               5                   10                  15
```

<210> 730
<211> 16
<212> PRT
<213> Homo sapiens

<400> 730

```
Val Pro Ser Pro Val Asp Cys Gln Val Thr Asp Leu Ala Gly Asn Glu
1               5                   10                  15
```

<210> 731
<211> 18
<212> PRT
<213> Homo sapiens

<400> 731

```
Asp Gly Leu Asn Ser Leu Thr Tyr Gln Val Leu Asp Val Gln Arg Tyr
1               5               10              15

Pro Leu
```

<210> 732
<211> 14
<212> PRT
<213> Homo sapiens

<400> 732

```
His Pro Val Leu Gln Arg Gln Gln Leu Asp Tyr Gly Ile Tyr
1               5               10
```

<210> 733
<211> 13
<212> PRT
<213> Homo sapiens

<400> 733

```
Leu Asn Ser Leu Thr Tyr Gln Val Leu Asp Val Gln Arg
1               5               10
```

<210> 734
<211> 15
<212> PRT
<213> Homo sapiens

<400> 734

```
Leu Asn Ser Leu Thr Tyr Gln Val Leu Asp Val Gln Arg Tyr Pro
1               5               10              15
```

<210> 735
<211> 16
<212> PRT
<213> Homo sapiens

<400> 735

```
Leu Asn Ser Leu Thr Tyr Gln Val Leu Asp Val Gln Arg Tyr Pro Leu
1               5               10              15
```

<210> 736
<211> 13
<212> PRT
<213> Homo sapiens

<400> 736

```
Leu Pro Gln Leu Val Gly Val Ser Thr Pro Leu Gln Gly
1               5               10
```

<210> 737
<211> 14

<212> PRT
<213> Homo sapiens

<400> 737

Leu Pro Gln Leu Val Gly Val Ser Thr Pro Leu Gln Gly Gly
1               5                   10

<210> 738
<211> 15
<212> PRT
<213> Homo sapiens

<400> 738

Leu Pro Gln Leu Val Gly Val Ser Thr Pro Leu Gln Gly Gly Ser
1               5                   10                  15

<210> 739
<211> 16
<212> PRT
<213> Homo sapiens

<400> 739

Arg Leu Pro Gln Leu Val Gly Val Ser Thr Pro Leu Gln Gly Gly Ser
1               5                   10                  15

<210> 740
<211> 14
<212> PRT
<213> Homo sapiens

<400> 740

Ser Pro His Lys Val Ala Ile Ile Ile Pro Phe Arg Asn Arg
1               5                   10

<210> 741
<211> 16
<212> PRT
<213> Homo sapiens

<400> 741

Ser Pro His Lys Val Ala Ile Ile Ile Pro Phe Arg Asn Arg Gln Glu
1               5                   10                  15

<210> 742
<211> 17
<212> PRT
<213> Homo sapiens

<400> 742

```
Ser Pro His Lys Val Ala Ile Ile Ile Pro Phe Arg Asn Arg Gln Glu
1               5                   10                  15

        His
```

<210> 743
<211> 12
<212> PRT
<213> Homo sapiens

<400> 743

```
        Ala Ile Val Gln Ala Val Ser Ala His Arg His Arg
        1               5                   10
```

<210> 744
<211> 13
<212> PRT
<213> Homo sapiens

<400> 744

```
        Ala Arg Asn Phe Glu Arg Asn Lys Ala Ile Lys Val Ile
        1               5                   10
```

<210> 745
<211> 15
<212> PRT
<213> Homo sapiens

<400> 745

```
        Ala Arg Asn Phe Glu Arg Asn Lys Ala Ile Lys Val Ile Ile Ala
        1               5                   10                  15
```

<210> 746
<211> 12
<212> PRT
<213> Homo sapiens

<400> 746

```
        Asn Phe Glu Arg Asn Lys Ala Ile Lys Val Ile Ile
        1               5                   10
```

<210> 747
<211> 13
<212> PRT
<213> Homo sapiens

<400> 747

```
        Asn Phe Glu Arg Asn Lys Ala Ile Lys Val Ile Ile Ala
        1               5                   10
```

<210> 748
<211> 12

<212> PRT
<213> Homo sapiens

<400> 748

```
Val Ala Ile Val Gln Ala Val Ser Ala His Arg His
1               5                   10
```

<210> 749
<211> 13
<212> PRT
<213> Homo sapiens

<400> 749

```
Val Ala Ile Val Gln Ala Val Ser Ala His Arg His Arg
1               5                   10
```

<210> 750
<211> 14
<212> PRT
<213> Homo sapiens

<400> 750

```
Val Ala Ile Val Gln Ala Val Ser Ala His Arg His Arg Ala
1               5                   10
```

<210> 751
<211> 15
<212> PRT
<213> Homo sapiens

<400> 751

```
Val Ala Ile Val Gln Ala Val Ser Ala His Arg His Arg Ala Arg
1               5                   10                  15
```

<210> 752
<211> 14
<212> PRT
<213> Homo sapiens

<400> 752

```
Glu Glu Val Ile Thr Leu Ile Arg Ser Asn Gln Gln Leu Glu
1               5                   10
```

<210> 753
<211> 15
<212> PRT
<213> Homo sapiens

<400> 753

```
Glu Glu Val Ile Thr Leu Ile Arg Ser Asn Gln Gln Leu Glu Asn
1               5                   10                  15
```

<210> 754
<211> 16
<212> PRT
<213> Homo sapiens

<400> 754

```
Ile Pro Ala Asp Thr Phe Ala Ala Leu Lys Asn Pro Asn Ala Met Leu
1               5               10              15
```

<210> 755
<211> 15
<212> PRT
<213> Homo sapiens

<400> 755

```
Leu Lys Gln Leu Leu Ser Asp Lys Gln Gln Lys Arg Gln Ser Gly
1               5               10              15
```

<210> 756
<211> 16
<212> PRT
<213> Homo sapiens

<400> 756

```
Leu Lys Gln Leu Leu Ser Asp Lys Gln Gln Lys Arg Gln Ser Gly Gln
1               5               10              15
```

<210> 757
<211> 12
<212> PRT
<213> Homo sapiens

<400> 757

```
Thr Pro Ser Tyr Val Ala Phe Thr Asp Thr Glu Arg
1               5               10
```

<210> 758
<211> 13
<212> PRT
<213> Homo sapiens

<400> 758

```
Thr Pro Ser Tyr Val Ala Phe Thr Asp Thr Glu Arg Leu
1               5               10
```

<210> 759
<211> 13
<212> PRT
<213> Homo sapiens

<400> 759

Glu Gly Leu Tyr Ser Arg Thr Leu Ala Gly Ser Ile Thr
1               5               10

<210> 760
<211> 16
<212> PRT
<213> Homo sapiens

<400> 760

Glu Gly Leu Tyr Ser Arg Thr Leu Ala Gly Ser Ile Thr Thr Pro Pro
1               5                   10                  15

<210> 761
<211> 13
<212> PRT
<213> Homo sapiens

<400> 761

Glu Lys Trp Tyr Ile Pro Asp Pro Thr Gly Lys Phe Asn
1               5                   10

<210> 762
<211> 14
<212> PRT
<213> Homo sapiens

<400> 762

Gly Ala Ile Ala Ala Ile Asn Ser Ile Gln His Asn Thr Arg

        1               5                   10

<210> 763
<211> 12
<212> PRT
<213> Homo sapiens

<400> 763

Leu Pro Ile Leu Val Pro Ser Ala Lys Lys Ala Ile
1               5                   10

<210> 764
<211> 13
<212> PRT
<213> Homo sapiens

<400> 764

Leu Pro Ile Leu Val Pro Ser Ala Lys Lys Ala Ile Tyr
1               5                   10

<210> 765
<211> 14

<212> PRT
<213> Homo sapiens

<400> 765

```
Leu Pro Ile Leu Val Pro Ser Ala Lys Lys Ala Ile Tyr Met
1               5                   10
```

<210> 766
<211> 15
<212> PRT
<213> Homo sapiens

<400> 766

```
Leu Pro Ile Leu Val Pro Ser Ala Lys Lys Ala Ile Tyr Met Asp
1               5                   10                  15
```

<210> 767
<211> 16
<212> PRT
<213> Homo sapiens

<400> 767

```
Leu Pro Ile Leu Val Pro Ser Ala Lys Lys Ala Ile Tyr Met Asp Asp
1               5                   10                  15
```

<210> 768
<211> 15
<212> PRT
<213> Homo sapiens

<400> 768

```
Val Glu Glu Gly Leu Tyr Ser Arg Thr Leu Ala Gly Ser Ile Thr
1               5                   10                  15
```

<210> 769
<211> 14
<212> PRT
<213> Homo sapiens

<400> 769

```
Trp Glu Lys Trp Tyr Ile Pro Asp Pro Thr Gly Lys Phe Asn
1               5                   10
```

<210> 770
<211> 12
<212> PRT
<213> Homo sapiens

<400> 770

```
Tyr Lys Ile Val Asn Phe Asp Pro Lys Leu Leu Glu
1               5                   10
```

<210> 771
<211> 13
<212> PRT
<213> Homo sapiens

<400> 771

```
Tyr Lys Ile Val Asn Phe Asp Pro Lys Leu Leu Glu Gly
1               5                   10
```

<210> 772
<211> 15
<212> PRT
<213> Homo sapiens

<400> 772

```
Tyr Lys Ile Val Asn Phe Asp Pro Lys Leu Leu Glu Gly Lys Val
1               5                   10                  15
```

<210> 773
<211> 12
<212> PRT
<213> Homo sapiens

<400> 773

```
Leu Pro Glu Phe Tyr Lys Thr Val Ser Pro Ala Leu
1               5                   10
```

<210> 774
<211> 14
<212> PRT
<213> Homo sapiens

<400> 774

```
Val Gly Gln Phe Ile Gln Asp Val Lys Asn Ser Arg Ser Thr
1               5                   10
```

<210> 775
<211> 15
<212> PRT
<213> Homo sapiens

<400> 775

```
Val Gly Gln Phe Ile Gln Asp Val Lys Asn Ser Arg Ser Thr Asp
1               5                   10                  15
```

<210> 776
<211> 14
<212> PRT
<213> Homo sapiens

<400> 776

```
        Val Val Gly Gln Phe Ile Gln Asp Val Lys Asn Ser Arg Ser
        1               5               10
```

<210> 777
<211> 15
<212> PRT
<213> Homo sapiens

<400> 777

```
        Val Val Gly Gln Phe Ile Gln Asp Val Lys Asn Ser Arg Ser Thr
        1               5                   10                  15
```

<210> 778
<211> 16
<212> PRT
<213> Homo sapiens

<400> 778

```
        Val Val Gly Gln Phe Ile Gln Asp Val Lys Asn Ser Arg Ser Thr Asp
        1               5                   10                  15
```

<210> 779
<211> 17
<212> PRT
<213> Homo sapiens

<400> 779

```
        Val Val Gly Gln Phe Ile Gln Asp Val Lys Asn Ser Arg Ser Thr Asp
        1               5                   10                  15

                                        Ser
```

<210> 780
<211> 16
<212> PRT
<213> Homo sapiens

<400> 780

```
        Asp Asn Gly His Leu Tyr Arg Glu Asp Gln Thr Ser Pro Ala Pro Gly
        1               5                   10                  15
```

<210> 781
<211> 18
<212> PRT
<213> Homo sapiens

<400> 781

```
        Asp Asn Gly His Leu Tyr Arg Glu Asp Gln Thr Ser Pro Ala Pro Gly
        1               5                   10                  15


        Leu Arg
```

<210> 782
<211> 16
<212> PRT
<213> Homo sapiens

<400> 782

```
Glu Val Gln Val Phe Ala Pro Ala Asn Ala Leu Pro Ala Arg Ser Glu
1               5                   10                  15
```

<210> 783
<211> 14
<212> PRT
<213> Homo sapiens

<400> 783

```
Gly His Leu Tyr Arg Glu Asp Gln Thr Ser Pro Ala Pro Gly
1               5                   10
```

<210> 784
<211> 15
<212> PRT
<213> Homo sapiens

<400> 784

```
Leu Pro Ala Arg Ser Glu Ala Ala Ala Val Gln Pro Val Ile Gly
1               5                   10                  15
```

<210> 785
<211> 15
<212> PRT
<213> Homo sapiens

<400> 785

```
Asn Gly His Leu Tyr Arg Glu Asp Gln Thr Ser Pro Ala Pro Gly
1               5                   10                  15
```

<210> 786
<211> 16
<212> PRT
<213> Homo sapiens

<400> 786

```
Asn Gly His Leu Tyr Arg Glu Asp Gln Thr Ser Pro Ala Pro Gly Leu
1               5                   10                  15
```

<210> 787
<211> 17
<212> PRT
<213> Homo sapiens

<400> 787

```
Asn Gly His Leu Tyr Arg Glu Asp Gln Thr Ser Pro Ala Pro Gly Leu
1               5                   10                  15
```

```
Arg
```

<210> 788
<211> 15
<212> PRT
<213> Homo sapiens

<400> 788

```
Val Phe Ala Pro Ala Asn Ala Leu Pro Ala Arg Ser Glu Ala Ala
1               5                   10                  15
```

<210> 789
<211> 15
<212> PRT
<213> Homo sapiens

<400> 789

```
Val Gln Val Phe Ala Pro Ala Asn Ala Leu Pro Ala Arg Ser Glu
1               5                   10                  15
```

<210> 790
<211> 14
<212> PRT
<213> Homo sapiens

<400> 790

```
Ala Ile Val Val Ser Asp Arg Asp Gly Val Pro Val Ile Lys
1               5                   10
```

<210> 791
<211> 15
<212> PRT
<213> Homo sapiens

<400> 791

```
Gly Leu His Ala Ile Val Val Ser Asp Arg Asp Gly Val Pro Val
1               5                   10                  15
```

<210> 792
<211> 17
<212> PRT
<213> Homo sapiens

<400> 792

```
Gly Leu His Ala Ile Val Val Ser Asp Arg Asp Gly Val Pro Val Ile
1               5                   10                  15
```

```
Lys
```

<210> 793
<211> 13
<212> PRT
<213> Homo sapiens

<400> 793

```
His Ala Ile Val Val Ser Asp Arg Asp Gly Val Pro Val
1               5                   10
```

<210> 794
<211> 18
<212> PRT
<213> Homo sapiens

<400> 794

```
Lys Leu Pro Ser Val Glu Gly Leu His Ala Ile Val Val Ser Asp Arg
1               5                   10                  15

Asp Gly
```

<210> 795
<211> 14
<212> PRT
<213> Homo sapiens

<400> 795

```
Leu His Ala Ile Val Val Ser Asp Arg Asp Gly Val Pro Val
1               5                   10
```

<210> 796
<211> 15
<212> PRT
<213> Homo sapiens

<400> 796

```
Leu His Ala Ile Val Val Ser Asp Arg Asp Gly Val Pro Val Ile
1               5                   10                  15
```

<210> 797
<211> 16
<212> PRT
<213> Homo sapiens

<400> 797

```
Leu His Ala Ile Val Val Ser Asp Arg Asp Gly Val Pro Val Ile Lys
1               5                   10                  15
```

<210> 798
<211> 15
<212> PRT
<213> Homo sapiens

<400> 798

```
        Leu Pro Ser Val Glu Gly Leu His Ala Ile Val Val Ser Asp Arg
        1               5                   10                  15
```

<210> 799
<211> 13
<212> PRT
<213> Homo sapiens

<400> 799

```
        Val Pro Val Ile Lys Val Ala Asn Asp Asn Ala Pro Glu
        1               5                   10
```

<210> 800
<211> 13
<212> PRT
<213> Homo sapiens

<400> 800

```
        Tyr Asn Thr Tyr Gln Val Val Gln Phe Asn Arg Leu Pro
        1               5                   10
```

<210> 801
<211> 14
<212> PRT
<213> Homo sapiens

<400> 801

```
        Tyr Asn Thr Tyr Gln Val Val Gln Phe Asn Arg Leu Pro Leu
        1               5                   10
```

<210> 802
<211> 15
<212> PRT
<213> Homo sapiens

<400> 802

```
        Tyr Asn Thr Tyr Gln Val Val Gln Phe Asn Arg Leu Pro Leu Val
        1               5                   10                  15
```

<210> 803
<211> 16
<212> PRT
<213> Homo sapiens

<400> 803

```
        Tyr Asn Thr Tyr Gln Val Val Gln Phe Asn Arg Leu Pro Leu Val Val
        1               5                   10                  15
```

<210> 804
<211> 14
<212> PRT

<213> Homo sapiens

<400> 804

```
        Tyr Tyr Asn Thr Tyr Gln Val Val Gln Phe Asn Arg Leu Pro
        1               5                   10
```

<210> 805
<211> 15
<212> PRT
<213> Homo sapiens

<400> 805

```
      Tyr Tyr Asn Thr Tyr Gln Val Val Gln Phe Asn Arg Leu Pro Leu
      1               5                   10                  15
```

<210> 806
<211> 16
<212> PRT
<213> Homo sapiens

<400> 806

```
    Tyr Tyr Asn Thr Tyr Gln Val Val Gln Phe Asn Arg Leu Pro Leu Val


        1               5                   10                  15
```

<210> 807
<211> 13
<212> PRT
<213> Homo sapiens

<400> 807

```
        Asp Lys Ile Tyr Phe Met Ala Gly Ser Ser Arg Lys Glu
        1               5                   10
```

<210> 808
<211> 20
<212> PRT
<213> Homo sapiens

<400> 808

```
    Asp Val Gly Thr Asp Glu Glu Glu Glu Thr Ala Lys Glu Ser Thr Ala
    1               5                   10                  15

    Glu Lys Asp Glu
                20
```

<210> 809
<211> 15
<212> PRT
<213> Homo sapiens

<400> 809

```
        Glu Val Thr Phe Lys Ser Ile Leu Phe Val Pro Thr Ser Ala Pro
        1               5               10                  15
```

<210> 810
<211> 13
<212> PRT
<213> Homo sapiens

<400> 810

```
        Lys Ser Glu Lys Phe Ala Phe Gln Ala Glu Val Asn Arg
        1               5               10
```

<210> 811
<211> 14
<212> PRT
<213> Homo sapiens

<400> 811

```
        Leu Pro Glu Phe Asp Gly Lys Arg Phe Gln Asn Val Ala Lys
        1               5               10
```

<210> 812
<211> 13
<212> PRT
<213> Homo sapiens

<400> 812

```
        Asp Gly Ser Tyr Arg Ile Phe Ser Lys Gly Ala Ser Glu
        1               5               10
```

<210> 813
<211> 12
<212> PRT
<213> Homo sapiens

<400> 813

```
        Gly Ser Tyr Arg Ile Phe Ser Lys Gly Ala Ser Glu
        1               5               10
```

<210> 814
<211> 14
<212> PRT
<213> Homo sapiens

<400> 814

```
        Ser Asp Gly Ser Tyr Arg Ile Phe Ser Lys Gly Ala Ser Glu
        1               5               10
```

<210> 815
<211> 14
<212> PRT

<213> Homo sapiens

<400> 815

Ser Val Lys Lys Met Met Lys Asp Asn Asn Leu Val Arg His
1               5                   10

<210> 816
<211> 13
<212> PRT
<213> Homo sapiens

<400> 816

Val Lys Lys Met Met Lys Asp Asn Asn Leu Val Arg His
1               5                   10

<210> 817
<211> 13
<212> PRT
<213> Homo sapiens

<400> 817

Asn Asn Met Arg Ile Phe Gly Glu Ala Ala Glu Lys Asn
1               5                   10

<210> 818
<211> 13
<212> PRT
<213> Homo sapiens

<400> 818

Val Asp Lys Val Leu Glu Arg Asp Gln Lys Leu Ser Glu
1               5                   10

<210> 819
<211> 15
<212> PRT
<213> Homo sapiens

<400> 819

Val Asp Lys Val Leu Glu Arg Asp Gln Lys Leu Ser Glu Leu Asp
1               5                   10                  15

<210> 820
<211> 16
<212> PRT
<213> Homo sapiens

<400> 820

Val Asp Lys Val Leu Glu Arg Asp Gln Lys Leu Ser Glu Leu Asp Asp
1               5                   10                  15

<210> 821

<211> 17
<212> PRT
<213> Homo sapiens

<400> 821

```
Val Asp Lys Val Leu Glu Arg Asp Gln Lys Leu Ser Glu Leu Asp Asp
1               5                   10                  15

Arg
```

<210> 822
<211> 14
<212> PRT
<213> Homo sapiens

<400> 822

```
Val Leu Glu Arg Asp Gln Lys Leu Ser Glu Leu Asp Asp Arg
1               5                   10
```

<210> 823
<211> 15
<212> PRT
<213> Homo sapiens

<400> 823

```
Ala Thr Arg Ser Ile Gln Val Asp Gly Lys Thr Ile Lys Ala Gln
1               5                   10                  15
```

<210> 824
<211> 16
<212> PRT
<213> Homo sapiens

<400> 824

```
Ala Thr Arg Ser Ile Gln Val Asp Gly Lys Thr Ile Lys Ala Gln Ile
1               5                   10                  15
```

<210> 825
<211> 15
<212> PRT
<213> Homo sapiens

<400> 825

```
Ile Gly Val Glu Phe Ala Thr Arg Ser Ile Gln Val Asp Gly Lys
1               5                   10                  15
```

<210> 826
<211> 12
<212> PRT
<213> Homo sapiens

<400> 826

```
                    Arg Ser Ile Gln Val Asp Gly Lys Thr Ile Lys Ala
                    1               5               10
```

<210> 827
<211> 13
<212> PRT
<213> Homo sapiens


<400> 827

```
                Arg Ser Ile Gln Val Asp Gly Lys Thr Ile Lys Ala Gln
                1               5               10
```

<210> 828
<211> 14
<212> PRT
<213> Homo sapiens


<400> 828

```
                Arg Ser Ile Gln Val Asp Gly Lys Thr Ile Lys Ala Gln Ile
                1               5               10
```

<210> 829
<211> 14
<212> PRT
<213> Homo sapiens


<400> 829

```
                Thr Arg Ser Ile Gln Val Asp Gly Lys Thr Ile Lys Ala Gln
                1               5               10
```

<210> 830
<211> 16
<212> PRT
<213> Homo sapiens


<400> 830

```
            Asp Ile Met Arg Val Asn Val Asp Lys Val Leu Glu Arg Asp Gln Lys
            1               5               10              15
```

<210> 831
<211> 17
<212> PRT
<213> Homo sapiens


<400> 831

```
            Asp Ile Met Arg Val Asn Val Asp Lys Val Leu Glu Arg Asp Gln Lys
            1               5               10              15
```

```
            Leu
```

<210> 832
<211> 15

<212> PRT
<213> Homo sapiens

<400> 832

Ile Met Arg Val Asn Val Asp Lys Val Leu Glu Arg Asp Gln Lys
1               5                   10                  15

<210> 833
<211> 13
<212> PRT
<213> Homo sapiens

<400> 833

Val Asp Lys Val Leu Glu Arg Asp Gln Lys Leu Ser Glu
1               5                   10

<210> 834
<211> 15
<212> PRT
<213> Homo sapiens

<400> 834

Val Asp Lys Val Leu Glu Arg Asp Gln Lys Leu Ser Glu Leu Asp
1               5                   10                  15

<210> 835
<211> 16
<212> PRT
<213> Homo sapiens

<400> 835

Val Asp Lys Val Leu Glu Arg Asp Gln Lys Leu Ser Glu Leu Asp Asp
1               5                   10                  15

<210> 836
<211> 17
<212> PRT
<213> Homo sapiens

<400> 836

Val Asp Lys Val Leu Glu Arg Asp Gln Lys Leu Ser Glu Leu Asp Asp
1               5                   10                  15

Arg

<210> 837
<211> 14
<212> PRT
<213> Homo sapiens

<400> 837

Val Leu Glu Arg Asp Gln Lys Leu Ser Glu Leu Asp Asp Arg
1               5                   10

<210> 838
<211> 15
<212> PRT
<213> Homo sapiens

<400> 838

Ala Thr Arg Ser Ile Gln Val Asp Gly Lys Thr Ile Lys Ala Gln
1               5                   10                  15

<210> 839
<211> 16
<212> PRT
<213> Homo sapiens

<400> 839

Ala Thr Arg Ser Ile Gln Val Asp Gly Lys Thr Ile Lys Ala Gln Ile
1               5                   10                  15

<210> 840
<211> 15
<212> PRT
<213> Homo sapiens

<400> 840

Ile Gly Val Glu Phe Ala Thr Arg Ser Ile Gln Val Asp Gly Lys
1               5                   10                  15

<210> 841
<211> 12
<212> PRT
<213> Homo sapiens

<400> 841

Arg Ser Ile Gln Val Asp Gly Lys Thr Ile Lys Ala
1               5                   10

<210> 842
<211> 13
<212> PRT
<213> Homo sapiens

<400> 842

Arg Ser Ile Gln Val Asp Gly Lys Thr Ile Lys Ala Gln
1               5                   10

<210> 843
<211> 14
<212> PRT
<213> Homo sapiens

<400> 843

```
Arg Ser Ile Gln Val Asp Gly Lys Thr Ile Lys Ala Gln Ile
1               5                   10
```

<210> 844
<211> 14
<212> PRT
<213> Homo sapiens

<400> 844

```
Thr Arg Ser Ile Gln Val Asp Gly Lys Thr Ile Lys Ala Gln
1               5                   10
```

<210> 845
<211> 12
<212> PRT
<213> Homo sapiens

<400> 845

```
Gly Ile Arg Val Ala Pro Val Pro Leu Tyr Asn Ser
1               5                   10
```

<210> 846
<211> 14
<212> PRT
<213> Homo sapiens

<400> 846

```
Gly Ile Arg Val Ala Pro Val Pro Leu Tyr Asn Ser Phe His
1               5                   10
```

<210> 847
<211> 17
<212> PRT
<213> Homo sapiens

<400> 847

```
Asn Pro Asn Gly Ile Arg Val Ala Pro Val Pro Leu Tyr Asn Ser Phe
1               5                   10                  15

His
```

<210> 848
<211> 17
<212> PRT
<213> Homo sapiens

<400> 848

```
Asp Asp Pro Ala Ile Asp Val Cys Lys Lys Leu Leu Gly Lys Tyr Pro
1               5                   10                  15

Asn
```

<210> 849
<211> 16
<212> PRT
<213> Homo sapiens

<400> 849

```
Asp Lys Gln Pro Tyr Ser Lys Leu Pro Gly Val Ser Leu Leu Lys Pro
1               5                   10                  15
```

<210> 850
<211> 17
<212> PRT
<213> Homo sapiens

<400> 850

```
Asp Lys Gln Pro Tyr Ser Lys Leu Pro Gly Val Ser Leu Leu Lys Pro
1               5                   10                  15

Leu
```

<210> 851
<211> 14
<212> PRT
<213> Homo sapiens

<400> 851

```
His Pro Arg Tyr Tyr Ile Ser Ala Asn Val Thr Gly Phe Lys
1               5                   10
```

<210> 852
<211> 13
<212> PRT
<213> Homo sapiens

<400> 852

```
Ser His Pro Arg Tyr Tyr Ile Ser Ala Asn Val Thr Gly
1               5                   10
```

<210> 853
<211> 15
<212> PRT
<213> Homo sapiens

<400> 853

```
Ser His Pro Arg Tyr Tyr Ile Ser Ala Asn Val Thr Gly Phe Lys
1               5                   10                  15
```

<210> 854
<211> 14
<212> PRT
<213> Homo sapiens

<400> 854

```
        Thr Ser His Pro Arg Tyr Tyr Ile Ser Ala Asn Val Thr Gly
        1                   5                   10
```

<210> 855
<211> 16
<212> PRT
<213> Homo sapiens

<400> 855

```
    Thr Ser His Pro Arg Tyr Tyr Ile Ser Ala Asn Val Thr Gly Phe Lys


        1                   5                   10                  15
```

<210> 856
<211> 12
<212> PRT
<213> Homo sapiens

<400> 856

```
            Ala Asp Ile Phe Val Asp Pro Val Leu His Thr Ala
            1                   5                   10
```

<210> 857
<211> 14
<212> PRT
<213> Homo sapiens

<400> 857

```
        Ala Asp Ile Phe Val Asp Pro Val Leu His Thr Ala Cys Ala
        1                   5                   10
```

<210> 858
<211> 15
<212> PRT
<213> Homo sapiens

<400> 858

```
        Asp Pro Gly Ala Asp Tyr Arg Ile Asp Arg Ala Leu Asn Glu Ala
        1                   5                   10                  15
```

<210> 859
<211> 12
<212> PRT
<213> Homo sapiens

<400> 859

```
Ile Ala Gln Asp Tyr Lys Val Ser Tyr Ser Leu Ala
1               5                   10
```

<210> 860
<211> 13
<212> PRT
<213> Homo sapiens

<400> 860

```
Ile Ala Gln Asp Tyr Lys Val Ser Tyr Ser Leu Ala Lys
1               5                   10
```

<210> 861
<211> 14
<212> PRT
<213> Homo sapiens

<400> 861

```
Ile Ser Arg Asp Trp Lys Leu Asp Pro Val Leu Tyr Arg Lys
1               5                   10
```

<210> 862
<211> 13
<212> PRT
<213> Homo sapiens

<400> 862

```
Leu Ile Ala Gln Asp Tyr Lys Val Ser Tyr Ser Leu Ala
1               5                   10
```

<210> 863
<211> 14
<212> PRT
<213> Homo sapiens

<400> 863

```
Arg Gln Lys Leu Ile Ala Gln Asp Tyr Lys Val Ser Tyr Ser
1               5                   10
```

<210> 864
<211> 15
<212> PRT
<213> Homo sapiens

<400> 864

```
Arg Gln Lys Leu Ile Ala Gln Asp Tyr Lys Val Ser Tyr Ser Leu
1               5                   10                  15
```

<210> 865
<211> 16
<212> PRT

<213> Homo sapiens

<400> 865

```
        Arg Gln Lys Leu Ile Ala Gln Asp Tyr Lys Val Ser Tyr Ser Leu Ala
        1               5                   10                  15
```

<210> 866
<211> 17
<212> PRT
<213> Homo sapiens

<400> 866

```
        Arg Gln Lys Leu Ile Ala Gln Asp Tyr Lys Val Ser Tyr Ser Leu Ala
        1               5                   10                  15


        Lys
```

<210> 867
<211> 14
<212> PRT
<213> Homo sapiens

<400> 867

```
        Ser Ala Leu Asp Tyr Arg Leu Asp Pro Gln Leu Gln Leu His
        1               5                   10
```

<210> 868
<211> 14
<212> PRT
<213> Homo sapiens

<400> 868

```
        Ser Lys Ala Asp Ile Phe Val Asp Pro Val Leu His Thr Ala
        1               5                   10
```

<210> 869
<211> 15
<212> PRT
<213> Homo sapiens

<400> 869

```
        Ser Pro Ser Lys Asn Tyr Ile Leu Ser Val Ile Ser Gly Ser Ile
        1               5                   10                  15
```

<210> 870
<211> 17
<212> PRT
<213> Homo sapiens

<400> 870

```
        Glu Thr Thr Gln Leu Thr Ala Asp Ser His Pro Ser Tyr His Thr Asp
        1               5                   10                  15

        Gly
```

<210> 871
<211> 19
<212> PRT
<213> Homo sapiens

<400> 871

```
        Ser Gly Glu Ser Leu Tyr His Val Leu Gly Leu Asp Lys Asn Ala Thr
        1               5                   10                  15

        Ser Asp Asp
```

<210> 872
<211> 14
<212> PRT
<213> Homo sapiens

<400> 872

```
            Thr Thr Gln Leu Thr Ala Asp Ser His Pro Ser Tyr His Thr
            1               5                   10
```

<210> 873
<211> 15
<212> PRT
<213> Homo sapiens

<400> 873

```
        Thr Thr Gln Leu Thr Ala Asp Ser His Pro Ser Tyr His Thr Asp
        1               5                   10                  15
```

<210> 874
<211> 16
<212> PRT
<213> Homo sapiens

<400> 874

```
        Thr Thr Gln Leu Thr Ala Asp Ser His Pro Ser Tyr His Thr Asp Gly
        1               5                   10                  15
```

<210> 875
<211> 13
<212> PRT
<213> Homo sapiens

<400> 875

```
            Ser Val Glu Glu Phe Leu Ser Glu Lys Leu Glu Arg Ile
            1               5                   10
```

<210> 876
<211> 12
<212> PRT
<213> Homo sapiens

<400> 876

Val Glu Glu Phe Leu Ser Glu Lys Leu Glu Arg Ile
1               5                   10

<210> 877
<211> 15
<212> PRT
<213> Homo sapiens

<400> 877

Asp Leu Ser Ser Ser Ile Leu Ala Gln Ser Arg Glu Arg Val Ala
1               5                   10                  15

<210> 878
<211> 16
<212> PRT
<213> Homo sapiens

<400> 878

Glu Lys Gly Val Arg Thr Leu Thr Ala Ala Ala Val Ser Gly Ala Gln
1               5                   10                  15

<210> 879
<211> 17
<212> PRT
<213> Homo sapiens

<400> 879

Glu Lys Gly Val Arg Thr Leu Thr Ala Ala Ala Val Ser Gly Ala Gln
1               5                   10                  15

Pro

<210> 880
<211> 18
<212> PRT
<213> Homo sapiens

<400> 880

Glu Lys Gly Val Arg Thr Leu Thr Ala Ala Ala Val Ser Gly Ala Gln
1               5                   10                  15

Pro Ile

<210> 881
<211> 14

<212> PRT
<213> Homo sapiens

<400> 881

Lys Gly Val Arg Thr Leu Thr Ala Ala Ala Val Ser Gly Ala
1               5                   10

<210> 882
<211> 15
<212> PRT
<213> Homo sapiens

<400> 882

Lys Gly Val Arg Thr Leu Thr Ala Ala Ala Val Ser Gly Ala Gln
1               5                   10                  15

<210> 883
<211> 17
<212> PRT
<213> Homo sapiens

<400> 883

Val Gly Pro Phe Ala Pro Gly Ile Thr Glu Lys Ala Pro Glu Glu Lys
1               5                   10                  15

Lys

<210> 884
<211> 14
<212> PRT
<213> Homo sapiens

<400> 884

Asp Pro Pro Leu Ile Ala Leu Asp Lys Asp Ala Pro Leu Arg
1               5                   10

<210> 885
<211> 15
<212> PRT
<213> Homo sapiens

<400> 885

Glu Ile Ile Thr Pro Asp Val Pro Phe Thr Val Asp Lys Asp Gly
1               5                   10                  15

<210> 886
<211> 14
<212> PRT
<213> Homo sapiens

<400> 886

```
Ile Ile Thr Pro Asp Val Pro Phe Thr Val Asp Lys Asp Gly
1               5                   10
```

<210> 887
<211> 13
<212> PRT
<213> Homo sapiens


<400> 887

```
Pro Pro Leu Ile Ala Leu Asp Lys Asp Ala Pro Leu Arg
1               5                   10
```

<210> 888
<211> 14
<212> PRT
<213> Homo sapiens


<400> 888

```
Thr Asn Val Lys Lys Ser His Lys Ala Thr Val His Ile Gln
1               5                   10
```

<210> 889
<211> 12
<212> PRT
<213> Homo sapiens


<400> 889

```
Asp Asp Asn Ile Lys Thr Tyr Ser Asp His Pro Glu
1               5                   10
```

<210> 890
<211> 13
<212> PRT
<213> Homo sapiens


<400> 890

```
Asp Asp Asn Ile Lys Thr Tyr Ser Asp His Pro Glu Lys
1               5                   10
```

<210> 891
<211> 14
<212> PRT
<213> Homo sapiens


<400> 891

```
Asp Ser Ala Val Phe Phe Glu Gln Gly Thr Thr Arg Ile Gly
1               5                   10
```

<210> 892
<211> 16
<212> PRT
<213> Homo sapiens

<400> 892

```
Gly Asp Lys Val Tyr Val His Leu Lys Asn Leu Ala Ser Arg Pro Tyr
1               5                   10                  15
```

<210> 893
<211> 17
<212> PRT
<213> Homo sapiens

<400> 893

```
Gly Asp Lys Val Tyr Val His Leu Lys Asn Leu Ala Ser Arg Pro Tyr
1               5                   10                  15

Thr
```

<210> 894
<211> 12
<212> PRT
<213> Homo sapiens

<400> 894

```
Val His Leu Lys Asn Leu Ala Ser Arg Pro Tyr Thr
1               5                   10
```

<210> 895
<211> 13
<212> PRT
<213> Homo sapiens

<400> 895

```
Val Tyr Val His Leu Lys Asn Leu Ala Ser Arg Pro Tyr
1               5                   10
```

<210> 896
<211> 14
<212> PRT
<213> Homo sapiens

<400> 896

```
Val Tyr Val His Leu Lys Asn Leu Ala Ser Arg Pro Tyr Thr
1               5                   10
```

<210> 897
<211> 16
<212> PRT
<213> Homo sapiens

<400> 897

```
Val Tyr Val His Leu Lys Asn Leu Ala Ser Arg Pro Tyr Thr Phe His
1               5                   10                  15
```

<210> 898
<211> 12
<212> PRT
<213> Homo sapiens

<400> 898

```
    Tyr Val His Leu Lys Asn Leu Ala Ser Arg Pro Tyr
    1               5                   10
```

<210> 899
<211> 13
<212> PRT
<213> Homo sapiens

<400> 899

```
    Tyr Val His Leu Lys Asn Leu Ala Ser Arg Pro Tyr Thr
    1               5                   10
```

<210> 900
<211> 15
<212> PRT
<213> Homo sapiens

<400> 900

```
    Tyr Val His Leu Lys Asn Leu Ala Ser Arg Pro Tyr Thr Phe His
    1               5                   10                  15
```

<210> 901
<211> 14
<212> PRT
<213> Homo sapiens

<400> 901

```
    Ser Asn Leu Ile Lys Leu Ala Gln Lys Val Pro Thr Ala Asp
    1               5                   10
```

<210> 902
<211> 12
<212> PRT
<213> Homo sapiens

<400> 902

```
    Tyr Asp Thr Arg Thr Ser Ala Leu Ser Ala Lys Ser
    1               5                   10
```

<210> 903
<211> 14
<212> PRT
<213> Homo sapiens

<400> 903

Ala Leu Met Thr Asp Pro Lys Leu Ile Thr Trp Ser Pro Val
1                   5                   10

<210> 904
<211> 16
<212> PRT
<213> Homo sapiens

<400> 904

Asn Asp Val Ala Trp Asn Phe Glu Lys Phe Leu Val Gly Pro Asp Gly

    1           5                   10                  15

<210> 905
<211> 13
<212> PRT
<213> Homo sapiens

<400> 905

Gln Ser Val Tyr Ala Phe Ser Ala Arg Pro Leu Ala Gly
1                   5                   10

<210> 906
<211> 17
<212> PRT
<213> Homo sapiens

<400> 906

Gln Ser Val Tyr Ala Phe Ser Ala Arg Pro Leu Ala Gly Gly Glu Pro
1                   5                   10                  15

Val

<210> 907
<211> 12
<212> PRT
<213> Homo sapiens

<400> 907

Trp Asn Phe Glu Lys Phe Leu Val Gly Pro Asp Gly
1                   5                   10

<210> 908
<211> 15
<212> PRT
<213> Homo sapiens

<400> 908

Asp Val Gly Met Phe Val Ala Leu Thr Lys Leu Gly Gln Pro Asp
1                   5                   10                  15

<210> 909
<211> 14
<212> PRT
<213> Homo sapiens

<400> 909

```
      Val Gly Met Phe Val Ala Leu Thr Lys Leu Gly Gln Pro Asp
      1               5               10
```

<210> 910
<211> 12
<212> PRT
<213> Homo sapiens

<400> 910

```
      Ala Gly Val Phe His Val Glu Lys Asn Gly Arg Tyr
      1               5               10
```

<210> 911
<211> 14
<212> PRT
<213> Homo sapiens

<400> 911

```
      Phe Ala Gly Val Phe His Val Glu Lys Asn Gly Arg Tyr Ser
      1               5               10
```

<210> 912
<211> 14
<212> PRT
<213> Homo sapiens

<400> 912

```
      Gly Pro Ile Thr Ile Thr Ile Val Asn Arg Asp Gly Thr Arg
      1               5               10
```

<210> 913
<211> 14
<212> PRT
<213> Homo sapiens

<400> 913

```
      Asn Gly Arg Tyr Ser Ile Ser Arg Thr Glu Ala Ala Asp Leu
      1               5               10
```

<210> 914
<211> 14
<212> PRT
<213> Homo sapiens

<400> 914

```
          Arg Lys Ser Arg Gln Gly Ser Leu Ala Met Glu Glu Leu Lys
          1               5                   10
```

<210> 915
<211> 15
<212> PRT
<213> Homo sapiens

<400> 915

```
          Arg Arg Lys Ser Arg Gln Gly Ser Leu Ala Met Glu Glu Leu Lys
          1               5                   10                  15
```

<210> 916
<211> 15
<212> PRT
<213> Homo sapiens

<400> 916

```
          Glu Glu Phe Lys Lys Leu Thr Ser Ile Lys Ile Gln Asn Asp Lys
          1               5                   10                  15
```

<210> 917
<211> 13
<212> PRT
<213> Homo sapiens

<400> 917

```
            Ile Asn Arg Arg Met Ala Asp Asp Asn Lys Leu Phe Arg
            1               5                   10
```

<210> 918
<211> 15
<212> PRT
<213> Homo sapiens

<400> 918

```
          Thr Ala Thr Ile Val Met Val Thr Asn Leu Lys Glu Arg Lys Glu
          1               5                   10                  15
```

<210> 919
<211> 14
<212> PRT
<213> Homo sapiens

<400> 919

```
          Glu Leu Phe Tyr Lys Gly Ile Arg Pro Ala Ile Asn Val Gly
          1               5                   10
```

<210> 920
<211> 13
<212> PRT
<213> Homo sapiens

<400> 920

```
        Gly Gln Lys Arg Ser Thr Val Ala Gln Leu Val Lys Arg
        1               5                   10
```

<210> 921
<211> 15
<212> PRT
<213> Homo sapiens

<400> 921

```
        Ser Asp Leu Asp Ala Ala Thr Gln Gln Leu Leu Ser Arg Gly Val
        1               5                   10                  15
```

<210> 922
<211> 14
<212> PRT
<213> Homo sapiens

<400> 922

```
        Phe Asp Phe Ser Gln Asn Thr Arg Val Pro Arg Leu Pro Glu
        1               5                   10
```

<210> 923
<211> 12
<212> PRT
<213> Homo sapiens

<400> 923

```
        Gly Asp Ala Pro Ala Ile Leu Phe Asp Lys Glu Phe
        1               5                   10
```

<210> 924
<211> 13
<212> PRT
<213> Homo sapiens

<400> 924

```
        Val Thr His Glu Ile Asp Arg Tyr Thr Ala Ile Ala Tyr
        1               5                   10
```

<210> 925
<211> 16
<212> PRT
<213> Homo sapiens

<400> 925

```
        Gly Gln Gly Tyr Leu Ile Lys Asp Gly Lys Leu Ile Lys Asn Asn Ala
        1               5                   10                  15
```

<210> 926
<211> 15
<212> PRT

<213> Homo sapiens

<400> 926

```
        Ile Asp Thr Thr Ser Lys Phe Gly His Gly Arg Phe Gln Thr Met
        1               5                   10                  15
```

<210> 927
<211> 19
<212> PRT
<213> Homo sapiens

<400> 927

```
        Ile Asp Val Ile Gly Val Thr Lys Gly Lys Gly Tyr Lys Gly Val Thr
        1               5                   10                  15


        Ser Arg Trp
```

<210> 928
<211> 15
<212> PRT
<213> Homo sapiens

<400> 928

```
        Met Gly Pro Leu Lys Lys Asp Arg Ile Ala Lys Glu Glu Gly Ala
        1               5                   10                  15
```

<210> 929
<211> 14
<212> PRT
<213> Homo sapiens

<400> 929

```
        Ala Ala Lys Tyr Gln Leu Asp Pro Thr Ala Ser Ile Ser Ala
        1               5                   10
```

<210> 930
<211> 15
<212> PRT
<213> Homo sapiens

<400> 930

```
        Ile Ala Ala Lys Tyr Gln Leu Asp Pro Thr Ala Ser Ile Ser Ala
        1               5                   10                  15
```

<210> 931
<211> 16
<212> PRT
<213> Homo sapiens

<400> 931

```
        Ile Ala Ala Lys Tyr Gln Leu Asp Pro Thr Ala Ser Ile Ser Ala Lys
        1               5                   10                  15
```

<210> 932
<211> 15
<212> PRT
<213> Homo sapiens


<400> 932

```
            Ala Gly Leu Gly Arg Ala Tyr Ala Leu Ala Phe Ala Glu Arg Gly
            1               5                   10                  15
```

<210> 933
<211> 14
<212> PRT
<213> Homo sapiens


<400> 933

```
            Asp Ala Phe Gly Arg Ile Asp Val Val Val Asn Asn Ala Gly
            1               5                   10
```

<210> 934
<211> 13
<212> PRT
<213> Homo sapiens


<400> 934

```
            Gly Leu Gly Arg Ala Tyr Ala Leu Ala Phe Ala Glu Arg
            1               5                   10
```

<210> 935
<211> 14
<212> PRT
<213> Homo sapiens


<400> 935

```
            Gly Leu Gly Arg Ala Tyr Ala Leu Ala Phe Ala Glu Arg Gly
            1               5                   10
```

<210> 936
<211> 15
<212> PRT
<213> Homo sapiens


<400> 936

```
            Ala Lys Phe Ala Leu Asn Gly Glu Glu Phe Met Asn Phe Asp Leu
            1               5                   10                  15
```

<210> 937
<211> 16
<212> PRT
<213> Homo sapiens

<400> 937

```
Ala Lys Phe Ala Leu Asn Gly Glu Glu Phe Met Asn Phe Asp Leu Lys
1               5                   10                  15
```

<210> 938
<211> 13
<212> PRT
<213> Homo sapiens

<400> 938

```
Ala Leu Asn Gly Glu Glu Phe Met Asn Phe Asp Leu Lys
1               5                   10
```

<210> 939
<211> 14
<212> PRT
<213> Homo sapiens

<400> 939

```
Lys Phe Ala Leu Asn Gly Glu Glu Phe Met Asn Phe Asp Leu
1               5                   10
```

<210> 940
<211> 14
<212> PRT
<213> Homo sapiens

<400> 940

```
Ser Asp Gly Ser Phe His Ala Ser Ser Ser Leu Thr Val Lys
1               5                   10
```

<210> 941
<211> 17
<212> PRT
<213> Homo sapiens

<400> 941

```
Glu Glu Arg Asn Leu Leu Ser Val Ala Tyr Lys Asn Val Val Gly Ala
1               5                   10                  15

Arg
```

<210> 942
<211> 16
<212> PRT
<213> Homo sapiens

<400> 942

```
Glu Arg Asn Leu Leu Ser Val Ala Tyr Lys Asn Val Val Gly Ala Arg
1               5                   10                  15
```

<210> 943
<211> 14
<212> PRT
<213> Homo sapiens

<400> 943

Ile Ala Glu Leu Asp Thr Leu Ser Glu Glu Ser Tyr Lys Asp

1                    5                      10

<210> 944
<211> 15
<212> PRT
<213> Homo sapiens

<400> 944

Ile Ala Glu Leu Asp Thr Leu Ser Glu Glu Ser Tyr Lys Asp Ser
1              5                  10                  15

<210> 945
<211> 16
<212> PRT
<213> Homo sapiens

<400> 945

Ala Asp Ser Tyr Leu Asp Glu Gly Phe Leu Leu Asp Lys Lys Ile Gly
1              5                  10                  15

<210> 946
<211> 13
<212> PRT
<213> Homo sapiens

<400> 946

Asp Ser Tyr Leu Asp Glu Gly Phe Leu Leu Asp Lys Lys
1                  5                  10

<210> 947
<211> 15
<212> PRT
<213> Homo sapiens

<400> 947

Asp Ser Tyr Leu Asp Glu Gly Phe Leu Leu Asp Lys Lys Ile Gly
1              5                  10                  15

<210> 948
<211> 15
<212> PRT
<213> Homo sapiens

<400> 948

```
        Val Asp Asn Ile Ile Lys Ala Ala Pro Arg Lys Arg Val Pro Asp
        1               5                   10                  15
```

<210> 949
<211> 13
<212> PRT
<213> Homo sapiens

<400> 949

```
        Ser Pro Pro Gln Phe Arg Val Asn Gly Ala Ile Ser Asn
        1               5                   10
```

<210> 950
<211> 15
<212> PRT
<213> Homo sapiens

<400> 950

```
        Ser Pro Pro Gln Phe Arg Val Asn Gly Ala Ile Ser Asn Phe Glu
        1               5                   10                  15
```

<210> 951
<211> 16
<212> PRT
<213> Homo sapiens

<400> 951

```
        Ser Pro Pro Gln Phe Arg Val Asn Gly Ala Ile Ser Asn Phe Glu Glu
        1               5                   10                  15
```

<210> 952
<211> 17
<212> PRT
<213> Homo sapiens

<400> 952

```
        Ser Pro Pro Gln Phe Arg Val Asn Gly Ala Ile Ser Asn Phe Glu Glu
        1               5                   10                  15

        Phe
```

<210> 953
<211> 15
<212> PRT
<213> Homo sapiens

<400> 953

```
        Val Gly Lys Met Phe Val Asp Val Tyr Phe Gln Glu Asp Lys Lys
        1               5                   10                  15
```

<210> 954
<211> 16
<212> PRT
<213> Homo sapiens

<400> 954

```
Val Gly Lys Met Phe Val Asp Val Tyr Phe Gln Glu Asp Lys Lys Glu
1               5                   10                  15
```

<210> 955
<211> 17
<212> PRT
<213> Homo sapiens

<400> 955

```
Asp Pro Lys Arg Thr Ile Ala Gln Asp Tyr Gly Val Leu Lys Ala Asp
1               5                   10                  15

Glu
```

<210> 956
<211> 18
<212> PRT
<213> Homo sapiens

<400> 956

```
Asp Pro Lys Arg Thr Ile Ala Gln Asp Tyr Gly Val Leu Lys Ala Asp
1               5                   10                  15

Glu Gly
```

<210> 957
<211> 17
<212> PRT
<213> Homo sapiens

<400> 957

```
Pro Lys Arg Thr Ile Ala Gln Asp Tyr Gly Val Leu Lys Ala Asp Glu
1               5                   10                  15

Gly
```

<210> 958
<211> 13
<212> PRT
<213> Homo sapiens

<400> 958

```
Gly Leu Phe Ile Ile Asp Asp Lys Gly Ile Leu Arg Gln
1               5                   10
```

<210> 959
<211> 15
<212> PRT
<213> Homo sapiens

<400> 959

```
Gly Leu Phe Ile Ile Asp Asp Lys Gly Ile Leu Arg Gln Ile Thr
1               5                   10                  15
```

<210> 960
<211> 13
<212> PRT
<213> Homo sapiens

<400> 960

```
Arg Gly Leu Phe Ile Ile Asp Asp Lys Gly Ile Leu Arg
1               5                   10
```

<210> 961
<211> 14
<212> PRT
<213> Homo sapiens

<400> 961

```
Arg Gly Leu Phe Ile Ile Asp Asp Lys Gly Ile Leu Arg Gln
1               5                   10
```

<210> 962
<211> 16
<212> PRT
<213> Homo sapiens

<400> 962

```
Arg Gly Leu Phe Ile Ile Asp Asp Lys Gly Ile Leu Arg Gln Ile Thr
1               5                   10                  15
```

<210> 963
<211> 15
<212> PRT
<213> Homo sapiens

<400> 963

```
Gly Asn Thr Val Ile His Leu Asp Gln Ala Leu Ala Arg Met Arg
1               5                   10                  15
```

<210> 964
<211> 14
<212> PRT
<213> Homo sapiens

<400> 964

```
        Asn Thr Val Ile His Leu Asp Gln Ala Leu Ala Arg Met Arg
        1               5                   10
```

<210> 965
<211> 15
<212> PRT
<213> Homo sapiens

<400> 965

```
        Asn Thr Val Ile His Leu Asp Gln Ala Leu Ala Arg Met Arg Glu
        1               5                   10                  15
```

<210> 966
<211> 15
<212> PRT
<213> Homo sapiens

<400> 966

```
        Glu Asn Asn Glu Ile Ile Ser Asn Ile Arg Asp Ser Val Ile Asn
        1               5                   10                  15
```

<210> 967
<211> 14
<212> PRT
<213> Homo sapiens

<400> 967

```
        Asn Asn Glu Ile Ile Ser Asn Ile Arg Asp Ser Val Ile Asn
        1               5                   10
```

<210> 968
<211> 14
<212> PRT
<213> Homo sapiens

<400> 968

```
        Ser Pro Thr Val Gln Val Phe Ser Ala Ser Gly Lys Pro Val
        1               5                   10
```

<210> 969
<211> 16
<212> PRT
<213> Homo sapiens

<400> 969

```
        Ser Ser Pro Thr Val Gln Val Phe Ser Ala Ser Gly Lys Pro Val Glu
        1               5                   10                  15
```

<210> 970
<211> 16
<212> PRT
<213> Homo sapiens

<400> 970

```
        Ala Glu Pro Asn Tyr His Ser Leu Pro Ser Ala Arg Thr Asp Glu Gln
        1               5                   10                  15
```

<210> 971
<211> 15
<212> PRT
<213> Homo sapiens

<400> 971

```
        Ser Ser Ile Leu Ala Lys Thr Ala Ser Asn Ile Ile Asp Val Ser
        1               5                   10                  15
```

<210> 972
<211> 15
<212> PRT
<213> Homo sapiens

<400> 972

```
        Leu Glu Ala Arg Ala Thr Ala Pro Pro Ala Pro Ser Ala Pro Asn
        1               5                   10                  15
```

<210> 973
<211> 12
<212> PRT
<213> Homo sapiens

<400> 973

```
            Ala Asp Asp Leu Glu Gly Glu Ala Phe Leu Pro Leu
            1               5                   10
```

<210> 974
<211> 13
<212> PRT
<213> Homo sapiens

<400> 974

```
            Ala Asp Asp Leu Glu Gly Glu Ala Phe Leu Pro Leu Arg
            1               5                   10
```

<210> 975
<211> 14
<212> PRT
<213> Homo sapiens

<400> 975

```
        Ala Asp Asp Leu Glu Gly Glu Ala Phe Leu Pro Leu Arg Glu
        1               5                   10
```

<210> 976
<211> 14
<212> PRT

<213> Homo sapiens

<400> 976

```
        Gly Ala Asp Asp Leu Glu Gly Glu Ala Phe Leu Pro Leu Arg
        1               5                   10
```

<210> 977
<211> 15
<212> PRT
<213> Homo sapiens

<400> 977

```
        Ala Gly Arg Glu Ile Asn Leu Val Asp Ala His Leu Lys Ser Glu
        1               5                   10                  15
```

<210> 978
<211> 17
<212> PRT
<213> Homo sapiens

<400> 978

```
        Ala Gly Arg Glu Ile Asn Leu Val Asp Ala His Leu Lys Ser Glu Gln
        1               5                   10                  15


        Thr
```

<210> 979
<211> 14
<212> PRT
<213> Homo sapiens

<400> 979

```
        Gly Arg Glu Ile Asn Leu Val Asp Ala His Leu Lys Ser Glu
        1               5                   10
```

<210> 980
<211> 15
<212> PRT
<213> Homo sapiens

<400> 980

```
        Lys Pro Gly Ile Val Tyr Ala Ser Leu Asn His Ser Val Ile Gly
        1               5                   10                  15
```

<210> 981
<211> 16
<212> PRT
<213> Homo sapiens

<400> 981

```
        Asn Lys Pro Gly Ile Val Tyr Ala Ser Leu Asn His Ser Val Ile Gly
        1               5                   10                  15
```

<210> 982
<211> 16
<212> PRT
<213> Homo sapiens

<400> 982

```
        Thr Thr Leu Tyr Val Thr Asp Val Lys Ser Ala Ser Glu Arg Pro Ser
        1               5                   10                  15
```

<210> 983
<211> 16
<212> PRT
<213> Homo sapiens

<400> 983

```
        Ala Pro Ser Thr Tyr Ala His Leu Ser Pro Ala Lys Thr Pro Pro Pro
        1               5                   10                  15
```

<210> 984
<211> 17
<212> PRT
<213> Homo sapiens

<400> 984

```
        Ala Pro Ser Thr Tyr Ala His Leu Ser Pro Ala Lys Thr Pro Pro Pro
        1               5                   10                  15
```

```
        Pro
```

<210> 985
<211> 18
<212> PRT
<213> Homo sapiens

<400> 985

```
        Ala Pro Ser Thr Tyr Ala His Leu Ser Pro Ala Lys Thr Pro Pro Pro
        1               5                   10                  15
```

```
        Pro Ala
```

<210> 986
<211> 15
<212> PRT
<213> Homo sapiens

<400> 986

```
        Arg Asp Asp Leu Tyr Asp Gln Asp Asp Ser Arg Asp Phe Pro Arg
        1               5                   10                  15
```

<210> 987
<211> 14
<212> PRT
<213> Homo sapiens

<400> 987

```
        Thr Arg Pro Tyr His Ser Leu Pro Ser Glu Ala Val Phe Ala
        1               5                   10
```

<210> 988
<211> 15
<212> PRT
<213> Homo sapiens

<400> 988

```
        Thr Arg Pro Tyr His Ser Leu Pro Ser Glu Ala Val Phe Ala Asn
        1               5                   10                  15
```

<210> 989
<211> 12
<212> PRT
<213> Homo sapiens

<400> 989

```
            Val Ala Val Phe Thr Phe His Asn His Gly Arg Thr
            1               5                   10
```

<210> 990
<211> 13
<212> PRT
<213> Homo sapiens

<400> 990

```
        Val Ala Val Phe Thr Phe His Asn His Gly Arg Thr Ala
        1               5                   10
```

<210> 991
<211> 15
<212> PRT
<213> Homo sapiens

<400> 991

```
        Val Ala Val Phe Thr Phe His Asn His Gly Arg Thr Ala Asn Leu
        1               5                   10                  15
```

<210> 992
<211> 16
<212> PRT
<213> Homo sapiens

<400> 992

```
Glu Asp Asp Tyr Ile Lys Ser Trp Glu Asp Asn Gln Gln Gly Asp Glu

        1               5               10              15
```

<210> 993
<211> 16
<212> PRT
<213> Homo sapiens

<400> 993

```
Glu Leu Glu Arg Ile Gln Ile Gln Glu Ala Ala Lys Lys Lys Pro Gly
1               5               10              15
```

<210> 994
<211> 13
<212> PRT
<213> Homo sapiens

<400> 994

```
Glu Arg Ile Gln Ile Gln Glu Ala Ala Lys Lys Lys Pro
1               5               10
```

<210> 995
<211> 14
<212> PRT
<213> Homo sapiens

<400> 995

```
Glu Arg Ile Gln Ile Gln Glu Ala Ala Lys Lys Lys Pro Gly
1               5               10
```

<210> 996
<211> 15
<212> PRT
<213> Homo sapiens

<400> 996

```
Glu Arg Ile Gln Ile Gln Glu Ala Ala Lys Lys Lys Pro Gly Ile
1               5               10              15
```

<210> 997
<211> 15
<212> PRT
<213> Homo sapiens

<400> 997

```
Leu Glu Arg Ile Gln Ile Gln Glu Ala Ala Lys Lys Lys Pro Gly
1               5               10              15
```

<210> 998
<211> 12

<212> PRT
<213> Homo sapiens

<400> 998

```
Leu Ser Ser Ile Ser Gln Tyr Ser Gly Lys Ile Lys
1               5                   10
```

<210> 999
<211> 12
<212> PRT
<213> Homo sapiens

<400> 999

```
Ser Pro Ala Lys Asp Ser Leu Ser Phe Glu Asp Phe
1               5                   10
```

<210> 1000
<211> 15
<212> PRT
<213> Homo sapiens

<400> 1000

```
Ser Pro Ala Lys Asp Ser Leu Ser Phe Glu Asp Phe Leu Asp Leu
1               5                   10                  15
```

<210> 1001
<211> 14
<212> PRT
<213> Homo sapiens

<400> 1001

```
Ile Asn Ser Arg Phe Pro Ile Pro Ser Ala Thr Asp Pro Asp
1               5                   10
```

<210> 1002
<211> 14
<212> PRT
<213> Homo sapiens

<400> 1002

```
Val Gln His Tyr Glu Leu Leu Asn Gly Gln Ser Val Phe Gly
1               5                   10
```

<210> 1003
<211> 14
<212> PRT
<213> Homo sapiens

<400> 1003

```
Asp Asn Gln Tyr Ala Val Leu Glu Asn Gln Lys Ser Ser His
1               5                   10
```

<210> 1004
<211> 13
<212> PRT
<213> Homo sapiens

<400> 1004

```
Gly Pro Pro Glu Ile Tyr Ser Asp Thr Gln Phe Pro Ser
1               5                   10
```

<210> 1005
<211> 15
<212> PRT
<213> Homo sapiens

<400> 1005

```
Gly Pro Pro Glu Ile Tyr Ser Asp Thr Gln Phe Pro Ser Leu Gln
1               5                   10                  15
```

<210> 1006
<211> 16
<212> PRT
<213> Homo sapiens

<400> 1006

```
Thr Pro Gln Gly Pro Pro Glu Ile Tyr Ser Asp Thr Gln Phe Pro Ser
1               5                   10                  15
```

<210> 1007
<211> 18
<212> PRT
<213> Homo sapiens

<400> 1007

```
Thr Pro Gln Gly Pro Pro Glu Ile Tyr Ser Asp Thr Gln Phe Pro Ser
1               5                   10                  15

Leu Gln
```

<210> 1008
<211> 20
<212> PRT
<213> Homo sapiens

<400> 1008

```
Thr Pro Gln Gly Pro Pro Glu Ile Tyr Ser Asp Thr Gln Phe Pro Ser
1               5                   10                  15

Leu Gln Ser Thr
            20
```

<210> 1009

<211> 14
<212> PRT
<213> Homo sapiens

<400> 1009

```
Ala Asn Leu Gln Arg Ala Tyr Ser Leu Ala Lys Glu Gln Arg
1               5                   10
```

<210> 1010
<211> 13
<212> PRT
<213> Homo sapiens

<400> 1010

```
Asn Leu Gln Arg Ala Tyr Ser Leu Ala Lys Glu Gln Arg
1               5                   10
```

<210> 1011
<211> 15
<212> PRT
<213> Homo sapiens

<400> 1011

```
Thr Pro Ser Gly Ile Thr Tyr Asp Arg Lys Asp Ile Glu Glu His
1               5                   10                  15
```

<210> 1012
<211> 14
<212> PRT
<213> Homo sapiens

<400> 1012

```
Val Ser Thr Leu Asn Ser Glu Asp Phe Val Leu Val Ser Arg
1               5                   10
```

<210> 1013
<211> 15
<212> PRT
<213> Homo sapiens

<400> 1013

```
Val Ser Thr Leu Asn Ser Glu Asp Phe Val Leu Val Ser Arg Gln
1               5                   10                  15
```

<210> 1014
<211> 16
<212> PRT
<213> Homo sapiens

<400> 1014

```
Val Ser Thr Leu Asn Ser Glu Asp Phe Val Leu Val Ser Arg Gln Gly
1               5                   10                  15
```

<210> 1015
<211> 14
<212> PRT
<213> Homo sapiens

<400> 1015

```
Gly Ser Ser Phe Phe Gly Glu Leu Phe Asn Gln Asn Pro Glu
1               5                   10
```

<210> 1016
<211> 15
<212> PRT
<213> Homo sapiens

<400> 1016

```
Ser Gly Ser Ser Phe Phe Gly Glu Leu Phe Asn Gln Asn Pro Glu
1               5                   10                  15
```

<210> 1017
<211> 10
<212> PRT
<213> Homo sapiens

<400> 1016

```
Glu Leu Ala Gly Ile Gly Ile Leu Thr Val
1               5                   10
```

<210> 1018
<211> 9
<212> PRT
<213> Homo sapiens

<400> 1016

```
Tyr Leu Leu Pro Ala Ile Val His Ile
1               5
```